(19) Europäisches Patentamt
European Patent Office
Office européen des brevets

(11) EP 4 353 732 A1

(12) **EUROPEAN PATENT APPLICATION**
published in accordance with Art. 153(4) EPC

(43) Date of publication:
**17.04.2024 Bulletin 2024/16**

(21) Application number: **22806710.4**

(22) Date of filing: **10.05.2022**

(51) International Patent Classification (IPC):
***C07H 19/16*** (2006.01) ***C07H 19/20*** (2006.01)
***C07H 19/207*** (2006.01) ***A61K 31/70*** (2006.01)
***A61K 31/7052*** (2006.01) ***A61K 31/7076*** (2006.01)
***A61P 35/00*** (2006.01)

(52) Cooperative Patent Classification (CPC):
**A61K 31/70; A61K 31/7052; A61K 31/7076; A61P 35/00; C07H 19/16; C07H 19/20; C07H 19/207**

(86) International application number:
**PCT/CN2022/091843**

(87) International publication number:
**WO 2022/237747 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.05.2021 CN 202110512304**
**02.07.2021 CN 202110747483**
**25.08.2021 CN 202110982246**
**14.10.2021 CN 202111196621**
**10.11.2021 CN 202111324541**

(71) Applicant: **Xizang Haisco Pharmaceutical Co., Ltd.**
**Lhoka, Tibet 856099 (CN)**

(72) Inventors:
- **LI, Yao**
  **Chengdu City, Sichuan 611130 (CN)**
- **CHEN, Lei**
  **Chengdu City, Sichuan 611130 (CN)**
- **SHI, Zongjun**
  **Chengdu City, Sichuan 611130 (CN)**
- **HU, Gang**
  **Chengdu City, Sichuan 611130 (CN)**
- **GENG, Pengxin**
  **Chengdu City, Sichuan 611130 (CN)**
- **WANG, Haodong**
  **Chengdu City, Sichuan 611130 (CN)**
- **SHI, Shaohui**
  **Chengdu City, Sichuan 611130 (CN)**
- **WANG, Yongli**
  **Chengdu City, Sichuan 611130 (CN)**
- **TANG, Pingming**
  **Chengdu City, Sichuan 611130 (CN)**
- **YU, Yan**
  **Chengdu City, Sichuan 611130 (CN)**
- **ZHANG, Chen**
  **Chengdu City, Sichuan 611130 (CN)**
- **YAN, Pangke**
  **Chengdu City, Sichuan 611130 (CN)**

(74) Representative: **Hoffmann Eitle**
**Patent- und Rechtsanwälte PartmbB**
**Arabellastraße 30**
**81925 München (DE)**

# (54) SMALL MOLECULAR CD73 ANTAGONIST AND USE THEREOF

(57) Disclosed is a compound of formula (I), and a stereoisomer, a pharmaceutically acceptable salt, a solvate, a co-crystal or a deuterated product thereof, or a pharmaceutical composition containing same, and the use thereof as a CD73 antagonist in the preparation of a drug for treating related diseases, wherein the definition of each group in formula (I) is consistent with the definition in the description.

EP 4 353 732 A1

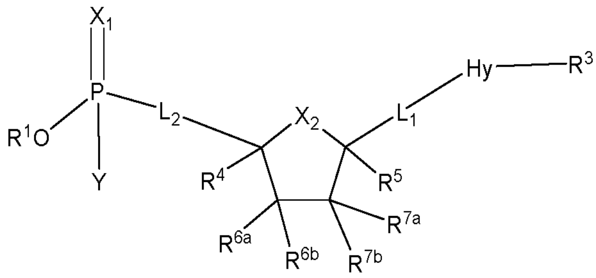

(I)

## Description

### Technical Field

The present invention belongs to the field of drugs, and in particular relates to a compound having a CD73 inhibitory activity, and a stereoisomer, a pharmaceutically acceptable salt, a solvate, a co-crystal or a deuterated product thereof, and the use thereof in the preparation of a drug for treating related diseases.

### Background Art

At present, the methods for treating cancers mainly comprise radiotherapy, surgery therapy and pharmaceutical therapy, and treatment with drugs targeting lesions has currently become the main means of clinical tumor treatment. However, due to the rapid development of drug resistance in tumor cells, people are basically at a loss for tumor metastasis and recurrence at the present stage.

CD73 is an extracellular 5'-nucleotidase. The C-terminal domain contains an AMP-binding site and is anchored to the plasma membrane by a GPI. Extracellular ATP and ADP or NAD+ are converted to AMP by different enzymes and further hydrolyzed to adenosine by CD73. CD73 is expressed in different tissues to different degrees, especially abundant in colon, kidney, brain, liver, heart, lung, spleen, lymph node and bone marrow (Trends Cancer. 2016 1;2(2):95-109.). After CD73 hydrolyzes AMP into adenosine (Ado), extracellular Ado binds to four G protein-coupled adenosine receptors (ARs) (A1R, A2AR, A2BR, and A3R) on the cell surface to promote anti-inflammatory responses and inhibit a series of signaling pathways related to immune activation, such as LCK, MAPK and PKC, regulate regulatory T cells, inhibit inflammation and use other ways to exert a wide range of immunosuppressive activities. Extracellular Ado can also enter the cell through balanced nucleoside transporters (such as ENTs), or be further metabolized into inosine (such as ADA/CD26), and Ado can activate a variety of signaling pathways, such as MAPK, PI3K, PLC, PKC and ion channels. Among them, A2AR and A2BR signals stimulate adenylyl cyclase (AC) to produce cyclic adenylate cyclase (cAMP), which activates PKA kinase. A1R and A3R signals inhibit AC to adjust the intracellular concentration of cAMP (Front Immunol. 2020 15; 11 :508.).

CD73 acts as a major immunosuppressive modulator in the tumor microenvironment through the production of extracellular adenosine. CD73 is expressed on the surface of various cells such as cancer cells, DC cells, Treg, NK cells, MDSC, and TAM. While suppressing immune cells (such as effector T cells, B cells, NK cells, and DC cells), it maintains the function of immunosuppressive cells (Current Opinion in Pharmacology 2020, 53:1-11). CD73 is highly expressed in different tumors, including colorectal cancer, lung cancer, pancreatic cancer, ovarian cancer, breast cancer, melanoma, and thyroid tumors, etc. It is closely related to the poor prognosis of various types of tumors and can be used as a key factor in the development of malignant tumors. Overexpression of CD73 can promote the proliferation and growth of tumors, and CD73 of both the tumor and host has important effects on tumor growth. In vivo and in vitro experiments have confirmed that inhibiting CD73 expression can inhibit the growth of tumor cells (Am J Physiol Cell Physiol. 2019 317(6):C1079-C1092.).

At present, the research and development of CD73 small molecule antagonists is still in a relatively preliminary stage globally. However, based on the existing preclinical and clinical data, drugs that inhibit the physiological function of CD73 in the tumor microenvironment have great potential, especially when combined with other immune checkpoint inhibitors such as PD-1, PD-L1 and A2aR antagonists, they have shown good efficacy or activity while ensuring safety. Therefore, the development of novel CD73 antagonists to relieve immune suppression by reducing extracellular adenosine in the tumor microenvironment provides a new therapeutic idea for inhibiting tumor development and metastasis.

### Summary of the Invention

The present invention provides a compound having a CD73 inhibitory activity, and a stereoisomer, a pharmaceutically acceptable salt, a solvate, a co-crystal or a deuterated product thereof, the preparation therefor and the use thereof for treating CD73-induced diseases. The compound of the present invention has good inhibitory effect on CD73, can inhibit tumor cell proliferation, and has good pharmacokinetic characteristics, high bioavailability, good safety, less toxic and side effects, no inhibition on CYP enzymes, and fast absorption.

1. The present invention provides a compound of formula (I), and a stereoisomer, a pharmaceutically acceptable salt, a solvate, a co-crystal or a deuterated product thereof,

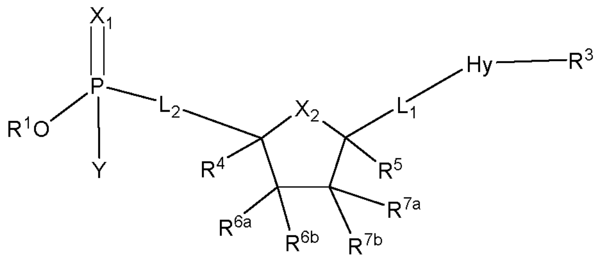

(I)

wherein

$X_1$ is selected from O or S;
$X_2$ is selected from $CR^{x2a}R^{x2b}$, O, S or $NR^{x2c}$;
Y is selected from $OR^2$, $-C(R^{y1}R^{y2})-P(=O)(OR^2)OR^2$, $-C(R^{y1}R^{y2})-P(=O)(OR^2)NHR^{y3}$, $-C(R^{y1}R^{y2})-P(=O)(OR^2)SR^{y3}$, $-C(R^{y1}R^{y2})-R^{y4}$, $-C(R^{y1}R^{y2})-P(=S)(OR^2)OR^2$, $-C(R^{y1}R^{y2})-P(=S)(OR^2)NHR^{y3}$ or $-C(R^{y1}R^{y2})-P(=S)(OR^2)SR^{y3}$;
$R^1$ and $R^2$ are each independently selected from H, deuterium, amino, $C_{1-6}$alkyl, $C_{2-6}$alkenyl, $C_{2-6}$alkynyl, $C_{3-10}$cycloalkyl, 3- to 10-membered heterocycloalkyl, $C_{6-10}$aryl, 5- to 10-membered heteroaryl, $-C(R^{2a}R^{2b})-O-C(O)-OR^{2c}$, $-C_{0-6}alkyl-S(O)_rR^{2c}$, $-C_{0-6}alkyl-C(O)R^{2c}$, $-C_{0-6}alkyl-C(O)OR^{2c}$ or $-C_{0-6}alkyl-C(O)NR^{2c}$, wherein the alkyl, cycloalkyl, heterocycloalkyl, aryl or heteroaryl is optionally further substituted with 1 or more groups selected from $R^x$;
r is selected from 1 or 2;
$R^{y1}$, $R^{y2}$, $R^{y3}$, $R^{x2a}$, $R^{x2b}$ and $R^{x2c}$ are each independently selected from H, deuterium, hydroxyl, cyano, $C_{1-6}$alkyl, deuterated $C_{1-6}$alkyl or halo $C_{1-6}$alkyl;
$R^{y4}$ is selected from 4- to 10-membered heterocycloalkyl, 5- to 10-membered heteroaryl, $C_{3-10}$cycloalkyl or $C_{6-10}$aryl, wherein the heterocycloalkyl, heteroaryl, cycloalkyl or aryl is optionally further substituted with 1 or more groups selected from $R^x$;
$R^{2a}$ and $R^{2b}$ are each independently selected from H, deuterium, hydroxyl, cyano, halogen, $C_{1-6}$alkyl, deuterated $C_{1-6}$alkyl, halo $C_{1-6}$alkyl, $C_{3-10}$cycloalkyl, 3-to 10-membered heterocycloalkyl, $C_{6-10}$aryl or 5- to 10-membered heteroaryl;
each $R^{2c}$ is independently selected from H, deuterium, $C_{1-6}$alkyl, deuterated $C_{1-6}$alkyl, halo $C_{1-6}$alkyl, $C_{3-10}$cycloalkyl, 3- to 10-membered heterocycloalkyl, $C_{6-10}$aryl or 5- to 10-membered heteroaryl, wherein the alkyl, cycloalkyl, heterocycloalkyl, aryl or heteroaryl is optionally further substituted with 1 or more groups selected from $R^x$;
$L_1$ is selected from a bond, S, O, NH, $N(C_{1-6}alkyl)$ or $C_{1-6}$alkyl, wherein the alkyl is optionally further substituted with 1 or more groups selected from $R^x$;
$L_2$ is selected from $-(CR^{L2a}R^{L2b})n-$, $*-Z-C_{6-10}aryl$, *-Z-(5- to 10-membered heteroaryl)-, $*-C_{6-10}aryl-Z-$, *-(5- to 10-membered heteroaryl)Z-, $C_{2-6}$alkenyl or $C_{2-6}$alkynyl, wherein the carbon atom in the alkyl is optionally replaced with 1 or more groups selected from N, O, S, S(O) or $S(O)_2$, the $L_2$ is optionally further substituted with 1 or more groups selected from $R^x$, and * denotes the site where the $L_2$ is connected to P atom;
$R^{L2a}$ and $R^{L2b}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl, or $-C_{1-6}alkyl-O-C_{1-6}alkyl$, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl;
or, $R^{L2a}$ and $R^{L2b}$ on the same carbon atom together with the carbon atom to which they are attached form $C_{3-6}$cycloalkyl or 4- to 6-membered heterocycloalkyl;
each n is independently selected from 1, 2, 3, 4, 5, 6, 7, 8, 9 or 10;
each Z is independently selected from a bond, -O- or $C_{1-6}$alkyl, wherein the alkyl is optionally further substituted with 1 or more groups selected from $R^x$;
$R_4$, $R_5$, $R^{6a}$, $R^{6b}$, $R^{7a}$, and $R^{7b}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1 or more groups selected from deuterium, halogen, hydroxyl or cyano; or $R_4$, $R_5$, $R^{6a}$, $R^{6b}$, $R^{7a}$, and $R^{7b}$ are each independently selected from H, deuterium, hydroxyl, cyano, halogen, azido, amino, $C_{1-6}$alkyl, $C_{2-6}$alkenyl, $C_{2-6}$alkynyl or $C_{3-6}$cycloalkyl, wherein the amino or alkyl is optionally further substituted with 1 or more groups selected from deuterium, halogen, hydroxyl, cyano or $C_{1-6}$alkoxy;

or, $R^{6a}$ and $R^{6b}$, or $R^{7a}$ and $R^{7b}$ together form $C_{2-6}$alkenyl, $C_{3-6}$cycloalkyl or 4-to 6-membered heterocycloalkyl, wherein the alkenyl is optionally further substituted with 1 or more groups selected from deuterium, halogen, hydroxyl, or cyano;

or, $R_4$ and $R_5$, or $R^{6a}$ and $X_2$, or $R^{L2a}$ and $R^5$, or $R^4$ and $R^{7a}$ together form $C_{5-7}$cycloalkyl or 5- to 7-membered heterocycloalkyl;

or, $R^4$ and $R^{6a}$, or $R^{6b}$ and $R^{7b}$, or $R^4$ and $R^{L2a}$, or $R^4$ and $X_2$ together form $C_{3-7}$cycloalkyl or 3- to 7-membered heterocycloalkyl;

or, $R^{x2a}$ and $R^4$, or $R^{x2a}$ and $R^5$, or $R^{6a}$ and $R^4$, or $R^{6b}$ and $R^{7b}$, or $R^{7a}$and $R^5$ together form one chemical bond, provided that at most one group forms one chemical bond;

Hy is selected from 5- to 15-membered heteroaryl or 5- to 15-membered heterocycloalkyl, wherein the heteroaryl or heterocycloalkyl is optionally further substituted with 1 or more groups selected from $R^{hy}$;

$R^3$ is selected from $L_3$-$R^{3a}$, 5- to 10-membered heterocycloalkyl, $C_{5-10}$cycloalkyl, 5- to 10-membered heteroaryl or $C_{6-10}$aryl, wherein the heterocycloalkyl, cycloalkyl, heteroaryl or aryl is optionally further substituted with 1 or more groups selected from $R^y$;

$L_3$ is selected from **-N($R^{L3}$)$C_{1-6}$alkyl-, -N($R^{L3}$)-, **-N($R^{L3}$)S(O)-, **-N($R^{L3}$)$S(O)_2$-, **-N($R^{L3}$)S(O)-$C_{1-6}$alkyl-, **-N($R^{L3}$)$S(O)_2$-$C_{1-6}$alkyl-, **-N($R^{L3}$)-N=CH-, or **-N($R^{L3}$)-O-, and ** denotes the site where $L_3$ is connected to Hy;

$R^{3a}$ is selected from H, $C_{1-6}$alkyl, 5- to 15-membered carbocyclyl, 5- to 15-membered heterocyclyl or benzyl, wherein the alkyl, benzyl, carbocyclyl or heterocyclyl is optionally further substituted with 1 or more groups selected from $R^y$;

$R^{L3}$ is each independently selected from H, deuterium, $C_{1-6}$alkyl, halo $C_{1-6}$alkyl or deuterated $C_{1-6}$alkyl;

or, $R^{L3}$ and $R^{hy}$ together with the atom to which they are attached form 5- to 8-membered heterocycloalkyl;

$R^{hy}$ and $R^x$ are each independently selected from deuterium, halogen, amino, hydroxyl, cyano, =O, $SF_5$, azido, $C_{1-6}$alkyl, $C_{1-6}$alkoxy, $C_{2-6}$alkenyl or $C_{2-6}$alkynyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, -O-$C_{1-6}$alkyl-$C_{6-10}$aryl or -O-$C_{1-6}$alkyl-(5- to 10-membered heteroaryl);

$R^y$ is selected from deuterium, halogen, amino, hydroxyl, cyano, =O, $SF_5$, azido, -P(=O)($C_{1-6}$alkyl)$_2$, -P(=S)($C_{1-6}$alkyl)$_2$,

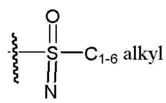
,
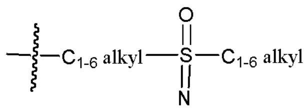

, -Si($C_{1-6}$alkyl)$_3$, -N=S(=O)($C_{1-6}$alkyl)$_2$, -$C_{1-6}$alkyl-S(=O)(=N), -$C_{1-6}$alkyl, $C_{1-6}$alkoxy, $C_{2-6}$alkenyl or $C_{2-6}$alkynyl, wherein the alkyl, alkoxy, amino, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy,

and other groups are as described in 1.

2. The compound of formula I, and the stereoisomer, pharmaceutically acceptable salt, solvate, co-crystal or deuterated product thereof of the present invention, wherein

$L_2$ is selected from *-O-($CR^{L2a}R^{L2b}$)n'-, *-($CR^{L2a}R^{L2b}$)n'-O-, -($CR^{L2a}R^{L2b}$)n'-, -O-, *-O-($CR^{L2a}R^{L2b}$)n'-O-($CR^{L2a}R^{L2b}$)m-, *-($CR^{L2a}R^{L2b}$)n'-O-($CR^{L2a}R^{L2b}$)m-, *-O-($CR^{L2a}R^{L2b}$)n'-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl-, or -$C_{2-6}$alkynyl-, wherein the alkyl, aryl, heteroaryl, alkenyl, and alkynyl are optionally further substituted with 1 or more groups selected from $R^x$;

$R^{L2a}$, $R^{L2b}$, and $R^{L2c}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl or -$C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl;

or, $R^{L2a}$ and $R^{L2b}$ on the same carbon atom together with the carbon atom to which they are attached form $C_{3-6}$cycloalkyl, or 4- to 6-membered heterocycloalkyl;

m, n, and n' are each independently selected from 1, 2, 3, 4 or 5.

Further, the compound of formula (I), and the stereoisomer, pharmaceutically acceptable salt, solvate, co-crystal or deuterated product thereof as provided by the present invention, wherein

$L_2$ is selected from *-O-($CR^{L2a}R^{L2b}$)n'-, *-($CR^{L2a}R^{L2b}$)n'-O-, -O-, *-($CR^{L2a}R^{L2b}$)n'-O-($CR^{L2a}R^{L2b}$)m-, *-O-($CR^{L2a}R^{L2b}$)n'-O-, *-O-N($R^{L2c}$)-, *-C(O)$C_{1-6}$alkyl, *-Z-$C_{6-10}$aryl-, -$C_{2-6}$alkenyl- or -$C_{2-6}$alkynyl-, wherein the alkyl, aryl, alkenyl or alkynyl is optionally further substituted with 1 or more groups selected from $R^x$;
m, n, and n' are each independently selected from 1, 2 or 3;
$R^{L2a}$, $R^{L2b}$, and $R^{L2c}$ are each independently selected from H, deuterium, halogen, amino, hydroxyl, cyano, $C_{1-4}$alkyl or -$C_{1-4}$alkyl-O-$C_{1-4}$alkyl, wherein the alkyl is optionally further substituted with 1 or more groups selected from deuterium, halogen, amino, hydroxyl, cyano, deuterated $C_{1-4}$alkyl, halo $C_{1-4}$alkyl, hydroxyl $C_{1-4}$alkyl, $C_{1-4}$alkoxy, deuterated $C_{1-4}$alkoxy, halo $C_{1-4}$alkoxy, hydroxyl $C_{1-4}$alkoxy, 5-membered heteroaryl, 6-membered heteroaryl or 8- to 10-membered bicyclic heteroaryl;
Z is independently selected from a bond, -O- or $C_{1-4}$alkyl, wherein the alkyl is optionally further substituted with 1 or more groups selected from $R^x$;
or, $R^{L2a}$ and $R^{L2b}$ on the same carbon atom together with the carbon atom to which they are attached form $C_{3-6}$cycloalkyl or 4- to 6-membered heterocycloalkyl.

Further, the compound of formula (I), and the stereoisomer, pharmaceutically acceptable salt, solvate, co-crystal or deuterated product thereof as provided by the present invention, wherein

$L_2$ is selected from *-O-($CR^{L2a}R^{L2b}$)n'-, *-($CR^{L2a}R^{L2b}$)n'-O-, -O-, *-($CR^{L2a}R^{L2b}$)n'-O-($CR^{L2a}R^{L2b}$)m-, *-O-($CR^{L2a}R^{L2b}$)n'-O-, *-O-N($R^{L2c}$)-, *-C(O)$C_{1-4}$alkyl, *-Z-$C_{6-10}$aryl-, -$C_{2-6}$alkenyl- or -$C_{2-6}$alkynyl-, wherein the alkyl, aryl, alkenyl or alkynyl is optionally further substituted with 1 or more groups selected from $R^x$;
m, n, and n' are each independently selected from 1, 2 or 3;
Z is independently selected from a bond, -O- or $C_{1-3}$alkyl, wherein the alkyl is optionally further substituted with 1 or more groups selected from $R^x$;
$R^{L2a}$, $R^{L2b}$, and $R^{L2c}$ are each independently selected from H, deuterium, halogen, amino, hydroxyl, cyano, $C_{1-4}$alkyl or -$C_{1-4}$alkyl-O-$C_{1-4}$alkyl, wherein the alkyl is optionally further substituted with 1 or more groups selected from deuterium, halogen, amino, hydroxyl, cyano, deuterated $C_{1-4}$alkyl, halo $C_{1-4}$alkyl, hydroxyl $C_{1-4}$alkyl, $C_{1-4}$alkoxy, deuterated $C_{1-4}$alkoxy, halo $C_{1-4}$alkoxy, hydroxyl $C_{1-4}$alkoxy, 5-membered heteroaryl, 6-membered heteroaryl or 8- to 10-membered bicyclic heteroaryl;
or, $R^{L2a}$ and $R^{L2b}$ on the same carbon atom together with the carbon atom to which they are attached form $C_{3-6}$cycloalkyl or 4- to 6-membered heterocycloalkyl.

Further, the compound of formula (I), and the stereoisomer, pharmaceutically acceptable salt, solvate, co-crystal or deuterated product thereof as provided by the present invention, wherein

$L_2$ is selected from *-O-($CR^{L2a}R^{L2b}$)$_2$-, *-C($R^{L2a}R^{L2b}$)-O-, -O-, *-C($R^{L2a}R^{L2b}$)-O-C($R^{L2a}R^{L2b}$)-, *-O-C($R^{L2a}R^{L2b}$)-O-, *-O-N($R^{L2c}$)-, *-C(O)$C_{1-3}$alkyl, *-Z-phenyl, $C_{2-4}$alkenyl or $C_{2-4}$alkynyl, wherein the alkyl, aryl, alkenyl or alkynyl is optionally further substituted with 1 or more groups selected from $R^x$;
Z is independently selected from a bond, -O-, methyl or ethyl, wherein the methyl or ethyl is optionally further substituted with 1 or more groups selected from $R^x$;
$R^{L2a}$, $R^{L2b}$, and $R^{L2c}$ are each independently selected from H, deuterium, halogen, amino, hydroxyl, cyano, methyl, ethyl, propyl, isopropyl, -methyl-O-methyl, -methyl-O-ethyl, -methyl-O-propyl, -ethyl-O-methyl, -ethyl-O-ethyl or -ethyl-O-propyl, wherein the methyl, ethyl, propyl or isopropyl is optionally further substituted with 1 or more groups selected from deuterium, halogen, amino, hydroxyl, cyano, deuterated $C_{1-2}$alkyl, fluoro $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, 5-membered heteroaryl, 6-membered heteroaryl or 8- to 10-membered bicyclic heteroaryl;
or, $R^{L2a}$ and $R^{L2b}$ on the same carbon atom together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl or 6-membered heterocycloalkyl.

3. The compound of formula I, and the stereoisomer, pharmaceutically acceptable salt, solvate, co-crystal or deuterated product thereof of the present invention, wherein,

$R^3$ is selected from $L_3$-$R^{3a}$;
$R^{3a}$ is selected from H, $C_{1-6}$alkyl, benzyl, 5- to 15-membered carbocyclyl or 5-15 heterocyclyl, wherein the alkyl, benzyl, carbocyclyl or heterocyclyl is further substituted with 1 or more groups selected from $R^y$; further, $R^{3a}$ is selected from H, $C_{1-6}$alkyl, 5- to 15-membered carbocyclyl or 5-15 heterocyclyl, wherein the carbocyclyl or heterocyclyl is further substituted with 1 or more groups selected from $R^y$; further, $R^{3a}$ is selected from phenyl, 5-membered heteroaryl, 6-membered heteroaryl, 5-membered monocyclic cycloalkyl, 6-membered monocyclic cycloalkyl, 5-membered monocyclic heterocycloalkyl, 6-membered monocyclic heterocycloalkyl, 8- to 10-membered bicyclic heteroaryl, 8- to 10-membered bicyclic heterocycloalkyl, 8- to 10-membered bicyclic aryl, 8- to 10-membered bicyclic cycloalkyl, 11- to 15-membered tricyclic cycloalkyl or 11- to 15-membered tricyclic heterocycloalkyl, wherein the cycloalkyl, heterocycloalkyl, aryl or heteroaryl is further substituted with 1 to 3 groups selected from $R^y$; further, $R^{3a}$ is selected from phenyl, pyridyl, pyridazinyl, pyrimidinyl, pyrazinyl, a benzo-fused 5-membered cycloalkyl, a benzo-fused 5-membered heterocycloalkyl, a benzo-fused 6-membered cycloalkyl, a benzo-fused 6-membered heterocycloalkyl, a benzo-fused 5-membered heteroaryl, a benzo-fused 6-membered heteroaryl, a pyrido-fused 5-membered cycloalkyl, a pyrido-fused 5-membered heterocycloalkyl, a pyrido-fused 6-membered cycloalkyl, a pyrido-fused 6-membered heterocycloalkyl, a pyrido-fused 5-membered heteroaryl, a pyrido-fused 6-membered heteroaryl, a 5-membered cycloalkyl fused phenyl, a 5-membered heterocycloalkyl fused phenyl, a 6-membered cycloalkyl fused phenyl, a 6-membered heterocycloalkyl fused phenyl, a 5-membered heteroaryl fused phenyl, a 6-membered heteroaryl fused phenyl, a 5-membered cycloalkyl fused pyridyl, a 5-membered heterocycloalkyl fused pyridyl, a 6-membered cycloalkyl fused pyridyl, a 6-membered heterocycloalkyl fused pyridyl, a 5-membered heteroaryl fused pyridyl or a 6-membered heteroaryl fused pyridyl, wherein the ring on the left of the word "fused" means a ring to which $L_3$ is connected to, and the phenyl, pyridyl, pyridazinyl, pyrimidinyl, pyrazinyl, cycloalkyl, heterocycloalkyl or heteroaryl is further substituted with 1, 2, or 3 groups selected from $R^y$; further, $R^{3a}$ is selected from phenyl, wherein the phenyl is further substituted with 1, 2, or 3 groups selected from $R^y$;

when $R^{3a}$ is substituted with two or more than two (e.g., three or four) $R^y$, $R^y$ is selected from deuterium, halogen, amino, hydroxyl, cyano, =O, $SF_5$, azido, - $P(=O)(C_{1-6}alkyl)_2$, $-P(=S)(C_{1-6}alkyl)_2$,

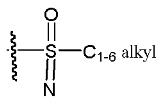 , 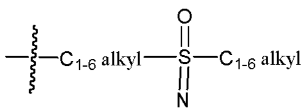 ,

$-Si(C_{1-6}alkyl)_3$, $-N=S(=O)(C_{1-6}alkyl)_2$, $-C_{1-6}alkyl-S(=O)(=N)$, $-C_{1-6}alkyl$, $C_{1-6}alkoxy$, $C_{2-6}alkenyl$, $C_{2-6}alkynyl$, wherein the alkyl, alkoxy, alkenyl, and alkynyl is optionally further substituted with 1 or more groups selected from deuterium, halogen, amino, hydroxyl, cyano, deuterated $C_{1-6}alkyl$, halo $C_{1-6}alkyl$, hydroxyl $C_{1-6}alkyl$, $C_{1-6}alkoxy$, deuterated $C_{1-6}alkoxy$, halo $C_{1-6}alkoxy$, or hydroxyl $C_{1-6}alkoxy$; further, $R^y$ is selected from deuterium, halogen, amino, hydroxyl, cyano, =O, $SF_5$, azido, - $P(=O)(C_{1-3}alkyl)_2$, $-P(=S)(C_{1-3}alkyl)_2$,

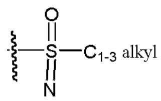 , 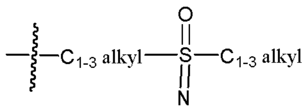 ,

$-Si(C_{1-3}alkyl)_3$, $-N=S(=O)(C_{1-3}alkyl)_2$, $-C_{1-3}alkyl-S(=O)(=N)$, $-C_{1-3}alkyl$, $C_{1-3}alkoxy$, $C_{2-4}alkenyl$ or $C_{2-4}alkynyl$, wherein the alkyl, alkoxy, alkenyl or alkynyl is optionally further substituted with 1 or more groups selected from deuterium, halogen, amino, hydroxyl, cyano, deuterated $C_{1-3}alkyl$, halo $C_{1-3}alkyl$, hydroxyl $C_{1-3}alkyl$, $C_{1-3}alkoxy$, deuterated $C_{1-3}alkoxy$, halo $C_{1-3}alkoxy$ or hydroxyl $C_{1-3}alkoxy$; further, at least one $R^y$ is selected from deuterium, halogen, amino, hydroxyl, cyano, =O, $SF_5$, azido, $-C_{1-3}alkyl$, $C_{1-3}alkoxy$, $C_{2-4}alkenyl$ or $C_{2-4}alkynyl$; provided that, when $R^{3a}$ is substituted with two $R^y$, $R^y$ is not a combination of F and $SF_5$;

when $R^{3a}$ is substituted with only one $R^y$, $R^y$ is selected from azido, - $P(=O)(C_{1-6}alkyl)_2$, $-P(=S)(C_{1-6}alkyl)_2$,

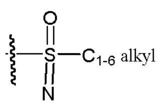 , 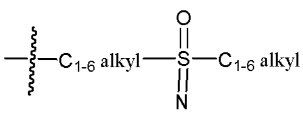 ,

$-Si(C_{1-6}alkyl)_3$, $-N=S(=O)(C_{1-6}alkyl)_2$ or $-C_{1-6}alkyl-S(=O)(=N)$, wherein the alkyl is optionally further substituted with 1 or more groups selected from deuterium, halogen, amino, hydroxyl, cyano, deuterated $C_{1-6}alkyl$, halo $C_{1-6}alkyl$, hydroxyl $C_{1-6}alkyl$, $C_{1-6}alkoxy$, deuterated $C_{1-6}alkoxy$, halo $C_{1-6}alkoxy$ or hydroxyl $C_{1-6}alkoxy$; further, $R^y$ is selected from azido, $-P(=O)(C_{1-3}alkyl)_2$, $-P(=S)(C_{1-3}alkyl)_2$,

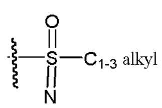, 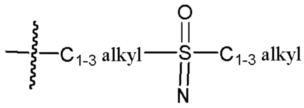,

$-Si(C_{1-3}alkyl)_3$, $-N=S(=O)(C_{1-3}alkyl)_2$ or $-C_{1-3}alkyl-S(=O)(=N)$, wherein the alkyl is optionally further substituted with 1, 2, or 3 groups selected from deuterium, F, Cl, amino, hydroxyl or cyano,
and other groups are as described in 1.

4. The compound of formula I, and the stereoisomer, pharmaceutically acceptable salt, solvate, co-crystal or deuterated product thereof of the present invention, wherein

$R_4$, $R_5$, $R^{6a}$, $R^{6b}$, $R^{7a}$, and $R^{7b}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1 or more groups selected from deuterium, halogen, hydroxyl or cyano;
or, $R^{6a}$ and $R^{6b}$, or $R^{7a}$ and $R^{7b}$ together form $C_{2-6}$alkenyl, $C_{3-6}$cycloalkyl or 4-to 6-membered heterocycloalkyl, wherein the alkenyl is optionally further substituted with 1 or more groups selected from deuterium, halogen, hydroxyl or cyano; further, $R^{6a}$ and $R^{6b}$, or $R^{7a}$ and $R^{7b}$ together form $C_{2-4}$alkenyl, $C_{3-4}$-membered cycloalkyl or 4- to 5-membered heterocycloalkyl, wherein the alkenyl is optionally further substituted with 1 or more groups selected from deuterium, halogen, hydroxyl or cyano; further, $R^{6a}$ and $R^{6b}$, or $R^{7a}$ and $R^{7b}$ together form ethenyl, propenyl, 3-membered cycloalkyl, 4-membered cycloalkyl, 4-membered heterocycloalkyl containing 1 atom selected from N or O, or 5-membered heterocycloalkyl containing 1 atom selected from N or O, wherein the alkenyl is optionally further substituted with 1, 2, or 3 groups selected from deuterium, F, Cl, hydroxyl, or cyano;
or, $R_4$, $R_5$ and $X_2$, or $R^4$, $R^{6a}$ and $X_2$, or $R^4$, $R^{7a}$ and $X_2$ together form $C_{5-7}$cycloalkyl or 5- to 7-membered heterocycloalkyl; further, $R_4$, $R_5$ and $X_2$, or $R^4$, $R^{6a}$ and $X_2$, or $R^4$, $R^{7a}$ and $X_2$ together form 5-membered cycloalkyl, 6-membered cycloalkyl, 7-membered cycloalkyl, 5-membered heterocycloalkyl, 6-membered heterocycloalkyl or 7-membered heterocycloalkyl; further, $R_4$, $R_5$ and $X_2$, or $R^4$, $R^{6a}$ and $X_2$, or $R^4$, $R^{7a}$ and $X_2$ together form 5-membered cycloalkyl, 6-membered cycloalkyl or 7-membered cycloalkyl; further, $R_4$, $R_5$ and $X_2$, or $R^4$, $R^{6a}$ and $X_2$, or $R^4$, $R^{7a}$ and $X_2$ together form 5-membered heterocycloalkyl, 6-membered heterocycloalkyl, or 7-membered heterocycloalkyl, and further, the heterocycloalkyl contains 1 or 2 oxygen atoms;
or, $R^4$ and $R^{6a}$, or $R^{6b}$ and $R^{7b}$, or $R^4$ and $X_2$ together with the atom to which they are attached form $C_{3-7}$cycloalkyl, or 3- to 7-membered heterocycloalkyl; further, $R^4$ and $R^{6a}$, or $R^{6b}$ and $R^{7b}$, or $R^4$ and $X_2$ together with the atom to which they are attached form 3-membered cycloalkyl, 4-membered cycloalkyl, 5-membered cycloalkyl, 6-membered cycloalkyl, 7-membered cycloalkyl, 4-membered heterocycloalkyl, 5-membered heterocycloalkyl, 6-membered heterocycloalkyl or 7-membered heterocycloalkyl, wherein the heterocycloalkyl contains 1 or 2 oxygen atoms;
or, $R^{x2a}$ and $R^4$, or $R^{x2a}$ and $R^5$, or $R^{6a}$ and $R^4$, or $R^{6b}$ and $R^{7b}$, or $R^{7a}$and $R^5$ together with the atom to which they are attached form one chemical bond, provided that at most one group forms one chemical bond;
provided that

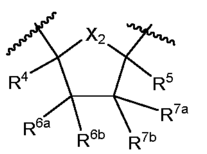

is not selected from the following structures:

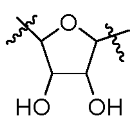 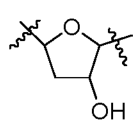 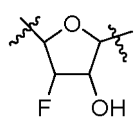 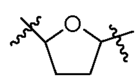 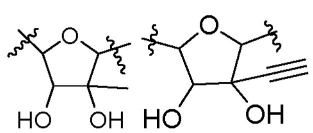

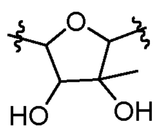,

and other groups are as described in 1.

5. The compound of formula I, and the stereoisomer, pharmaceutically acceptable salt, solvate, co-crystal or deuterated product thereof of the present invention, wherein

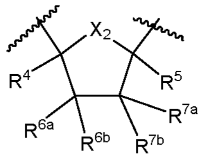

is selected from:

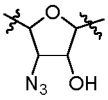 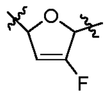 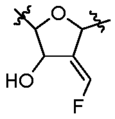 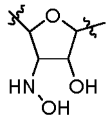 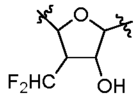 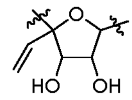 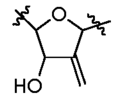 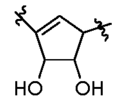

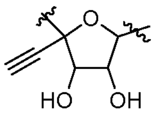 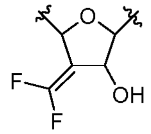 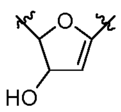 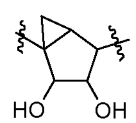 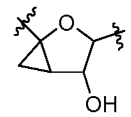 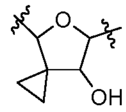

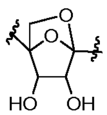 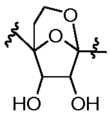 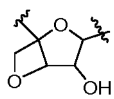 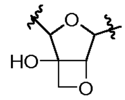 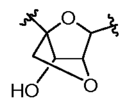 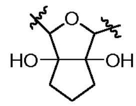 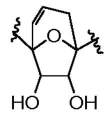 ,

and other groups are as described in 1.

6. The compound of formula I, and the stereoisomer, pharmaceutically acceptable salt, solvate, co-crystal or deuterated product thereof of the present invention, wherein

$R^{L2a}$, $R^5$ and $X_2$ together with the atom to which they are attached form $C_{5-7}$cycloalkyl, or 5- to 7-membered heterocycloalkyl; further, $R^{L2a}$, $R^5$ and $X_2$ together with the atom to which they are attached form 5-membered cycloalkyl, 6-membered cycloalkyl, 7-membered cycloalkyl, 5-membered heterocycloalkyl, 6-membered heterocycloalkyl or 7-membered heterocycloalkyl; further, $R^{L2a}$, $R^5$ and $X_2$ together with the atom to which they are attached form 5-membered heterocycloalkyl, 6-membered heterocycloalkyl or 7-membered heterocycloalkyl, wherein the heterocycloalkyl contains 1 or 2 oxygen atoms; further, $R^{L2a}$, $R^5$ and $X_2$ together with the atom to which they are attached form 6-membered heterocycloalkyl or 7-membered heterocycloalkyl, wherein the heterocycloalkyl contains 1 or 2 oxygen atoms;

or, $R^4$ and $R^{L2a}$ together with the atom to which they are attached form $C_{3-7}$cycloalkyl, or 3- to 7-membered heterocycloalkyl; further, $R^4$ and $R^{L2a}$ together with the atom to which they are attached form 3-membered cycloalkyl, 4-membered cycloalkyl, 5-membered cycloalkyl, 6-membered cycloalkyl, 7-membered cycloalkyl, 4-membered heterocycloalkyl, 5-membered heterocycloalkyl, 6-membered heterocycloalkyl or 7-membered heterocycloalkyl, wherein the heterocycloalkyl contains 1 or 2 oxygen atoms; further, $R^4$ and $R^{L2a}$ together with the atom to which they are attached form 3-membered cycloalkyl, 4-membered cycloalkyl or 5-membered cycloalkyl,

and other groups are as described in 1.

7. The compound of formula I, and the stereoisomer, pharmaceutically acceptable salt, solvate, co-crystal or deuterated product thereof of the present invention, wherein the compound has a structure of formula (II):

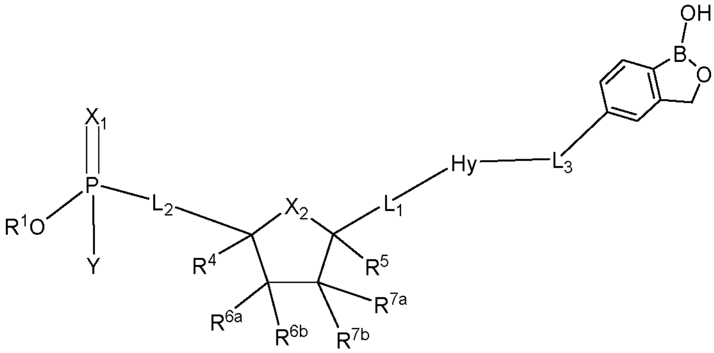

(II)

and other groups are as described in 1.

8. The compound of formula I, and the stereoisomer, pharmaceutically acceptable salt, solvate, co-crystal or deuterated product of the present invention, wherein the compound has a structure of formula (III), (IV), (V), (VI), (III-1), (IV-1), (V-1), or (VI-1):

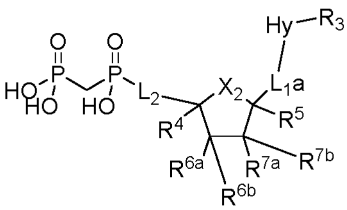

(III)

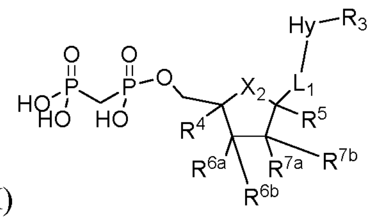

(IV)

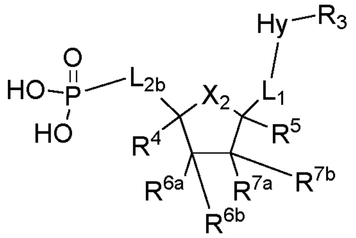

(V)

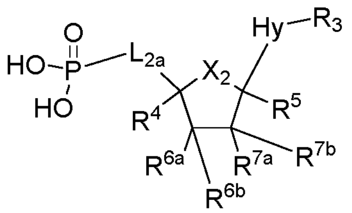

(VI)

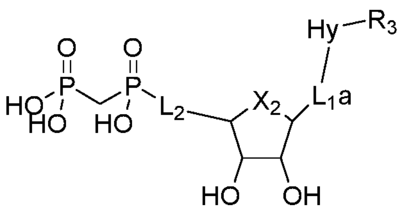

(III-1)

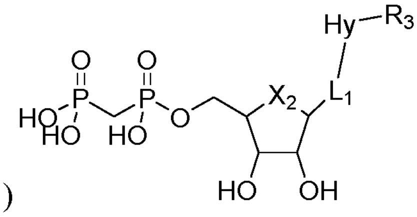

(IV-1)

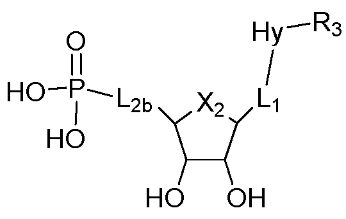

(V-1)

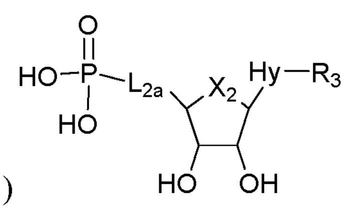

(VI-1)

$L_1$ is selected from S, O, NH, $N(CH_3)$ or $-CH_2-$; further, $L_1$ is selected from - $CH_2$-; further, $L_1$ is selected from O, NH, or $N(CH_3)$; further, $L_1$ is selected from O; further, $L_1$ is selected from NH or $N(CH_3)$;

$L_{1a}$ is selected from a bond, S, O, NH, $N(CH_3)$ or $-CH_2-$; further, $L_{1a}$ is selected from a bond, O, NH, $N(CH_3)$ or $-CH_2-$; further, $L_{1a}$ is selected from a bond or $-CH_2-$;

$L_2$ is selected from $*\text{-O-}(CR^{L2a}R^{L2b})_2\text{-}$, $*\text{-}(CR^{L2a}R^{L2b})n'\text{-O-}$, -O-, $*\text{-O-}(CR^{L2a}R^{L2b})n'\text{-O-}(CR^{L2a}R^{L2b})m\text{-}$,

*-($CR^{L2a}R^{L2b}$)n'-O-($CR^{L2a}R^{L2b}$)m-, *-O-($CR^{L2a}R^{L2b}$)n'-O-, *-O-N($R^{L2c}$)-, *-C(O)$C_{1-6}$alkyl, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$; further, $L_2$ is selected from *-O-($CR^{L2a}R^{L2b}$)$_2$-, *-C($R^{L2a}R^{L2b}$)-O-, *-($CR^{L2a}R^{L2b}$)$_2$-O-, -O-, *-O-C($R^{L2a}R^{L2b}$)-O-C($R^{L2a}R^{L2b}$)-, *-O-($CR^{L2a}R^{L2b}$)$_2$-O-($CR^{L2a}R^{L2b}$)$_2$-, *-C($R^{L2a}R^{L2b}$)-O-C($R^{L2a}R^{L2b}$)-, *-($CR^{L2a}R^{L2b}$)$_2$-O-($CR^{L2a}R^{L2b}$)$_2$-, *-O-C($R^{L2a}R^{L2b}$)-O-, *-O-($CR^{L2a}R^{L2b}$)$_2$-O-, *-O-N($R^{L2c}$)-, *-C(O)$C_{1-3}$alkyl-, *-Z-phenyl-, *-Z-(5-membered heteroaryl)-, *-Z-(6-membered heteroaryl)-, -$C_{2-4}$alkenyl- or -$C_{2-4}$alkynyl-, wherein the alkyl, phenyl, heteroaryl, alkenyl or alkynyl is optionally further substituted with 1 to 3 groups selected from $R^x$, and * denotes the site where the $L_2$ is connected to P atom;

$L_{2a}$ is selected from *-O-($CR^{L2a}R^{L2b}$)$_2$-, *-($CR^{L2a}R^{L2b}$)$_{n'}$-O-, *-O-($CR^{L2a}R^{L2b}$)$_{n'}$-O-($CR^{L2a}R^{L2b}$)$_m$-, *-($CR^{L2a}R^{L2b}$)$_2$-O-($CR^{L2a}R^{L2b}$)$_m$-, *-O-($CR^{L2a}R^{L2b}$)$_{n'}$-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2a}$ is selected from *-O-($CR^{L2a}R^{L2b}$)$_2$-, *-($CR^{L2a}R^{L2b}$)n'-O-, *-O-($CR^{L2a}R^{L2b}$)$_{n'}$-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2a}$ is selected from *-O-($CR^{L2a}R^{L2b}$)$_2$-, *-($CR^{L2a}R^{L2b}$)n'-O-, *-O-($CR^{L2a}R^{L2b}$)$_{n'}$-O-, *-C(O)$C_{1-3}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1, 2, or 3 groups selected from $R^x$, and * denotes the site where the $L_2$ is connected to P atom; further, $L_{2a}$ is selected from -O-$CH_2CH_2CH_2$-O-, -O-$CH_2CH_2$-O-, -O-$CH_2CH_2$-, *-C(O)$CH_2$-, phenyl, *-$CH_2$-phenyl-, *-O-phenyl-, ethenyl, propenyl or ethynyl, wherein the phenyl, ethenyl, propenyl or ethynyl is optionally further substituted with 1 or 2 groups selected from F and Cl, and * denotes the site where the $L_2$ is connected to P atom;

$L_{2b}$ is selected from *-O-($CR^{L2a}R^{L2b}$)$_2$-, *-($CR^{L2a}R^{L2b}$)n'-O-, -O-, *-O-($CR^{L2a}R^{L2b}$)n'-O-($CR^{L2a}R^{L2b}$)m-, *-($CR^{L2a}R^{L2b}$)$_{n'}$-O-($CR^{L2a}R^{L2b}$)$_m$-, *-O-($CR^{L2a}R^{L2b}$)$_{n'}$-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2b}$ is selected from *-O-($CR^{L2a}R^{L2b}$)$_2$-, *-($CR^{L2a}R^{L2b}$)n'-O-, -O-, *-($CR^{L2a}R^{L2b}$)$_{n'}$-O-($CR^{L2a}R^{L2b}$)$_m$-, *-O-($CR^{L2a}R^{L2b}$)n'-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2b}$ is selected from *-($CR^{L2a}R^{L2b}$)n'-O- or *-($CR^{L2a}R^{L2b}$)$_{n'}$-O-($CR^{L2a}R^{L2b}$)$_m$-; further, $L_{2b}$ is selected from *-($CR^{L2a}R^{L2b}$)-O- or *-($CR^{L2a}R^{L2b}$)-O-($CR^{L2a}R^{L2b}$)-;

$R^{L2a}$, $R^{L2b}$, and $R^{L2c}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl or -$C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl; further, $R^{L2a}$, $R^{L2b}$, and $R^{L2c}$ are each independently selected from H, deuterium, halogen, amino, hydroxyl, cyano, $C_{1-4}$alkyl, $C_{1-4}$alkoxy, $C_{2-4}$alkenyl, $C_{2-4}$alkynyl or -$C_{1-4}$alkyl-O-$C_{1-4}$alkyl, wherein the alkyl, alkoxy, alkenyl or alkynyl is optionally further substituted with 1 or more groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, 5-to 6-membered heteroaryl, 8- to 10-membered bicyclic heteroaryl, phenyl, $C_{8-10}$bicyclic aryl, 4- to 6-membered monocyclic heterocycloalkyl, 6- to 10-membered bicyclic heterocycloalkyl, $C_{3-6}$monocyclic cycloalkyl or $C_{6-10}$bicyclic cycloalkyl; further, $R^{L2a}$, $R^{L2b}$, and $R^{L2c}$ are each independently selected from H, deuterium, F, Cl, amino, hydroxyl, cyano, methyl, ethyl, propyl, isopropyl, butyl, methoxy, ethoxy, propoxy, isopropoxy, butoxy, ethenyl, propenyl, ethynyl, propynyl, - methyl-O-methyl-, -methyl-O-ethyl- or -ethyl-O-ethyl-, and the above-mentioned groups are optionally further substituted with 1, 2, or 3 groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, 5- to 6-membered heteroaryl, phenyl, 4- to 6-membered monocyclic heterocycloalkyl, $C_{3-6}$monocyclic cycloalkyl or $C_{6-10}$bicyclic cycloalkyl;

or, $R^{L2a}$ and $R^{L2b}$ on the same carbon atom together with the carbon atom to which they are attached form $C_{3-6}$cycloalkyl or 4- to 6-membered heterocycloalkyl; further, $R^{L2a}$ and $R^{L2b}$ on the same carbon atom together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl or 6-membered heterocycloalkyl;

$X_2$ is selected from $CH_2$ or O; further, $X_2$ is selected from $CH_2$; further, $X_2$ is selected from O;

each Z is independently selected from a bond, -O- or $C_{1-6}$alkyl, wherein the alkyl is optionally further substituted with 1 or more groups selected from $R^x$; further, each Z is independently selected from a bond, -O-, or $C_{1-3}$alkyl,

wherein the alkyl is optionally further substituted with 1, 2, or 3 groups selected from $R^x$; further, each Z is independently selected from a bond, -O-, methyl, ethyl, or propyl, wherein the methyl, ethyl, and propyl are optionally further substituted with 1, 2, or 3 groups selected from $R^x$;

m, n, and n' are each independently selected from 1, 2 or 3; further, each m is independently selected from 1 or 2, and n and n' are each independently selected from 1, 2, or 3;

provided that, in formula (V), when $L_{2b}$ is selected from *-C($R^{L2a}R^{L2b}$)-O-C($R^{L2a}R^{L2b}$)-, $R^{L2a}$ and $R^{L2b}$ are not both H, and other groups are as described in 1.

9. The compound, and the stereoisomer, pharmaceutically acceptable salt, solvate, co-crystal or deuterated product of the present invention, wherein the compound has a structure of formula (III-2), (III-3), (IV-2), (IV-3), (V-2), (V-3), (VI-2), or (VI-3):

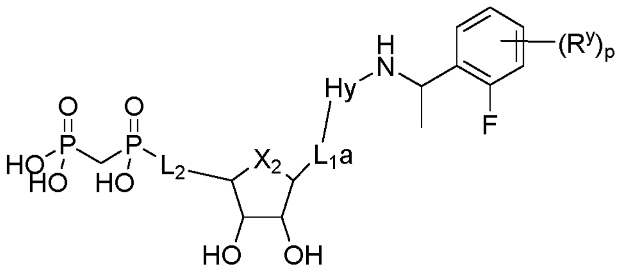

(III-2)

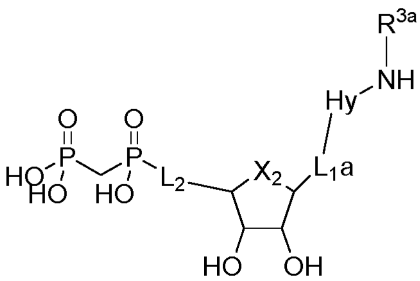

(III-3)

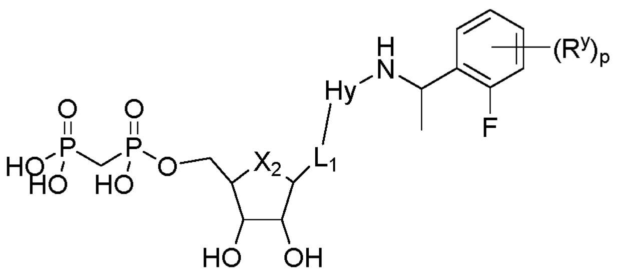

(IV-2)

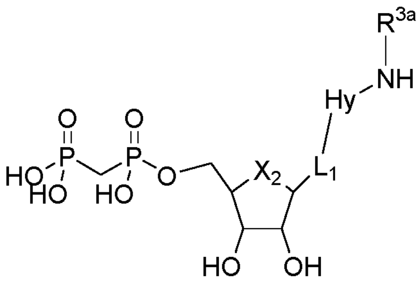

(IV-3)

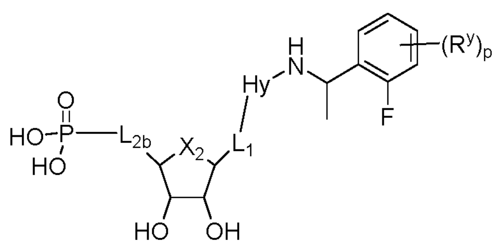

(V-2)

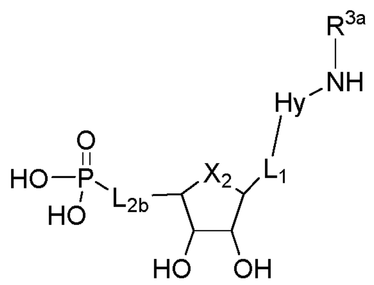

(V-3)

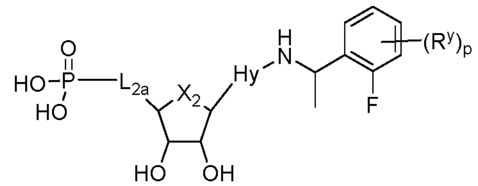

(VI-2)

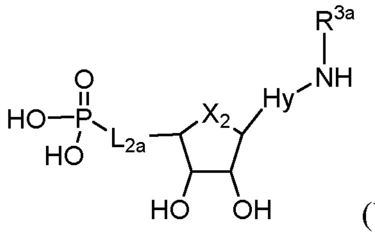

(VI-3)

p is selected from 0, 1 or 2; further, p is selected from 0 or 1, and furthermore, p is selected from 0;

$R^{3a}$ is selected from 5- to 6-membered monocyclic cycloalkyl, 5- to 6-membered monocyclic heterocycloalkyl, 8- to 10-membered bicyclic cycloalkyl, 8-to 10-membered bicyclic heterocycloalkyl, 11- to 15-membered tricyclic cycloalkyl or 11- to 15-membered tricyclic heterocycloalkyl, wherein the cycloalkyl or heterocycloalkyl is optionally further substituted with 1 to 3 groups selected from $R^y$; further, $R^{3a}$ is selected from 5-membered monocyclic cycloalkyl, 6-membered monocyclic cycloalkyl, 5-membered monocyclic heterocycloalkyl, 6-membered monocyclic heterocycloalkyl, a benzo-fused 5-membered cycloalkyl, a benzo-fused 5-membered heterocycloalkyl, a benzo-fused 6-membered cycloalkyl, a benzo-fused 6-membered heterocycloalkyl, a benzo-fused 5-membered heteroaryl, a benzo-fused 6-membered heteroaryl, a pyrido-fused 5-membered cycloalkyl, a pyrido-fused 5-membered heterocycloalkyl, a pyrido-fused 6-membered cycloalkyl, a pyrido-fused 6-membered heterocycloalkyl, a pyrido-fused 5-membered heteroaryl, a pyrido-fused 6-membered heteroaryl, a 5-membered cycloalkyl fused phenyl, a 5-membered heterocycloalkyl fused phenyl, a 6-membered cycloalkyl fused phenyl, a 6-membered heterocycloalkyl fused phenyl, a 5-membered heteroaryl fused phenyl, a 6-membered heteroaryl fused phenyl, a 5-membered cycloalkyl fused pyridyl, a 5-membered heterocycloalkyl fused pyridyl, a 6-membered cycloalkyl fused pyridyl, a 6-membered heterocycloalkyl fused pyridyl, a 5-membered heteroaryl fused pyridyl or a 6-membered heteroaryl fused pyridyl, wherein the ring on the left of the word "fused" means a ring to which a nitrogen atom is connected to, and the phenyl, pyridyl, cycloalkyl, heterocycloalkyl, or heteroaryl is further substituted with 1, 2, or 3 groups selected from $R^y$; further, $R^{3a}$ is selected from 5-membered monocyclic cycloalkyl, 6-membered monocyclic cycloalkyl, 5-membered monocyclic heterocycloalkyl, 6-membered monocyclic heterocycloalkyl, a 5-membered cycloalkyl fused phenyl, a 5-membered heterocycloalkyl fused phenyl, a 6-membered cycloalkyl fused phenyl or a 6-membered heterocycloalkyl fused phenyl, wherein the ring on the left of the word "fused" means a ring to which a nitrogen atom is connected to, and the phenyl, cycloalkyl, heterocycloalkyl, or heteroaryl is further substituted with 1, 2, or 3 groups selected from $R^y$;

$L_2$ is selected from *-O-$(CR^{L2a}R^{L2b})_2$-, *-$(CR^{L2a}R^{L2b})$n'-O-, -O-, *-C$(R^{L2a}R^{L2b})$-O-C$(R^{L2a}R^{L2b})$-, *-O-C$(R^{L2a}R^{L2b})$-O-, *-O-N$(R^{L2c})$-, *-C(O)$C_{1-3}$alkyl-, *-Z-phenyl-, *-Z-(5-membered heteroaryl)-, *-Z-(6-membered heteroaryl)-, -$C_{2-4}$alkenyl- or -$C_{2-4}$alkynyl-, wherein the alkyl, aryl, heteroaryl, alkenyl or alkynyl is optionally further substituted with 1 to 3 groups selected from $R^x$; further, $L_2$ is selected from *-O-$(CR^{L2a}R^{L2b})$n'-, *-$(CR^{L2a}R^{L2b})$n'-O-, -O-, *-$(CR^{L2a}R^{L2b})$n'-O-$(CR^{L2a}R^{L2b})$m-, *-O-$(CR^{L2a}R^{L2b})$n'-O-, *-O-N$(R^{L2c})$-, *-C(O)$C_{1-4}$alkyl, *-Z-$C_{6-10}$aryl-, -$C_{2-6}$alkenyl- or -$C_{2-6}$alkynyl-, wherein the alkyl, aryl, alkenyl or alkynyl is optionally further substituted with 1 or more groups selected from $R^x$; further, $L_2$ is selected from *-O-$(CR^{L2a}R^{L2b})_2$-, *-C$(R^{L2a}R^{L2b})$-O-, -O-, *-C$(R^{L2a}R^{L2b})$-O-C$(R^{L2a}R^{L2b})$-, *-O-C$(R^{L2a}R^{L2b})$-O-, *-O-N$(R^{L2c})$-, *-C(O)$C_{1-3}$alkyl-, *-Z-phenyl-, - $C_{2-4}$alkenyl- or -$C_{2-4}$alkynyl-, wherein the alkyl, aryl, alkenyl or alkynyl is optionally further substituted with 1 or more groups selected from $R^x$; further, $L_2$ is selected from *-O-$(CR^{L2a}R^{L2b})_2$-, *-C$(R^{L2a}R^{L2b})$-O-, *-$(CR^{L2a}R^{L2b})_2$-O-, -O-, *-O-C$(R^{L2a}R^{L2b})$-O-C$(R^{L2a}R^{L2b})$-, *-O-$(CR^{L2a}R^{L2b})_2$-O-$(CR^{L2a}R^{L2b})_2$-, *-C$(R^{L2a}R^{L2b})$-O-C$(R^{L2a}R^{L2b})$-, *-$(CR^{L2a}R^{L2b})_2$-O-$(CR^{L2a}R^{L2b})_2$-, *-O-C$(R^{L2a}R^{L2b})$-O-, *-O-$(CR^{L2a}R^{L2b})_2$-O-, *-O-N$(R^{L2c})$-, *-C(O)$C_{1-3}$alkyl-, *-Z-phenyl-, *-Z-(5-membered heteroaryl)--, *-Z-(6-membered heteroaryl)-, -$C_{2-4}$alkenyl- or -$C_{2-4}$alkynyl-, wherein the alkyl, phenyl, heteroaryl, alkenyl or alkynyl is optionally further substituted with 1, 2, or 3 groups selected from $R^x$;

$L_{2a}$ is selected from *-O-$(CR^{L2a}R^{L2b})_2$-, *-$(CR^{L2a}R^{L2b})_{n'}$-O-, *-O-$(CR^{L2a}R^{L2b})_{n'}$-O-$(CR^{L2a}R^{L2b})_{m}$-,

*-$(CR^{L2a}R^{L2b})_2$-O-$(CR^{L2a}R^{L2b})_m$-, *-O-$(CR^{L2a}R^{L2b})_{n'}$-O-, *-O-N($R^{L2c}$)-, *-C(O)$C_{1-6}$alkyl, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2a}$ is selected from *-O-$(CR^{L2a}R^{L2b})_2$-, *-$(CR^{L2a}R^{L2b})_{n'}$-O-, *-O-$(CR^{L2a}R^{L2b})_{n'}$-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2a}$ is selected from *-O-$(CR^{L2a}R^{L2b})_2$-, *-$(CR^{L2a}R^{L2b})$n'-O-, *-O-$(CR^{L2a}R^{L2b})_{n'}$-O-, *-C(O)$C_{1-3}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1, 2, or 3 groups selected from $R^x$, and * denotes the site where the $L_2$ is connected to P atom; further, $L_{2a}$ is selected from -O-$CH_2CH_2CH_2$-O-, -O-$CH_2CH_2$-O-, -O-$CH_2CH_2$-, *-C(O)$CH_2$-, phenyl, *-$CH_2$-phenyl-, *-O-phenyl-, ethenyl, propenyl or ethynyl, wherein the phenyl, ethenyl, propenyl or ethynyl is optionally further substituted with 1 or 2 groups selected from F and Cl, and * denotes the site where the $L_2$ is connected to P atom;

$L_{2b}$ is selected from *-O-$(CR^{L2a}R^{L2b})_2$-, *-$(CR^{L2a}R^{L2b})$n'-O-, -O-, *-O-$(CR^{L2a}R^{L2b})$n'-O-$(CR^{L2a}R^{L2b})$m-, *-$(CR^{L2a}R^{L2b})_{n'}$-O-$(CR^{L2a}R^{L2b})_m$-, *-O-$(CR^{L2a}R^{L2b})_{n'}$-O-, *-O-N($R^{L2c}$)-, *-C(O)$C_{1-6}$alkyl, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2b}$ is selected from *-O-$(CR^{L2a}R^{L2b})_2$-, *-$(CR^{L2a}R^{L2b})$n'-O-, -O-, *-$(CR^{L2a}R^{L2b})_{n'}$-O-$(CR^{L2a}R^{L2b})_m$-, *-O-$(CR^{L2a}R^{L2b})$n'-O-, *-O-N($R^{L2c}$)-, *-C(O)$C_{1-6}$alkyl-, *-Z-$C_{6-10}$aryl-, *-Z-(5- to 10-membered heteroaryl)-, -$C_{2-6}$alkenyl- or -$C_{2-6}$alkynyl-, wherein the alkyl, aryl, heteroaryl, alkenyl or alkynyl is optionally further substituted with 1 to 3 groups selected from $R^x$, and * denotes the site where the $L_2$ is connected to P atom; further, $L_{2b}$ is selected from *-$(CR^{L2a}R^{L2b})$n'-O- or *-$(CR^{L2a}R^{L2b})_{n'}$-O-$(CR^{L2a}R^{L2b})_m$-; further, $L_{2b}$ is selected from *-$(CR^{L2a}R^{L2b})$-O-, or *-$(CR^{L2a}R^{L2b})$-O-$(CR^{L2a}R^{L2b})$-;

$R^{L2a}$, $R^{L2b}$, and $R^{L2c}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl or -$C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl; further, $R^{L2a}$, $R^{L2b}$, and $R^{L2c}$ are each independently selected from H, deuterium, halogen, amino, hydroxyl, cyano, $C_{1-4}$alkyl, $C_{1-4}$alkoxy, $C_{2-4}$alkenyl, $C_{2-4}$alkynyl or -$C_{1-4}$alkyl-O-$C_{1-4}$alkyl, wherein the alkyl, alkoxy, alkenyl or alkynyl is optionally further substituted with 1 or more groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, 5-to 6-membered heteroaryl, 8- to 10-membered bicyclic heteroaryl, phenyl, $C_{8-10}$bicyclic aryl, 4- to 6-membered monocyclic heterocycloalkyl, 6- to 10-membered bicyclic heterocycloalkyl, $C_{3-6}$monocyclic cycloalkyl or $C_{6-10}$bicyclic cycloalkyl; further, $R^{L2a}$, $R^{L2b}$, and $R^{L2c}$ are each independently selected from H, deuterium, F, Cl, amino, hydroxyl, cyano, methyl, ethyl, propyl, isopropyl, butyl, methoxy, ethoxy, propoxy, isopropoxy, butoxy, ethenyl, propenyl, ethynyl, propynyl, - methyl-O-methyl-, -methyl-O-ethyl- or -ethyl-O-ethyl-, and the above-mentioned groups are optionally further substituted with 1, 2, or 3 groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, 5- to 6-membered heteroaryl, phenyl, 4- to 6-membered monocyclic heterocycloalkyl, $C_{3-6}$monocyclic cycloalkyl or $C_{6-10}$bicyclic cycloalkyl;

or, $R^{L2a}$ and $R^{L2b}$ on the same carbon atom together with the carbon atom to which they are attached form $C_{3-6}$cycloalkyl or 4- to 6-membered heterocycloalkyl; further, $R^{L2a}$ and $R^{L2b}$ on the same carbon atom together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl or 6-membered heterocycloalkyl;

each Z is independently selected from a bond, -O- or $C_{1-6}$alkyl, wherein the alkyl is optionally further substituted with 1 or more groups selected from $R^x$; further, each Z is independently selected from a bond, -O- or $C_{1-3}$alkyl, wherein the alkyl is optionally further substituted with 1, 2, or 3 groups selected from $R^x$; further, each Z is independently selected from a bond, -O-, methyl, ethyl or propyl, wherein the methyl, ethyl or propyl is optionally further substituted with 1, 2, or 3 groups selected from $R^x$;

m, n, and n' are each independently selected from 1, 2 or 3; further, each m is independently selected from 1 or 2, and n and n' are each independently selected from 1, 2, or 3;

provided that, in formula (V-2) or (V-3), when $L_{2b}$ is selected from *-C($R^{L2a}R^{L2b}$)-O-C($R^{L2a}R^{L2b}$)-, $R^{L2a}$ and $R^{L2b}$ are not both H.

10. The compound, and the stereoisomer, pharmaceutically acceptable salt, solvate, co-crystal or deuterated product thereof of the present invention, wherein

Hy is selected from 6-membered heteroaryl, a 5-membered heteroaryl fused 5-membered heteroaryl, a 5-membered heteroaryl fused phenyl, a 5-membered heteroaryl fused 5-membered heterocycloalkyl, a 5-membered heteroaryl fused 6-membered heterocycloalkyl, a 5-membered heterocycloalkyl fused 5-membered heteroaryl, a 5-membered heterocycloalkyl fused 6-membered heteroaryl, a 5-membered heterocycloalkyl fused phenyl, a 5-membered heterocycloalkyl fused 5-membered heterocycloalkyl, a 5-membered heterocycloalkyl fused 6-membered heterocycloalkyl, a 6-membered heteroaryl fused 5-membered heteroaryl, a 6-membered heteroaryl fused 6-membered heteroaryl, a 6-membered heteroaryl fused phenyl, a 6-membered heteroaryl fused 5-membered heterocycloalkyl, a 6-membered heteroaryl fused 6-membered heterocycloalkyl, a 6-membered heterocycloalkyl fused 5-membered heteroaryl, a 6-membered heterocycloalkyl fused 6-membered heteroaryl, a 6-membered heterocycloalkyl fused phenyl, a 6-membered heterocycloalkyl fused 5-membered heterocycloalkyl, a 6-membered heterocycloalkyl fused 6-membered heterocycloalkyl or

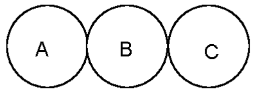
;

A ring is selected from 5- to 6-membered heteroaryl, 5- to 6-membered heterocycloalkyl or phenyl;
B ring is selected from 5- to 6-membered heteroaryl, 4- to 6-membered heterocycloalkyl or 4-6cycloalkyl;
C ring is selected from 5- to 6-membered heteroaryl, 5- to 6-membered heterocycloalkyl or phenyl;
the heteroaryl, aryl, phenyl or heterocycloalkyl is optionally further substituted with 1 or more groups selected from $R^{hy}$;
each $R^{hy}$ is substituted with a group independently selected from H, deuterium, halogen, amino, hydroxyl, cyano, $C_{1-6}$alkyl, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy,
and other groups are as described in 1.

11. The compound, and the stereoisomer, pharmaceutically acceptable salt, solvate, co-crystal or deuterated product thereof of the present invention, wherein

Hy is selected from

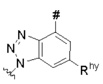 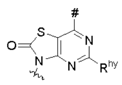 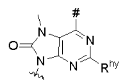 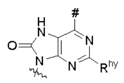 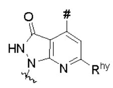 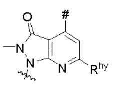 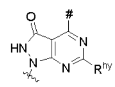 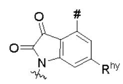

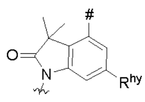 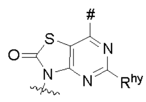 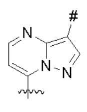 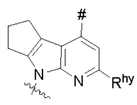 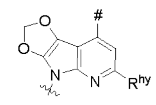 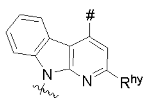 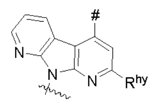
;

each $R^{hy}$ is independently selected from deuterium, halogen, amino, hydroxyl, cyano, $SF_5$, azido, $C_{1-4}$alkyl, $C_{1-4}$alkoxy, $C_{2-4}$alkenyl, or $C_{2-4}$alkynyl, wherein the alkyl, alkoxy, alkenyl, or alkynyl is optionally further substituted with 1 to 3 groups selected from deuterium, halogen, amino, hydroxyl, cyano, deuterated $C_{1-4}$alkyl, halo $C_{1-4}$alkyl, hydroxyl $C_{1-4}$alkyl, $C_{1-4}$alkoxy, deuterated $C_{1-4}$alkoxy, halo $C_{1-4}$alkoxy or hydroxyl $C_{1-4}$alkoxy,
and other groups are as described in 1.

12. The compound, and the stereoisomer, pharmaceutically acceptable salt, solvate, co-crystal or deuterated product thereof of the present invention, wherein

Y is selected from $-C(R^{y1}R^{y2})-R^{y4}$, $-C(R^{y1}R^{y2})-P(=S)(OR^2)OR^2$, $-C(R^{y1}R^{y2})-P(=S)(OR^2)NR^{y3}$, or $-C(R^{y1}R^{y2})-P(=S)(OR^2)SR^{y3}$;
$R^{y1}$, $R^{y2}$, $R^{y3}$, $R^{x2a}$, $R^{x2b}$ and $R^{x2c}$ are each independently selected from H, deuterium, hydroxyl, cyano, $C_{1-6}$alkyl, deuterated $C_{1-6}$alkyl or halo $C_{1-6}$alkyl; further, $R^{y1}$, $R^{y2}$, $R^{y3}$, $R^{x2a}$, $R^{x2b}$, and $R^{x2c}$ are each independently selected from H, deuterium, hydroxyl, cyano, $C_{1-3}$alkyl, deuterated $C_{1-3}$alkyl or halo $C_{1-3}$alkyl; further, $R^{y1}$, $R^{y2}$, $R^{y3}$, $R^{x2a}$, $R^{x2b}$, and $R^{x2c}$ are each independently selected from H, deuterium or hydroxyl;
$R^{y4}$ is selected from 4- to 10-membered heterocycloalkyl, 5- to 10-membered heteroaryl, $C_{3-10}$cycloalkyl or $C_{6-10}$aryl, wherein the heterocycloalkyl, heteroaryl, cycloalkyl or aryl is optionally further substituted with 1 or more groups selected from $R^x$; further, $R^{y4}$ is selected from 5-membered heterocycloalkyl, 6-membered heterocycloalkyl or 7-membered heterocycloalkyl, wherein the heterocycloalkyl is optionally further substituted with 1, 2, or 3 groups selected from $R^x$,
and other groups are as described in 1.

13. The compound, and the stereoisomer, pharmaceutically acceptable salt, solvate, co-crystal or deuterated product thereof of the present invention, wherein the compound has a structure of formula (VII):

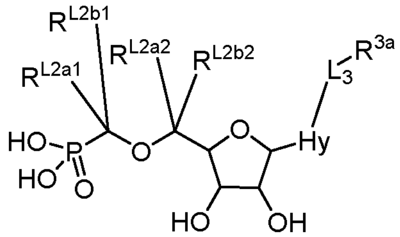
(VII)

$R^{L2a1}$, $R^{L2b1}$, $R^{L2a2}$, and $R^{L2b2}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl, or -$C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy; further, $R^{L2a1}$, $R^{L2b1}$, $R^{L2a2}$, and $R^{L2b2}$ are each independently selected from H, deuterium, halogen, amino, hydroxyl, cyano, $C_{1-4}$alkyl, $C_{1-4}$alkoxy, $C_{2-4}$alkenyl, $C_{2-4}$alkynyl, or -$C_{1-4}$alkyl-O-$C_{1-4}$alkyl, wherein the alkyl, alkoxy, alkenyl or alkynyl is optionally further substituted with 1 or more groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy or hydroxyl $C_{1-2}$alkoxy;
or, $R^{L2a1}$ and $R^{L2b1}$ on the same carbon atom together with the carbon atom to which they are attached form $C_{3-6}$cycloalkyl or 4- to 6-membered heterocycloalkyl; further, $R^{L2a1}$ and $R^{L2b1}$ on the same carbon atom together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl or 6-membered heterocycloalkyl;
or, $R^{L2a2}$ and $R^{L2b2}$ on the same carbon atom together with the carbon atom to which they are attached form $C_{3-6}$cycloalkyl or 4- to 6-membered heterocycloalkyl; further, $R^{L2a2}$ and $R^{L2b2}$ on the same carbon atom together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl or 6-membered heterocycloalkyl;
provided that, at least three of $R^{L2a1}$, $R^{L2b1}$, $R^{L2a2}$ and $R^{L2b2}$ are not selected from H;
$L_3$ is selected from **-N($R^{L3}$)$C_{1-6}$alkyl- or -N($R^{L3}$)-; further, $L_3$ is selected from **-N($R^{L3}$)$C_{1-2}$alkyl- or-N(H)-;
$R^{3a}$ is selected from 5- to 15-membered carbocyclyl or 5- 15heterocyclyl, wherein the carbocyclyl or heterocyclyl is optionally further substituted with 1 or more groups selected from $R^y$; further, $R^{3a}$ is selected from 5- to 6-membered monocyclic cycloalkyl, 5- to 6-membered monocyclic heterocycloalkyl, 8- to 10-membered bicyclic cycloalkyl, 8- to 10-membered bicyclic heterocycloalkyl, 11- to 15-membered tricyclic cycloalkyl, 11- to 15-membered tricyclic heterocycloalkyl, wherein the cycloalkyl or heterocycloalkyl is optionally further substituted with 1 to 3 groups selected from $R^y$; further, $R^{3a}$ is selected from 5-membered monocyclic cycloalkyl, 6-membered monocyclic cycloalkyl, 5-membered monocyclic heterocycloalkyl, 6-membered monocyclic heterocycloalkyl, a benzo-fused 5-membered cycloalkyl, a benzo-fused 5-membered heterocycloalkyl, a benzo-fused 6-membered cycloalkyl, a benzo-fused 6-membered heterocycloalkyl, a benzo-fused 5-membered heteroaryl, a benzo-fused 6-membered heteroaryl, a pyrido-fused 5-membered cycloalkyl, a pyrido-fused 5-membered heterocycloalkyl, a pyrido-fused 6-membered cycloalkyl, a pyrido-fused 6-membered heterocycloalkyl, a pyrido-fused 5-membered heteroaryl, a pyrido-fused 6-membered heteroaryl, a 5-membered cycloalkyl fused phenyl, a 5-membered heterocycloalkyl fused phenyl, a 6-membered cycloalkyl fused phenyl, a 6-membered heterocycloalkyl fused phenyl, a 5-membered heteroaryl fused phenyl, a 6-membered heteroaryl fused phenyl, a 5-membered cycloalkyl fused pyridyl, a 5-membered heterocycloalkyl fused pyridyl, a 6-membered cycloalkyl fused pyridyl, a 6-membered heterocycloalkyl fused pyridyl, a 5-membered heteroaryl fused pyridyl or a 6-membered heteroaryl fused pyridyl, wherein the ring on the left of the word "fused" means a ring to which a nitrogen atom is connected to, and the phenyl, pyridyl, cycloalkyl, heterocycloalkyl, or heteroaryl is further substituted with 1, 2, or 3 groups selected from $R^y$; further, $R^{3a}$ is selected from 5-membered monocyclic cycloalkyl, 6-membered monocyclic cycloalkyl, 5-membered monocyclic heterocycloalkyl, 6-membered monocyclic heterocycloalkyl, a 5-membered cycloalkyl fused phenyl, a 5-membered heterocycloalkyl fused phenyl, a 6-membered cycloalkyl fused phenyl or a 6-membered heterocycloalkyl fused phenyl, wherein the

ring on the left of the word "fused" means a ring to which a nitrogen atom is connected to, and the phenyl, cycloalkyl, heterocycloalkyl, or heteroaryl is further substituted with 1, 2, or 3 groups selected from $R^y$,
and other groups are as described in 1.

14. The compound of formula I, and the stereoisomer, pharmaceutically acceptable salt, solvate, co-crystal or deuterated product thereof, wherein the compound has a structure of formula (VIII) as follows:

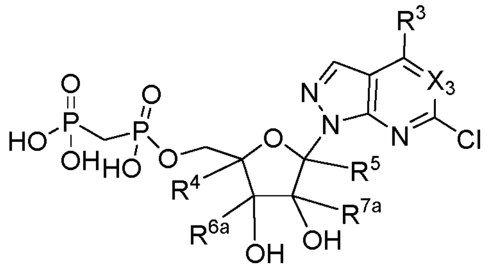

(VIII)

wherein, $X_3$ is selected from CH or N;
$R_4$, $R_5$, $R^{6a}$, and $R^{7a}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino, alkyl, cycloalkyl or alkynyl is optionally further substituted with 1, 2, or 3 groups selected from deuterium, halogen, hydroxyl, cyano or $C_{1-6}$alkoxy; furthermore, $R_4$, $R_5$, $R^{6a}$, and $R^{7a}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino or alkyl is optionally further substituted with 1, 2, or 3 groups selected from deuterium, halogen, hydroxyl, cyano or $C_{1-6}$alkoxy; furthermore, $R_4$, $R_5$, $R^{6a}$, and $R^{7a}$ are each independently selected from H, deuterium, hydroxyl, $C_{1-6}$alkyl or $C_{2-6}$alkynyl, wherein the alkyl is optionally further substituted with 1, 2, or 3 groups selected from halogen or hydroxyl; furthermore, $R_4$, $R_5$, $R^{6a}$, and $R^{7a}$ are each independently selected from H, $C_{1-6}$alkyl or $C_{2-4}$alkynyl, wherein the alkyl is optionally further substituted with 1, 2, or 3 groups selected from halogen or hydroxyl; furthermore, $R_4$, $R_5$, and $R^{7a}$ are H, and $R^{6a}$ is $C_{1-4}$alkyl or $C_{2-4}$alkynyl, wherein the alkyl is optionally further substituted with 1, 2, or 3 groups selected from halogen or hydroxyl; furthermore, $R_4$, $R_5$, and $R^{7a}$ are H, and $R^{6a}$ is methyl, ethyl, propyl or ethyne, which is optionally further substituted with 1, 2, or 3 groups selected from halogen or hydroxyl;
$R^3$ is selected from -NH-O-$R^{3a}$, 3- to 6-membered monocyclic cycloalkyl, 4-to 6-membered monocyclic heterocycloalkyl, 5- to 10-membered spiro ring, 4- to 10-membered fused ring, or 5- to 9-membered bridged ring, wherein the cycloalkyl, heterocycle cycloalkyl, spiro ring, bridged ring or fused ring is optionally further substituted with 1, 2, or 3 groups selected from $R^y$; furthermore, $R^3$ is selected from -NH-O-$R^{3a}$, 3- to 6-membered monocyclic cycloalkyl, or 7- to 10-membered heterocycle fused ring containing 1 to 3 heteroatoms selected from N, S, or O, wherein the cycloalkyl and heterocycle fused ring are optionally further substituted with 1, 2, or 3 groups selected from $R^y$; furthermore, $R^3$ is selected from -NH-O-$R^{3a}$, cyclopentyl, cyclohexyl, or 8- to 10-membered heterocycle fused ring containing 1 to 3 heteroatoms selected from N, S, or O, wherein the cycloalkyl and heterocycle fused ring are optionally further substituted with 1, 2, or 3 groups selected from $R^y$; furthermore, $R^3$ is selected from -NH-O-$R^{3a}$, cyclopentyl, cyclohexyl,

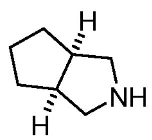 , 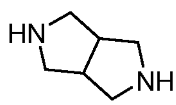 , 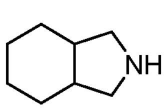 or 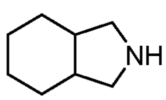 ,

wherein the above-mentioned ring is optionally further substituted with 1, 2, or 3 groups selected from $R^y$;
$R^{3a}$ is selected from $C_{1-6}$alkyl, benzyl, 3- to 6-membered monocyclic cycloalkyl, 4- to 6-membered monocyclic heterocycloalkyl, 5- to 12-membered spiro ring, 4- to 10-membered fused ring, or 5- to 9-membered bridged ring, wherein the cycloalkyl, heterocycloalkyl, spiro ring, bridged ring or fused ring is optionally further substituted with 1, 2, or 3 groups selected from $R^y$; furthermore, $R^{3a}$ is selected from $C_{1-6}$alkyl, benzyl, or 3- to 6-membered monocyclic cycloalkyl, wherein the alkyl, benzyl or cycloalkyl is optionally further substituted with 1, 2, or 3 groups selected from $R^y$; furthermore, $R^{3a}$ is selected from $C_{1-4}$alkyl, benzyl, cyclopropyl, cyclobutyl, cyclopentyl or cyclohexyl, wherein the alkyl, benzyl or cycloalkyl is optionally further substituted with 1, 2, or 3 groups selected from $R^y$; furthermore, $R^{3a}$ is selected from methyl, ethyl, isopropyl, propyl, benzyl, cyclopropyl, cyclobutyl, cyclopentyl or cyclohexyl, wherein the above-mentioned alkyl, benzyl or cycloalkyl is optionally further substituted with 1, 2, or 3 groups selected

from $R^y$;
$R^y$ is selected from deuterium, halogen, amino, hydroxyl, cyano, =O, $SF_5$ or azido; furthermore, $R^y$ is selected from deuterium, halogen, amino or hydroxyl;
provided that, when $R_3$ is substituted or unsubstituted 3- to 6-membered monocyclic cycloalkyl, 4- to 6-membered monocyclic heterocycloalkyl, 5- to 12-membered spiro ring, 4- to 10-membered fused ring, or 5- to 9-membered bridged ring, $R_4$, $R_5$, $R^{6a}$, and $R^{7a}$ are not H or deuterium at the same time,
and other groups are as described in 1.

15. The compound of formula VIII, and the stereoisomer, pharmaceutically acceptable salt, solvate, co-crystal or deuterated product thereof, wherein, $R_4$, $R_5$, $R^{6a}$, and $R^{7a}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino or alkyl is optionally further substituted with 1, 2, or 3 groups selected from deuterium, halogen, hydroxyl, cyano or $C_{1-6}$alkoxy, provided that $R_4$, $R_5$, $R^{6a}$, and $R^{7a}$ are not H or deuterium at the same time; furthermore, $R_4$, $R_5$, $R^{6a}$, and $R^{7a}$ are each independently selected from H, deuterium, hydroxyl, $C_{1-6}$alkyl, $C_{2-6}$alkenyl or $C_{2-6}$alkynyl, wherein the amino or alkyl is optionally further substituted with 1, 2, or 3 groups selected from halogen or hydroxyl, provided that, $R_4$, $R_5$, $R^{6a}$, and $R^{7a}$ are not H or deuterium at the same time; furthermore, $R_4$, $R_5$, $R^{6a}$, and $R^{7a}$ are each independently selected from H, $C_{1-6}$alkyl, or $C_{2-6}$alkynyl, wherein the alkyl is optionally further substituted with 1, 2, or 3 groups selected from halogen or hydroxyl, provided that, $R_4$, $R_5$, $R^{6a}$, and $R^{7a}$ are not H or deuterium at the same time;

$R^3$ is selected from 3- to 6-membered monocyclic cycloalkyl, 4- to 6-membered monocyclic heterocycloalkyl, 5- to 10-membered spiro ring, 4- to 10-membered fused ring or 5- to 9-membered bridged ring, wherein the cycloalkyl, heterocycle cycloalkyl, spiro ring, bridged ring or fused ring is optionally further substituted with 1, 2, or 3 groups selected from $R^y$; furthermore, $R^3$ is selected from 3- to 6-membered monocyclic cycloalkyl, or 7- to 10-membered heterocycle fused ring containing 1 to 3 heteroatoms selected from N, S, or O, wherein the cycloalkyl and heterocycle fused ring are optionally further substituted with 1, 2, or 3 groups selected from $R^y$; furthermore, $R^3$ is selected from -NH-O-$R_{3a}$, cyclopentyl, cyclohexyl, or 8- to 10-membered heterocycle fused ring containing 1 to 3 heteroatoms selected from N, S, or O, wherein the cycloalkyl and heterocycle fused ring are optionally further substituted with 1, 2, or 3 groups selected from $R^y$; furthermore, $R^3$ is selected from -NH-O-$R_{3a}$, cyclopentyl, cyclohexyl,

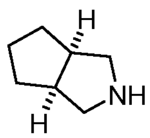,

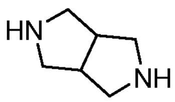, 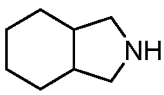 or 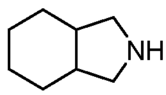,

wherein the above-mentioned ring is optionally further substituted with 1, 2, or 3 groups selected from $R^y$;
$R^y$ is selected from deuterium, halogen, amino, hydroxyl, cyano, =O, $SF_5$ or azido; furthermore, $R^y$ is selected from deuterium, halogen, amino or hydroxyl,
and other groups are as described in 14.

16. The compound, and the stereoisomer, pharmaceutically acceptable salt, solvate, co-crystal or deuterated product thereof of preceding 15, wherein, $R_4$, $R_5$, and $R^{7a}$ are selected from H;

$R^{6a}$ is selected from $C_{1-6}$alkyl or $C_{2-6}$alkynyl, wherein the alkyl is optionally further substituted with 1, 2, or 3 groups selected from deuterium, halogen, hydroxyl, cyano or $C_{1-2}$alkoxy; furthermore, $R^{6a}$ is $C_{1-4}$alkyl or $C_{2-4}$alkynyl, wherein the alkyl is optionally further substituted with 1, 2, or 3 groups selected from halogen or hydroxyl; furthermore, $R^{6a}$ is methyl, ethyl, propyl or ethyne, which is optionally further substituted with 1, 2, or 3 groups selected from halogen or hydroxyl;
$R^3$ is selected from 4- to 10-membered fused ring, wherein the fused ring is optionally further substituted with 1, 2, or 3 groups selected from $R^y$; furthermore, $R^3$ is selected from 7- to 10-membered heterocycle fused ring containing 1 to 3 heteroatoms selected from N, S, or O, wherein the heterocycle fused ring is optionally further substituted with 1, 2, or 3 groups selected from $R^y$; furthermore, $R^3$ is selected from or is 8- to 10-membered heterocycle fused ring containing 1 to 3 heteroatoms selected from N, S, or O, wherein the cycloalkyl and heterocycle fused ring are

optionally further substituted with 1, 2, or 3 groups selected from $R^y$; furthermore, $R^3$ is selected from

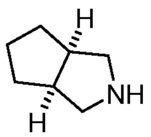, 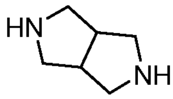, 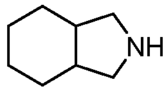

or

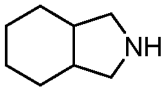,

wherein the above-mentioned ring is optionally further substituted with 1, 2, or 3 groups selected from $R^y$; furthermore, $R^3$ is selected from

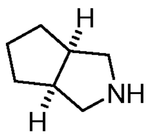,

which is optionally further substituted with 1, 2, or 3 groups selected from $R^y$;
$R^y$ is selected from deuterium, halogen, amino, hydroxyl or =O; furthermore, $R^y$ is selected from deuterium, halogen or hydroxyl,
and other groups are as described in 15.

17. The compound, and the stereoisomer, pharmaceutically acceptable salt, solvate, co-crystal or deuterated product thereof of preceding 14, wherein the compound has a structure of formula (IX) as follows:

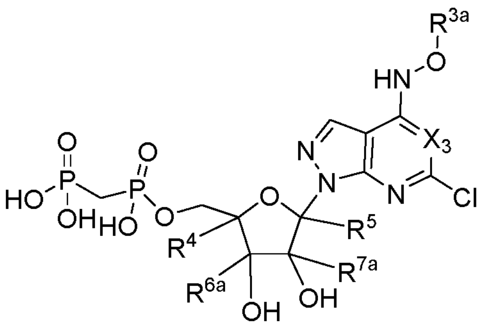

(IX)

wherein, $X_3$ is CH or N;
$R_4$, $R_5$, $R^{6a}$, and $R^{7a}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1, 2, or 3 groups selected from deuterium, halogen, hydroxyl or cyano; furthermore, $R_4$, $R_5$, $R^{6a}$, and $R^{7a}$ are each independently selected from H, deuterium, hydroxyl, $C_{1-6}$alkyl, $C_{2-6}$alkenyl or $C_{2-6}$alkynyl, wherein the alkyl, alkenyl or alkynyl is optionally further substituted with 1, 2, or 3 groups selected from deuterium, halogen, hydroxyl, or cyano; furthermore, $R_4$, $R_5$, $R^{6a}$, and $R^{7a}$ are each independently selected from H;
$R^{3a}$ is selected from $C_{1-6}$alkyl, benzyl, 3- to 6-membered monocyclic cycloalkyl, 4- to 6-membered monocyclic heterocycloalkyl, 5- to 12-membered spiro ring, 4- to 10-membered fused ring or 5- to 9-membered bridged ring, wherein the cycloalkyl, heterocycle cycloalkyl, spiro ring, bridged ring or fused ring is optionally further substituted with 1, 2, or 3 groups selected from $R^y$; furthermore, $R^{3a}$ is selected from $C_{1-4}$alkyl, benzyl, 3- to 6-membered monocyclic cycloalkyl or 4- to 6-membered monocyclic heterocycloalkyl, and is optionally further substituted with 1, 2, or 3 groups selected from $R^y$; furthermore, $R^{3a}$ is selected from cyclopropyl, cyclopentyl, cyclohexyl, benzyl, methyl, ethyl, propyl, isopropyl, azacyclobutyl, tetrahydropyrrolyl, piperidyl or tetrahydrofuryl, and is optionally further

substituted with 1, 2, or 3 groups selected from $R^y$;

$R^y$ is selected from deuterium, halogen, amino, hydroxyl, cyano, =O, $SF_5$ or azido; furthermore, $R^y$ is selected from deuterium, halogen or hydroxyl,

and other groups are as described in 14.

18. The compound, and the stereoisomer, pharmaceutically acceptable salt, solvate, co-crystal or deuterated product thereof of preceding 17,

wherein, $R_4$, $R_5$, $R^{6a}$ or $R^{7a}$ is selected from H;

$R^{3a}$ is selected from $C_{1-6}$alkyl, benzyl or 3- to 6-membered monocyclic cycloalkyl, wherein the cycloalkyl is optionally further substituted with 1, 2, or 3 groups selected from $R^y$; furthermore, $R^{3a}$ is selected from $C_{1-4}$alkyl, benzyl, cyclopropyl, cyclobutyl, cyclopentyl or cyclohexyl, and is optionally further substituted with 1, 2, or 3 groups selected from $R^y$; furthermore, $R^{3a}$ is selected from methyl, ethyl, propyl, isopropyl, benzyl, cyclopropyl, cyclobutyl, cyclopentyl or cyclohexyl, and is optionally further substituted with 1, 2, or 3 groups selected from $R^y$;

$R^y$ is selected from deuterium, halogen, amino, hydroxyl or =O; furthermore, $R^y$ is selected from deuterium, halogen or hydroxyl,

and other groups are as described in 17.

19. The compound of formula I, and the stereoisomer, pharmaceutically acceptable salt, solvate, co-crystal or deuterated product thereof, wherein the compound has a structure of formula (X) as follows:

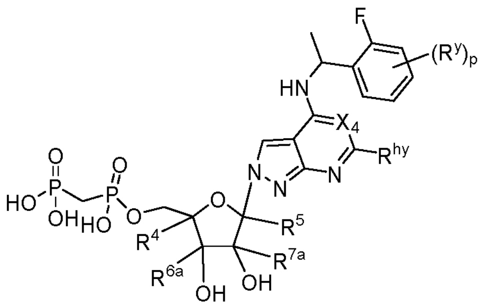

(X)

$X_4$ is CH or N;

$R_4$, $R_5$, $R^{6a}$, and $R^{7a}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1, 2, or 3 groups selected from deuterium, halogen, hydroxyl or cyano; furthermore, $R_4$, $R_5$, $R^{6a}$, and $R^{7a}$ are each independently selected from H, deuterium, hydroxyl, $C_{1-4}$alkyl, $C_{2-4}$alkenyl or $C_{2-4}$alkynyl, wherein the alkyl is optionally further substituted with 1, 2, or 3 groups selected from deuterium, halogen or hydroxyl; furthermore, $R_4$, $R_5$, and $R^{7a}$ are each independently selected from H, and $R^{6a}$ is selected from $C_{1-4}$alkyl, $C_{2-4}$alkenyl or $C_{2-4}$alkynyl, wherein the alkyl is optionally further substituted with 1, 2, or 3 groups selected from halogen or hydroxyl;

$R^y$ is selected from deuterium, halogen, amino, hydroxyl, cyano, =O, $SF_5$, azido, $-P(=O)(C_{1-3}alkyl)_2$, $-P(=S)(C_{1-3}alkyl)_2$,

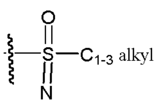,

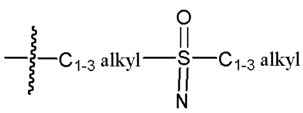,

$-Si(C_{1-3}alkyl)_3$, $-N=S(=O)(C_{1-3}alkyl)_2$, $-C_{1-3}$alkyl-S(=O)(=N), $-C_{1-3}$alkyl, $C_{1-3}$alkoxy, $C_{2-4}$alkenyl or $C_{2-4}$alkynyl, wherein the alkyl, alkoxy, alkenyl or alkynyl is optionally further substituted with 1 or more groups selected from deuterium, halogen, amino, hydroxyl, cyano, deuterated $C_{1-3}$alkyl, halo $C_{1-3}$alkyl, hydroxyl $C_{1-3}$alkyl, $C_{1-3}$alkoxy, deuterated $C_{1-3}$alkoxy, halo $C_{1-3}$alkoxy or hydroxyl $C_{1-3}$alkoxy; furthermore, $R^y$ is selected from deuterium, halogen, amino, hydroxyl, cyano, =O or $SF_5$;

p is selected from 0, 1, or 2; furthermore, p is selected from 0 or 1;

$R^{hy}$ is selected from deuterium, halogen, amino, hydroxyl, cyano, =O, $SF_5$, azido, $C_{1-6}$alkyl, $C_{1-6}$alkoxy, $C_{2-6}$alkenyl or $C_{2-6}$alkynyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, $-O-C_{1-6}$alkyl-$C_{6-10}$aryl or $-O-C_{1-6}$alkyl-(5-

to 10-membered heteroaryl); furthermore, $R^{hy}$ is selected from deuterium, halogen, hydroxyl, $C_{1-6}$alkyl, $C_{1-6}$alkoxy, $C_{2-6}$alkenyl or $C_{2-6}$alkynyl, which is optionally further substituted with 1 or more groups selected from deuterium, halogen, amino, hydroxyl or cyano,
and other groups are as described in 1.

20. The compound, and the stereoisomer, pharmaceutically acceptable salt, solvate, co-crystal or deuterated product thereof of preceding 19,

wherein, $R_4$, $R_5$, and $R^{7a}$ are selected from H;
$R^{6a}$ is selected from $C_{1-6}$alkyl, wherein the alkyl is optionally further substituted with 1, 2, or 3 groups selected from deuterium, halogen, hydroxyl, cyano or $C_{1-2}$alkoxy; furthermore, $R^{6a}$ is selected from $C_{1-4}$alkyl, and is optionally further substituted with 1, 2, or 3 groups selected from deuterium, halogen or hydroxyl; furthermore, $R^{6a}$ is selected from methyl, ethyl, propyl or isopropyl, and is optionally further substituted with 1, 2, or 3 groups selected from halogen or hydroxyl;
p is selected from 0;
$R^{hy}$ is selected from F or Cl; furthermore, $R^{hy}$ is selected from Cl.

The compound, and the stereoisomer, pharmaceutically acceptable salt, solvate, co-crystal or deuterated product thereof of the present invention, wherein, $X_1$ is selected from O,
and other groups are as described in 19.

The compound, and the stereoisomer, pharmaceutically acceptable salt, solvate, co-crystal or deuterated product thereof of the present invention, wherein, $X_2$ is selected from $CR^{x2a}R^{x2b}$ or O; further, $X_2$ is selected from $CH_2$ or O; further, $X_2$ is selected from O.

The compound, and the stereoisomer, pharmaceutically acceptable salt, solvate, co-crystal or deuterated product thereof of the present invention, wherein, Y is selected from OH, $-CH_2-P(=O)(OH)OH$, $-CH_2-P(=O)(OH)ONH_2$, $-CH_2-P(=O)(OH)NH(OH)$, $-CH_2-P(=O)(OH)SH$, $-CH_2-R^{y4}$, $-CH_2-P(=S)(OH)OH$, $-CH_2-P(=S)(OH)NH(OH)$, or $-CH_2-P(=S)(OH)SH$; further, Y is selected from OH, $-CH_2-P(=O)(OH)OH$, $-CH_2-P(=O)(OH)NH(OH)$, $-CH_2-P(=O)(OH)ONH_2$, $-CH_2-P(=O)(OH)SH$, $-CH_2$-5-membered heterocycloalkyl or $-CH_2-P(=S)(OH)SH$; further, Y is selected from OH or $-CH_2-P(=O)(OH)OH$; further, Y is selected from $-CH_2-P(=O)(OH)OH$; further, Y is selected from OH.

The compound, and the stereoisomer, pharmaceutically acceptable salt, solvate, co-crystal or deuterated product thereof of the present invention, wherein, $R^1$ and $R^2$ are each independently selected from H, deuterium, amino, $C_{1-4}$alkyl, $C_{2-4}$alkenyl, $C_{2-4}$alkynyl, $C_{3-6}$cycloalkyl, 3- to 6-membered heterocycloalkyl, phenyl, $C_{8-10}$bicyclic aryl, 5- to 6-membered heteroaryl, 8- to 10-membered bicyclic heteroaryl, $-CH_2-O-C(O)-OR^{2c}$, $-C_{0-4}alkyl-S(O)_2R^{2c}$, $-C_{0-4}alkyl-C(O)R^{2c}$, $-C_{0-4}alkyl-C(O)OR^{2c}$ or $-C_{0-4}alkyl-C(O)NR^{2c}$, and the alkyl, cycloalkyl, heterocycloalkyl, aryl or heteroaryl is optionally further substituted with 1 or more groups selected from $R^x$; further, $R^1$ and $R^2$ are each independently selected from H, deuterium, or amino.

The compound, and the stereoisomer, pharmaceutically acceptable salt, solvate, co-crystal or deuterated product thereof of the present invention, wherein, $R^{y1}$, $R^{y2}$, $R^{y3}$, $R^{x2a}$, $R^{x2b}$, and $R^{x2c}$ are each independently selected from H, deuterium, hydroxyl, cyano, $C_{1-4}$alkyl, deuterated $C_{1-4}$alkyl or halo $C_{1-4}$alkyl; further, $R^{y1}$, $R^{y2}$, $R^{y3}$, $R^{x2a}$, $R^{x2b}$, and $R^{x2c}$ are each independently selected from H, deuterium or hydroxyl; further, $R^{y1}$, $R^{y2}$, $R^{y3}$, $R^{x2a}$, $R^{x2b}$, and $R^{x2c}$ are each independently selected from H or deuterium.

The compound, and the stereoisomer, pharmaceutically acceptable salt, solvate, co-crystal or deuterated product thereof of the present invention, wherein, $R^{y4}$ is selected from 4- to 6-membered heterocycloalkyl, 8- to 10-membered bicyclic heterocycloalkyl, 5- to 6-membered heteroaryl, 8- to 10-membered bicyclic heteroaryl, $C_{3-6}$cycloalkyl, $C_{8-10}$bicyclic cycloalkyl, phenyl or $C_{8-10}$bicyclic aryl, and the heterocycloalkyl, heteroaryl, cycloalkyl, or aryl is optionally further substituted with 1 or more groups selected from $R^x$; further, $R^{y4}$ is selected from 4-to 6-membered heterocycloalkyl or 8- to 10-membered bicyclic heterocycloalkyl, wherein the heterocycloalkyl is optionally further substituted with 1-3 groups selected from $R^x$; further, $R^{y4}$ is selected from 4-membered heterocycloalkyl, 5-membered heterocycloalkyl or 6-membered heterocycloalkyl, wherein the heterocycloalkyl is optionally further substituted with 1-3 groups selected from deuterium, halogen, amino, hydroxyl, cyano, =O, $SF_5$, azido, $C_{1-3}$alkyl, $C_{1-3}$alkoxy, $C_{2-4}$alkenyl or $C_{2-4}$alkynyl; further, $R^{y4}$ is selected from 4-membered heterocycloalkyl, 5-membered heterocycloalkyl or 6-membered heterocycloalkyl, wherein the heterocycloalkyl is optionally further substituted with 1-3 groups selected from deuterium, halogen, amino, hydroxyl, cyano, =O, $SF_5$, azido, $C_{1-3}$alkyl, $C_{1-3}$alkoxy, $C_{2-4}$alkenyl or $C_{2-4}$alkynyl; further, $R^{y4}$ is selected from

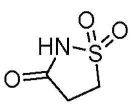
.

The compound, and the stereoisomer, pharmaceutically acceptable salt, solvate, co-crystal or deuterated product thereof of the present invention, wherein, $R^{2a}$ and $R^{2b}$ are each independently selected from H, deuterium, hydroxyl, cyano, halogen, $C_{1-4}$alkyl, deuterated $C_{1-4}$alkyl, halo $C_{1-4}$alkyl, $C_{3-6}$cycloalkyl, 3- to 6-membered heterocycloalkyl, phenyl or 5- to 6-membered heteroaryl; further, $R^{2a}$ and $R^{2b}$ are each independently selected from H, deuterium, hydroxyl, cyano, halogen, $C_{1-4}$alkyl, deuterated $C_{1-4}$alkyl or halo $C_{1-4}$alkyl; further, $R^{2a}$ and $R^{2b}$ are each independently selected from H, deuterium or $C_{1-3}$alkyl; further, $R^{2a}$ and $R^{2b}$ are each independently selected from H or deuterium.

The compound, and the stereoisomer, pharmaceutically acceptable salt, solvate, co-crystal or deuterated product thereof of the present invention, wherein, each $R^{2c}$ is independently selected from H, deuterium, $C_{1-4}$alkyl, deuterated $C_{1-4}$alkyl, halo $C_{1-4}$alkyl, $C_{3-6}$cycloalkyl, 3- to 6-membered heterocycloalkyl, phenyl or 5- to 6-membered heteroaryl, and the alkyl, cycloalkyl, heterocycloalkyl, phenyl or heteroaryl is optionally further substituted with 1 or more groups selected from $R^x$; further, each $R^{2c}$ is independently selected from H, deuterium, or $C_{1-4}$alkyl.

The compound, and the stereoisomer, pharmaceutically acceptable salt, solvate, co-crystal or deuterated product thereof of the present invention, wherein, $L_1$ is selected from a bond, S, O, NH, N($C_{1-2}$alkyl) or $C_{1-2}$alkyl, and the alkyl is optionally further substituted with 1 or more groups selected from $R^x$; further, $L_1$ is selected from a bond, NH, N($CH_3$) or methyl; further, $L_1$ is selected from a bond, NH or methyl; further, $L_1$ is selected from a bond; further, $L_1$ is selected from NH; further, $L_1$ is selected from methyl.

The compound, and the stereoisomer, pharmaceutically acceptable salt, solvate, co-crystal or deuterated product thereof of the present invention, wherein, $L_2$ is selected from -($CR^{L2a}R^{L2b}$)n-, *-Z-phenyl, *-Z-$C_{8-10}$bicyclic aryl, *-Z-(5- to 6-membered heteroaryl), *-Z-(8- to 10-membered bicyclic heteroaryl), *-phenyl-Z-, *-$C_{8-10}$bicyclic aryl-Z-, *-(5- to 6-membered heteroaryl)-Z-, *-(8- to 10-membered bicyclic heteroaryl)-Z-, $C_{2-4}$alkenyl or $C_{2-4}$alkynyl, and the carbon atom in the - ($CR^{L2a}R^{L2b}$)n- is optionally replaced with 1 or more groups selected from N, O, S, S(O) or $S(O)_2$, the $L_2$ is optionally further substituted with 1 or more groups selected from $R^x$, and * denotes the site where the $L_2$ is connected to P atom; further, $L_2$ is selected from *-O-($CR^{L2a}R^{L2b}$)n-, *-($CR^{L2a}R^{L2b}$)n-O-, -($CR^{L2a}R^{L2b}$)n-, -O-, *-O-($CR^{L2a}R^{L2b}$)n-O-($CR^{L2a}R^{L2b}$)m-, *-($CR^{L2a}R^{L2b}$)n-O-($CR^{L2a}R^{L2b}$)m-, *-O-($CR^{L2a}R^{L2b}$)n-O-, *-O-N($R^{L2c}$)-, *-C(O)$C_{1-6}$alkyl, *-Z-phenyl, *-Z-$C_{8-10}$bicyclic aryl, *-Z-(5- to 6-membered heteroaryl), *-Z-(8- to 10-membered bicyclic heteroaryl), *-phenyl-Z-, *-$C_{8-10}$bicyclic aryl-Z-, *-(5- to 6-membered heteroaryl)-Z-, *-(8- to 10-membered bicyclic heteroaryl)-Z-, $C_{2-4}$alkenyl or $C_{2-4}$alkynyl, and the alkyl, aryl, heteroaryl, alkenyl or alkynyl is optionally further substituted with 1 or more groups selected from $R^x$; further, *-O-($CR^{L2a}R^{L2b}$)n-, *-($CR^{L2a}R^{L2b}$)n-O-, -O-, *-O-($CR^{L2a}R^{L2b}$)n-O-, *-O-N($R^{L2c}$)-, *-Z-phenyl, *-($CR^{L2a}R^{L2b}$)n-O-($CR^{L2a}R^{L2b}$)m-, $C_{2-4}$alkenyl or $C_{2-4}$alkynyl, wherein the alkyl, aryl, heteroaryl, alkenyl or alkynyl is optionally further substituted with 1 or more groups selected from $R^x$.

The compound, and the stereoisomer, pharmaceutically acceptable salt, solvate, co-crystal or deuterated product thereof of the present invention, wherein, $R^{L2a}$ and $R^{L2b}$ on the same carbon atom together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl or 6-membered heterocycloalkyl; further, $R^{L2a}$ and $R^{L2b}$ on the same carbon atom together with the carbon atom to which they are attached form 3-membered cycloalkyl, 4-membered cycloalkyl, or 4-membered heterocycloalkyl.

The compound, and the stereoisomer, pharmaceutically acceptable salt, solvate, co-crystal or deuterated product thereof of the present invention, wherein, each n is independently selected from 1, 2, 3, 4, 5, 6, 7, 8, 9 or 10; further, each n is independently selected from 1, 2, or 3; further, each n is independently selected from 1 or 2.

The compound, and the stereoisomer, pharmaceutically acceptable salt, solvate, co-crystal or deuterated product thereof of the present invention, wherein, each n' is independently selected from 1, 2, 3, 4, 5, 6, 7, or 8; further, each n' is independently selected from 1, 2, 3, 4, or 5; further, each n is independently selected from 1, 2, or 3; further, each n is independently selected from 1 or 2.

The compound, and the stereoisomer, pharmaceutically acceptable salt, solvate, co-crystal or deuterated product thereof of the present invention, wherein, each n' is independently selected from 1, 2, 3, 4, 5, 6, 7, or 8; further, each n' is independently selected from 1, 2, 3, 4, or 5; further, each n is independently selected from 1, 2, or 3; further, each n is independently selected from 1 or 2.

The compound, and the stereoisomer, pharmaceutically acceptable salt, solvate, co-crystal or deuterated product thereof of the present invention, wherein, each m is independently selected from 1, 2, 3, 4, 5, 6, 7, 8, 9 or 10; further, each m is independently selected from 1, 2, or 3; further, each m is independently selected from 1 or 2.

The compound, and the stereoisomer, pharmaceutically acceptable salt, solvate, co-crystal or deuterated product thereof of the present invention, wherein, each Z is independently selected from a bond, -O-, or $C_{1-2}$alkyl, and the alkyl is optionally further substituted with 1 or more groups selected from $R^x$; further, each Z is independently selected from a bond, -O-, or methyl.

The compound, and the stereoisomer, pharmaceutically acceptable salt, solvate, co-crystal or deuterated product thereof of the present invention, wherein, $R_4$, $R_5$, $R^{6a}$, $R^{6b}$, $R^{7a}$, and $R^{7b}$ are each independently selected from H, deuterium, hydroxyl, cyano, halogen, azido, amino, $C_{1-2}$alkyl, $C_{2-4}$alkenyl or $C_{2-4}$alkynyl, and the amino or alkyl is optionally further substituted with 1 or more groups selected from deuterium, halogen, hydroxyl or cyano; further, $R_4$, $R_5$,

$R^{6a}$, $R^{6b}$, $R^{7a}$, and $R^{7b}$ are each independently selected from H, deuterium, hydroxyl, cyano, halogen, azido, $C_{2-4}$alkenyl or $C_{2-4}$alkynyl, wherein the alkyl is optionally further substituted with 1-3 groups selected from deuterium, halogen, hydroxyl or cyano.

The compound, and the stereoisomer, pharmaceutically acceptable salt, solvate, co-crystal or deuterated product thereof of the present invention, wherein, $R^{6a}$ and $R^{6b}$ together form $C_{2-4}$alkenyl, $C_{3-6}$cycloalkyl or 4- to 6-membered heterocycloalkyl, and the alkenyl is optionally further substituted with 1 or more groups selected from deuterium, halogen, hydroxyl or cyano; further, $R^{6a}$ and $R^{6b}$ together form ethenyl or ethynyl, wherein the ethenyl or ethynyl is optionally further substituted with 1-3 groups selected from deuterium, halogen, hydroxyl or cyano.

The compound, and the stereoisomer, pharmaceutically acceptable salt, solvate, co-crystal or deuterated product thereof of the present invention, wherein, $R^{7a}$ and $R^{7b}$ together form $C_{2-4}$alkenyl, $C_{3-6}$cycloalkyl or 4- to 6-membered heterocycloalkyl, and the alkenyl is optionally further substituted with 1 or more groups selected from deuterium, halogen, hydroxyl or cyano; further, $R^{7a}$ and $R^{7b}$ together form ethenyl or ethynyl, wherein the ethenyl or ethynyl is optionally further substituted with 1-3 groups selected from deuterium, halogen, hydroxyl or cyano.

The compound, and the stereoisomer, pharmaceutically acceptable salt, solvate, co-crystal or deuterated product thereof of the present invention, wherein, $R^4$, $R^5$ and $X_2$ together form 5-membered cycloalkyl, 6-membered cycloalkyl, 7-membered cycloalkyl, 5-membered heterocycloalkyl, 6-membered heterocycloalkyl, or 7-membered heterocycloalkyl.

The compound, and the stereoisomer, pharmaceutically acceptable salt, solvate, co-crystal or deuterated product thereof of the present invention, wherein, $R^4$, $R^{6a}$ and $X_2$ together form 5-membered cycloalkyl, 6-membered cycloalkyl, 7-membered cycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^{L2a}$, $R^5$ and $X_2$ together form 5-membered cycloalkyl, 6-membered cycloalkyl, 7-membered cycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^4$, $R^{7a}$ and $X_2$ together form 5-membered cycloalkyl, 6-membered cycloalkyl, 7-membered cycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^4$ and $R^{6a}$ together form 3-membered cycloalkyl, 4-membered cycloalkyl, 5-membered cycloalkyl, 6-membered cycloalkyl, 7-membered cycloalkyl, 4-membered heterocycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^{6b}$ and $R^{7b}$ together form 3-membered cycloalkyl, 4-membered cycloalkyl, 5-membered cycloalkyl, 6-membered cycloalkyl, 7-membered cycloalkyl, 4-membered heterocycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^4$ and $R^{L2a}$ together form 3-membered cycloalkyl, 4-membered cycloalkyl, 5-membered cycloalkyl, 6-membered cycloalkyl, 7-membered cycloalkyl, 4-membered heterocycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^4$ and $X_2$ together form 3-membered cycloalkyl, 4-membered cycloalkyl, 5-membered cycloalkyl, 6-membered cycloalkyl, 7-membered cycloalkyl, 4-membered heterocycloalkyl, 5-membered heterocycloalkyl, 6-membered heterocycloalkyl or 7-membered heterocycloalkyl.

The compound, and the stereoisomer, pharmaceutically acceptable salt, solvate, co-crystal or deuterated product thereof of the present invention, wherein, $R^{x2a}$ and $R^4$ together form one chemical bond, which together with a bond on the ring forms a double bond.

The compound, and the stereoisomer, pharmaceutically acceptable salt, solvate, co-crystal or deuterated product thereof of the present invention, wherein, $R^{x2a}$ and $R^5$ together form one chemical bond, which together with a bond on the ring forms a double bond.

The compound, and the stereoisomer, pharmaceutically acceptable salt, solvate, co-crystal or deuterated product thereof of the present invention, wherein, $R^{6a}$ and $R^4$ together form one chemical bond, which together with a bond on the ring forms a double bond.

The compound, and the stereoisomer, pharmaceutically acceptable salt, solvate, co-crystal or deuterated product thereof of the present invention, wherein, $R^{6b}$ and $R^{7b}$ together form one chemical bond, which together with a bond on the ring forms a double bond.

The compound, and the stereoisomer, pharmaceutically acceptable salt, solvate, co-crystal or deuterated product thereof of the present invention, wherein, $R^{7a}$ and $R^5$ together form one chemical bond, which together with a bond

on the ring forms a double bond.

$R^{x2a}$ and $R^4$, or $R^{x2a}$ and $R^5$, or $R^{6a}$ and $R^4$, or $R^{6b}$ and $R^{7b}$, or $R^{7a}$and $R^5$ of the present invention together form one chemical bond, provided that at most one group forms one chemical bond.

The compound, and the stereoisomer, pharmaceutically acceptable salt, solvate, co-crystal or deuterated product thereof of the present invention, wherein, $R^3$ is selected from $L_3$-$R^{3a}$ or

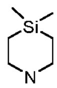;

further, $R^3$ is selected from $L_3$-$R^{3a}$.

The compound, and the stereoisomer, pharmaceutically acceptable salt, solvate, co-crystal or deuterated product thereof of the present invention, wherein, $L_3$ is selected from **-N($R^{L3}$)$C_{1-4}$alkyl-, -N($R^{L3}$)-, **-N($R^{L3}$)S(O)-, **-N($R^{L3}$)$S(O)_2$-, **-N($R^{L3}$)S(O)-$C_{1-4}$alkyl-, **-N($R^{L3}$)$S(O)_2$-$C_{1-4}$alkyl- or **-N($R^{L3}$)-N=CH-, and ** denotes the site where $L_3$ is connected to Hy; further, $L_3$ is selected from **-N($R^{L3}$)CH($CH_3$)-, -N($R^{L3}$)-, **-N($R^{L3}$)$S(O)_2$- or **-N($R^{L3}$)-N=CH-, and ** denotes the site where $L_3$ is connected to Hy; further, $L_3$ is selected from **-NHCH($CH_3$)-, -NH-, **-NHS$(O)_2$- or **-NH-N=CH-, and ** denotes the site where $L_3$ is connected to Hy; further, $L_3$ is selected from **-NHCH($CH_3$)- or -NH-; further, $L_3$ is selected from **-NHS$(O)_2$- or **-NH-N=CH-.

The compound, and the stereoisomer, pharmaceutically acceptable salt, solvate, co-crystal or deuterated product thereof of the present invention, wherein, $R^{3a}$ is selected from 5- to 6-membered cycloalkyl, 5- to 6-membered heterocycloalkyl, phenyl, 5- to 6-membered heteroaryl, 8- to 10-membered bicyclic heterocyclyl, 8- to 10-membered bicyclic cycloalkyl, 8- to 10-membered bicyclic heteroaryl or 8- to 10-membered bicyclic aryl, and the carbocyclyl or heterocyclyl is optionally further substituted with 1 or more groups selected from $R^y$; further, $R^{3a}$ is selected from 5- to 6-membered cycloalkyl, 5- to 6-membered heterocycloalkyl, phenyl or 5- to 6-membered heteroaryl, wherein the carbocyclyl or heterocyclyl is optionally further substituted with 1 or more groups selected from $R^y$.

The compound, and the stereoisomer, pharmaceutically acceptable salt, solvate, co-crystal or deuterated product thereof of the present invention, wherein, each $R^{L3}$ is independently selected from H, deuterium, $C_{1-4}$alkyl, halo $C_{1-4}$alkyl or deuterated $C_{1-4}$alkyl; further, each $R^{L3}$ is independently selected from H, deuterium, $C_{1-2}$alkyl, halo $C_{1-2}$alkyl or deuterated $C_{1-2}$alkyl; further, each $R^{L3}$ is independently selected from H, deuterium, or methyl.

The compound, and the stereoisomer, pharmaceutically acceptable salt, solvate, co-crystal or deuterated product thereof of the present invention, wherein, $R^{L3}$ and $R^{hy}$ together with the atom to which they are attached form 5-membered heterocycloalkyl, 6-membered heterocycloalkyl, 7-membered heterocycloalkyl, or 8-membered heterocycloalkyl; further, $R^{L3}$ and $R^{hy}$ together with the atom to which they are attached form 5-membered heterocycloalkyl, 6-membered heterocycloalkyl, or 7-membered heterocycloalkyl; further, $R^{L3}$ ad $R^{hy}$ together with the atom to which they are attached form 5-membered heterocycloalkyl or 6-membered heterocycloalkyl; further, $R^{L3}$ and $R^{hy}$ together with the atom to which they are attached form 5-membered heterocycloalkyl.

The compound, and the stereoisomer, pharmaceutically acceptable salt, solvate, co-crystal or deuterated product thereof of the present invention, wherein, each $R^x$ is independently selected from deuterium, F, Cl, amino, hydroxyl, cyano, =O, $C_{1-3}$alkyl, $C_{1-3}$alkoxy, $C_{2-4}$alkenyl or $C_{2-4}$alkynyl, and the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, -O-$C_{1-6}$alkyl-$C_{6-10}$aryl or -O-$C_{1-6}$alkyl-(5- to 10-membered heteroaryl); further, each $R^x$ is independently selected from deuterium, F, Cl, amino, hydroxyl, cyano, =O, $C_{1-3}$alkyl or $C_{1-3}$alkoxy, wherein the alkyl or alkoxy is optionally further substituted with 1 or more groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, -O-$C_{1-2}$alkyl-$C_{6-10}$aryl or -O-$C_{1-2}$alkyl-(5- to 10-membered heteroaryl); further, each $R^x$ is independently selected from deuterium, F, Cl, amino, hydroxyl, cyano, =O, $C_{1-3}$alkyl or $C_{1-3}$alkoxy, wherein the alkyl or alkoxy is optionally further substituted with 1, 2, or 3 groups selected from deuterium, halogen, amino, hydroxyl, cyano or $C_{1-2}$alkoxy, deuterated $C_{1-2}$alkoxy, halo $C_{1-2}$alkoxy, hydroxyl $C_{1-2}$alkoxy, -O-$C_{1-2}$alkyl-phenyl or -O-$C_{1-2}$alkyl-(5- to 6-membered heteroaryl).

The compound, and the stereoisomer, pharmaceutically acceptable salt, solvate, co-crystal or deuterated product thereof of the present invention, wherein, each $R^{hy}$ is independently selected from deuterium, halogen, amino, hydroxyl, cyano, =O, $SF_5$, azido, $C_{1-4}$alkyl, $C_{1-4}$alkoxy, $C_{2-4}$alkenyl or $C_{2-4}$alkynyl, and the alkyl, alkoxy, alkenyl or alkynyl is optionally further substituted with 1 or more groups selected from deuterium, halogen, amino, hydroxyl, cyano, deuterated $C_{1-2}$alkyl, halo $C_{1-2}$alkyl, hydroxyl $C_{1-2}$alkyl, $C_{1-2}$alkoxy, deuterated $C_{1-2}$alkoxy, halo $C_{1-2}$alkoxy, hydroxyl $C_{1-2}$alkoxy, -O-$C_{1-2}$alkyl-$C_{6-10}$aryl or -O-$C_{1-2}$alkyl-(5- to 10-membered heteroaryl); further, each $R^{hy}$ is independently selected from deuterium, F, Cl, methyl or ethyl.

The compound, and the stereoisomer, pharmaceutically acceptable salt, solvate, co-crystal or deuterated product thereof of the present invention, wherein, $R^y$ is selected from deuterium, halogen, amino, hydroxyl, cyano, =O, $SF_5$, azido, - $P(=O)(C_{1-2}alkyl)_2$, $-P(=S)(C_{1-2}alkyl)_2$,

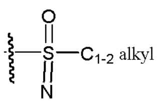 , 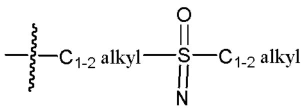 ,

$-Si(C_{1-2}alkyl)_3$, $-N=S(=O)(C_{1-2}alkyl)_2$, $-C_{1-2}alkyl-S(=O)(=N)$, $-C_{1-4}alkyl$, $C_{1-4}alkoxy$, $C_{2-4}alkenyl$ or $C_{2-4}alkynyl$, and the alkyl, alkoxy, amino, alkenyl or alkynyl is optionally further substituted with 1 or more groups selected from deuterium, halogen, amino, hydroxyl, cyano, deuterated $C_{1-2}alkyl$, halo $C_{1-2}alkyl$, hydroxyl $C_{1-2}alkyl$, $C_{1-2}alkoxy$, deuterated $C_{1-2}alkoxy$, halo $C_{1-2}alkoxy$ or hydroxyl $C_{1-2}alkoxy$; further, $R^y$ is selected from deuterium, halogen, amino, hydroxyl, cyano, =O, $SF_5$, azido, $-P(=O)(C_{1-2}alkyl)_2$, $-P(=S)(C_{1-2}alkyl)_2$,

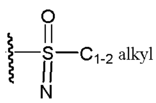 , 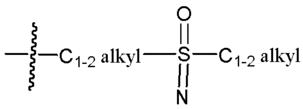 ,

$-Si(C_{1-2}alkyl)_3$, $-N=S(=O)(C_{1-2}alkyl)_2$ or $-C_{1-2}alkyl-S(=O)(=N)$.

The compound, and the stereoisomer, pharmaceutically acceptable salt, solvate, co-crystal or deuterated product thereof of the present invention, wherein, when $R^{3a}$ is substituted with one substituent, $R^y$ is selected from $-P(=O)(C_{1-2}alkyl)_2$, $-P(=S)(C_{1-2}alkyl)_2$,

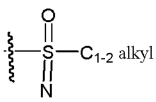 , 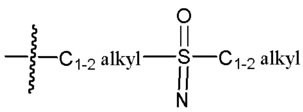 ,

$-Si(C_{1-2}alkyl)_3$, - $N=S(=O)(C_{1-2}alkyl)_2$ or $-C_{1-2}alkyl-S(=O)(=N)$.

The compound, and the stereoisomer, pharmaceutically acceptable salt, solvate, co-crystal or deuterated product thereof of the present invention, wherein, when $R^{3a}$ is sustituted with 2 substituents, one $R^y$ is selected from halogen, and the other $R^y$ is selected from azido, $-P(=O)(C_{1-2}alkyl)_2$, $-P(=S)(C_{1-2}alkyl)_2$,

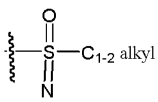

, 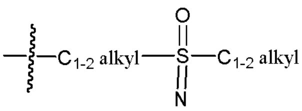 ,

$-Si(C_{1-2}alkyl)_3$, $-N=S(=O)(C_{1-2}alkyl)_2$ or $-C_{1-2}alkylS(=O)(=N)$.

"Substituted with 1 or more" substituents described herein includes all hydrogen atoms in the structure being substituted, further it may be substituted with 1-6 substituents, further it may be substituted with 1-5 substituents, further it may be substituted with 1-3 substituents, further substituted with 1-2 substituents, and further substituted with 1 substituent.

The compound, and the stereoisomer, pharmaceutically acceptable salt, solvate, co-crystal or deuterated product thereof of the present invention, wherein, p is selected from 0, 1 or 2; further, p is selected from 0 or 1; further, p is selected from 0; further, p is selected from 1.

The compound, and the stereoisomer, pharmaceutically acceptable salt, solvate, co-crystal or deuterated product thereof of the present invention, wherein the compound has a structure of formula (VIII) as follows:

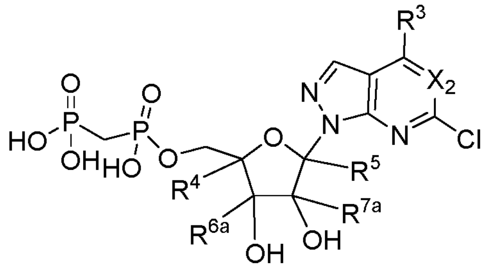

(VIII)

$X_2$ is selected from CH or N;

$R_4$, $R_5$, $R^{6a}$, and $R^{7a}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino or alkyl is optionally further substituted with 1, 2, or 3 groups selected from deuterium, halogen, hydroxyl, cyano or $C_{1-6}$alkoxy, provided that $R_4$, $R_5$, $R^{6a}$, and $R^{7a}$ are not H or deuterium at the same time;

$R^3$ is selected from 3- to 6-membered monocyclic cycloalkyl, 4- to 6-membered monocyclic heterocycloalkyl, 5- to 10-membered spiro ring, 4- to 10-membered fused ring or 5- to 9-membered bridged ring, wherein the cycloalkyl, heterocycle cycloalkyl, spiro ring, bridged ring or fused ring is optionally further substituted with 1, 2, or 3 groups selected from $R^y$;

$R^y$ is selected from deuterium, halogen, amino, hydroxyl, cyano, =O, $SF_5$ or azido.

The compound, and the stereoisomer, pharmaceutically acceptable salt, solvate, co-crystal or deuterated product thereof of the present invention, wherein the compound has a structure of formula (IX) as follows:

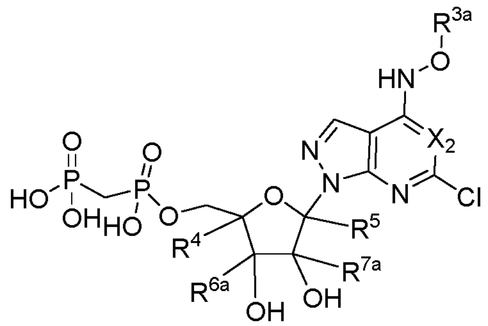

(IX)

$R_4$, $R_5$, $R^{6a}$, and $R^{7a}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1, 2, or 3 groups selected from deuterium, halogen, hydroxyl or cyano; further, $R_4$, $R_5$, $R^{6a}$, and $R^{7a}$ are each independently selected from H, deuterium, hydroxyl, cyano, halogen, azido, amino, $C_{1-4}$alkyl, $C_{2-4}$alkenyl or $C_{2-4}$alkynyl, wherein the amino or alkyl is optionally further substituted with 1, 2, or 3 groups selected from deuterium, halogen, hydroxyl or cyano; further, $R_4$ and $R_5$ are selected from H or deuterium, and $R^{6a}$ and $R^{7a}$ are each independently selected from H, deuterium, hydroxyl, cyano, halogen, azido, amino, $C_{1-4}$alkyl, $C_{2-4}$alkenyl or $C_{2-4}$alkynyl, wherein the amino or alkyl is optionally further substituted with 1, 2, or 3 groups selected from deuterium, halogen, hydroxyl or cyano;

$R^{3a}$ is selected from $C_{1-6}$alkyl, benzyl, 3- to 6-membered monocyclic cycloalkyl, 4- to 6-membered monocyclic heterocycloalkyl, 5- to 10-membered spiro ring, 4- to 10-membered fused ring or 5- to 9-membered bridged ring, wherein the cycloalkyl, heterocycle cycloalkyl, spiro ring, bridged ring or fused ring is optionally further substituted with 1, 2, or 3 groups selected from $R^y$; further, $R^{3a}$ is selected from $C_{1-4}$alkyl, benzyl, 3-membered monocyclic cycloalkyl, 4-membered monocyclic cycloalkyl, 5-membered monocyclic cycloalkyl, 6-membered monocyclic cycloalkyl, 4-membered monocyclic heterocycloalkyl, 5-membered monocyclic heterocycloalkyl, 6-membered monocyclic heterocycloalkyl, 5-membered spiro ring, 6-membered spiro ring, 7-membered spiro ring, 8-membered spiro ring, 9-membered spiro ring, 10-membered spiro ring, 11-membered spiro ring, 4-membered fused ring, 5-membered fused ring, 6-membered fused ring, 7-membered fused ring, 8-membered fused ring, 9-membered fused ring, 10-membered fused ring, 5-membered bridged ring, 6-membered bridged ring, 7-membered bridged ring, 8-membered bridged ring or 9-membered bridged ring, wherein the cycloalkyl, heterocycle cycloalkyl, spiro ring, bridged ring or fused ring is optionally further substituted with 1, 2, or 3 groups selected from $R^y$;

$R^y$ is selected from deuterium, halogen, amino, hydroxyl, cyano, =O, $SF_5$ or azido.

The compound, and the stereoisomer, pharmaceutically acceptable salt, solvate, co-crystal or deuterated product thereof of the present invention, wherein the compound has a structure of formula (X) as follows:

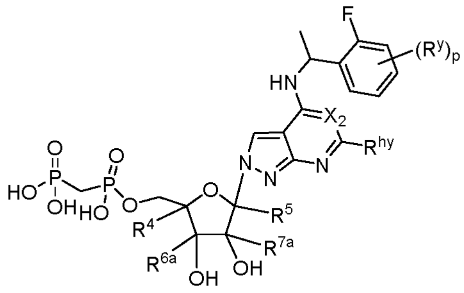

(X)

$R_4$, $R_5$, $R^{6a}$, and $R^{7a}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1, 2, or 3 groups selected from deuterium, halogen, hydroxyl or cyano; further, $R_4$, $R_5$, $R^{6a}$, and $R^{7a}$ are each independently selected from H, deuterium, hydroxyl, cyano, halogen, azido, amino, $C_{1-4}$alkyl, $C_{2-4}$alkenyl or $C_{2-4}$alkynyl, wherein the amino or alkyl is optionally further substituted with 1, 2, or 3 groups selected from deuterium, halogen, hydroxyl or cyano; further, $R_4$ and $R_5$ are selected from H or deuterium, and $R^{6a}$ and $R^{7a}$ are each independently selected from H, deuterium, hydroxyl, cyano, halogen, azido, amino, $C_{1-4}$alkyl, $C_{2-4}$alkenyl or $C_{2-4}$alkynyl, wherein the amino or alkyl is optionally further substituted with 1, 2, or 3 groups selected from deuterium, halogen, hydroxyl or cyano;

$R^y$ is selected from deuterium, halogen, amino, hydroxyl, cyano, =O, $SF_5$, azido, -P(=O)($C_{1-6}$alkyl)$_2$, -P(=S)($C_{1-6}$alkyl)$_2$,

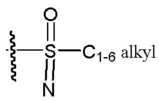 , 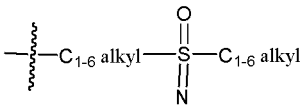 ,

-Si($C_{1-6}$alkyl)$_3$, -N=S(=O)($C_{1-6}$alkyl)$_2$, -$C_{1-6}$alkyl-S(=O)(=N), -$C_{1-6}$alkyl, $C_{1-6}$alkoxy, $C_{2-6}$alkenyl or $C_{2-6}$alkynyl, wherein the alkyl, alkoxy, alkenyl o alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy; further, $R^y$ is selected from deuterium, halogen, amino, hydroxyl, cyano, =O, $SF_5$, azido, -P(=O)($C_{1-3}$alkyl)$_2$, -P(=S)($C_{1-3}$alkyl)$_2$,

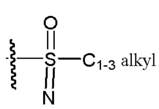 , 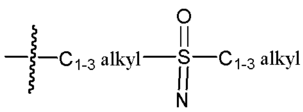 ,

-Si($C_{1-3}$alkyl)$_3$, -N=S(=O)($C_{1-3}$alkyl)$_2$, -$C_{1-3}$alkyl-S(=O)(=N), -$C_{1-3}$alkyl, $C_{1-3}$alkoxy, $C_{2-4}$alkenyl or $C_{2-4}$alkynyl, wherein the alkyl, alkoxy, alkenyl or alkynyl is optionally further substituted with 1 or more groups selected from deuterium, halogen, amino, hydroxyl, cyano, deuterated $C_{1-3}$alkyl, halo $C_{1-3}$alkyl, hydroxyl $C_{1-3}$alkyl, $C_{1-3}$alkoxy, deuterated $C_{1-3}$alkoxy, halo $C_{1-3}$alkoxy or hydroxyl $C_{1-3}$alkoxy; further, $R^y$ is selected from deuterium, halogen, amino, hydroxyl, cyano, =O, $SF_5$, azido, -$C_{1-3}$alkyl, $C_{1-3}$alkoxy, $C_{2-4}$alkenyl or $C_{2-4}$alkynyl;

p is selected from 0, 1, or 2; further, p is selected from 0 or 1; further, p is selected from 0;

$R^{hy}$ is selected from deuterium, halogen, amino, hydroxyl, cyano, =O, $SF_5$, azido, $C_{1-6}$alkyl, $C_{1-6}$alkoxy, $C_{2-6}$alkenyl or $C_{2-6}$alkynyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, -O-$C_{1-6}$alkyl-$C_{6-10}$aryl or -O-$C_{1-6}$alkyl-(5- to 10-membered heteroaryl).

The compound, and the stereoisomer, pharmaceutically acceptable salt, solvate, co-crystal or deuterated product thereof of the present invention, wherein the compound has a structure selected from one of the following:

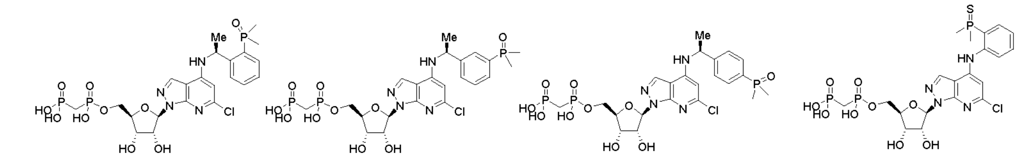

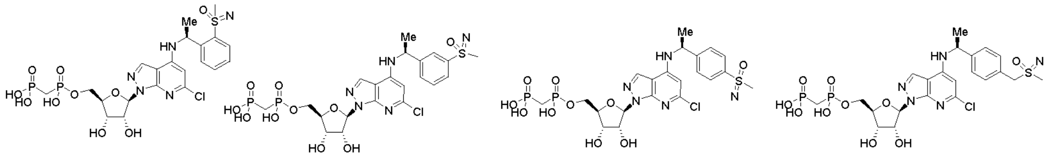

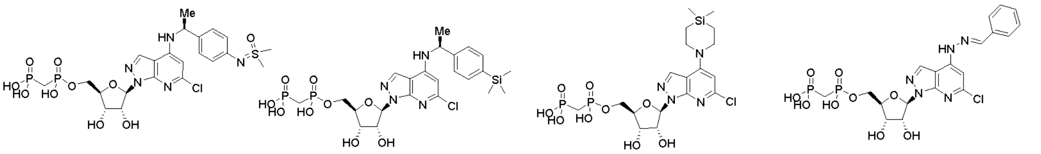

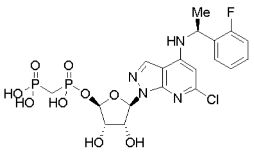 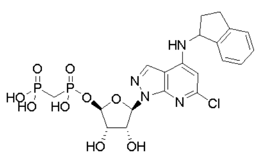 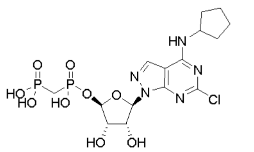 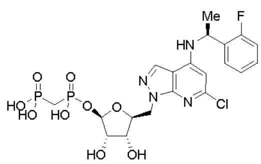

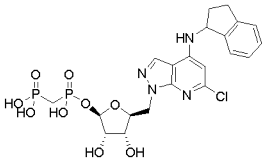 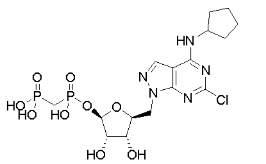 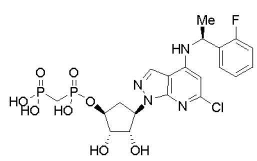 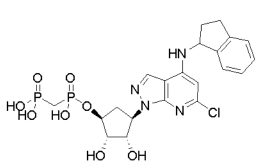

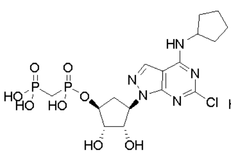 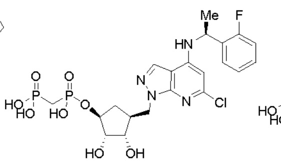 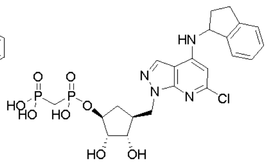 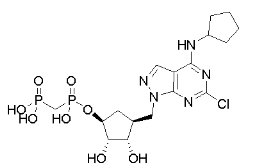

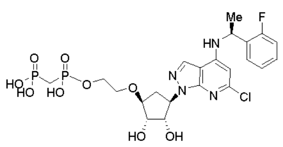 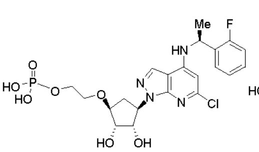 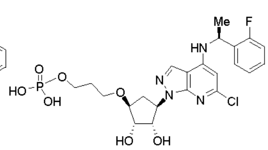 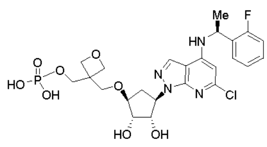

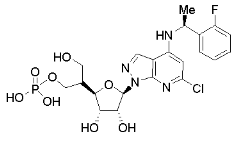 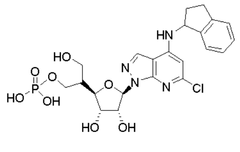 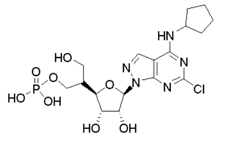 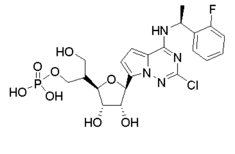

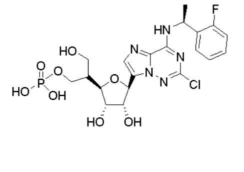 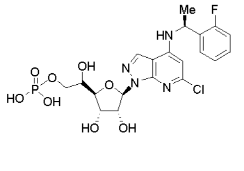 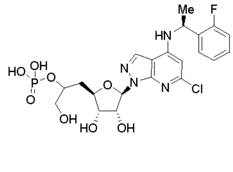 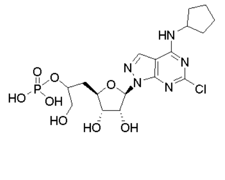

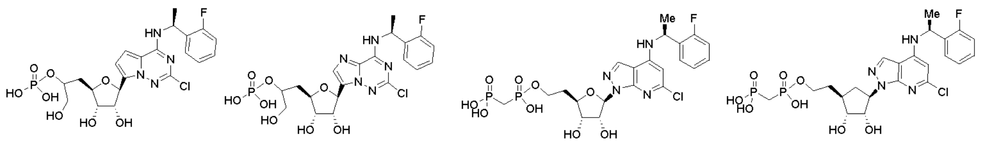

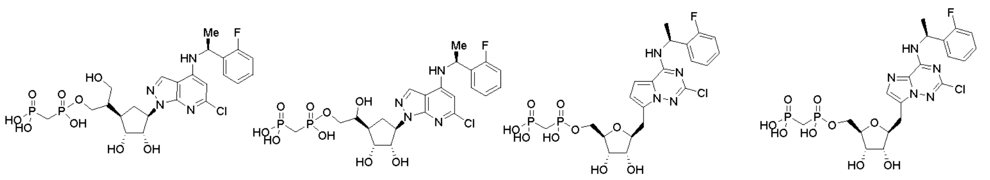

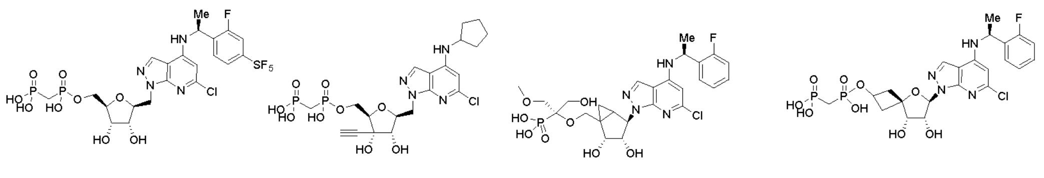

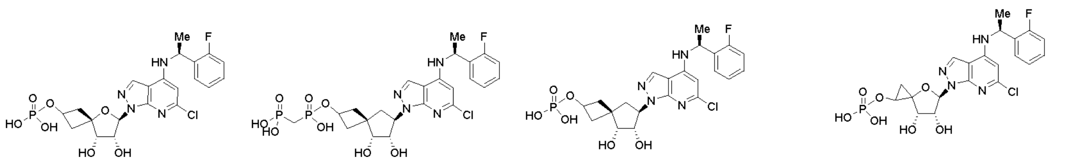

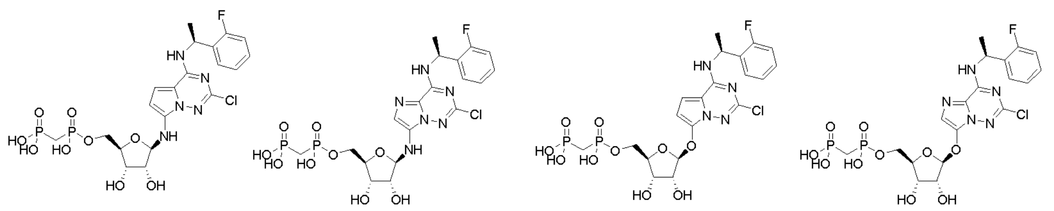

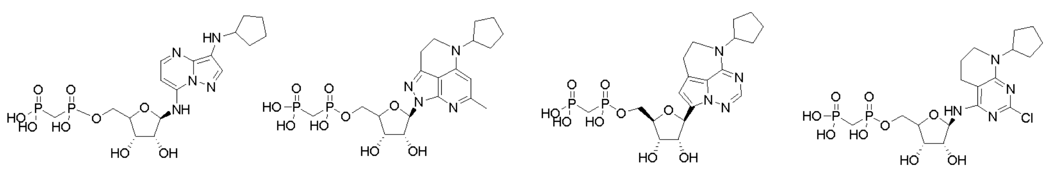

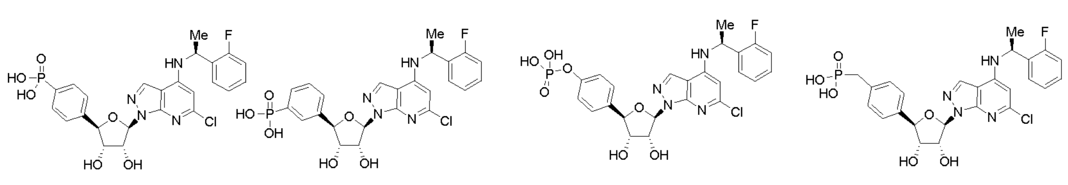

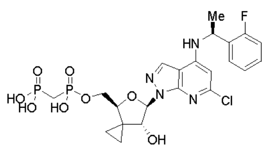
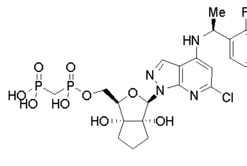
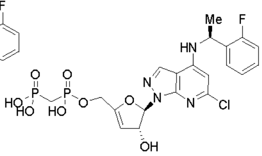
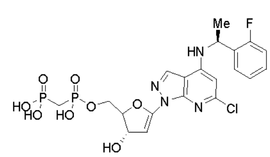
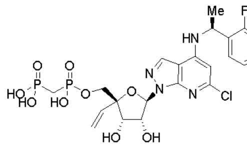
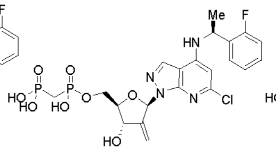
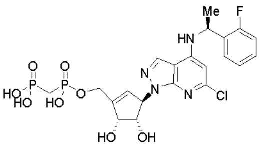
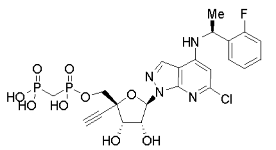
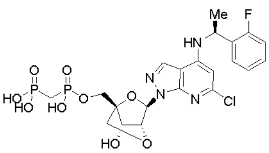
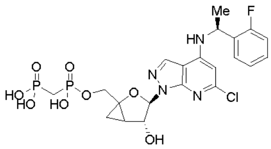
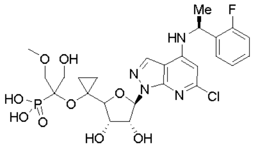
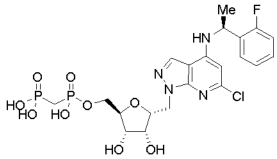
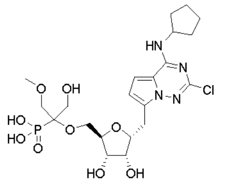
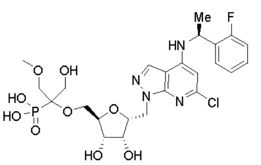
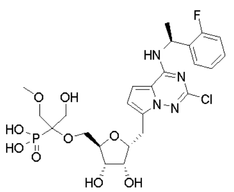
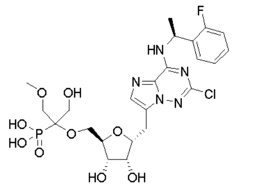
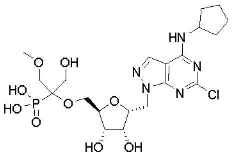
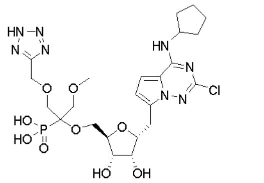
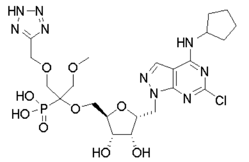
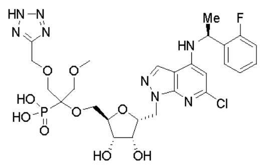
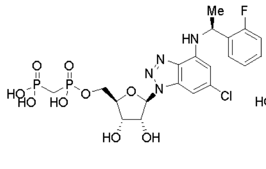
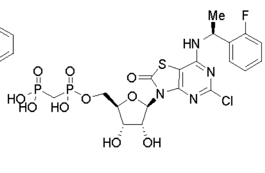
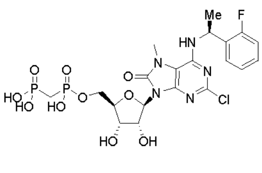
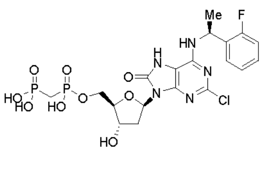
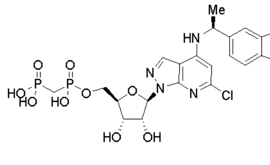
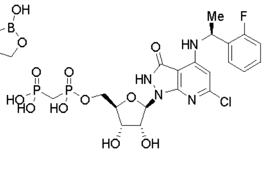
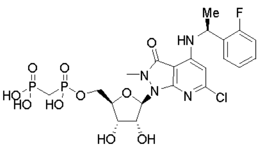
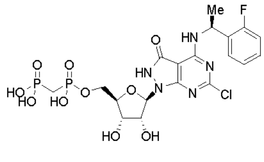
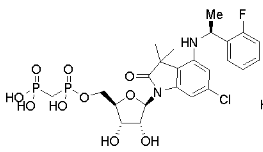
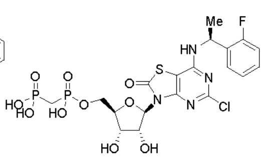
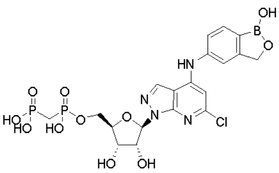
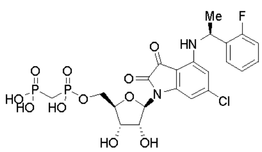

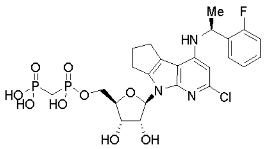
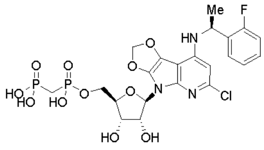
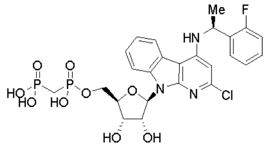
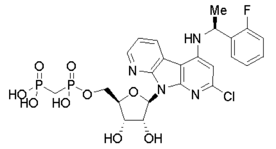

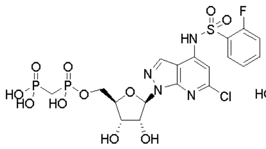
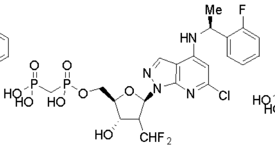
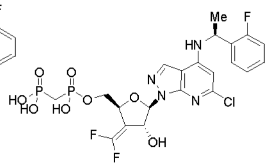
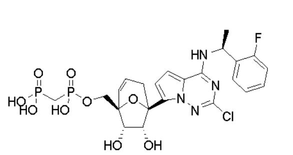

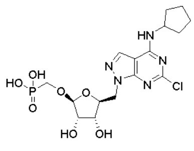
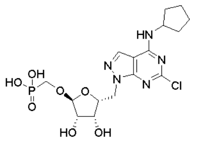
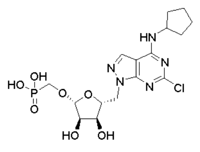
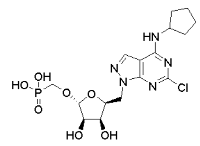
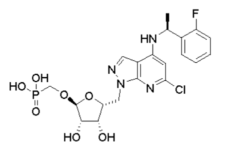

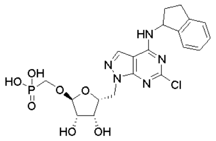
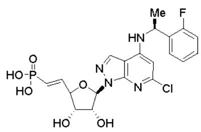
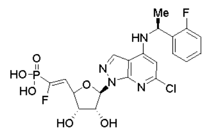
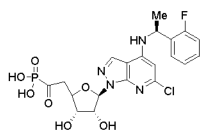
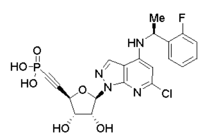

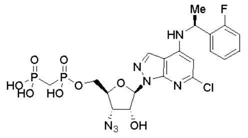
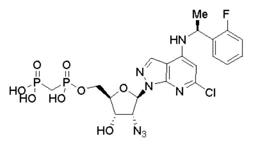
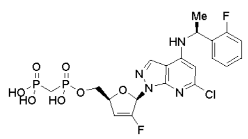
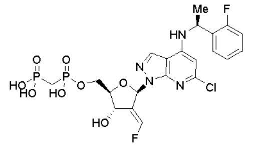

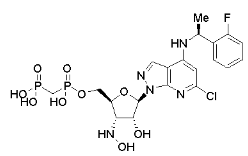
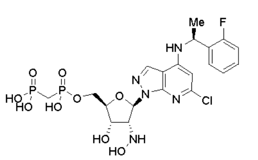
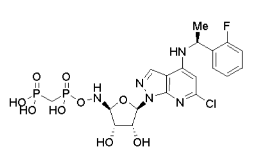
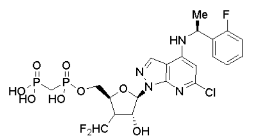

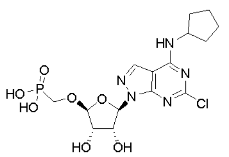
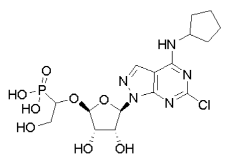
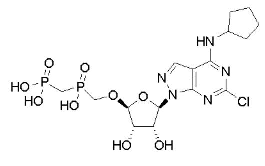
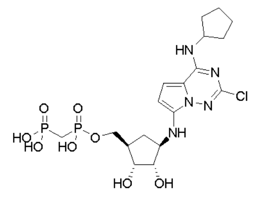

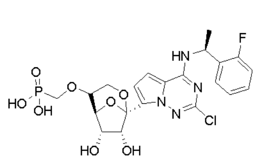
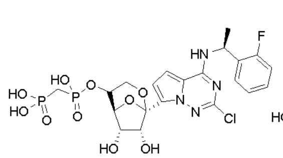
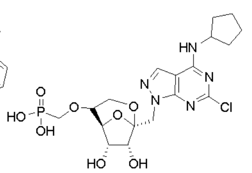
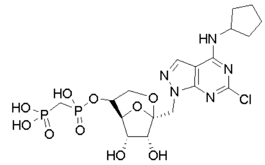

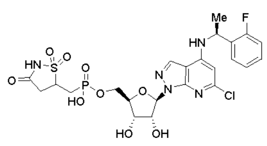
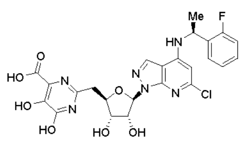
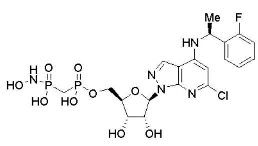
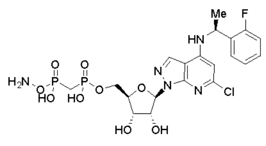

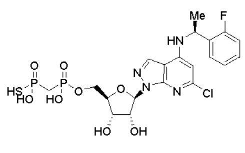
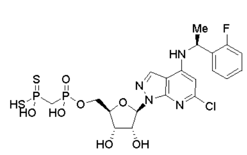

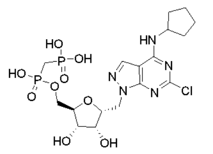
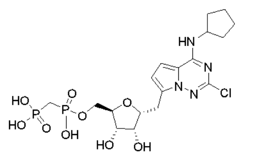
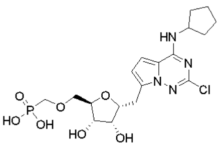
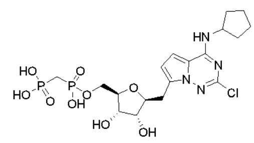

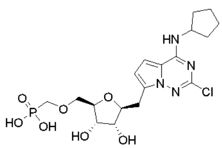
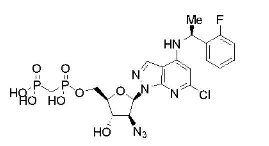
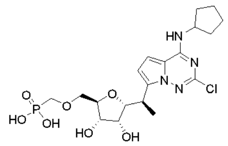
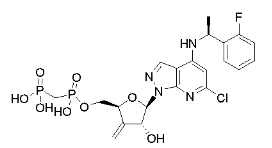

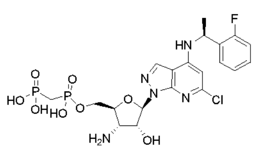
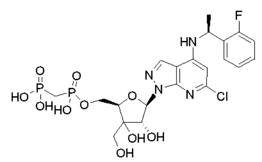
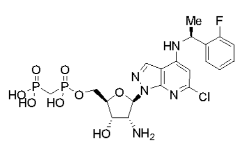
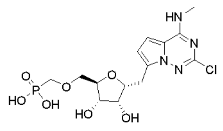

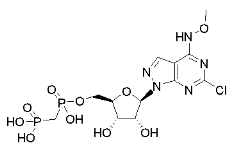
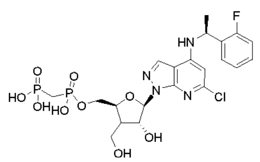
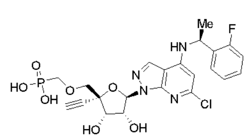

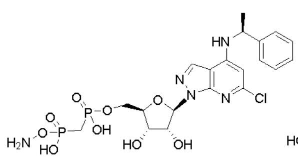
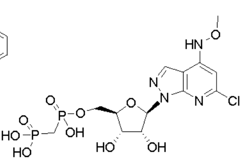
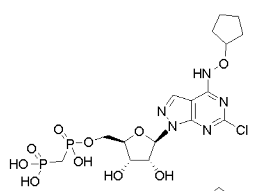
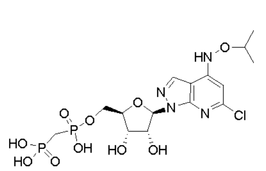

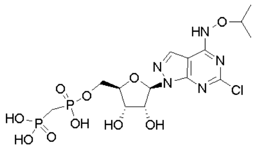
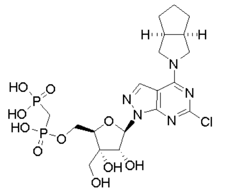
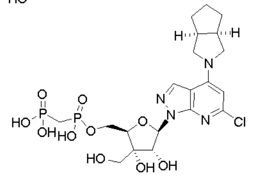
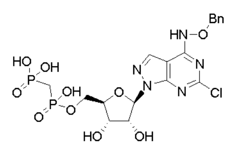

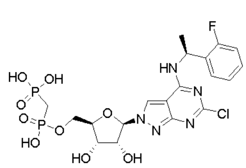
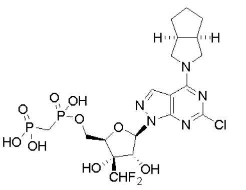
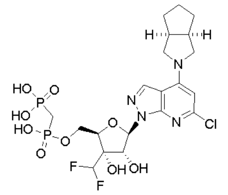
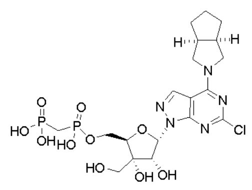

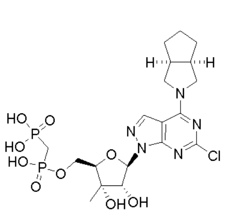
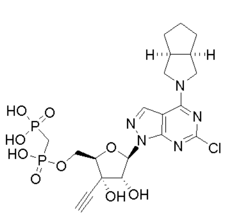
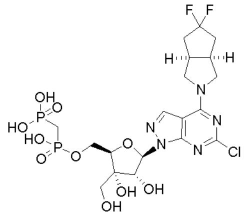
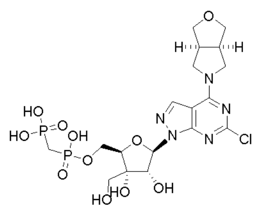

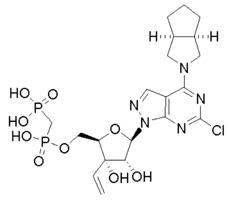
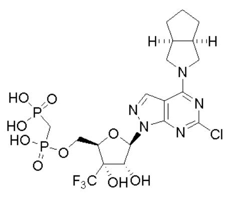
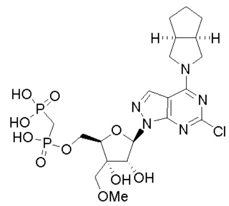

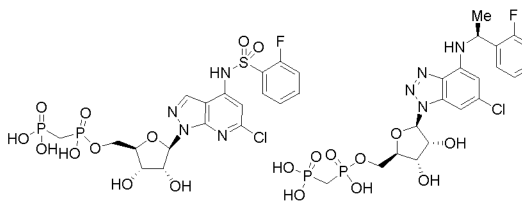
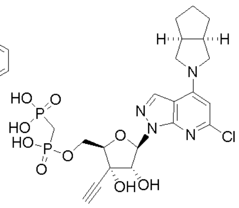
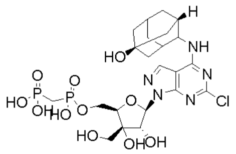

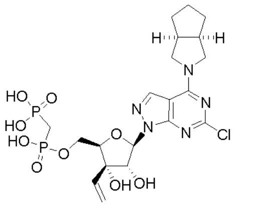
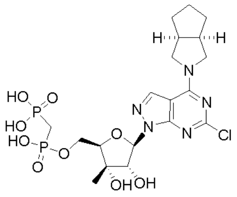
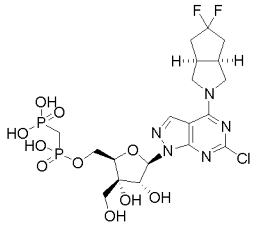
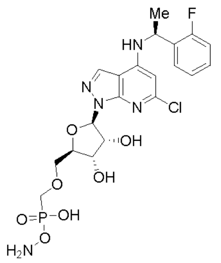

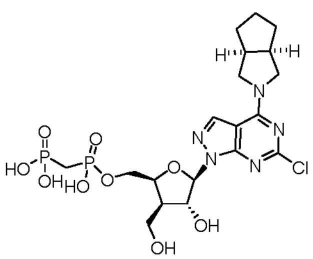
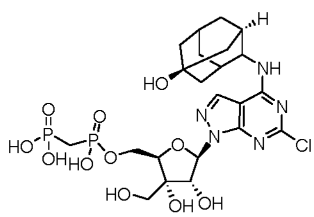

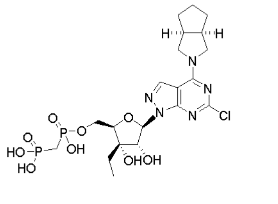 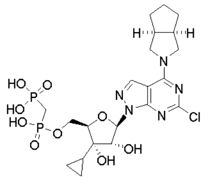 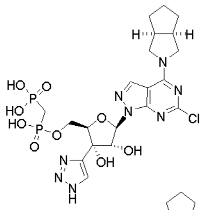 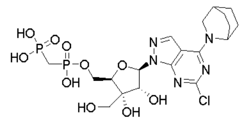

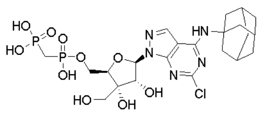 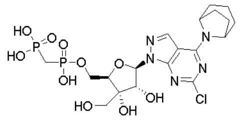 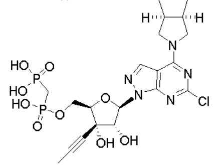 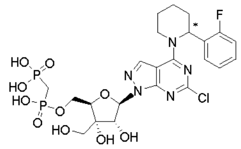

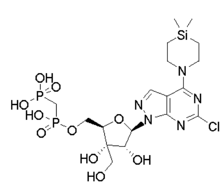 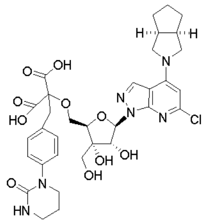 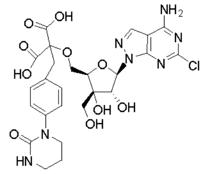 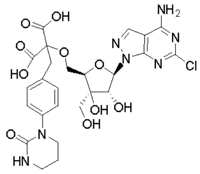.

The present invention further relates to a pharmaceutical composition comprising the compound, and the stereoisomer, pharmaceutically acceptable salt, solvate, co-crystal or deuterated product thereof of the present invention, and a pharmaceutically acceptable adjuvant and/or carrier.

Furthermore, the aforementioned pharmaceutical composition further contains anti-PD-1 antibodies.

The present invention further relates to the use of the compound, and the stereoisomer, pharmaceutically acceptable salt, solvate, co-crystal or deuterated product thereof, or the pharmaceutical composition of the present invention in the preparation of a drug for treating a CD73-mediated disease, wherein the CD73-mediated disease is a tumor or an autoimmune disease. Furthermore, the use comprises combined use of anti-PD-1 antibodies.

The present invention further relates to a method for treating a CD73-mediated disease, which comprises administering the compound, and the stereoisomer, pharmaceutically acceptable salt, solvate, co-crystal or deuterated product thereof, or the pharmaceutical composition of the present invention, wherein the CD73-mediated disease is a tumor or an autoimmune disease. Furthermore, the treatment method further comprises administering another drug, the drug being an anti-PD-1 antibody.

## Synthetic Route

Patent documents such as WO 2017120508 A1 and WO 2019213174 A1 introduce a method for preparing a CD73 antagonist, and those skilled in the art would have been able to prepare the compounds of the present invention by means of combining the documents and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial

Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalog are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

**Term**

Unless otherwise specified, the terms of the present invention have the following meanings.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen.

The term "deuterated" or "deuterated product" refers to the case where a hydrogen atom on alkyl, cycloalkyl, alkylene, aryl, heteroaryl, mercapto, heterocycloalkyl, alkenyl, alkynyl and other groups is substituted with at least one isotope deuterium, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, more preferably 1-10 deuterium atoms, more preferably 1-6 deuterium atoms, further preferably 1-3 deuterium atoms.

The term "alkyl "refers to a monovalent straight or branched saturated aliphatic hydrocarbon group, and non-limiting examples of alkyl include methyl, ethyl, n-propyl, isopropyl, n-butyl, sec-butyl, neobutyl, tert-butyl, n-pentyl, isopentyl, neopentyl, n-hexyl and various branched isomers thereof. The alkyl can be further substituted with any substituent.

The term "cycloalkyl" refers to a monovalent saturated or unsaturated non-aromatic carbocyclic hydrocarbon group and can be a monocyclic ring, a bicyclic ring, a spiro ring, a bridged ring or a fused ring, and non-limiting examples of cycloalkyl include cyclopropyl, cyclobutyl, cyclopentyl, cyclohexyl, cycloheptyl, cyclopentenyl,

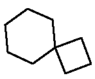, 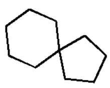, 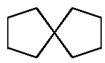, 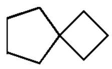, 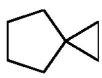, 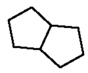, 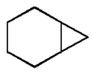,

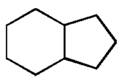 and 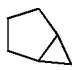, 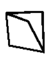, 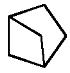, 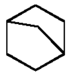,

etc. The cycloalkyl can be optionally further substituted with any substituent.

The term "heterocycloalkyl "refers to a substituted or unsubstituted, saturated or unsaturated non-aromatic ring. Without particular limitation, the heterocycloalkyl contains 1 to 5 heteroatoms selected from N, O, P, Si or S, and further contains 1 to 4 or 1 to 3 heteroatoms selected from N, O, P, Si or S, and may be a monocyclic ring or polycyclic ring, and specifically, the polycyclic ring may be a bridged ring, a fused ring or a spiro ring. Without particular limitation, the heterocycloalkyl is 3- to 12-membered heterocycle, more preferably 4- to 12-membered heterocycle, and more preferably 4- to 10-membered heterocycle. The optionally substituted N, S and P in the ring of heterocyclyl may be oxidized to various oxidation states. Non-limiting examples include azacyclopropyl, oxecyclopropyl, thiacyclopropyl, azacyclobutyl, azacyclopentyl, piperidine, oxetanyl, oxacyclopentyl, oxacyclohexyl, thiacyclobutyl, pyrrolidinyl, pyrazolidinyl, tetrahydrofuryl, tetrahydrothienyl, azaadamantyl, oxaspiro[3.3]heptanyl,

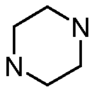, 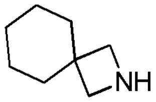, 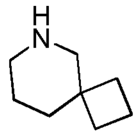, 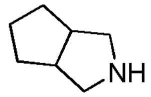, 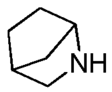,

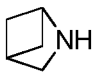, 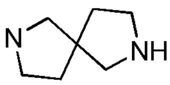, 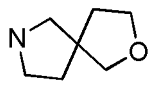, 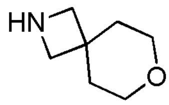, 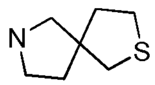, 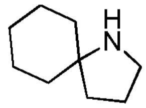,

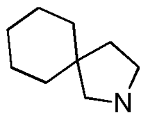, 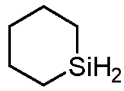, 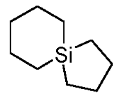, 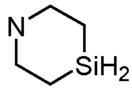,

etc. The heterocycloalkyl can be optionally further substituted with any substituent.

The term "heterocycle" or "heterocyclyl" refers to a substituted or unsubstituted, saturated or unsaturated aromatic ring or non-aromatic ring. Without particular limitation, the heterocycle or heterocyclyl contains 1 to 5 heteroatoms selected from N, O or S, further contains 1 to 4 heteroatoms selected from N, O or S, and further contains 1 to 3 heteroatoms selected from N, O and S, including monocyclic heterocycle or polycyclic heterocycle, specifically the polycyclic heterocycle including bridged bicyclic ring heterocycle, bicyclic fused heterocycle, bicyclic spiro heterocycle, etc. Without particular limitation, the heterocycle is 3-to 12-membered heterocycle, more preferably 4- to 12-membered heterocycle, more preferably 4- to 10-membered heterocycle, and further preferably 4- to 7-membered heterocycle. The definition thereof comprises heterocycloalkyl and heteroaryl. The N and S in the heterocyclyl ring may be oxidized to various oxidation states. Heterocyclyl can be connected to a heteroatom or a carbon atom, and non-limiting examples of heterocyclyl include epoxyethyl, azacyclopropyl, oxetanyl, azacyclobutyl, 1,3-dioxolanyl, 1,4-dioxolanyl, 1,3-dioxanyl, azacycloheptyl, pyridyl, furyl, thienyl, pyranyl, N-alkylpyrrolyl, pyrimidinyl, pyrazinyl, pyrazolyl, pyridazinyl, imidazoly, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl,

, 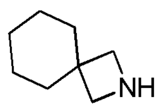, 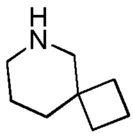, 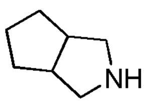, 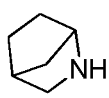,

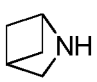, 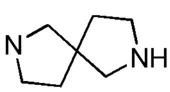, 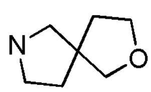, 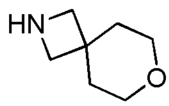, 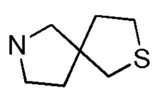, 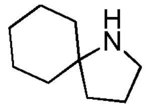,

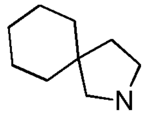,

etc.

The term "carbocycle" or "carbocyclyl" refers to a substituted or unsubstituted, saturated or unsaturated, aromatic or non-aromatic carbocycle group, including monocyclic or polycyclic carbocycle, specifically the polycyclic carbocycle including bridged bicyclic ring, bicyclic fused ring, bicyclic spiro ring, etc. Unless otherwise specified, the carbocycle or carbocyclyl contains 3 to 12 carbon atoms, preferably 3-10 carbon atoms, further preferably 3-6 carbon atoms. The definition thereof comprises cycloalkyl and aryl. In non-limiting embodiments, a monocyclic carbocyclic ring includes cyclopropyl, cyclobutyl, cyclopentyl, cyclohexyl, cycloheptyl or phenyl,

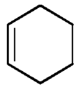,

*etc.*, a bicyclic bridged ring includes

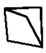, 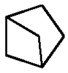, 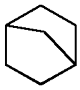,

*etc.*, a bicyclic fused ring includes

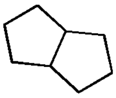, 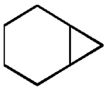,

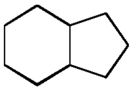, 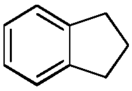, 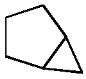,

*etc.*, and a bicyclic spiro ring includes

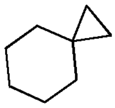,

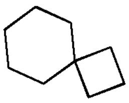, 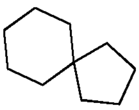, 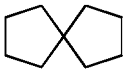, 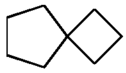, 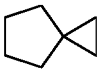,

*etc.*

The term "aryl" refers to a substituted or unsubstituted aromatic 5- to 15-membered carbocycle, and includes monocyclic aryl and fused aryl. 5- to 10-membered aromatic ring is preferred, and 5- to 8-membered aromatic ring is further preferred. Non-limiting examples thereof include phenyl, naphthyl, anthryl, phenanthryl, etc. The aryl ring can be fused to a heteroaryl, heterocycloalkyl or cycloalkyl ring, wherein the ring connected to the parent structure is an aryl ring, and non-limiting examples include

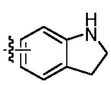, 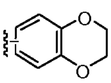, 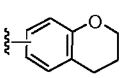, 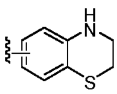, 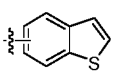, 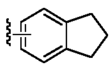, 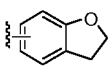, 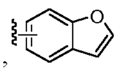,

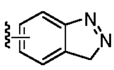, 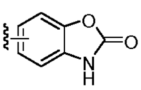, 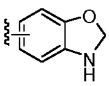, 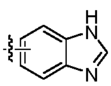, 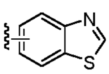, 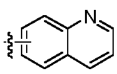 and 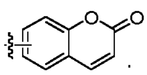.

The aryl can be optionally further substituted with any substituent.

The term "heteroaryl "refers to a substituted or unsubstituted 5- to 15-membered aromatic ring containing 1 to 5 heteroatoms selected from N, O, P, Si or S and various oxidized forms of the heteroatoms, preferably a 5- to 10-membered heteroaromatic ring, further preferably a 5- to 8-membered heteroaromatic ring. Non-limiting examples of heteroaryl include, but are not limited to furyl, oxazolyl, furyl, thienyl, N-alkylpyrrolyl, pyrazinyl, pyridazinyl, piperidyl, morpholine, thiomorpholine, 1,3-dithiane, benzimidazole, piperadinyl,

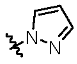 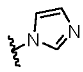 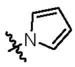 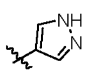 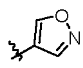 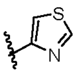 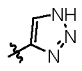 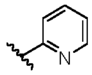 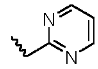

. The heteroaryl ring can be fused to an aryl, heterocycloalkyl or cycloalkyl ring, wherein the ring connected to the parent structure is a heteroaryl ring, and non-limiting examples include

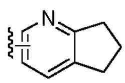, 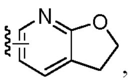, 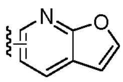, 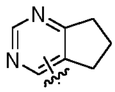, 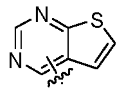, 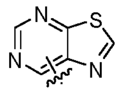, 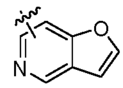

and

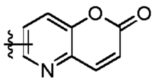.

The heteroaryl can be optionally further substituted with any substituent.

The term "alkynyl" refers to a linear or branched monovalent unsaturated hydrocarbon group containing one or more carbon-carbon triple bonds. Unless otherwise specified, the alkynyl contains 2-6 carbon atoms, preferably contains 2-4 carbon atoms, and non-limiting examples of the alkynyl are ethynyl, propynyl, propargyl, etc.

The term "alkenyl" refers to a linear or branched monovalent unsaturated hydrocarbon group containing one or more carbon-carbon double bonds. Unless otherwise specified, the alkenyl contains 2-6 carbon atoms, preferably contains 2-4 carbon atoms, and non-limiting examples of the alkenyl are ethenyl, propenyl, allyl, 2-butenyl, 1-butenyl etc.

The term "alkoxy" or "alkyloxy" refers to -O-alkyl. Without particular limitation, alkoxy or alkyloxy is -O-$C_{1-8}$ alkyl, preferably -O-$C_{1-6}$alkyl, more preferably -O-$C_{1-4}$ alkyl, and further preferably -O-$C_{1-2}$ alkyl. Non-limiting examples of the alkoxy or alkyloxy include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Without particular limitation, haloalkoxy is -O-halo $C_{1-8}$alkyl, preferably -O-halo $C_{1-6}$ alkyl, more preferably -O-halo $C_{1-4}$ alkyl, and further preferably -O-halo $C_{1-2}$ alkyl. Non-limiting examples of haloalkoxy include monofluoromethoxy, difluoromethoxy, trifluoromethoxy, difluoroethyloxy, *etc.*

The term "alkylamino" or "alkamino" refers to amino substituted with one or two alkyl, and is also written as -N-$(alkyl)_2$ or -NH-alkyl, wherein the latter is also known as monoalkylamino. Non-limiting examples of alkylamino or alkamino include dimethylamino, monomethylamino, diethylamino, monoethylamino, etc.

In the present invention, "$C_0$alkyl" means a bond.

Unless otherwise specified, the term "substitute" or "substituent" refers to any substitution at a position allowed by chemical theory, and the number of substituents conforms to the rules of chemical bonding. Exemplary substituents include, but are not limited to: cyano, halogen, nitro, amino, hydroxyl, $C_{1-6}$alkyl, $C_{2-6}$alkenyl, $C_{2-6}$alkynyl, $C_{3-8}$heteroalkyl, $C_{5-12}$aryl, 5- to 12-membered heteroaryl, $C_{1-6}$alkoxy, $C_{5-12}$aryloxy, thiol, $C_{1-6}$alkylthio, $C_{1-6}$alkylthiocarbonyl, $C_{1-6}$alkylcarbamoyl, N-carbamoyl, silyl, sulfinyl, sulfonyl, sulfoxide, halo $C_{1-6}$alkyl, halo $C_{1-6}$alkoxy, phosphonic acid, -$CO_2$($C_{1-6}$alkyl), -OC(=O)($C_{1-6}$alkyl), -$OCO_2$($C_{1-6}$alkyl), -C(=O)$NH_2$, -C(=O)N($C_{1-6}$alkyl)$_2$, -OC(=O)NH($C_{1-6}$alkyl), -NHC(=O)($C_{1-6}$alkyl), -N($C_{1-6}$alkyl)C(=O)($C_{1-6}$alkyl), -$NHCO_2$($C_{1-6}$alkyl), -NHC(=O)N($C_{1-6}$alkyl)$_2$, -HC(=O)NH($C_{1-6}$alkyl), -NHC(=O)$NH_2$, -$NHSO_2$($C_{1-6}$alkyl), -$SO_2$N($C_{1-6}$alkyl)$_2$, -$SO_2$NH($C_{1-6}$alkyl), -$SO_2NH_2$, -$SO_2C_{1-6}$alkyl, etc.

The term "optional" or "optionally" refers to that the event or circumstance subsequently described may but not necessarily occur, and the description includes the occasions where the events or circumstances occur or do not occur. For example, "alkyl optionally substituted with F" means that an alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein and the stereoisomers, solvates, pharmaceutically acceptable salts or co-crystals thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the

drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavoring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

## Brief Description of the Drawings

Figure 1 is the mouse body weight change curve of the B16F10 xenograft tumor model experiment in vivo.
Figure 2 is the tumor volume growth curve of the B16F10 xenograft tumor model experiment in vivo.

## Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

### Test Method

The structures of the compounds are determined by nuclear magnetic resonance (NMR) or (and) mass spectrometry (MS). The NMR shift (δ) is given in the unit of 10-6 (ppm). NMR is determined with Bruker Avance III 400 and Bruker Avance 300; the solvent for determination is deuterated dimethyl sulfoxide (DMSO-d6), deuterated chloroform ($CDCl_3$) and deuterated methanol ($CD_3OD$); and the internal standard is tetramethylsilane (TMS).

MS is measured with (Agilent 6120B(ESI) and Agilent 6120B(APCI));
HPLC is measured with Agilent 1260DAD high pressure liquid chromatography (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.

For the column chromatography, Yantai Huanghai silica gel of 200-300 mesh silica gel is generally used as a carrier.

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

**Example 1:**

(((((2R,3S,4R,5R)-5-((6-chloro-4-(cyclopentylamino)-1H-pyrazolo[3,4-d]pyrimidin-1-yl)methyl)-3,4-dihydroxytetrahydrofuran-2-yl)methoxy)(hydroxy)phosphoryl)methyl)phosphonic acid (Compound 1)

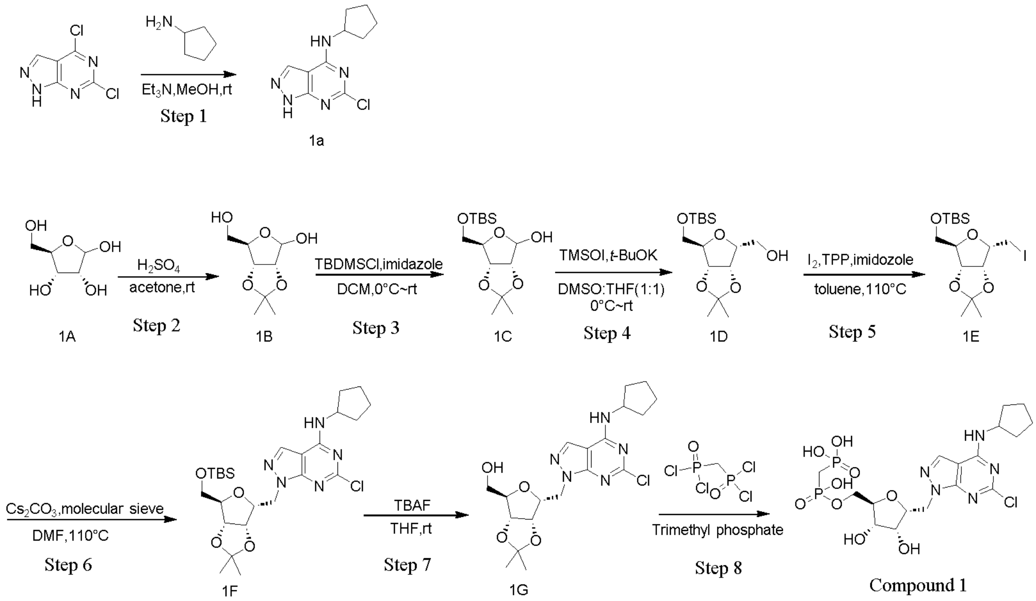


Step 1: 6-chloro-N-cyclopentyl-1H-pyrazolo[3,4-d]pyrimidin-4-amine (1a)

4,6-dichloro-1H-pyrazolo[3,4-d]pyrimidine (5g, 26.5mmol) was dissolved in methanol (50 ml), triethylamine (8.02g, 79.4mmol) was added, the reaction was stirred at room temperature, after the reaction was completed, the reaction solution was concentrated, to the resulting residue were added water (25 ml) and dichloromethane (25 ml), the organic phases were separated, the aqueous phase was extracted with dichloromethane (25 ml × 2), the organic phases were combined, dried over anhydrous sodium sulfate, concentrated, and the resulting residue was purified by silica gel column chromatography (eluent: ethyl acetate: petroleum ether = 0%-50%) to obtain the compound 1a (5.02 g, 80.1%).

LCMS m/z = 238.6 $[M+1]^+$.

Step 2: (3aR,6R,6aR)-6-(hydroxymethyl)-2,2-dimethyltetrahydrofuro[3,4-d][1,3]dioxol-4-ol (1B)

Compound 1A (15 g, 150.1 mmol) was dissolved in acetone (150 ml), sulfuric acid (0.9 ml) was added, the reaction was stirred at room temperature overnight, after the reaction was completed, solid sodium bicarbonate was added to adjust the system to a neutral pH, the mixture was filtered, and concentrated, and the resulting residue was purified by silica gel column chromatography (eluent: ethyl acetate: petroleum ether = 0%-30%) to obtain the compound 1B (14.96 g, 78.7%).

$^1H$ NMR (400 MHz, $CDCl_3$) δ 5.42 (s, 1H), 4.84 (d, 1H), 4.59 (d, 1H), 4.41 (t, 1H), 3.74 (dd, 2H), 1.49 (s, 3H), 1.33 (s, 3H).

Step 3: (3aR,6R,6aR)-6-(((tert-butyldimethylsilyl)oxy)methyl)-2,2-dimethyltetrahydrofuro[3,4-d][1,3]dioxol-4-ol (1C)

Compound 1B (14.96 g, 78.2 mmol) and imidazole (6.914 g, 101.7mmol) were dissolved in anhydrous dichloromethane (150 ml), the reaction was stirred at room temperature for 30 min, cooled to 0°C, tert-butyldimethylsilyl chloride (13.08g, 86.0mmol) was added, the mixture was warmed to room temperature and reacted for 4 h, after the reaction was completed, water (150 ml) was added, organic phases were separated, the aqueous phase was extracted with dichloromethane (150 ml × 2), the organic phases were combined, dried over anhydrous sodium sulfate, concentrated, and the resulting residue was purified by silica gel column chromatography (eluent: ethyl acetate: petroleum ether = 0%-5%) to obtain the compound 1C (17.57g, 73.4%).

$^1H$ NMR (400 MHz, $CDCl_3$) δ 5.28 (s, 1H), 4.70 (s, 1H), 4.51 (s, 1H), 4.35 (s, 1H), 3.75 (s, 2H), 1.49 (s, 3H), 1.33 (s, 3H), 0.93 (s, 9H), 0.14 (d, 6H).

Step 4: ((3aS,4R,6R,6aR)-6-(((tert-butyldimethylsilyl)oxy)methyl)-2,2-dimethyltetrahydrofuro[3,4-d][1,3]dioxol-4-yl)methanol (1D)

Trimethylsulfoxonium iodide (1.10g, 5.02mmol) and potassium tert-butoxide (562 mg, 5.02 mmol) were added into a reaction flask, stirred at 0°C, then anhydrous dimethyl sulfoxide (10 ml) was added, stirred at 0°C for 30min, and the mixture was warmed to room temperature and stirred for 30 min. The reaction was cooled to 0°C, compound 1C (1.017 g, 3.35 mmol) was dissolved in anhydrous tetrahydrofuran (10 ml), the mixture was added into a reaction flask, kept at 0°C and stirred for 30 min, the mixture was warmed to room temperature and stirred overnight, after the reaction was completed, saturated ammonium chloride (5 ml) was added, followed by water (20 ml) and ethyl acetate (20 ml), organic phases were separated, the aqueous phase was extracted with ethyl acetate (20 ml × 2), the organic phases were combined, washed with saturated sodium bicarbonate solution (20 ml), saturated brine (20 ml) and water (20 ml), dried over anhydrous sodium sulfate, concentrated, and the resulting residue was purified by silica gel column chromatography (eluent: ethyl acetate: petroleum ether = 0%-15%) to obtain the compound 1D (355mg, 33.4%).

$^1H$ NMR (400 MHz, $CDCl_3$) δ 4.80 (dt, 2H), 4.21 (dd, 1H), 4.15 (t, 1H), 3.89 - 3.80 (m, 2H), 3.75 - 3.70 (m, 2H), 1.51 (s, 3H), 1.35 (s, 3H), 0.89 (s, 9H), 0.06 (s, 6H).

Step 5: tert-butyl(((3aR,4R,6S,6aR)-6-(iodomethyl)-2,2-dimethyltetrahydrofuro[3,4-d][1,3]dioxol-4-yl)methoxy)dimethylsilane (1E)

Compound 1D (330 mg, 1.04 mmol), iodine (580 mg, 2.28 mmol), imidazole (212 mg, 3.11 mmol) and triphenylphosphine (565 mg, 2.08 mmol) were dissolved in toluene (10 ml), the mixture was refluxed and reacted at 110°C for 3 h, after the reaction was completed, water (10 ml) and ethyl acetate (10 ml) were added, organic phases were separated, the aqueous phase was extracted with ethyl acetate (10 ml × 2), the organic phases were combined, washed with saturated brine (20 ml), dried over anhydrous sodium sulfate, concentrated, and the resulting residue was purified by silica gel column chromatography (eluent: ethyl acetate: petroleum ether = 0%-5%) to obtain the compound 1E (310 mg, 69.6%).

$^1H$ NMR (400 MHz, $CDCl_3$) δ 4.86 (d, 1H), 4.80 - 4.72 (m, 1H), 4.42 (ddd, 1H), 4.16 - 4.08 (m, 1H), 3.78 - 3.65 (m, 2H), 3.35 - 3.18 (m, 2H), 1.49 (s, 3H), 1.36 (s, 3H), 0.90 (s, 9H), 0.07 (s, 6H).

Step 6: 1-(((3aS,4R,6R,6aR)-6-(((tert-butyldimethylsilyl)oxy)methyl)-2,2-dimethyltetrahydrofuro[3,4-d][1,3]dioxol-4-yl)methyl)-6-chloro-N-cyclopentyl-1H-pyrazolo[3,4-d]pyrimidin-4-amine (1F)

Compounds 1E (250 mg, 0.58 mmol) and 1a (141 mg, 0.58 mmol) were dissolved in anhydrous N,N-dimethylformamide (5 ml), cesium carbonate (570 mg, 1.74 mmol) and a molecular sieve for drying (2 g) were added, the mixture was refluxed and reacted at 110°C overnight, after the reaction was completed, the mixture was concentrated, to the residue were added water (5 ml) and ethyl acetate (5ml), organic phases were separated, the aqueous phase was extracted with ethyl acetate (5 ml × 2), the organic phases were combined, dried over anhydrous sodium sulfate, concentrated, and the resulting residue was purified by silica gel column chromatography (eluent: ethyl acetate: petroleum ether = 0%-30%) to obtain the compound 1F (150 mg, 48.1%).

LCMS m/z = 538.3 $[M+1]^+$.

Step 7: ((3aR,4R,6R,6aS)-6-((6-chloro-4-(cyclopentylamino)-1H-pyrazolo [3,4-d]pyrimidin-1-yl)methyl)-2,2-dimethyltetrahydrofuro[3,4-d][1,3]dioxol-4-yl)methanol (1G)

Compound 1F (250 mg, 0.47 mmol) was dissolved in anhydrous tetrahydrofuran (5 ml), 1M of tetrabutylammonium fluoride in tetrahydrofuran (1.40 ml, 1.40 mmol) was added, stirred at room temperature for 30 min, after the

reaction was completed, water (5 ml) and ethyl acetate (5 ml) were added, organic phases were separated, the aqueous phase was extracted with ethyl acetate (5 ml × 2), the organic phases were combined, dried over anhydrous sodium sulfate, concentrated, and the resulting residue was purified by silica gel column chromatography (eluent: ethyl acetate: petroleum ether = 0%-100%) to obtain the compound 1G (160 mg, 81.2%).

LCMS m/z = 424.1 $[M+1]^+$.

Step 8: (((((2R,3S,4R,5R)-5-((6-chloro-4-(cyclopentylamino)-1H-pyrazolo [3,4-d]pyrimidin-1-yl)methyl)-3,4-dihydroxytetrahydrofuran-2-yl)methoxy)(hydroxy)phosphoryl)methyl)phosphonic acid(Compound 1)

Compound 1G (160 mg, 0.38 mmol) was dissolved in trimethyl phosphate (2 ml), and stirred at 0°C, methylenebis(phosphonic dichloride) (285 mg, 1.14 mmol) was dissolved in trimethyl phosphate (1 ml) and the mixture was added into a reaction flask, and stirred at 0°C for 2h, after the substrate was completely converted, water (0.6 ml) was added, and stirred at room temperature overnight, after the reaction was completed, the mixture was concentrated, and the residue was separated and purified by a preparative liquid chromatography column with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 10.0 min, to obtain the compound 1 (80 mg, 36.2%).

$^1$H NMR (400 MHz, $CDCl_3$) δ 8.62 (d, 1H), 8.45 (s, 1H), 4.41 (dd, 3H), 3.98 (dd, 5H), 2.21 (t, 2H), 1.99 (s, 2H), 1.72 (s, 2H), 1.57 (s, 3H), 1.24 (s, 2H).

MS M/Z (ESI) : m/z = 542.2 $[M+1]^+$.

**Example 2:**

(((((2R,3S,4R,5R)-5-((2-chloro-4-(cyclopentylamino)pyrrolo[2,1-f][1,2,4]triazin-7-yl)methyl)-3,4-dihydroxytetrahydrofuran-2-yl)methoxy)(hydroxy)phosphoryl)methyl)phosphonic acid(Compound 2)

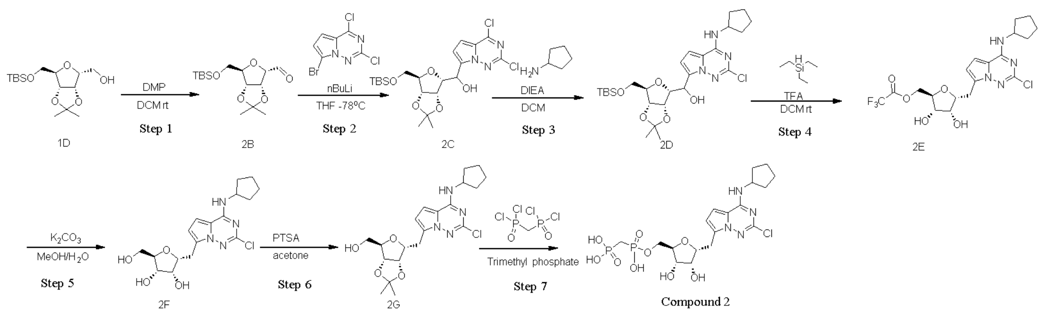


Step 1: (3aR,4S,6R,6aR)-6-(((tert-butyldimethylsilyl)oxy)methyl)-2,2-dimethyltetrahydrofuro[3,4-d][1,3]dioxole-4-carbaldehyde (2B)

Compound 1D (2.0 g, 6.28 mmol) was dissolved in dichloromethane (20 ml), Dess-Martin reagent (7.99 g, 18.84 mmol) was added in portions, after the addition, the mixture was reacted at room temperature for 2 hours, after the reaction was completed, water (50 ml) was added into the reaction solution to quench the reaction, the mixture was filtered, the filter cake was rinsed with dichloromethane, the liquid separation was conducted, the aqueous phase was extracted twice with dichloromethane (100 ml × 2), the organic layers were combined, washed with saturated brine, dried over anhydrous sodium sulfate, and concentrated to obtain the compound 2B (1.9 g, 95%), which was directly used in the next step without purification.

Step 2: ((3aS,4R,6R,6aR)-6-(((tert-butyldimethylsilyl)oxy)methyl)-2,2-dimethyltetrahydrofuro[3,4-d][1,3]dioxol-4-yl)(2,4-dichloropyrrolo[2,1-f][1,2,4]triazin-7-yl)methanol (2C)

7-Bromo-2,4-dichloropyrrolo[2,1-f][1,2,4]triazine (1.92 g, 7.2 mmol) was dissolved in dry tetrahydrofuran (20 ml), the reaction system was subjected to nitrogen replacement three times, cooled to -78°C using an ethanol dry ice system, with the temperature was controlled at -78°C, n-butyllithium (2.64 ml, 6.6 mmol, 2.5mol/L) was added dropwise,

after the addition, the mixture was reacted at -78°C for 10 minutes, with the temperature was controlled at -78°C, compound 2B (1.9 g, 6.0 mmol) was added dropwise, after the addition, with the temperature was maintained at -78°C, the mixture was reacted for 1 hour, after the reaction was completed, the reaction was quenched with glacial acetic acid (1.5 ml), water (30 ml) was added, the reaction was extracted with ethyl acetate (100 ml × 2), the organic layers were combined, washed with saturated brine, dried and concentrated, and then the residue was separated and purified by silica gel column chromatography (petroleum ether : ethyl acetate (v/v) = 5:1) to obtain the title compound 2C (0.52 g, 17%).

LC-MS (ESI): m/z = 526.1 $[M+Na]^+$; 486.1 $[M+1-H_2O]^+$.

Step 3: ((3aS,4R,6R,6aR)-6-(((tert-butyldimethylsilyl)oxy)methyl)-2,2-dimethyltetrahydrofuro[3,4-d][1,3]dioxol-4-yl)(2-chloro-4-(cyclopentylamino)pyrrolo[2,1-f][1,2,4]triazin-7-yl)methanol (2D)

Compound 2C (0.52 g, 1.03mmol) and cyclopentylamine (0.18, 2.06 mmol) were dissolved in dichloromethane (10ml), N,N-diisopropyl ethylamine (0.51 g, 4.12 mmol) was added, and then the mixture was reacted at room temperature for 3 hours, and after the reaction was completed, the reaction solution was directly concentrated, and the residue was separated and purified by silica gel column chromatography (petroleum ether: ethyl acetate (v/v) = 5 : 1) to obtain the title compound 2D (0.48 g, 84%).

LC-MS (ESI): m/z = 553.2 $[M+H]^+$.

Step 4: ((2R,3 S,4R,5R)-5-((2-chloro-4-(cyclopentylamino)pyrrolo[2,1-f][1,2,4]triazin-7-yl)methyl)-3,4-dihydroxytetrahydrofuran-2-yl)methyl 2,2,2-trifluoroacetate (2E)

Compound 2D (0.48 g, 0.87 mmol) was dissolved in dichloromethane (5 ml), trifluoroacetic acid (2 ml) was added, the temperature was decreased by 0-5°C, triethylsilane (1.4 ml, 8.7 mmol) was added dropwise, and after the addition, the mixture was naturally warmed to room temperature and reacted for 16 hours, and after the reaction was completed, the mixture was directly concentrated to obtain the target compound 2E (crude, 0.36 g, 86%), which was directly used in the next reaction.

LC-MS (ESI): m/z = 479.1$[M+H]^+$.

Step 5: (2R,3R,4S,5R)-2-((2-chloro-4-(cyclopentylamino)pyrrolo[2,1-f][1,2,4] triazin-7-yl)methyl)-5-(hydroxymethyl)tetrahydrofuran-3,4-diol (2F)

Compound 2E (0.36 g, 0.75 mmol) was dissolved in methanol (5ml) and water (1ml), potassium carbonate (0.1 g, 0.75 mmol) was added, after the addition, the mixture was reacted at room temperature for 1 hour, after the reaction was completed, water (10 ml) was added and the mixture was extracted with dichloromethane (50 ml × 2), washed with saturated brine, dried over anhydrous sodium sulfate, filtered, and the filtrate was concentrated under reduced pressure to obtain the target compound 2F (crude, 0.27 g, 94%), which was directly used in the next step.

LC-MS (ESI): m/z = 383.1$[M+H]^+$.

Step 6: ((3aR,4R,6R,6aS)-6-((2-chloro-4-(cyclopentylamino)pyrrolo[2,1-f][1,2,4]triazin-7-yl)methyl)-2,2-dimethyltetrahydrofuro[3,4-d][1,3]dioxol-4-yl)methanol (2G)

Compound 2F (0.27 g, 0.71 mmol) was dissolved in acetone (5 ml), a catalytic amount of p-toluenesulfonic acid was added, followed by 2,2-dimethoxypropane (0.37 g, 3.55 mmol), after the addition, the mixture was reacted at room temperature for 2 hours, after the reaction was completed, the reaction was concentrated to remove acetone, water (100 ml) was added, the mixture was extracted with dichloromethane (50ml × 2), washed with saturated brine, dried over anhydrous sodium sulfate, filtered, and concentrated, and the residue was separated and purified by silica gel column chromatography (petroleum ether: ethyl acetate (v/v) = 1 : 1) to obtain the title compound 2G (0.26 g, 94%).

LC-MS (ESI): m/z = 423.1$[M+H]^+$.

Step 7: (((((2R,3S,4R,5R)-5-((2-chloro-4-(cyclopentylamino)pyrrolo[2,1-f][1,2,4]triazin-7-yl)methyl)-3,4-dihydroxytetrahydrofuran-2-yl)methoxy)(hydroxy)phosphoryl)methyl)phosphonic acid (Compound 2)

Compound 2G (0.12 g, 0.31 mmol) was dissolved in trimethyl phosphate (3 ml), the temperature was decreased to 0°C under an ice bath, methylenebis(phosphonic dichloride) (0.23 g, 0.91 mmol) was dissolved in trimethyl phosphate (0.5 ml), and the mixture was slowly added dropwise into the reaction solution, after the addition, the mixture was naturally warmed to room temperature and reacted for 15 hours, upon completion monitored by LCMS, water (1 ml) was added, followed by 4N hydrochloric acid (1 ml), after the addition, the mixture was stirred for 5 hours, and after the reaction was

completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.5 min, to obtain the compound 2 (50 mg, 30%).

LC-MS (ESI): m/z = 539.0[M-H]-.

1H NMR (400 MHz, DMSO-d6) δ 8.36 (d, 1H), 6.98 (d, 1H),6.50 (d, 1H), 4.46-4.43 (m, 1H), 4.24-4.20 (m,1H), 4.06-3.85 (m,5H), 3.13-2.99 (m, 2H), 2.22 (t, 2H), 1.99-1.98 (m, 2H), 1.73-1.58 (m, 6H).

**Example 3:**

((((2R,3S,4R,SR)-5-((2-chloro-4-(cyclopentylamino)pyrrolo[2,1-f][1,2,4]triazin-7-yl)methyl)-3,4-dihydroxytetrahydrofuran-2-yl)methoxy)methyl)phosphonic acid (Compound 3)

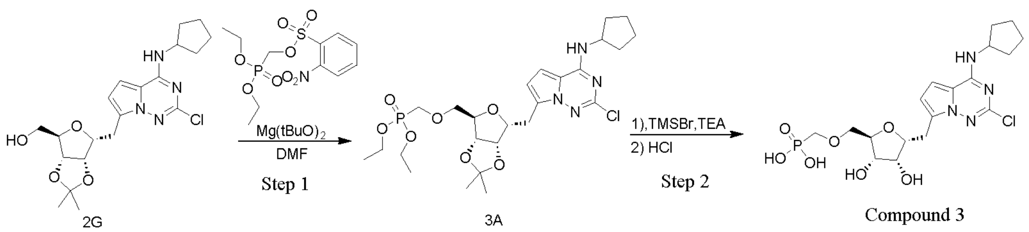


2G 3A Compound 3

Step 1: diethyl((((3aR,4R,6R,6aS)-6-((2-chloro-4-(cyclopentylamino)pyrrolo [2,1-f][1,2,4]triazin-7-yl)methyl)-2,2-dimethyltetrahydrofuro[3,4-d][1,3]dioxol-4-yl)methoxy)methyl)phosphonate (3A)

Compound 2G (0.14 g, 0.33 mmol) and methyl (diethoxyphosphoryl) 2-nitrobenzenesulfonate (0.17 g, 0.49 mmol) were dissolved in dry N,N-dimethylformamide (5 ml), the reaction system was subjected to nitrogen replacement three times, magnesium tert-butoxide (0.17 g, 0.99 mmol) was added, after the addition, the mixture was warmed to 80°C and reacted for 3 hours, after the reaction was completed, the reaction was cooled to room temperature and quenched with saturated ammonium chloride solution, then extracted with ethyl acetate (50 ml × 2), the organic layers were combined, washed with saturated brine, dried over anhydrous sodium sulfate, filtered and concentrated, and then the residue was separated and purified by column chromatography (petroleum ether : ethyl acetate (v/v) = 1 : 1-1 : 10) to obtain the compound 3A (0.15 g, 79%).

LC-MS (ESI): m/z = 573.1[M+H]+.

Step 2: ((((2R,3S,4R,5R)-5-((2-chloro-4-(cyclopentylamino)pyrrolo[2,1-f][1,2,4]triazin-7-yl)methyl)-3,4-dihydroxytetrahydrofuran-2-yl)methoxy)methyl)phosphonic acid(Compound 3)

Compound 3A (0.15 g, 0.26 mmol) was dissolved in acetonitrile (5 ml), triethylamine (0.79 g, 7.8 mmol) was added, bromotrimethylsilane (0.8 g, 5.2 mmol) was slowly added dropwise, after the addition, the mixture was reacted for 15 hours, after the reaction was completed, the mixture was filtered, the filter cake was washed with a small amount of acetonitrile, the filtrate was concentrated and 4N hydrochloric acid (1 ml) was added, after the addition, the reaction was stirred for 5 hours, and upon completion monitored by LCMS, the reaction system was directly subjected to preparative HPLC with preparative HPLC separation methods: 1. Instrument: waters 2767 (preparative liquid phase chromatographic instrument), chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: the content of mobile phase A: 5% to 50%; c. flow rate: 12 mL/min; d. elution time: 20 min, retention time: 10.2 min, to obtain the compound 3 (45 mg, 36%).

LC-MS (ESI): m/z = 475.0[M-H]-.

$^1$H NMR (400 MHz, DMSO-$d_6$) δ 8.36(d, 1H), 6.97 (d, 1H), 6.48 (d, 1H), 4.48-4.43 (m, 1H), 4.20-4.17 (m,1H), 3.91-3.80 (m,3H), 3.64-3.48 (m,4H), 3.12-2.98 (m, 2H), 1.98-1.95 (m,2H), 1.73-1.54 (m,6H).

**Example 4:**

(((((2R,3S,4R,5S)-5-((2-chloro-4-(cyclopentylamino)pyrrolo[2,1-f][1,2,4]triazin-7-yl)methyl)-3,4-dihydroxytetrahydrofuran-2-yl)methoxy)(hydroxy)phosphoryl)methyl)phosphonic acid(Compound 4)

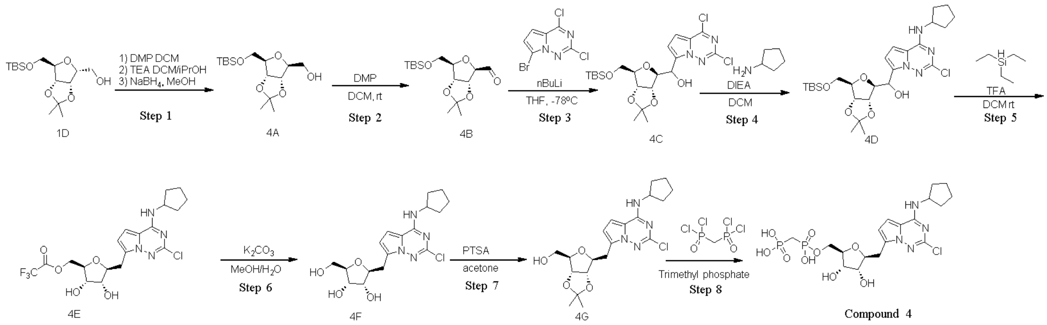


Step 1: ((3aS,4S,6R,6aR)-6-(((tert-butyldimethylsilyl)oxy)methyl)-2,2-dimethyltetrahydrofuro[3,4-d][1,3]dioxol-4-yl)methanol (4A)

Compound 1D (8.7 g, 28.32 mmol) was dissolved in dichloromethane (100 ml), Dess-Martin reagent (34.76 g, 81.96 mmol) was added in portions, after the addition, the mixture was reacted at room temperature for 2 hours, after the reaction was completed, water (50 ml) was added into the reaction solution to quench the reaction, the mixture was filtered, the filter cake was rinsed with dichloromethane, the liquid separation was conducted, the aqueous phase was extracted twice with dichloromethane (150 ml × 2), the organic layers were combined, washed with saturated brine, dried over anhydrous sodium sulfate, filtered, the filtrate was concentrated, the product was dissolved in dichloromethane (50 ml) and isopropanol (50 ml), triethylamine (8.29 g, 81.96 mmol) was added, after the addition, the mixture was stirred at room temperature for 2 days, and concentrated to remove the solvent, methanol (50 ml) was added, after the addition, sodium borohydride (2.07 g, 37.83 mmol) was added in portions, after the reaction was completed, water was added to quench the reaction, and the reaction was filtered. The filtrate was extracted with dichloromethane (150 ml × 2) twice, the organic phases were combined, washed with saturated brine, dried over anhydrous sodium sulfate, filtered and the filtrate was concentrated, and the residue was separated and purified by column chromatography (petroleum ether : ethyl acetate (v/v) = 10 : 3) to obtain the compound 4A (2.5 g, 29%).
LC-MS (ESI): m/z = 318.1[M+H]$^+$.

Step 2: (3aR,4R,6R,6aR)-6-(((tert-butyldimethylsilyl)oxy)methyl)-2,2-dimethyltetrahydrofuro[3,4-d][1,3]dioxole-4-carbaldehyde(4B)

Compound 4A (2.50 g, 7.85 mmol) was dissolved in dichloromethane (20 ml), Dess-Martin reagent (9.99 g, 23.55 mmol) was added in portions, after the addition, the mixture was reacted at room temperature for 2 hours, after the reaction was completed, water (50 ml) was added into the reaction solution to quench the reaction, the mixture was filtered, the filter cake was rinsed with dichloromethane, the liquid separation was conducted, the aqueous phase was extracted twice with dichloromethane (100 ml × 2), the organic layers were combined, washed with saturated brine, dried over anhydrous sodium sulfate, and concentrated to obtain the compound 4B (2.4 g, 96%), which was directly used in the next reaction.

Step 3: ((3aS,4S,6R,6aR)-6-(((tert-butyldimethylsilyl)oxy)methyl)-2,2-dimethyltetrahydrofuro[3,4-d][1,3]dioxol-4-yl)(2,4-dichloropyrrolo[2,1-f][1,2,4]triazin-7-yl)methanol (4C)

7-Bromo-2,4-dichloropyrrolo[2,1-f][1,2,4]triazine (2.43 g, 9.1 mmol) was dissolved in dry tetrahydrofuran (40 ml), the reaction system was subjected to nitrogen replacement three times, cooled to -78°C using an ethanol dry ice system, with the temperature was controlled at -78 °C, n-butyllithium (3.34 ml, 8.3 mmol, 2.5mol/L) was added dropwise, after the addition, the mixture was reacted at -78°C for 10 minutes, with the temperature was controlled at -78 °C, compound 4B (2.4 g, 7.6 mmol) was added dropwise, after the addition, with the temperature was maintained at -78°C, the mixture was reacted for 1 hour, after the reaction was completed, the reaction was quenched with glacial acetic acid (1.5 ml), water (30 ml) was added, the reaction was extracted with ethyl acetate (100 ml × 2), the organic layers were

combined, washed with saturated brine, dried and concentrated, and then the residue was separated and purified by silica gel column chromatography (petroleum ether : ethyl acetate (v/v) = 5 : 1) to obtain the title compound 4C (0.81g, 21%).

LC-MS (ESI): m/z = 526.1 $[M+Na]^+$; 486.1 $[M+1-H_2O]^+$.

Step 4: ((3aS,4S,6R,6aR)-6-(((tert-butyldimethylsilyl)oxy)methyl)-2,2-dimethyltetrahydrofuro[3,4-d][1,3]dioxol-4-yl)(2-chloro-4-(cyclopentylamino)pyrrolo[2,1-f][1,2,4]triazin-7-yl)methanol (4D)

Compound 4C (0.81 g, 1.61 mmol) and cyclopentylamine (0.21 g, 2.41 mmol) were dissolved in dichloromethane (10 ml), N,N-diisopropyl ethylamine (0.62 g, 4.83 mmol) was added, and then the mixture was reacted at room temperature for 3 hours, and after the reaction was completed, the reaction solution was directly concentrated, and the residue was separated and purified by silica gel column chromatography (petroleum ether : ethyl acetate (v/v) = 5 : 1) to obtain the title compound 4D (0.31 g, 35%).

LC-MS (ESI): m/z = 553.2 $[M+H]^+$.

Step 5: ((2R,3S,4R,5S)-5-((2-chloro-4-(cyclopentylamino)pyrrolo[2,1-f][1,2,4]triazin-7-yl)methyl)-3,4-dihydroxytetrahydrofuran-2-yl)methyl 2,2,2-trifluoroacetate (4E)

Compound 4D (0.3 g, 0.54 mmol) was dissolved in dichloromethane (5 ml), trifluoroacetic acid (2 ml) was added, the temperature was decreased by 0-5°C, triethylsilane (0.86 ml, 5.4 mmol) was added dropwise, and after the addition, the mixture was naturally warmed to room temperature and reacted for 16 hours, and after the reaction was completed, the mixture was directly concentrated to obtain the compound 4E (crude, 0.2 g, 77%), which was directly used in the next reaction.

LC-MS (ESI): m/z = 479.1$[M+H]^+$.

Step 6: (2S,3R,4S,5R)-2-((2-chloro-4-(cyclopentylamino)pyrrolo[2,1-f][1,2,4] triazin-7-yl)methyl)-5-(hydroxymethyl)tetrahydrofuran-3,4-diol (4F)

Compound 4E (0.2 g, 0.42 mmol) was dissolved in methanol (5 ml) and water (1 ml), potassium carbonate (58 mg, 0.42 mmol) was added, after the addition, the mixture was reacted at room temperature for 1 hour, after the reaction was completed, water was added and the mixture was extracted with dichloromethane (50 ml × 2), washed with saturated brine, dried over anhydrous sodium sulfate, filtered, and the filtrate was concentrated under reduced pressure to obtain the compound 4F (crude, 0.15 g, 93%), which was directly used in the next reaction.

LC-MS (ESI): m/z = 383.1$[M+H]^+$.

Step 7: ((3aR,4R,6S,6aS)-6-((2-chloro-4-(cyclopentylamino)pyrrolo[2,1-f][1,2,4]triazin-7-yl)methyl)-2,2-dimethyltetrahydrofuro[3,4-d][1,3]dioxol-4-yl)methanol (4G)

Compound 4F (0.15 g, 0.39 mmol) was dissolved in acetone (5 ml), a catalytic amount of p-toluenesulfonic acid was added, followed by 2,2-dimethoxypropane (0.2 g, 1.95 mmol), after the addition, the mixture was reacted at room temperature for 2 hours, after the reaction was completed, the reaction was concentrated to remove acetone, water (20 ml) was added, the mixture was extracted with dichloromethane (50 ml × 2), washed with saturated brine, dried over anhydrous sodium sulfate, filtered, and concentrated, and the residue was separated and purified by silica gel column chromatography (petroleum ether : ethyl acetate (v/v) = 1 : 1) to obtain the title compound 4G (0.11 g, 72%).

LC-MS (ESI): m/z = 423.1$[M+H]^+$.

Step 8: (((((2R,3S,4R,5S)-5-((2-chloro-4-(cyclopentylamino)pyrrolo[2,1-f][1,2,4]triazin-7-yl)methyl)-3,4-dihydroxytetrahydrofuran-2-yl)methoxy)(hydroxy)phosphoryl)methyl)phosphonic acid (Compound 4)

Compound 4G (0.050 g, 0.13 mmol) was dissolved in trimethyl phosphate (2 ml), the temperature was decreased to 0°C under an ice bath, methylenebis(phosphonic dichloride) (0.098 g, 0.39 mmol) was dissolved in trimethyl phosphate (0.5 ml), and the mixture was slowly added dropwise into the reaction solution, after the addition, the mixture was naturally warmed to room temperature and reacted for 15 hours, upon completion monitored by LCMS, water (1 ml) was added, followed by 4N hydrochloric acid (1 ml), after the addition, the mixture was stirred for 5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B:

water (containing 0.1% TFA); b. gradient elution: mobile phase A: 5% to 50%; c. flow rate: 12 mL/min; d. elution time: 20 min, retention time: 8.0 min, to obtain the compound 4 (17 mg, 24%).

LC-MS (ESI): m/z = 539.0[M-H]$^-$.

$^1$H NMR (400 MHz, DMSO-d6) δ 8.38(d, 1H), 6.98 (d, 1H), 6.51 (d, 1H), 4.48-4.43 (m, 1H), 4.02-3.89 (m, 3H), 3.82-3.76 (m, 2H), 3.70-3.68 (m, 1H), 3.08-2.90 (m, 2H), 2.23 (t, 2H), 1.99-1.98 (m, 2H), 1.73-1.58 (m, 6H).

**Example 5:**

((((2R,3S,4R,5S)-5-((2-chloro-4-(cyclopentylamino)pyrrolo[2,1-f][1,2,4]triazin-7-yl)methyl)-3,4-dihydroxytetrahydrofuran-2-yl)methoxy)methyl)phosphonic acid (Compound 5)

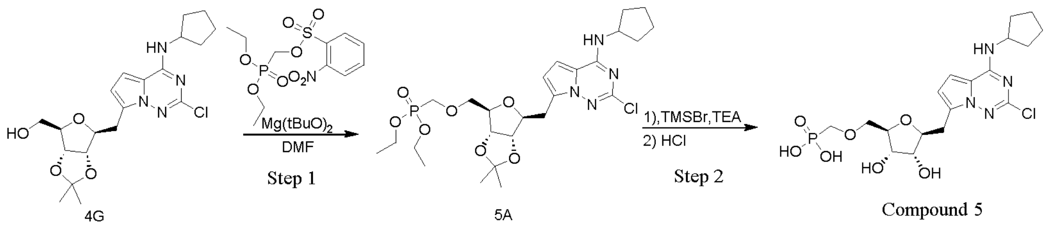


Step 1: diethyl((((3aR,4R,6S,6aS)-6-((2-chloro-4-(cyclopentylamino)pyrrolo [2,1-f][1,2,4]triazin-7-yl)methyl)-2,2-dimethyltetrahydrofuro[3,4-d][1,3]dioxol-4-yl)methoxy)methyl)phosphonate (5A)

Compound 4G (0.057 g, 0.13 mmol) and methyl (diethoxyphosphoryl) 2-nitrobenzenesulfonate (0.069 g, 0.2 mmol) were dissolved in dry N,N-dimethylformamide (2 ml), the reaction system was subjected to nitrogen replacement three times, magnesium tert-butoxide (0.066 g, 0.39 mmol) was added, after the addition, the mixture was warmed to 80°C and reacted for 3 hours, after the reaction was completed, the reaction was cooled to room temperature and quenched with saturated ammonium chloride solution, then extracted with ethyl acetate (30 ml × 2), the organic layers were combined, washed with saturated brine, dried over anhydrous sodium sulfate, filtered and concentrated, and then the residue was separated and purified by column chromatography (petroleum ether : ethyl acetate (v/v) = 1 : 1-1 : 10) to obtain the compound 5A (0.052 g, 70%).

LC-MS (ESI): m/z = 573.1[M+H]$^+$.

Step 2: ((((2R,3S,4R,5S)-5-((2-chloro-4-(cyclopentylamino)pyrrolo[2,1-f][1,2,4]triazin-7-yl)methyl)-3,4-dihydroxytetrahydrofuran-2-yl)methoxy)methyl)phosphonic acid (Compound 5)

Compound 5A (0.052 g, 0.09 mmol) was dissolved in acetonitrile (3 ml), triethylamine (0.28 g, 2.76 mmol) was added, bromotrimethylsilane (0.28 g, 1.84 mmol) was slowly added dropwise, after the addition, the mixture was reacted for 15 hours, after the reaction was completed, the mixture was filtered, the filter cake was washed with a small amount of acetonitrile, the filtrate was concentrated and 4N hydrochloric acid (1ml) was added, after the addition, the reaction was stirred for 5 hours, and upon completion monitored by LCMS, the reaction system was directly subjected to preparative HPLC with preparative HPLC separation methods: 1. Instrument: waters 2767 (preparative liquid phase chromatographic instrument), chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 9.8 min, to obtain the compound 5 (20 mg, 45%).

LC-MS (ESI): m/z = 475.0[M-H]$^-$.

$^1$H NMR (400 MHz, DMSO-d6) δ 8.37(d, 1H), 6.97 (d, 1H), 6.49 (d, 1H), 4.48-4.43 (m, 1H), 3.98-3.94 (m, 1H), 3.73-3.48 (m, 7H), 3.07-2.91 (m, 2H), 1.99-1.98 (m, 2H), 1.73-1.58 (m, 6H).

**Example 6**

(((((2R,3S,4R,5R)-5-(6-chloro-4-(((S)-1-(2-fluorophenyl)ethyl)amino)-1H-pyrazolo[3,4-b]pyridin-1-yl)-2-ethynyl-3,4-dihydroxytetrahydrofuran-2-yl)methoxy)(hydroxy)phosphoryl)methyl)phosphonic acid (Compound 6)

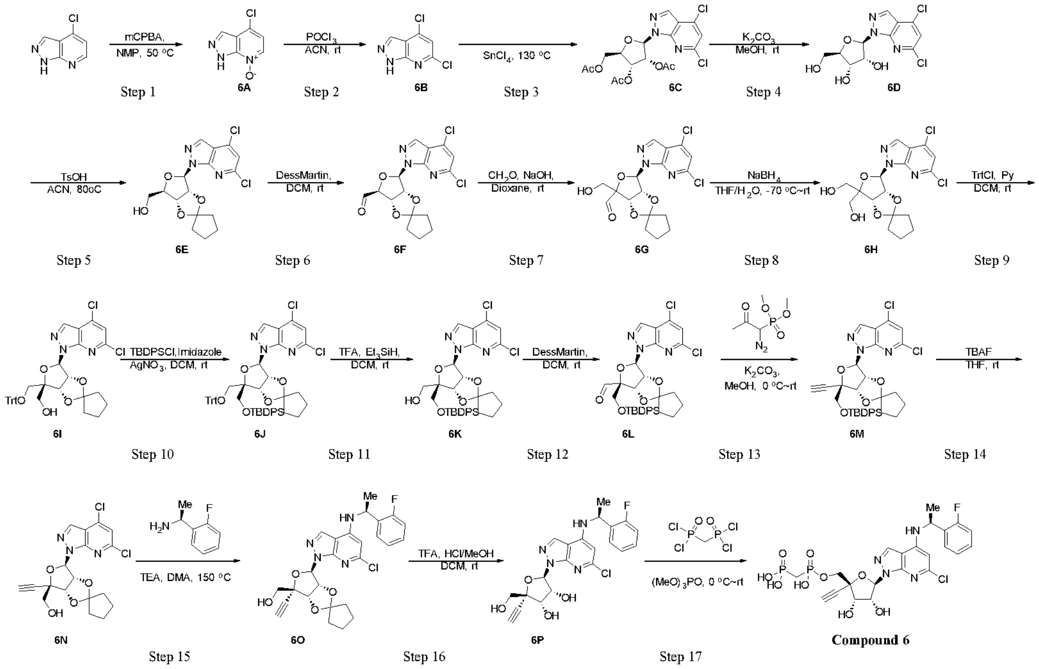


Step 1: 4-chloro-1H-pyrazolo[3,4-b]pyridine 7-oxide (6A)

4-Chloro-7-azaindazole (13.0 g, 84.65 mmol) and N-methylpyrrolidone (260 ml) were added into a flask, m-chloroperoxybenzoic acid (33.6 g, 194.69 mmol) was added, and the mixture was reacted at 50 °C overnight, and a product was generated as monitored by LCMC. Methyl tert-butyl ether (50 ml) was added, cooled, and filtered to obtain the title compound 6A (7.9 g, 55.04%).

LC-MS (ESI): m/z = 170.1 $[M+H]^+$.

Step 2: 4,6-dichloro-1H-pyrazolo[3,4-b]pyridine (6B)

6A (7.9 g, 46.59 mmol) and acetonitrile (160 ml) were added into a flask, phosphorus oxychloride (8.5 ml, 93.18 mmol) was added dropwise under an ice bath and the mixture was reacted at room temperature for 2 hours. Upon complete depletion of raw materials monitored by LCMS, a product was generated. The reaction solution was slowly poured into ice water, saturated sodium carbonate was added and dissolved to adjust the solution to pH 9, the mixture was stirred, and filtered, dichloromethane was added to dissolve the filter cake, and the mixture was washed, dried, and concentrated to obtain the title compound 6B (8.5 g, 97.0%).

LC-MS (ESI): m/z = 188.0 $[M+H]^+$.

Step 3: (2R,3R,4R,5R)-2-(acetoxymethyl)-5-(4,6-dichloro-1H-pyrazolo[3,4-b]pyridin-1-yl)tetrahydrofuran-3,4-diyl diacetate (6C)

Tetraacetyl ribose (23.7 g, 74.47 mmol) was added into a flask, heated to 90°C and stirred for 10 minutes, 6B (7.0 g, 37.23 mmol) and stannum tetrachloride (0.12 ml, 1.01 mmol) were added, warmed to 130°C and stirred under reduced pressure for 20 minutes. Then the mixture was cooled, and upon complete depletion of raw materials monitored by LCMS, a product was generated. Dichloromethane was added, and the reaction was directly passed through a column (PE : EA = 1 : 0-4 : 1), to obtain the title compound 6C (17 g, 100%).

LC-MS (ESI): m/z = 446.0 $[M+H]^+$.

Step 4: (2R,3R,4S,SR)-2-(4,6-dichloro-1H-pyrazolo[3,4-b]pyridin-1-yl)-5-(hydroxymethyl)tetrahydrofuran-3,4-diol (6D)

6C (17.0 g, 38.10 mmol) and methanol (350 ml) were added into a flask, followed by potassium carbonate (15.8 g, 114.91 mmol), and the mixture was reacted at room temperature for 1 hour. Upon complete depletion of raw materials monitored by LCMS, a product was generated. The mixture was extracted with dichloromethane, washed, dried and concentrated to directly obtain the title compound 6D (8.2 g, 67.2%).

LC-MS (ESI): m/z = 320.1 $[M+H]^+$.

Step 5: ((3a'R,4'R,6'R,6a'R)-4'-(4,6-dichloro-1H-pyrazolo[3,4-b]pyridin-1-yl) tetrahydrospiro[cyclopentane-1,2'-furo[3,4-d][1,3]dioxol]-6'-yl)methanol (6E)

6D (8.0 g, 24.99 mmol), cyclopentanone (9.5 g, 112.45 mmol), trimethyl orthoformate (8.0 g, 74.97 mmol) and acetonitrile (100 ml) were added into a flask, followed by p-toluenesulfonic acid (0.43 g, 2.5 mmol), and then the mixture was reacted at 80°C for 2 hours. Upon complete depletion of raw materials monitored by LCMS, a product was generated. The product was extracted with dichloromethane, washed, dried, concentrated and passed through a column (PE : EA = 3 : 1) to obtain the title compound 6E (7.2 g, 74.6%).

LC-MS (ESI): m/z = 386.1 $[M+H]^+$.

Step 6: (3a'R,4'R,6'S,6a'S)-4'-(4,6-dichloro-1H-pyrazolo[3,4-b]pyridin-1-yl) tetrahydrospiro[cyclopentane-1,2'-furo[3,4-d][1,3]dioxole]-6'-carbaldehyde (6F)

6E (7.2 g, 18.64 mmol) and dichloromethane (200 ml) were added into a flask, followed by Dess-Martin reagent (9.5 g, 22.37 mmol), and then the mixture was reacted at room temperature for 16 hours. Upon complete depletion of raw materials monitored by LCMS, a product was generated. The mixture was extracted with dichloromethane, washed, dried, concentrated and passed through a column (PE : EA = 3 : 1) to obtain the title compound 6F (5.4 g, 75.4%).

LC-MS (ESI): m/z = 384.1 $[M+H]^+$.

Step 7: (3a'S,6'R,6a'R)-6'-(4,6-dichloro-1H-pyrazolo[3,4-b]pyridin-1-yl)-4'-(hydroxymethyl)tetrahydrospiro[cyclopentane-1,2'-furo[3,4-d][1,3]dioxole]-4'-carbaldehyde (6G)

6F (5.4 g, 14.05 mmol) and dioxane (55 ml) were added into a flask, carbaldehyde solution (5.15 ml, 140.5 mmol) and sodium hydroxide solution (2N, 10.5 ml) were added, then the mixture was reacted at room temperature for 3 h, and upon complete depletion of raw materials monitored by LCMS, water was added, and the mixture was extracted with ethyl acetate, washed, dried, and concentrated to obtain the title compound 6G (6 gram of crude product). The reaction was directly used in the next reaction.

LC-MS (ESI): m/z = 414.2 $[M+H]^+$.

Step 8: ((3a'R,4'R,6a'S)-4'-(4,6-dichloro-1H-pyrazolo[3,4-b]pyridin-1-yl)tetrahydrospiro[cyclopentane-1,2'-furo[3,4-d][1,3]dioxole]-6',6'-diyl)dimethanol (6H)

To 6G (6.0 g, 14.48 mmol) and tetrahydrofuran/water (60 ml/6 ml), sodium borohydride (0.55 g, 14.48 mmol) was added at -78°C. The temperature was naturally warmed to room temperature and the reaction was stirred for 2 hours. Upon complete depletion of raw materials monitored by LCMS, a product was generated. The reaction solution was slowly poured into water, extracted with dichloromethane, washed, dried and concentrated to obtain the title compound 6H (4.0 g, 66.36%).

LC-MS (ESI): m/z = 416.1 $[M+H]^+$.

Step 9: ((3a'R,4'R,6'S,6a'S)-4'-(4,6-dichloro-1H-pyrazolo[3,4-b]pyridin-1-yl)-6'-((trityloxy)methyl)tetrahydrospiro[cyclopentane-1,2'-furo[3,4-d][1,3]dioxol]-6'-yl)methanol (6I)

6H (4.0 g, 9.61 mmol), dichloromethane (80 ml), and pyridine (2.33 ml, 28.83 mmol) were added into a flask, triphenylmethyl chloride (4.02 g, 14.41 mmol) was added at room temperature and the reaction was stirred for 4 hours. Upon complete depletion of raw materials monitored by TLC (PE : EA = 3 : 1), a product was generated. The reaction solution was slowly poured into water, extracted with dichloromethane, washed, dried, concentrated and passed through a column (PE : EA = 1 : 0-3 : 1) to obtain the title compound 6I (4.1 g, 64.8%).

Step 10: 1-((3a'S,4'R,6'R,6a'R)-4'-(((tert-butyldiphenylsilyl)oxy)methyl)-4'-((trityloxy)methyl)tetrahydrospiro[cyclopentane-1,2'-furo[3,4-d] [1,3]dioxol]-6'-yl)-4,6-dichloro-1H-pyrazolo[3,4-b]pyridine (6J)

6I (4.1 g, 6.23 mmol), dichloromethane (80 ml), imidazole (1.27 g, 18.69 mmol), and silver nitrate (3.17 g, 18.69 mmol) were added into a flask, and tert-butylchlorodiphenylsilane (3.42 g, 12.46 mmol) was added dropwise at room temperature and the reaction was stirred for 16 hours. Upon complete depletion of raw materials monitored by TLC, a product was generated. The reaction solution was slowly poured into water, extracted with dichloromethane, washed, dried, concentrated and passed through a column (PE : EA = 1 : 0-5 : 1) to obtain the title compound 6J (4.8 g, 85.9%).

Step 11: ((3a'S,4'R,6'R,6a'R)-4'-(((tert-butyldiphenylsilyl)oxy)methyl)-6'-(4,6-dichloro-1H-pyrazolo[3,4-b]pyridin-1-yl)tetrahydrospiro[cyclopentane-1,2'-furo[3,4-d][1,3]dioxol]-4'-yl)methanol (6K)

6J (4.0g, 4.46 mmol) and dichloromethane (40 ml) were added into a flask, followed by trifluoroacetic acid (2 ml, 26.76 mmol) and triethylsilane (1 ml, 6.69 mmol), and then the mixture was reacted at room temperature for 1 hour. Upon complete depletion of raw materials monitored by LCMS, a product was generated. The product was extracted with dichloromethane, washed, dried, concentrated and passed through a column (PE : EA = 1 : 0-2 : 1) to obtain the title compound 6K (2.9 g, 99.3%).

Step 12: (3a'S,4'R,6'R,6a'R)-4'-(((tert-butyldiphenylsilyl)oxy)methyl)-6'-(4,6-dichloro-1H-pyrazolo[3,4-b]pyridin-1-yl)tetrahydrospiro[cyclopentane-1,2'-furo[3,4-d][1,3]dioxole]-4'-carbaldehyde (6L)

6K (2.9 g, 4.43 mmol) and dichloromethane (60 ml) were added into a flask, followed by Dess-Martin reagent (4.7 g, 11.07 mmol), and then the mixture was reacted at room temperature for 4 hours. Upon complete depletion of raw materials monitored by LCMS, a product was generated. The product was extracted with dichloromethane, washed, dried, concentrated and passed through a column (PE : EA = 0 : 1-3 : 1) to obtain the title compound 6L (2.8 g, 96.8%).

Step 13: 1-((3a'S,4'R,6'R,6a'R)-4'-(((tert-butyldiphenylsilyl)oxy)methyl)-4'-ethynyltetrahydrospiro[cyclopentane-1,2'-furo[3,4-d][1,3]dioxol]-6'-yl)-4,6-dichloro-1H-pyrazolo[3,4-b]pyridine (6M)

6L (1.6 g, 2.45 mmol) and methanol (20 ml) were added into a flask, dimethyl (1-diazo-2-oxopropyl)phosphonate (1.43 g, 7.35 mmol) in methanol (5 ml) was added dropwise under an ice bath, and then reacted at 0 °C for 4 hours. Upon complete depletion of raw materials monitored by LCMS, a product was generated. The product was extracted with dichloromethane, washed, dried, concentrated and passed through a column (PE : EA = 1 : 0-3 : 1) to obtain the title compound 6M (1.1 g, 66.1%).

Step 14: ((3a'R,4'R,6'R,6a'S)-4'-(4,6-dichloro-1H-pyrazolo[3,4-b]pyridin-1-yl)-6'-ethynyltetrahydrospiro[cyclopentane-1,2'-furo[3,4-d][1,3]dioxol]-6'-yl)methanol (6N)

6M (1.0 g, 1.54 mmol) and tetrahydrofuran (10 ml) were added into a flask, followed by tetrabutylammonium fluoride (1.2 g, 4.62 mmol), and then the mixture was reacted at room temperature for 2 hours. Upon complete depletion of raw materials monitored by LCMS, a product was generated. The product was extracted with dichloromethane, washed, dried, concentrated and passed through a column (PE : EA = 0 : 1-1 : 1) to obtain the title compound 6N (0.27 g, 42.7%).

LC-MS (ESI): m/z = 410.1 $[M+H]^+$.

Step 15: ((3a'R,4'R,6'R,6a'S)-4'-(6-chloro-4-(((S)-1-(2-fluorophenyl)ethyl) amino)-1H-pyrazolo[3,4-b]pyridin-1-yl)-6'-ethynyltetrahydrospiro[cyclopentane-1,2'-furo[3,4-d][1,3]dioxol]-6'-yl)methanol (6O)

6N (0.27 g, 0.66 mmol) and N,N-dimethyl acetamide (10 ml) were added into a flask, followed by (S)-1-(2-fluorophenyl)ethylamine (0.28 g, 1.98 mmol) and triethylamine (0.33 g, 3.30 mmol), and then the mixture was reacted at 150°C for 4 hours. Upon complete depletion of raw materials monitored by LCMS, a product was generated. The product was extracted with ethyl acetate, washed, dried, concentrated and passed through a column (PE : EA = 1 : 0-0 : 1) to obtain the title compound 6O (0.25 g, 73.8%).

LC-MS (ESI): m/z = 513.1 $[M+H]^+$.

Step 16: (2R,3S,4R,SR)-5-(6-chloro-4-(((S)-1-(2-fluorophenyl)ethyl)amino)-1H-pyrazolo[3,4-b]pyridin-1-yl)-2-ethynyl-2-(hydroxymethyl)tetrahydrofuran-3,4-diol (6P)

6O (0.12 g, 0.23 mmol) and dichloromethane (10 ml) were added into a flask, and trifluoroacetic acid (0.3 ml) and hydrogen chloride methanol (0.3 ml) were added at room temperature. The reaction was stirred and reacted at room temperature for 2 hours. Upon complete depletion of raw materials monitored by LCMS, a product was generated. Water was added, sodium carbonate solution was added to adjust the solution to pH 10, the mixture was extracted with dichloromethane, washed, dried, and concentrated to obtain the title compound 6P (0.1 g, 97.3%).

LC-MS (ESI): m/z = 447.1 $[M+H]^+$.

Step 17: (((((2R,3S,4R,5R)-5-(6-chloro-4-(((S)-1-(2-fluorophenyl)ethyl)amino) -1H-pyrazolo[3,4-b]pyridin-1-yl)-2-ethynyl-3,4-dihydroxytetrahydrofuran-2-yl)methoxy)(hydroxy)phosphoryl)methyl)phosphonic acid (Compound 6)

6P (100 mg, 0.22 mmol) and trimethyl phosphate (3ml) were added into a flask, and methylenebis(phosphonic dichloride) (270 mg, 1.1 mmol) was added under an ice bath. The temperature was naturally warmed to room temperature and the reaction was stirred for 3 hours. Upon complete depletion of raw materials monitored by LCMS, a product was generated. 1 ml of water was added and the solution was stirred for 10 minutes, and the reaction solution was subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.5 min, and the preparative liquid was concentrated and lyophilized to obtain the compound 6 (50 mg, 37.6%).

$^1H$ NMR (400 MHz, DMSO-$d_6$) δ 8.39 (s, 1H), 8.23 (d, 1H), 7.40 - 7.38 (m, 1H), 7.33 - 7.30 (m, 1H), 7.24 - 7.15 (m, 2H), 6.15 (d, 1H), 6.02 (s, 1H), 5.07 (s, 1H), 4.77 (t, 1H), 4.32 (d, 1H), 4.09 - 4.05 (m, 1H), 3.92 - 3.88 (m, 1H), 3.53 (s, 1H), 2.21 (t, 2H), 1.58 (d, 3H).

LC-MS (ESI): m/z = 605.1 $[M+H]^+$.

**Example 7**

(((((2R,3S,4S,5R)-4-azido-5-(6-chloro-4-(((S)-1-(2-fluorophenyl)ethyl)amino)-1H-pyrazolo[3,4-b]pyridin-1-yl)-4-hydroxytetrahydrofuran-2-yl)methoxy) (hydroxy)phosphoryl)methyl)phosphonic acid (Compound 7)

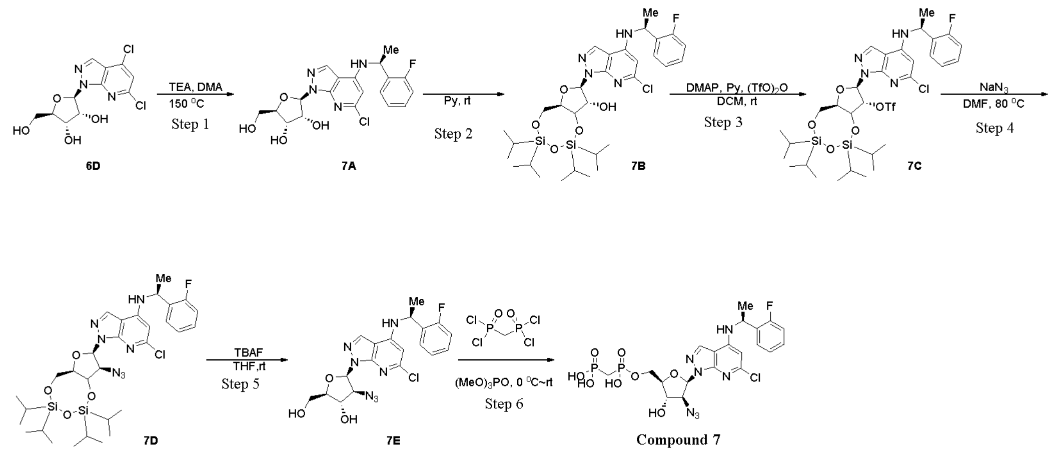


Step 1: (2R,3R,4S,5R)-2-(6-chloro-4-(((S)-1-(2-fluorophenyl)ethyl)amino)-1H -pyrazolo[3,4-b]pyridin-1-yl)-5-(hydroxymethyl)tetrahydrofuran-3,4-diol (7A)

6D (6.2 g, 19.37 mmol) and N,N-dimethyl acetamide (80 ml) were added into a flask, followed by (S)-1-(2-fluorophenyl)ethylamine (6.74 g, 48.43 mmol) and triethylamine (9.8 g, 96.85 mmol), and then the mixture was reacted at 150°C for 4 hours. Upon complete depletion of raw materials monitored by LCMS, a product was generated. The product was extracted with ethyl acetate, washed, dried, concentrated and passed through a column (PE : EA = 1 : 0-0 : 1) to obtain the title compound 7A (5.1 g, 61.1%).

LC-MS (ESI): m/z = 423.2 $[M+H]^+$.

Step 2: (6aR,8R,9R)-8-(6-chloro-4-(((S)-1-(2-fluorophenyl)ethyl)amino)-1H-pyrazolo[3,4-b]pyridin-1-yl)-2,2,4,4-tetraisopropyltetrahydro-6H-furo[3,2-f][1,3,5,2,4]trioxadisilocin-9-ol (7B)

7A (5.0 g, 11.82 mmol) and pyridine (50 ml) were added into a flask, 1,3-dichloro-1,1,3,3-tetraisopropyldisiloxane (4.1 g, 13.00 mmol) was added dropwise at room temperature, and then reacted at room temperature for 1 hour. Upon complete depletion of raw materials monitored by TLC, a product was generated. The product was extracted with dichloromethane, washed, dried, concentrated and passed through a column (PE : EA = 1 : 0-3 : 1) to obtain the title

compound 7B (7.8 g, 99.2%).

$^1H$ NMR (400 MHz, $cDCl_3$) δ 7.90 (s, 1H), 7.31-7.24(m, 2H), 7.18-7.07(m, 2H), 6.42 (s, 1H), 6.01 (s, 1H), 5.30 (s, 1H), 5.08-5.04 (m, 2H), 4.62 (t, 1H), 4.09-4.00 (m, 1H), 3.97-3.94 (m, 2H), 1.67 (d, 3H), 1.20-0.88 (m, 28H).

Step 3: (6aR,8R,9R)-8-(6-chloro-4-(((S)-1-(2-fluorophenyl)ethyl)amino)-1H-pyrazolo[3,4-b]pyridin-1-yl)-2,2,4,4-tetraisopropyltetrahydro-6H-furo[3,2-f][1,3,5,2,4]trioxadisilocin-9-yl trifluoromethanesulfonate (7C)

7B (2.0 g, 3.01 mmol), pyridine (2.14 g, 27.09 mmol), 4-dimethylaminopyridine (0.44 g, 3.61 mmol) and dichloromethane (20 ml) were added into a flask, trifluoromethanesulfonic anhydride (1.7 g, 6.02 mmol) was added dropwise at room temperature, and then the mixture was reacted at room temperature for 2 hours. Upon complete depletion of raw materials monitored by TLC (PE : EA = 10 : 1), a product was generated. The product was extracted with dichloromethane, washed, dried, concentrated and passed through a column (PE : EA = 1 : 0-5 : 1) to obtain the title compound 7C (1.5 g, 62.5%).

$^1H$ NMR (400 MHz, $CDCl_3$) δ 7.89 (s, 1H), 7.30-7.24(m, 2H), 7.13-7.07(m, 2H), 6.59 (s, 1H), 6.13 (s, 1H), 5.70 (s, 1H), 5.28-5.26 (m, 2H), 5.02 (t, 1H), 4.08-4.05 (m, 1H), 3.99-3.95 (m, 2H), 1.67 (d, 3H), 1.26-0.99 (m, 28H).

Step 4: 1-((6aR,8R,9S)-9-azido-2,2,4,4-tetraisopropyltetrahydro-6H-furo[3,2-f][1,3,5,2,4]trioxadisilocin-8-yl)-6-chloro-N-((S)-1-(2-fluorophenyl)ethyl)-1H-pyrazolo[3,4-b]pyridin-4-amine (7D)

7C (1.5 g, 1.88 mmol) and N,N-dimethylformamide (20 ml) were added into a flask, followed by sodium azide (0.37 g, 5.64 mmol), and then the mixture was reacted at 80 °C for 8 hours. Upon complete depletion of raw materials monitored by LCMS, a product was generated. The product was extracted with dichloromethane, washed, dried, concentrated and passed through a column (PE : EA = 1 : 0-5 : 1) to obtain the title compound 7D (0.45 g, 34.7%).

$^1H$ NMR (400 MHz, $CDCl_3$) δ 7.93 (s, 1H), 7.31-7.24(m, 2H), 7.13-7.07(m, 2H), 6.65 (d, 1H), 6.10 (s, 1H), 5.29-5.26 (m, 2H), 5.03 (t, 1H), 4.40 (t, 1H), 4.14-4.00 (m, 1H), 3.99-3.89 (m, 2H), 1.66 (d, 3H), 1.42-0.92 (m, 28H).

Step 5: (2R,3S,4S,5R)-4-azido-5-(6-chloro-4-(((S)-1-(2-fluorophenyl)ethyl) amino)-1H-pyrazolo[3,4-b]pyridin-1-yl)-2-(hydroxymethyl)tetrahydrofuran-3-ol (7E)

7D (450 mg, 0.65 mmol) and tetrahydrofuran (10 ml) were added into a flask, followed by tetrabutylammonium fluoride (0.51 g, 1.95 mmol), and then the mixture was reacted at room temperature for 4 hours. Upon complete depletion of raw materials monitored by LCMS, a product was generated. The product was extracted with dichloromethane, washed, dried, concentrated and passed through a column (PE : EA = 1 : 0-5 : 1) to obtain the title compound 7E (160 mg, 55.0%).

LC-MS (ESI): m/z = 448.2 $[M+H]^+$.

Step 6: (((((2R,3S,4S,SR)-4-azido-5-(6-chloro-4-(((S)-1-(2-fluorophenyl)ethyl) amino)-1H-pyrazolo[3,4-b]pyridin-1-yl)-4-hydroxytetrahydrofuran-2-yl)methoxy)(hydroxy)phosphoryl)methyl)phosphonic acid (Compound 7)

7E (70 mg, 0.16 mmol) and trimethyl phosphate (3 ml) were added into a flask, and methylenebis(phosphonic dichloride) (200 mg, 0.8 mmol) was added under an ice bath. The temperature was naturally warmed to room temperature and the reaction was stirred for 3 hours. Upon complete depletion of raw materials monitored by LCMS, a product was generated. 1 ml of water was added and the solution was stirred for 10 minutes, and the reaction solution was subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.5 min, and the preparative liquid was concentrated and lyophilized to obtain the title compound 7 (26 mg, 26.8%).

$^1H$ NMR (400 MHz, DMSO-$d_6$) δ 8.39 (s, 1H), 8.24 (d, 1H), 7.44 (t, 1H), 7.43 - 7.33 (m, 1H), 7.26 - 7.18 (m, 2H), 6.51 (d, 1H), 6.02 (s, 1H), 5.07 (s, 1H), 4.70 (t, 1H), 4.58 - 4.55 (m, 1H), 4.31 - 4.20 (m, 1H), 4.07 - 3.98 (m, 2H), 2.18 (t, 2H), 1.59 (d, 3H).

LC-MS (ESI): m/z = 606.2 $[M+H]^+$.

**Example 8**

((((2R,3S,4R,SR)-5-(1-(2-chloro-4-(cyclopentylamino)pyrrolo[2,1-f][1,2,4] triazin-7-yl)ethyl)-3,4-dihydroxytetrahydrofuran-2-yl)methoxy)methyl)phosphonic acid (Compound 8)

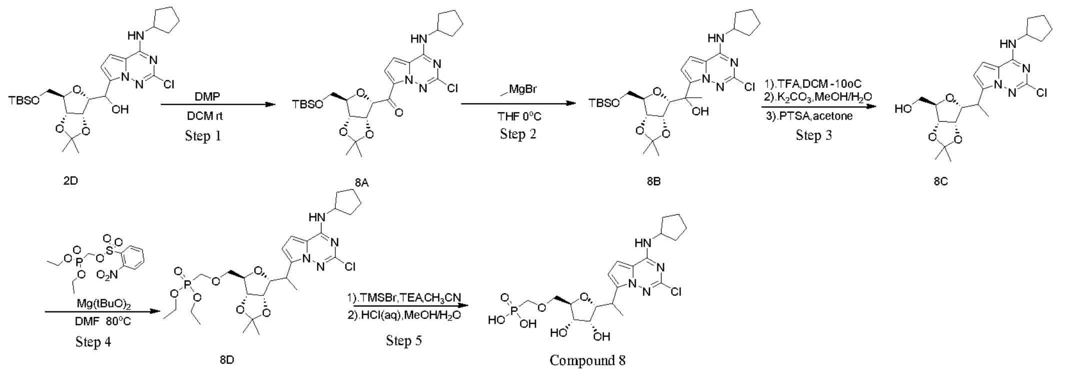

2D 8A 8B 8C 8D Compound 8

Step 1: ((3aR,4S,6R,6aR)-6-(((tert-butyldimethylsilyl)oxy)methyl)-2,2-dimethyltetrahydrofuro[3,4-d][1,3]dioxol-4-yl)(2-chloro-4-(cyclopentylamino) pyrrolo[2,1-f][1,2,4]triazin-7-yl)methanone (8A)

Compound 2D (0.6 g, 1.08 mmol) was dissolved in dichloromethane (10 ml), Dess-Martin reagent (0.69 g, 1.62 mmol) was added in portions, after the addition, the mixture was reacted at room temperature for 2 hours, after the reaction was completed, water (10 ml) was added into the reaction solution to quench the reaction, filtered, the filter cake was rinsed with dichloromethane, the liquid separation was conducted, the aqueous phase was extracted twice with dichloromethane (50 ml × 2), the organic layers were combined, washed with saturated brine, dried over anhydrous sodium sulfate, filtered, the filtrate was concentrated, and the residue was separated and purified by silica gel column chromatography (petroleum ether : ethyl acetate (v/v) = 4 : 1) to obtain the title compound 8A (0.25 g, 42%).

LC-MS (ESI): m/z = 551.2 $[M+H]^+$.

Step 2: 1-((3aR,4S,6R,6aR)-6-(((tert-butyldimethylsilyl)oxy)methyl)-2,2-dimethyltetrahydrofuro[3,4-d][1,3]dioxol-4-yl)-1-(2-chloro-4-(cyclopentylamino) pyrrolo[2,1-f][1,2,4]triazin-7-yl)ethan-1-ol (8B)

Under nitrogen protection, compound 8A (0.25 g, 0.45 mmol) was dissolved in tetrahydrofuran (5 ml), the temperature was cooled to 0°C, methylmagnesium bromide solution (0.45 ml, 1.35 mmol, 3.0 mol/L) was slowly added dropwise, after the addition, the mixture was reacted at room temperature for 2 hours, after the reaction was completed, saturated ammonium chloride (10 ml) was added into the reaction solution to quench the reaction, the reaction was extracted with dichloromethane (50 ml × 2) twice, the organic layers were combined, washed with saturated brine, dried over anhydrous sodium sulfate, filtered, the filtrate was concentrated, and then the residue was separated and purified by silica gel column chromatography (petroleum ether : ethyl acetate (v/v) = 4 : 1) to obtain the title compound 8B (crude, 0.25 g, 98%), which was directly used in the next step without purification.

LC-MS (ESI): m/z = 567.2 $[M+H]^+$.

Step 3: ((3aR,4R,6R,6aS)-6-(1-(2-chloro-4-(cyclopentylamino)pyrrolo[2,1-f][1,2,4]triazin-7-yl)ethyl)-2,2-dimethyltetrahydrofuro[3,4-d][1,3]dioxol-4-yl)methanol (8C)

Compound 8B (0.21 g, 0.37 mmol) was dissolved in dichloromethane (5 ml), triethylsilane (0.43 g, 3.7 mmol) was added, after the addition, the temperature was cooled to -10°C, trifluoroacetic acid (0.5 ml) was slowly added dropwise, and after the addition, the mixture was naturally warmed to room temperature and reacted for 16 hours. After the reaction was completed, the mixture was directly concentrated to obtain a product, which was dissolved in a mixed solvent of methanol (5 ml) and water (1 ml), potassium carbonate (88 mg, 0.64 mmol) was added, and after the addition, the mixture was reacted at room temperature for 1 hour. After the reaction was completed, water was added, the mixture was extracted with dichloromethane (50 ml × 2), washed with saturated brine, dried over anhydrous sodium sulfate, filtered, and the filtrate was concentrated under reduced pressure to obtain a product, which was dissolved in acetone (5 ml), a catalytic amount of p-toluenesulfonic acid was added, followed by 2,2-dimethoxypropane (0.15 g, 1.4 mmol), after the addition, the mixture was reacted at room temperature for 2 hours, after the reaction was completed, the reaction

was concentrated to remove acetone, water (20 ml) was added, extracted with dichloromethane (50 ml × 2), washed with saturated brine, dried over anhydrous sodium sulfate, filtered, and the filtrate was concentrated, and the residue was separated and purified by silica gel column chromatography (petroleum ether : ethyl acetate (v/v) = 4 : 1-2 : 1) to obtain the title compound 8C (0.12 g, 74%).

LC-MS (ESI): m/z = 437.2 $[M+H]^+$.

Step 4: diethyl((((3aR,4R,6R,6aS)-6-(1-(2-chloro-4-(cyclopentylamino) pyrrolo[2,1-f][1,2,4]triazin-7-yl)ethyl)-2,2-dimethyltetrahydrofuro[3,4-d][1,3]dioxol-4-yl)methoxy)methyl)phosphonate (8D)

Compound 8C (0.12 g, 0.27 mmol) and methyl (diethoxyphosphoryl) 2-nitrobenzenesulfonate (0.29 g, 0.81 mmol) were dissolved in dry N,N-dimethylformamide (5 ml), the reaction system was subjected to nitrogen replacement three times, magnesium tert-butoxide (0.23 g, 1.35 mmol) was added, after the addition, the mixture was warmed to 80°C and reacted for 15 hours, after the reaction was completed, the reaction was cooled to room temperature and quenched with saturated ammonium chloride solution, water (20 ml) was added, then extracted with ethyl acetate (50 ml × 2), the organic layers were combined, washed with saturated brine, dried over anhydrous sodium sulfate, filtered, the filtrate was concentrated, and then the residue was separated and purified by silica gel column chromatography (petroleum ether : ethyl acetate (v/v) = 1 : 1-1 : 9) to obtain the title compound 8D (0.1g, 63%).

LC-MS (ESI): m/z = 587.2 $[M+H]^+$.

Step 5: ((((2R,3S,4R,5R)-5-(1-(2-chloro-4-(cyclopentylamino)pyrrolo[2,1-f] [1,2,4]triazin-7-yl)ethyl)-3,4-dihydroxytetrahydrofuran-2-yl)methoxy)methyl) phosphonic acid (Compound 8)

Compound 8D (0.1 g, 0.17 mmol) was dissolved in acetonitrile (5 ml), triethylamine (0.52 g, 5.1 mmol) was added, bromotrimethylsilane (0.52 g, 3.4 mmol) was slowly added dropwise, after the addition, the mixture was reacted for 15 hours, after the reaction was completed, the mixture was filtered, the filter cake was washed with a small amount of acetonitrile, the filtrate was concentrated and 4N hydrochloric acid (1 ml) was added, after the addition, the reaction was stirred for 5 hours, and upon completion monitored by LCMS, the reaction system was directly subjected to preparative HPLC with preparative HPLC separation methods: 1. Instrument: waters 2767 (preparative liquid phase chromatographic instrument), chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 10.8 min, to obtain the compound 8 (46 mg, 55%).

LC-MS (ESI): m/z = 489.1 $[M-H]^-$.

$^1H$ NMR (400 MHz, DMSO-$d_6$) δ 8.30 (d, 1H), 6.95 (d, 1H), 6.43 (d, 1H), 4.48-4.43 (m, 1H), 4.21-4.18 (m, 1H), 3.98-3.96 (m, 2H), 3.73-3.44 (m, 6H), 2.01-1.98 (m, 2H), 1.73-1.58 (m, 6H), 1.11 (d, 3H).

**Example 9**

(((((2S,4R,5R)-5-(6-chloro-4-(((S)-1-(2-fluorophenyl)ethyl)amino)-1H-pyrazolo[3,4-b]pyridin-1-yl)-4-hydroxy-3-methyleneteтrahydrofuran-2-yl)methoxy)(hydroxy)phosphoryl)methyl)phosphonic acid (Compound 9)

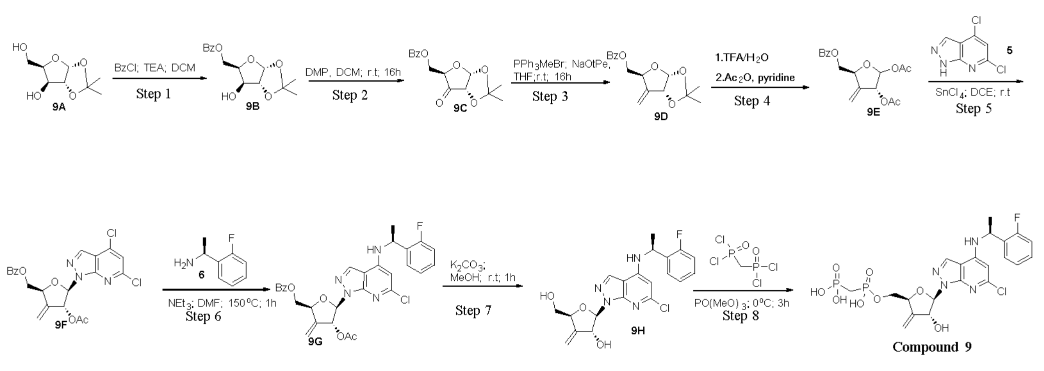

Step 1: ((3aR,5R,6S,6aR)-6-hydroxy-2,2-dimethyltetrahydrofuro[2,3-d][1,3] dioxol-5-yl)methyl benzoate (9B)

Compound 9A (20 g, 105.16 mmol) and triethylamine (31.92 g, 315.48 mmol) were dissolved in dichloromethane (150 ml), benzoyl chloride (12.22 mL, 105.16 mmol) was added in portions under an ice-water bath, after the addition, the mixture was reacted at room temperature for 2 hours, after the reaction was completed, water (50 ml) was added into the reaction solution to quench the reaction, the aqueous phase was extracted with dichloromethane (150 ml × 2) twice, the organic layers were combined, washed with saturated brine, dried over anhydrous sodium sulfate, filtered, and concentrated, and then the residue was separated and purified by column chromatography (petroleum ether : ethyl acetate (v/v) = 2 : 1) to obtain the compound 9B (22 g, 71%).

$^1H$ NMR (400 MHz, $CDCl_3$) δ 8.09 - 8.01 (m, 2H), 7.60 (t, 1H), 7.46 (t, 2H), 5.96 (d, 1H), 4.81 (dd, 1H), 4.60 (d, 1H), 4.42 - 4.32 (m, 2H), 4.18 (d, 1H), 1.51 (s, 3H), 1.32 (s, 3H).

Step 2: ((3aR,5R,6aS)-2,2-dimethyl-6-oxotetrahydrofuro[2,3-d][1,3]dioxol-5-yl)methyl benzoate (9C)

Compound 9B (22 g, 74.75 mmol) was dissolved in dichloromethane (200 ml), Dess-Martin reagent (63.4 g, 149.5 mmol) was added in portions, after the addition, the mixture was reacted at room temperature for 2 hours, after the reaction was completed, water (200 ml) was added into the reaction solution to quench the reaction, filtered, the filter cake was rinsed with dichloromethane, the liquid separation was conducted, the aqueous phase was extracted twice with dichloromethane (100 ml × 2), the organic layers were combined, washed with saturated brine, dried over anhydrous sodium sulfate, and concentrated to obtain the compound 9C (16 g, 73%), which was directly used in the next reaction.

Step 3: ((3aR,5S,6aR)-2,2-dimethyl-6-methylenetetrahydrofuro[2,3-d][1,3] dioxol-5-yl)methyl benzoate (9D)

Methyltriphenylphosphonium bromide (55 g, 153.96 mmol) was dissolved in dry tetrahydrofuran (500 ml), sodium tert-pentoxide (16.96 g, 153.96 mmol) was added under an ice-water bath, after the addition, the mixture was reacted at 0°C for 30 minutes, compound 9C (15 g, 7.6 mmol) was added at 0°C in portions, after the addition, the mixture was reacted at room temperature for 2 hours, after the reaction was completed, the reaction was quenched with saturated ammonium chloride (200 ml), water (300 ml) was added, the mixture was extracted with ethyl acetate (300 ml × 2), the organic layers were combined, washed with saturated brine, dried and concentrated, and then the residue was separated and purified by silica gel column chromatography (petroleum ether : ethyl acetate (v/v) = 5 : 1) to obtain the title compound 9D (7.9 g, 53%).

Step 4: (3R,5S)-5-((benzoyloxy)methyl)-4-methylenetetrahydrofuran-2,3-diyl diacetate (9E)

Compound 9D (3.5 g, 12.06 mmol) was dissolved in trifluoroacetic acid (10 mL) and water (5 mL), after the addition, the mixture was reacted at room temperature for 3 hours, after the reaction was completed, the mixture was concentrated to remove trifluoroacetic acid, the aqueous phase was extracted with ethyl acetate (50 mL × 2), the organic phases were combined and concentrated, then acetic anhydride (5 mL) and pyridine (15 mL) were added, the mixture was reacted at room temperature overnight, the reaction solution was directly concentrated, and the residue was separated and purified by silica gel column chromatography (petroleum ether : ethyl acetate (v/v) = 5 : 1) to obtain the title compound 9E (0.8 g, 20%).

$^1H$ NMR (400 MHz, $CDCl_3$) δ 8.07 (dd, 2H), 7.61 - 7.52 (m, 1H), 7.49 - 7.40 (m, 2H), 6.24 (s, 1H), 5.74 - 5.69 (m, 1H), 5.56 (s, 1H), 5.48 (s, 1H), 5.09 (dd, 1H), 4.52 (dd, 1H), 4.43 (dd, 1H), 2.11 (s, 3H), 2.01 (s, 3H).

Step 5: ((2S,4R,5R)-4-acetoxy-5-(4,6-dichloro-1H-pyrazolo[3,4-b]pyridin-1-yl)-3-methylenetetrahydrofuran-2-yl)methyl benzoate (9F)

Compound 9E (0.7 g, 2.09 mmol) was added into a 50 mL reaction flask, heated to 90°C, 4,6-dichloro-1H-pyrazolo[3,4-b]pyridine (0.39 g, 2.09 mmol) was added, after the addition, two drops of stannum tetrachloride were added dropwise, then warmed to 130°C and reacted under reduced pressure for 30 minutes, cooled to room temperature, and the residue was separated and purified by silica gel column chromatography (petroleum ether : ethyl acetate (v/v) = 10 : 1-5 : 1) to obtain the title compound 9F (0.8 g, 83%).

LC-MS (ESI): m/z = 462.0$[M+H]^+$.

Step 6: ((2S,4R,5R)-4-acetoxy-5-(6-chloro-4-(((S)-1-(2-fluorophenyl)ethyl) amino)-1H-pyrazolo[3,4-b]pyridin-1-yl)-3-methylenetetrahydrofuran-2-yl)methyl benzoate (9G)

Compound 9F (0.70 g, 1.51 mmol) was dissolved in DMF (5 ml), triethylamine (0.46 g, 4.53 mmol) and (S)-1-(2-fluorophenyl)ethyl-1-amine (0.40 g, 3.02 mmol) were added, after the addition, the mixture was reacted at 150°C for 1 hour, after the reaction was completed, water was added, the mixture was extracted with ethyl acetate (50 ml × 2), washed with saturated brine, dried over anhydrous sodium sulfate, filtered, and the filtrate was concentrated under reduced pressure, and the crude was separated and purified by silica gel column chromatography (petroleum ether : ethyl acetate (v/v) = 5 : 1-1 : 1) to obtain the title compound 9G (0.3 g, 35%).

LC-MS (ESI): m/z = 565.2[M+H]$^+$.

Step 7: (2R,3R,SS)-2-(6-chloro-4-(((S)-1-(2-fluorophenyl)ethyl)amino)-1H-pyrazolo[3,4-b]pyridin-1-yl)-5-(hydroxymethyl)-4-methylenetetrahydrofuran-3-ol (9H)

Compound 9G (0.25 g, 0.44 mmol) was dissolved in methanol (5 ml), potassium carbonate (0.24 g, 1.76 mmol) was added, after the addition, the mixture was reacted at room temperature for 2 hours, water (20 ml) was added, solids were precipitated, filtered, the filter cake was washed with water (2 mL) once, and the filter cake was azeotropically dried by distilling off toluene and water, and spun to dryness under reduced pressure to obtain the title compound 9H (0.16 g, 86%).

LC-MS (ESI): m/z = 419.2[M+H]$^+$.

Step 8: (((((2S,4R,5R)-5-(6-chloro-4-(((S)-1-(2-fluorophenyl)ethyl)amino)-1H-pyrazolo[3,4-b]pyridin-1-yl)-4-hydroxy-3-methylenetetrahydrofuran-2-yl)methoxy)(hydroxy)phosphoryl)methyl)phosphonic acid (Compound 9)

Compound 9H (0.080 g, 0.19 mmol) was dissolved in trimethyl phosphate (2 ml), the temperature was decreased to 0°C under an ice bath, methylenebis(phosphonic dichloride) (0.14 g, 0.57 mmol) was dissolved in trimethyl phosphate (0.5 ml), and slowly added dropwise into the reaction solution, after the addition, the mixture was reacted at 0°C for 3 hours, upon completion monitored by LCMS, water (1 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.0 min, to obtain the compound 9 (50 mg, 46%).

LC-MS (ESI): m/z = 575.0[M-H]$^-$.

$^1$H NMR (400 MHz, MeOD) δ 8.27 (s, 1H), 7.37 (t, 1H), 7.34 - 7.24 (m, 1H), 7.13 (dd, 2H), 6.14 - 6.02 (m, 2H), 5.44 (t, 1H), 5.39 - 5.28 (m, 2H), 5.07 (d, 1H), 4.90 (s, 1H), 4.30 - 4.21 (m, 1H), 4.17 (dd, 1H), 2.43 (t, 2H), 1.64 (d, 3H).

**Example 10**

(((((2S,3S,4R,5R)-3-azido-5-(6-chloro-4-(((S)-1-(2-fluorophenyl)ethyl)amino)-1H-pyrazolo[3,4-b]pyridin-1-yl)-4-hydroxytetrahydrofuran-2-yl)methoxy) (hydroxy)phosphoryl)methyl)phosphonic acid (Compound 10)

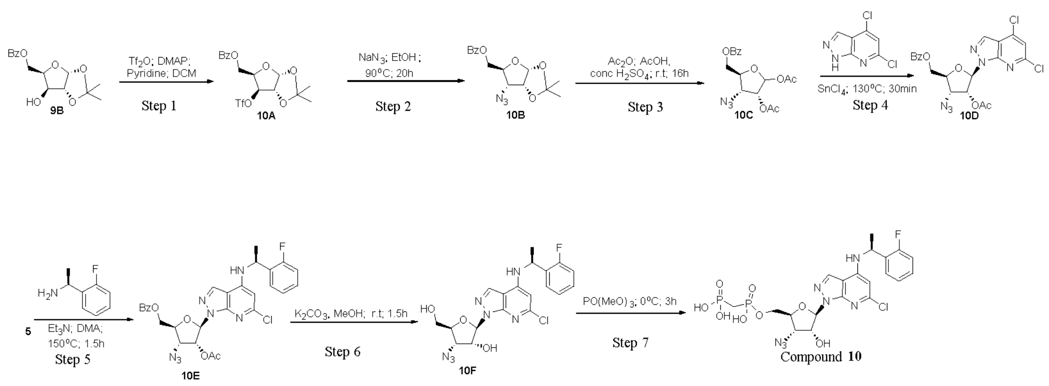

Step 1: ((3aR,5R,6S,6aR)-2,2-dimethyl-6-(((trifluoromethyl)sulfonyl)oxy) tetrahydrofuro[2,3-d][1,3]dioxol-5-yl)methyl benzoate (10A)

Compound 9B (6.0 g, 20.39 mmol), N,N-dimethylpyridin-4-amine (2.49 g, 20.39 mmol) and pyridine (16.46 mL, 203.9 mmol) were dissolved in dichloromethane (100 ml), trifluoromethanesulfonic anhydride (5.14 mL, 30.59 mmol) was added in portions under an ice-water bath, after the addition, the mixture was reacted at room temperature for 2 hours, after the reaction was completed, the reaction solution was concentrated and then the residue was separated and purified by column chromatography (petroleum ether : ethyl acetate (v/v) = 10 : 1-3 : 1) to obtain the compound 10A (8.0 g, 92%).

Step 2: ((3aR,5S,6R,6aR)-6-azido-2,2-dimethyltetrahydrofuro[2,3-d][1,3] dioxol-5-yl)methyl benzoate (10B)

Compound 10A (5.0 g, 11.73 mmol) was dissolved in ethanol (100 ml), sodium azide (2.29 g, 35.19 mmol) was added, after the addition, the mixture was reacted at 85°C for 48 hours, after the reaction was completed, water (200 ml) was added into the reaction solution to quench the reaction, the aqueous phase was extracted with ethyl acetate (300 ml × 2) twice, the organic layers were combined, washed with saturated brine, dried over anhydrous sodium sulfate, and concentrated, and the residue was separated and purified by column chromatography (petroleum ether : ethyl acetate (v/v) = 3 : 1) to obtain the compound 10B (1.4 g, 41%).

Step 3: (3R,4R,5S)-4-azido-5-((benzoyloxy)methyl)tetrahydrofuran-2,3-diyl diacetate (10C)

Compound 10B (0.55 g, 1.72 mmol) was dissolved in acetic acid (30 mL) and acetic anhydride (2 mL), concentrated sulfuric acid (0.9 mL) was added dropwise, after the addition, the mixture was reacted at room temperature for 16 hours, after the reaction was completed, water (100 mL) was added, the aqueous phase was extracted with ethyl acetate (100 mL × 2), the organic phases were combined, washed with water (100 mL × 2), washed with aqueous sodium bicarbonate solution, the liquid separation was conducted, and the organic phase was dried and concentrated to obtain the title compound 10C (0.4 g, 64%), which was directly used in the next step.
$^1$H NMR (400 MHz, $CDCl_3$) δ 8.08 (dd, 2H), 7.59 (t, 1H), 7.46 (td, 2H), 6.17 (s, 1H), 5.38 (d, 1H), 4.67 (dd, 1H), 4.50 - 4.44 (m, 1H), 4.38 (dt, 1H), 4.22 (dd, 1H), 2.19 (s, 3H), 1.92 (s, 3H).

Step 4: ((2S,3R,4R,5R)-4-acetoxy-3-azido-5-(4,6-dichloro-1H-pyrazolo[3,4-b]pyridin-1-yl)tetrahydrofuran-2-yl)methyl benzoate (10D)

Compound 10C (0.5 g, 1.38 mmol) was added into a 50 mL reaction flask, heated to 90°C, 4,6-dichloro-1H-pyrazolo[3,4-b]pyridine (0.26 g, 1.38 mmol) was added, after the addition, two drops of stannum tetrachloride were added dropwise, then the mixture was warmed to 130°C and reacted under reduced pressure for 30 minutes, cooled to room temperature, and the residue was separated and purified by silica gel column chromatography (petroleum ether : ethyl acetate (v/v) = 10 : 1-5 : 1) to obtain the title compound 10D (0.45 g, 66%).
LC-MS (ESI): m/z = 491.0[M+H]$^+$.

Step 5: ((2S,3R,4R,SR)-4-acetoxy-3-azido-5-(6-chloro-4-(((S)-1-(2-fluorophenyl)ethyl)amino)-1H-pyrazolo[3,4-b]pyridin-1-yl)tetrahydrofuran-2-yl)methyl benzoate (10E)

Compound 10D (0.45 g, 1.51 mmol) was dissolved in N,N-diethyl acetamide (5 ml), triethylamine (0.28 g, 2.76 mmol) and (S)-1-(2-fluorophenyl)ethyl-1-amine (0.26 g, 3.02 mmol) were added, after the addition, the mixture was reacted at 150°C for 1 hour, after the reaction was completed, water was added, the mixture was extracted with ethyl acetate (50 ml × 2), washed with saturated brine, dried over anhydrous sodium sulfate, filtered, and the filtrate was concentrated under reduced pressure, and the crude was separated and purified by silica gel column chromatography (petroleum ether : ethyl acetate (v/v) = 5 : 1-1 : 1) to obtain the title compound 10E (0.25 g, 46%).
LC-MS (ESI): m/z = 594.1[M+H]$^+$.

Step 6: (2R,3R,4S,5S)-4-azido-2-(6-chloro-4-(((S)-1-(2-fluorophenyl)ethyl) amino)-1H-pyrazolo[3,4-b]pyridin-1-yl)-5-(hydroxymethyl)tetrahydrofuran-3-ol (10F)

Compound 10E (0.25 g, 0.42 mmol) was dissolved in methanol (5 ml), potassium carbonate (0.23 g, 1.68 mmol) was added, after the addition, the mixture was reacted at room temperature for 2 hours, water (20 ml) was added, solids were precipitated, filtered, the filter cake was washed with water (2 mL) once, and the filter cake was azeotropically dried by distilling off toluene and water, and spun to dryness under reduced pressure to obtain the title compound 10F (0.15

g, 79%).

LC-MS (ESI): m/z = 448.2[M+H]$^+$.

Step 7: (((((2S,3S,4R,SR)-3-azido-5-(6-chloro-4-(((S)-1-(2-fluorophenyl)ethyl) amino)-1H-pyrazolo[3,4-b]pyridin-1-yl)-4-hydroxytetrahydrofuran-2-yl)methoxy)(hydroxy)phosphoryl)methyl)phosphonic acid (Compound 10)

Compound 10F (0.15 g, 0.33 mmol) was dissolved in trimethyl phosphate (2 ml), the temperature was decreased to 0°C under an ice bath, methylenebis(phosphonic dichloride) (0.25 g, 0.99 mmol) was dissolved in trimethyl phosphate (0.1 ml), and slowly added dropwise into the reaction solution, after the addition, the mixture was reacted at 0°C for 3 hours, upon completion monitored by LCMS, water (1 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.0 min, to obtain the compound 10 (40 mg, 20%).

LC-MS (ESI): m/z = 604.1[M-H]$^-$.

$^1$H NMR (400 MHz, DMSO) δ 8.39 (s, 1H), 8.24 (d, 1H), 7.40 (t, 1H), 7.32 (dd, 1H), 7.20 (dt, 2H), 7.01 (d, 1H), 6.07 (t, 2H), 5.06 (s, 1H), 4.96 (t, 1H), 4.41 - 4.32 (m, 1H), 4.16 - 4.01 (m, 2H), 3.90 (dd, 1H), 2.13 (t, 2H), 1.58 (d, 3H).

**Example 11:**

(((((2S,3S,4R,SR)-3-amino-5-(6-chloro-4-(((S)-1-(2-fluorophenyl) ethyl)amino)-1H-pyrazolo[3,4-b]pyridin-1-yl)-4-hydroxytetrahydrofuran-2-yl) methoxy)(hydroxy)phosphoryl)methyl)phosphonic acid (Compound 11)

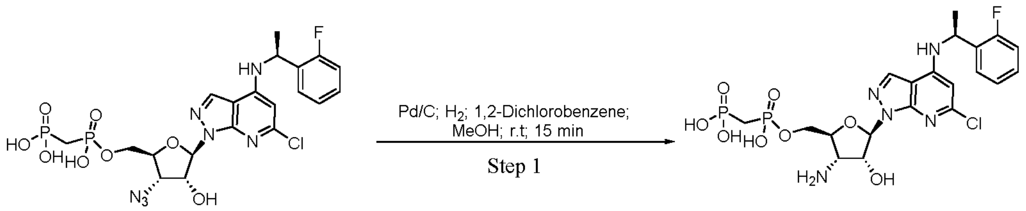


Compound **10**

Compound **11**

Compound 10 (0.035 g, 0.066 mmol) was dissolved in methanol (5 ml), o-dichlorobenzene (1 mL) Pd/C (10 mg) were added, the reaction system was subjected to hydrogen replacement three times, reacted at room temperature for 15 minutes, upon completion monitored by LCMS, the mixture was concentrated to remove methanol, water (5 ml) and ethyl acetate (5 mL) were added, the liquid separation was conducted, and the aqueous phase was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.0 min, to obtain the compound 11 (10 mg, 26%).

LC-MS (ESI): m/z = 580.1[M+H]$^+$.

$^1$H NMR (400 MHz, MeOD) δ 8.21 (s, 1H), 7.36 (dd, 1H), 7.32 - 7.24 (m, 1H), 7.13 (dd, 2H), 6.35 (s, 1H), 6.04 (s, 1H), 5.08-5.03 (m, 1H), 4.47 (s, 2H), 4.23-4.17 (m, 1H), 4.00-3.94 (m, 1H), 2.28 (t, 2H), 1.64 (d, 3H).

**Example 12:**

(((((2R,3S,4R,5R)-5-(6-chloro-4-(((S)-1-(2-fluorophenyl)ethyl)amino)-1H-pyrazolo[3,4-b]pyridin-1-yl)-3,4-dihydroxy-3-(hydroxymethyl)tetrahydrofuran-2-yl)methoxy)(hydroxy)phosphoryl)methyl)phosphonic acid (Compound 12)

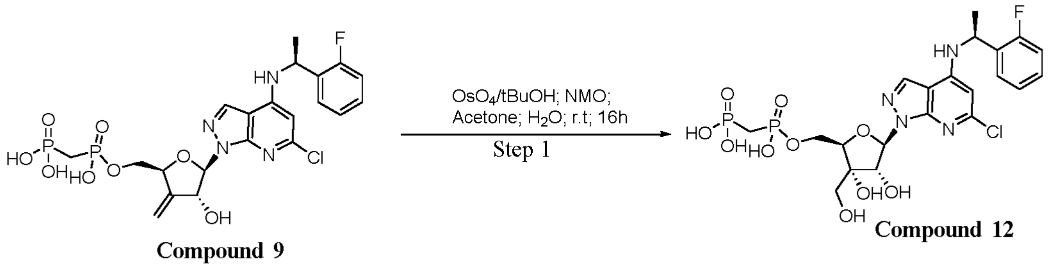


Compound 9 Compound 12

Compound 9 (0.050 g, 0.087 mmol) and N-methylmorpholine oxide (0.030 g, 0.26 mmol) were dissolved in acetone (2 ml) and water (1 mL), osmium tetroxide in tert-butanol (1 mL, 2 mg/mL) was added dropwise at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.0 min, to obtain the compound 12 (20 mg, 38%).

LC-MS (ESI): m/z = 609.0[M-H]$^-$.

$^1$H NMR (400 MHz, $D_2O$) δ 8.28 (s, 1H), 7.40 (t, 1H), 7.31 (dd, 1H), 7.15 (dt, 2H), 6.24 - 6.16 (m, 2H), 5.11 (d, 1H), 4.93 (d, 1H), 4.32 (t, 1H), 4.11 (dd, 1H), 4.05 (dd, 1H), 3.96 (d, 1H), 3.84 (d, 1H), 2.07 (td, 2H), 1.62 (d, 3H).

## Example 13

(((((2R,3S,4R,5R)-4-azido-5-(6-chloro-4-(((S)-1-(2-fluorophenyl)ethyl) amino)-1H-pyrazolo[3,4-b]pyridin-1-yl)-3-hydroxytetrahydrofuran-2-yl)methoxy) (hydroxy)phosphoryl)methyl)phosphonic acid (Compound 13)

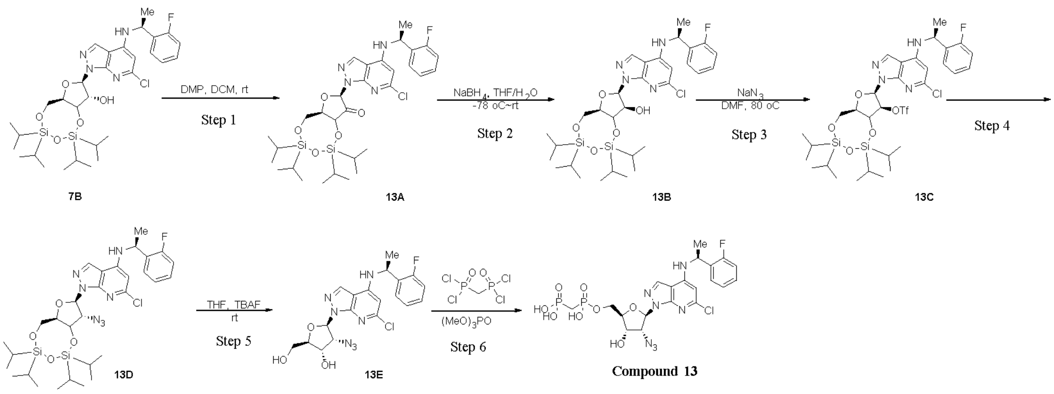


7B 13A 13B 13C 13D 13E Compound 13

Step 1: (6aR,8R)-8-(6-chloro-4-(((S)-1-(2-fluorophenyl)ethyl)amino)-1H-pyrazolo[3,4-b]pyridin-1-yl)-2,2,4,4-tetraisopropyldihydro-6H-furo[3,2-f][1,3,5,2,4]trioxadisilocin-9(8H)-one (13A)

Compound 7B (5.0 g, 7.51 mmol) was dissolved in dichloromethane (100 ml), Dess-Martin reagent (7.96 g, 18.77 mmol) was added in portions, after the addition, the mixture was reacted at room temperature for 16 hours, after the reaction was completed, water (50 ml) was added into the reaction solution to quench the reaction, the mixture was filtered, the filter cake was rinsed with dichloromethane, the liquid separation was conducted, the aqueous phase was extracted twice with dichloromethane (100 ml × 2), the organic layers were combined, washed with saturated brine, dried over anhydrous sodium sulfate, concentrated and passed through a column (PE : EA = 1 : 0-3 : 1) to obtain the title compound 13A (1.48 g, 29.7%).

$^1$H NMR (400 MHz, $CDCl_3$) δ 7.90 (s, 1H), 7.30-7.24(m, 2H), 7.12-7.07(m, 2H), 6.18 (s, 1H), 6.08 (s, 1H), 5.21 (d, 1H), 5.03 (s, 1H), 4.14-4.09 (m, 2H), 4.03-4.00 (m, 1H), 1.71-1.66 (m, 3H), 1.67 (d, 3H), 1.26-0.92 (m, 28H).

Step 2: (6aR,8R,9S)-8-(6-chloro-4-(((S)-1-(2-fluorophenyl)ethyl)amino)-1H-pyrazolo[3,4-b]pyridin-1-yl)-2,2,4,4-tetraisopropyltetrahydro-6H-furo[3,2-f][1,3,5,2,4]trioxadisilocin-9-ol (13B)

13A (1.48 g, 2.23 mmol), tetrahydrofuran (20 ml), and water (2 ml) were added into a flask, sodium borohydride (84 mg, 2.23 mmol) was added at -78°C, and the mixture was reacted at room temperature for 1 hour. Upon complete depletion of raw materials monitored by TLC, a product was generated. The product was extracted with dichloromethane, washed, dried, concentrated and passed through a column (PE : EA = 1 : 0-3 : 1) to obtain the title compound 13B (1.4 g, 94.4%).

$^1$H NMR (400 MHz, $CDCl_3$) δ 7.96 (s, 1H), 7.30-7.24 (m, 2H), 7.12-7.07 (m, 2H), 6.53 (d, 1H), 6.09 (s, 1H), 5.03 (s, 1H), 4.88 (t, 1H), 4.64 (t, 1H), 3.91-3.88 (m, 3H), 1.67 (d, 3H), 1.26-0.88 (m, 28H).

Step 3: (6aR,8R,9S)-8-(6-chloro-4-(((S)-1-(2-fluorophenyl)ethyl)amino)-1H-pyrazolo[3,4-b]pyridin-1-yl)-2,2,4,4-tetraisopropyltetrahydro-6H-furo[3,2-f][1,3,5,2,4]trioxadisilocin-9-yltrifluoromethanesulfonate (13C)

13B (1.4 g, 2.10 mmol), pyridine (1.5 g, 18.90 mmol), 4-dimethylaminopyridine (0.31 g, 2.52 mmol) and dichloromethane (20 ml) were added into a flask, trifluoromethanesulfonic anhydride (1.7 g, 6.02 mmol) was added dropwise at room temperature, and then reacted at room temperature for 2 hours. Upon complete depletion of raw materials monitored by TLC (PE : EA = 10 : 1), a product was generated. The product was extracted with dichloromethane, washed, dried, concentrated and passed through a column (PE : EA = 1 : 0-5 : 1) to obtain the title compound 13C (1.1 g, 65.7%).

$^1$H NMR (400 MHz, $CDCl_3$) δ 7.95 (s, 1H), 7.30-7.26 (m, 2H), 7.12-7.08 (m, 2H), 6.77 (s, 1H), 6.14 (s, 1H), 5.63 (t, 1H), 5.49 (t, 1H), 5.03 (s, 1H), 4.17-4.09 (m, 1H), 3.97-3.89 (m, 2H), 1.67 (d, 3H), 1.26-0.88 (m, 28H).

Step 4: 1-((6aR,8R,9R)-9-azido-2,2,4,4-tetraisopropyltetrahydro-6H-furo[3,2-f][1,3,5,2,4]trioxadisilocin-8-yl)-6-chloro-N-((S)-1-(2-fluorophenyl)ethyl)-1H-pyrazolo[3,4-b]pyridin-4-amine (13D)

13C (1.4 g, 1.76 mmol) and N,N-dimethylformamide (20 ml) were added in to a flask, followed by sodium azide (0.34 g, 5.28 mmol), and then the mixture was reacted at 80 °C for 8 hours. Upon complete depletion of raw materials monitored by LCMS, a product was generated. The product was extracted with dichloromethane, washed, dried, concentrated and passed through a column (PE : EA = 1 : 0-5 : 1) to obtain the title compound 13D (1.1 g, 90.5%).

$^1$H NMR (400 MHz, $CDCl_3$) δ 7.91 (s, 1H), 7.31-7.24 (m, 2H), 7.12-7.07 (m, 2H), 6.28 (s, 1H), 6.13 (s, 1H), 5.34-5.30 (m, 1H), 5.04 (s, 1H), 4.58 (d, 1H), 4.06-4.03 (m, 1H), 3.95 (t, 2H), 1.68 (d, 3H), 1.24-0.88 (m, 28H).

Step 5: (2R,3S,4R,5R)-4-azido-5-(6-chloro-4-(((S)-1-(2-fluorophenyl)ethyl) amino)-1H-pyrazolo[3,4-b]pyridin-1-yl)-2-(hydroxymethyl)tetrahydrofuran-3-ol (13E)

13D (1.1 g, 1.59 mmol) and tetrahydrofuran (20 ml) were added into a flask, followed by tetrabutylammonium fluoride (1.25 g, 4.77 mmol), and then the mixture was reacted at room temperature for 4 hours. Upon complete depletion of raw materials monitored by LCMS, a product was generated. The product was extracted with dichloromethane, washed, dried, concentrated and passed through a column (PE : EA = 1 : 0-0 : 1) to obtain the title compound 13E (600 mg, 84.3%).

LC-MS (ESI): m/z = 448.2 $[M+H]^+$.

Step 6: (((((2R,3S,4R,SR)-4-azido-5-(6-chloro-4-(((S)-1-(2-fluorophenyl)ethyl) amino)-1H-pyrazolo[3,4-b]pyridin-1-yl)-3-hydroxytetrahydrofuran-2-yl)methoxy)(hydroxy)phosphoryl)methyl)phosphonic acid (Compound 13)

13E (200 mg, 0.45 mmol) and trimethyl phosphate (4 ml) were added into a flask, and methylenebis(phosphonic dichloride) (340 mg, 1.35 mmol) was added under an ice bath. The temperature was naturally warmed to room temperature and the reaction was stirred for 3 hours. Upon complete depletion of raw materials monitored by LCMS, a product was generated. 1 ml of water was added and stirred for 10 minutes, and the reaction solution was subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.56 min, and the preparative liquid was concentrated and lyophilized to obtain the title compound 13F (110 mg, 40.3%).

$^1$H NMR (400 MHz, DMSO-$d_6$) δ 8.41 (s, 1H), 8.26 (d, 1H), 7.40 (t, 1H), 7.34 - 7.30(m, 1H), 7.24 - 7.16 (m, 2H), 6.12 (d, 1H), 6.04 (s, 1H), 5.07 (s, 1H), 4.66 (t, 2H), 4.06 (d, 2H), 3.96 - 3.91 (m, 1H), 2.02 (t, 2H), 1.58 (d, 3H).

LC-MS (ESI): m/z = 606.2 $[M+H]^+$.

**Example 14:**

(2-(((2R,3S,4R,5R)-5-((2-chloro-4-(cyclopentylamino)pyrrolo[2,1-f][1,2,4] triazin-7-yl)methyl)-3,4-dihydroxytetrahydrofuran-2-yl)methoxy)-1-hydroxy-3-methoxypropan-2-yl)phosphonic acid (Compound 14)

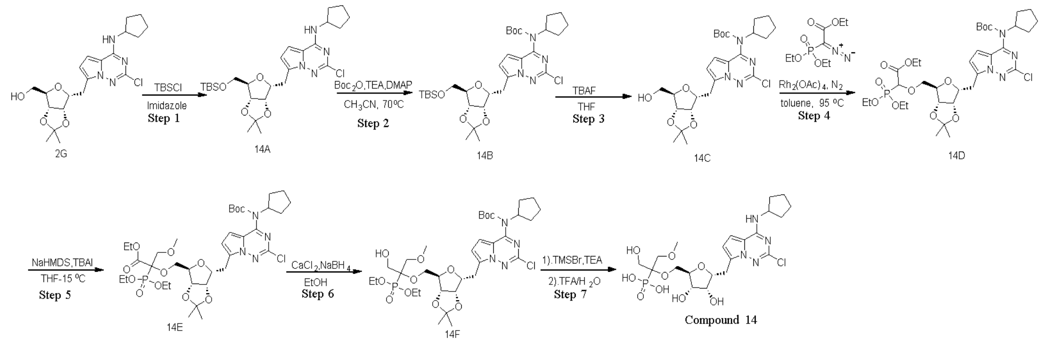


2G, 14A, 14B, 14C, 14D, 14E, 14F, Compound 14

Step 1: 7-(((3aS,4R,6R,6aR)-6-(((tert-butyldimethylsilyl)oxy)methyl)-2,2-dimethyltetrahydrofuro[3,4-d][1,3]dioxol-4-yl)methyl)-2-chloro-N-cyclopentylpyrrolo[2,1-f][1,2,4]triazin-4-amine (14A)

Compound 2G (1.18 g, 2.79 mmol) was dissolved in DCM (15 ml), imidazole (0.57 g, 8.37 mmol) was added, after the addition, under nitrogen protection, the temperature was decreased by 0-5°C, TBSCl (0.63 g, 4.19 mmol) was added in portions, after the addition, the mixture was naturally warmed to room temperature and reacted for 2 hours, after the reaction was completed, water was added to quench the reaction, the mixture was extracted with dichloromethane (50 ml × 2), the organic layers were combined, washed with saturated brine, dried and concentrated, and then the residue was separated and purified by silica gel column chromatography (petroleum ether : ethyl acetate (v/v) = 5 : 1) to obtain the title compound 14A (1.4 g, 93%).

LC-MS (ESI): m/z = 537.2 $[M+H]^+$.

Step 2: tert-butyl(7-(((3aS,4R,6R,6aR)-6-(((tert-butyldimethylsilyl)oxy) methyl)-2,2-dimethyltetrahydrofuro[3,4-d][1,3]dioxol-4-yl)methyl)-2-chloropyrrolo[2,1-f][1,2,4]triazin-4-yl)(cyclopentyl)carbamate (14B)

Compound 14A (1.4 g, 2.61 mmol) was dissolved in acetonitrile (20 ml), ditert-butyl dicarbonate (2.85 g, 13.05 mmol), triethylamine (2.64 g, 26.1 mmol) and DMAP (0.64 g, 5.22 mol) were added, after the addition, the mixture was warmed to 75°C and reacted for 15 hours, after the reaction was completed, water was added to quench the reaction, the mixture was extracted with ethyl acetate (100ml × 2), the organic layers were combined, washed with saturated brine, dried and concentrated, and then the residue was separated and purified by silica gel column chromatography (petroleum ether : ethyl acetate (v/v) = 10 : 1) to obtain the title compound 14B (1.3 g, 78%).

LC-MS (ESI): m/z = 637.2 $[M+H]^+$.

Step 3: tert-butyl(2-chloro-7-(((3aS,4R,6R,6aR)-6-(hydroxymethyl)-2,2-dimethyltetrahydrofuro[3,4-d][1,3]dioxol-4-yl)methyl)pyrrolo[2,1-f][1,2,4]triazin-4-yl)(cyclopentyl)carbamate (14C)

Compound 14B (1.3 g, 2.04 mmol) was dissolved in tetrahydrofuran (15 ml), tetrabutylammonium fluoride in tetrahydrofuran (2.45 ml, 2.45 mmol, 1.0 mol/L) was added, after the addition, the mixture was reacted at room temperature for 2 hours, and after the reaction was completed, the reaction solution was directly concentrated. After concentration, the residue was separated and purified by silica gel column chromatography (petroleum ether : ethyl acetate (v/v) = 2 : 1) to obtain the title compound 14C (0.90 g, 84%).

LC-MS (ESI): m/z = 467.2 $[M-tBu+H]^+$.

Step 4: ethyl2-(((3aR,4R,6R,6aS)-6-((4-((tert-butoxycarbonyl)(cyclopentyl) amino)-2-chloropyrrolo[2,1-f][1,2,4]triazin-7-yl)methyl)-2,2-dimethyltetrahydrofuro [3,4-d][1,3]dioxol-4-yl)methoxy)-2-(diethoxyphosphoryl) acetate (14D)

Compound 14C (0.90 g, 1.72 mmol) was dissolved in toluene (15 ml), ethyl 2-diazo-2-(diethoxyphosphoryl) acetate (1.29 g, 5.16 mmol) and rhodium(II) acetate dimmer (0.076 g, 0.17 mmol) were added, after the addition, under nitrogen protection, the mixture was warmed to 100°C and reacted for 3 hours, after the reaction was completed, the

reaction solution was directly concentrated, and after concentration, the residue was separated and purified by silica gel column chromatography (petroleum ether : ethyl acetate (v/v) = 4 : 1) to obtain the title compound 14D (1.1 g, 85%).

LC-MS (ESI): m/z = 745.3 $[M+H]^+$.

Step 5: ethyl2-(((3aR,4R,6R,6aS)-6-((4-((tert-butoxycarbonyl)(cyclopentyl) amino)-2-chloropyrrolo[2,1-f][1,2,4]triazin-7-yl)methyl)-2,2-dimethyltetrahydrofuro [3,4-d][1,3]dioxol-4-yl)methoxy)-2-(diethoxyphosphoryl)-3-methoxypropanoate (14E)

Compound 14D (1.1 g, 1.48 mmol) was dissolved in dry tetrahydrofuran (15 ml), the reaction system was subjected to nitrogen replacement three times, cooled to -15°C using an ethanol dry ice system, with the temperature was controlled at - 15°C, sodium bis(trimethylsilyl)amide (1.48 ml, 2.96 mmol, 2.0 mol/L) was added dropwise, after the addition, the mixture was reacted at -15°C for 20 minutes, with the temperature was controlled at -15°C, tetrabutylammonium iodide (0.27 g, 0.74 mmol) was added, bromomethyl methyl ether (0.55 g, 4.44 mmol) was slowly added dropwise, after the addition, with the temperature was kept at -15°C, the mixture was reacted for 3 hours, after the reaction was completed, saturated aqueous ammonium chloride solution was added to quench the reaction, water was added and the mixture was extracted with ethyl acetate (100 ml × 2), the organic layers were combined, washed with saturated brine, and dried and concentrated to obtain the title compound 14E (1.0 g, 85%), which crude was directly used in the next reaction without purification.

LC-MS (ESI): m/z = 789.3 $[M+H]^+$.

Step 6: tert-butyl(2-chloro-7-(((3aS,4R,6R,6aR)-6-(((2-(diethoxyphosphoryl)-1-hydroxy-3-methoxypropan-2-yl)oxy)methyl)-2,2-dimethyltetrahydrofuro[3,4-d][1,3]dioxol-4-yl)methyl)pyrrolo[2,1-f][1,2,4]triazin-4-yl)(cyclopentyl)carbamate (14F)

Compound 14E (1.0 g, 1.27 mmol) was dissolved in ethanol (15 ml), anhydrous calcium chloride (0.28 g, 2.54 mmol) was added, after the addition, under nitrogen protection, the temperature was decreased by 0-5°C, sodium borohydride (0.1 g, 2.54 mmol) was added in portions, after the addition, the mixture was naturally warmed to room temperature and reacted for 3 hours, after the reaction was completed, water was added to quench the reaction, the mixture was extracted with dichloromethane (50 ml × 2), the organic layers were combined, washed with saturated brine, dried over anhydrous sodium sulfate, dried and concentrated, and then the residue was separated and purified by silica gel column chromatography (petroleum ether : ethyl acetate (v/v) = 1 : 5) to obtain the title compound 14F (0.5 g, 52%).

LC-MS (ESI): m/z = 747.3 $[M+H]^+$.

Step 7: (2-(((2R,3S,4R,5R)-5-((2-chloro-4-(cyclopentylamino)pyrrolo[2,1-f] [1,2,4]triazin-7-yl)methyl)-3,4-dihydroxytetrahydrofuran-2-yl)methoxy)-1-hydroxy-3-methoxypropan-2-yl)phosphonic acid (Compound 14)

Compound 14F (0.069 g, 0.092 mmol) was dissolved in acetonitrile (2 ml), triethylamine (0.28 g, 2.76 mmol) was added, the compound bromotrimethylsilane (0.28g, 1.84mmol) was slowly added dropwise, after the addition, the mixture was reacted for 15 hours, after the reaction was completed, the mixture was filtered, the filter cake was washed with a small amount of acetonitrile, after the filtrate was concentrated, a mixture of trifluoroacetic acid and water (2 ml, 1 : 1) was added, after the addition, the mixture was stirred for 15 hours, after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: the content of mobile phase A: 5% to 50%; c. flow rate: 12 mL/min; d. elution time: 20 min, retention time: 7.3 min, to obtain the compound 14 (20 mg, 39%).

LC-MS (ESI): m/z = 549.1$[M-H]^-$

$^1H$ NMR (400 MHz, DMSO-$d_6$) δ 8.35(d, 1H), 6.97 (d, 1H), 6.49 (d, 1H), 4.48-4.43 (m, 1H), 4.20-4.18 (m, 1H), 4.00-3.95 (m, 1H), 3.86-3.60 (m, 8H), 3.25 (s, 3H), 3.11-2.98 (m, 2H), 1.99-1.98 (m, 2H), 1.73-1.58 (m, 6H).

**Example 15:**

((((2R,3S,4R,5R)-5-((2-chloro-4-(methylamino)pyrrolo[2,1-f][1,2,4]triazin-7-yl)methyl)-3,4-dihydroxytetrahydrofuran-2-yl)methoxy)methyl)phosphonic acid (Compound 15)

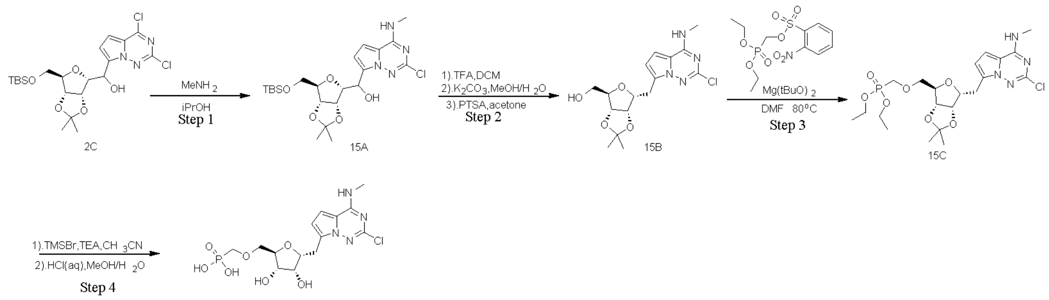


2C 15A 15B 15C

Compound 15

Step 1: (R)-((3aS,4R,6R,6aR)-6-(((tert-butyldimethylsilyl)oxy)methyl)-2,2-dimethyltetrahydrofuro[3,4-d][1,3]dioxol-4-yl)(2-chloro-4-(methylamino)pyrrolo [2,1-f][1,2,4]triazin-7-yl)methanol (15A)

Compound 2C (0.50 g, 0.99 mmol) was dissolved in isopropanol (5 ml), methylamine alcohol solution (2.0 ml, 1.98 mmol, 1.0 mol/L) was then added, after the addition, the mixture was reacted at room temperature for 3 hours, after the reaction was completed, the reaction solution was directly concentrated, and after concentration, the residue was separated and purified by silica gel column chromatography (petroleum ether : ethyl acetate (v/v) = 3 : 1) to obtain the title compound 15A (0.40 g, 81%).

LC-MS (ESI): m/z = 499.2 $[M+H]^+$.

Step 2: ((3aR,4R,6R,6aS)-6-((2-chloro-4-(methylamino)pyrrolo[2,1-f][1,2,4] triazin-7-yl)methyl)-2,2-dimethyltetrahydrofuro[3,4-d][1,3]dioxol-4-yl)methanol (15B)

Compound 15A (0.40 g, 0.80 mmol) was dissolved in dichloromethane (5 ml), triethylsilane (0.80 g, 8.0 mmol) was added, after the addition, trifluoroacetic acid (0.5 ml) was slowly added dropwise, and after the addition, the mixture was reacted at room temperature for 16 hours. After the reaction was completed, the mixture was directly concentrated to obtain a product, which was dissolved in a mixed solvent of methanol (5 ml) and water (1 ml), potassium carbonate (0.11 g, 0.80 mmol) was added, and after the addition, the mixture was reacted at room temperature for 1 hour. After the reaction was completed, water was added, the mixture was extracted with ethyl acetate (50 ml $\times$ 2), washed with saturated brine, dried over anhydrous sodium sulfate, filtered, and the filtrate was concentrated under reduced pressure to obtain a product, which was dissolved in acetone (5 ml), a catalytic amount of p-toluenesulfonic acid was added, followed by 2,2-dimethoxypropane (0.42 g, 4.0 mmol), after the addition, the mixture was reacted at room temperature for 2 hours, after the reaction was completed, the reaction was concentrated to remove acetone, water (20 ml) was added, extracted with dichloromethane (50 ml $\times$ 2), washed with saturated brine, dried over anhydrous sodium sulfate, filtered, and the filtrate was concentrated, and the residue was separated and purified by silica gel column chromatography (petroleum ether: ethyl acetate (v/v) = 1 : 1-1 : 5) to obtain the title compound 15B (0.21 g, 77%).

LC-MS (ESI): m/z = 369.1$[M+H]^+$.

Step 3: diethyl((((3aR,4R,6R,6aS)-6-((2-chloro-4-(methylamino)pyrrolo[2,1-f][1,2,4]triazin-7-yl)methyl)-2,2-dimethyltetrahydrofuro[3,4-d][1,3]dioxol-4-yl)methoxy)methyl)phosphonate (15C)

Compound 15B (0.21 g, 0.57 mmol) and methyl (diethoxyphosphoryl) 2-nitrobenzenesulfonate (0.3 g, 0.85 mmol) were dissolved in dry N,N-dimethylformamide (5 ml), the reaction system was subjected to nitrogen replacement three times, the compound magnesium tert-butoxide (0.29 g, 1.7 mmol) was added, after the addition, the mixture was warmed to 80°C and reacted for 3 hours, after the reaction was completed, the reaction was cooled to room temperature and quenched with saturated ammonium chloride solution, water was added, then extracted with ethyl acetate (50 ml $\times$ 2), the organic layers were combined, washed with saturated brine, dried over anhydrous sodium sulfate, filtered, the filtrate was concentrated, and then the residue was separated and purified by silica gel column chromatography (petroleum ether : ethyl acetate (v/v) = 1 : 5) to obtain the title compound 15C (0.24 g, 81%).

LC-MS (ESI): m/z = 519.2$[M+H]^+$.

Step 4: ((((2R,3S,4R,5R)-5-((2-chloro-4-(methylamino)pyrrolo[2,1-f][1,2,4] triazin-7-yl)methyl)-3,4-dihydroxytetrahydrofuran-2-yl)methoxy)methyl) phosphonic acid (Compound 15)

Compound 15C (0.24 g, 0.46 mmol) was dissolved in acetonitrile (5 ml), triethylamine (1.4 g, 13.8 mmol) was added, bromotrimethylsilane (1.4 g, 9.2 mmol) was slowly added dropwise, after the addition, the mixture was reacted

for 15 hours, after the reaction was completed, the mixture was filtered, the filter cake was washed with a small amount of acetonitrile, the filtrate was concentrated and 4N hydrochloric acid (1 ml) was added, after the addition, the reaction was stirred for 5 hours, and after the reaction was completed, the reaction system was directly subjected to preparative HPLC with preparative HPLC separation methods: 1. Instrument: waters 2767 (preparative liquid phase chromatographic instrument), chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.5 min, to obtain the compound 15 (110 mg, 56%).

LC-MS (ESI): m/z = 475.1[M-H]$^-$.

$^1$H NMR (400 MHz, DMSO-$d_6$) δ 8.57 (d, 1H), 6.85 (d, 1H), 6.49 (d, 1H), 4.23-4.18 (m, 1H), 3.91-3.82 (m,3H), 3.85-3.48 (m,4H), 3.13-2.96 (m,5H).

**Example 16**

((((3S,4R,5R)-5-((6-chloro-4-(cyclopentylamino)-1H-pyrazolo[3,4-d]pyrimidin -1-yl)methyl)-3,4-dihydroxytetrahydrofuran-2-yl)oxy)methyl)phosphonic acid (Compound 16)

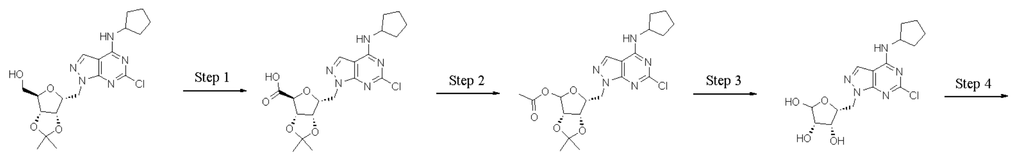


1G 16A 16B 16C

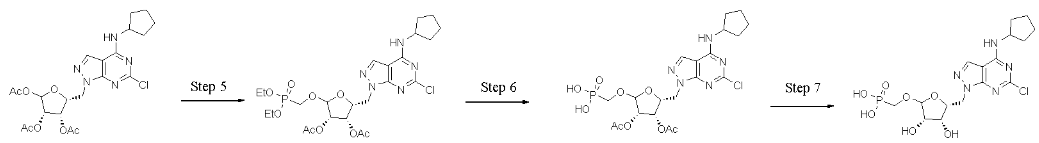


16D 16E 16F Compound 16

Step 1: (3aS,4S,6R,6aS)-6-((6-chloro-4-(cyclopentylamino)-1H-pyrazolo[3,4-d]pyrimidin-1-yl)methyl)-2,2-dimethyltetrahydrofuro[3,4-d][1,3]dioxole-4-carboxylic acid (16A)

((3aR, 4R, 6R, 6aS)-6-((6-chloro-4-(cyclopentylamino)-1H-pyrazolo[3, 4-d]pyrimidin-1-yl)methyl)-2,2-dimethyltetrahydrofuro[3,4-d][1,3]dioxol-4-yl)methanol (1G) (1.1 g, 2.5 mmol) was added into acetonitrile (4 mL) and water (4 mL), (diacetoxyiodo)benzene (1.84 g, 5.5 mmol) and TEMPO (0.1 g, 0.6 mmol) were added, the mixture was stirred at room temperature for 16 hours, extracted with water (50 mL) and EA (50 mL), and EA phase was concentrated to dryness, to obtain 1.3 g of 16A crude, which was directly used in the next reaction.

LCMS m/z = 438.2 [M+1]$^+$.

Step 2: (3aS,6R,6aS)-6-((6-chloro-4-(cyclopentylamino)-1H-pyrazolo[3,4-d]pyrimidin-1-yl)methyl)-2,2-dimethyltetrahydrofuro[3,4-d][1,3]dioxol-4-yl acetate(16B)

(3aS,4S,6R,6aS)-6-((6-chloro-4-(cyclopentylamino)-1H-pyrazolo[3,4-d]pyrimidin-1-yl)methyl)-2,2-dimethyltetrahydrofuro[3,4-d][1,3]dioxole-4-carboxylic acid (16A) (1.3 g, 3.0 mmol) was added into anhydrous DMF (12 mL), lead tetraacetate (3.6 g, 9.0 mmol) was added, under nitrogen protection, the mixture was reacted for 16 hours under light conditions, and extracted with water (50 mL) and ethyl acetate (50 mL), the ethyl acetate phase was concentrated to dryness, and purified by column chromatography (petroleum ether/ethyl acetate = 2 : 1) to obtain 1.0 g of 16B (two-step yield = 94%).

LCMS m/z = 452.2 [M+1]$^+$.

Step 3: (3S,4R,5R)-5-((6-chloro-4-(cyclopentylamino)-1H-pyrazolo[3,4-d] pyrimidin-1 -yl)methyl)tetrahydrofuran-2,3,4-triol (16C)

To (3aS,6R,6aS)-6-((6-chloro-4-(cyclopentylamino)-1H-pyrazolo[3,4-d]pyrimidin -1-yl)methyl)-2,2-dimethyltetrahydrofuro[3,4-d][1,3]dioxol-4-yl acetate (16B) (0.4 g, 0.88 mmol), 90% aqueous TFA solution (4 mL) was added, and the mixture was reacted at room temperature for 3h (there were raw materials and intermediate states that had not been fully reacted), TFA was dried by nitrogen blow, the products were dissolved in water and purified by reversed phase column chromatography (acidic: 0.5% TFA in $H_2O$), (ACN: $H_2O$ = 15%), and directly lyophilized to obtain 80 mg of 16C (yield = 25%).

LCMS m/z = 370.2 $[M+1]^+$.

Step 4: (3S,4S,5R)-5-((6-chloro-4-(cyclopentylamino)-1H-pyrazolo[3,4-d] pyrimidin-1-yl)methyl)tetrahydrofuran-2,3,4-triyl triacetate (16D)

(3S, 4R, 5R)-5-((6-chloro-4-(cyclopentylamino)-1H-pyrazolo[3, 4-d]pyrimidin-1-yl)methyl)tetrahydrofuran-2, 3, 4-triol (16J) (80 mg, 0.217 mmol) was added into DCM (2 mL), followed by acetic anhydride (110 mg, 1.08 mmol), triethylamine (175 mg, 1.736 mmol) and DMAP (13 mg, 0.108 mmol). The mixture was stirred at room temperature overnight, water (10 mL) and DCM (10 mL) were added for extraction, the DCM phase was concentrated to dryness, and the residue was subjected to thin layer chromatography to obtain 40 mg of 16D (yield = 30%).

LCMS m/z = 496.2 $[M+1]^+$.

Step 5: (2R,3S,4S)-2-((6-chloro-4-(cyclopentylamino)-1H-pyrazolo[3,4-d] pyrimidin-1-yl)methyl)-5-((diethoxyphosphoryl)methoxy)tetrahydrofuran-3,4-diyl diacetate (16E)

Diethyl (hydroxymethyl)phosphonate (100 mg, 0.59 mmol) and saccharin (11 mg, 0.059 mmol) were added into bis(trimethylsilyl)amine (2 mL), under nitrogen protection, the mixture was stirred at 100°C for 8 hours, cooled and then concentrated to remove excess solvent, azeotropically dried by distilling off toluene and water twice, under nitrogen protection, ultra-dried acetonitrile (2 mL) and (3S,4S,SR)-5-((6-chloro-4-(cyclopentylamino)-1H-pyrazolo[3,4-d]pyrimidin-1-yl)methyl)tetrahydrofuran-2,3,4-triyl triacetate (16D) (40 mg, 0.08 mmol) were added, stannum tetrachloride (307 mg, 1.18 mmol) was added dropwise, and the mixture was stirred at room temperature for 1 hour. Saturated sodium carbonate was added to quench the reaction, the reaction mixture was extracted with ethyl acetate and concentrated to dryness, and the residue was purified by thin layer chromatography to obtain 30 mg of 16E (yield = 60%).

LCMS m/z = 604.2 $[M+1]^+$.

Step 6: ((((3S,4S,SR)-3,4-diacetoxy-5-((6-chloro-4-(cyclopentylamino)-1H-pyrazolo[3,4-d]pyrimidin-1-yl)methyl)tetrahydrofuran-2-yl)oxy)methyl) phosphonic acid (16F)

(2R,3S,4S)-2-((6-chloro-4-(cyclopentylamino)-1H-pyrazolo[3,4-d]pyrimidin-1-yl)methyl)-5-((diethoxyphosphoryl)methoxy)tetrahydrofuran-3,4-diyl diacetate (16E) (30 mg, 0.05 mmol) was added into acetonitrile (2 mL), followed by triethylamine (750 mg, 7.5 mmol) and bromotrimethylsilane (760 mg, 5 mmol), and the mixture was stirred at room temperature for 16 hours, filtered, washed with acetonitrile, and concentrated to dryness at a low temperature, and the residue was purified by reversed phase column chromatography to obtain 20 mg (16F) (yield = 74%).

LCMS m/z = 548.2 $[M+1]^+$.

Step 7: ((((3S,4R,5R)-5-((6-chloro-4-(cyclopentylamino)-1H-pyrazolo[3,4-d]pyrimidin-1-yl)methyl)-3,4-dihydroxytetrahydrofuran-2-yl)oxy)methyl) phosphonic acid (Compound 16)

((((3 S,4S,SR)-3,4-diacetoxy-5-((6-chloro-4-(cyclopentylamino)-1H-pyrazolo[3,4-d]pyrimidin-1-yl)methyl)tetrahydrofuran-2-yl)oxy)methyl)phosphonic acid (16F) (15 mg, 0.027 mmol) was added into methanol (2 mL) and water (0.5 mL), sodium carbonate (29 mg, 0.27 mmol) was added and the mixture was stirred at room temperature for 1 hour, and the mixture was separated and purified by a preparative liquid chromatography column (liquid phase preparative conditions: C18 reversed phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 4.25 min), to obtain 4 mg (compound 16) (yield = 32%).

$^1$HNMR (400 MHz, MeOD and DMSO-$d_6$) $\delta$ 7.17 (s, 1H), 6.65 (s, 1H), 5.34-5.33 (d, 1H), 4.60-4.25 (m, 7H), 3.18-3.14 (m, 1H), 2.05-1.90 (m, 4H), 1.70-1.48 (m, 4H).

LCMS m/z = 464.1 $[M+1]^+$.

## Example 17

(((((2R,3S,4R,5R)-5-(6-chloro-4-(((S)-1-(2-fluorophenyl)ethyl)amino)-1H-benzo[d][1,2,3]triazol-1-yl)-3,4-dihydroxytetrahydrofuran-2-yl)methoxy)(hydroxy) phosphoryl)methyl)phosphonic acid (Compound 17)

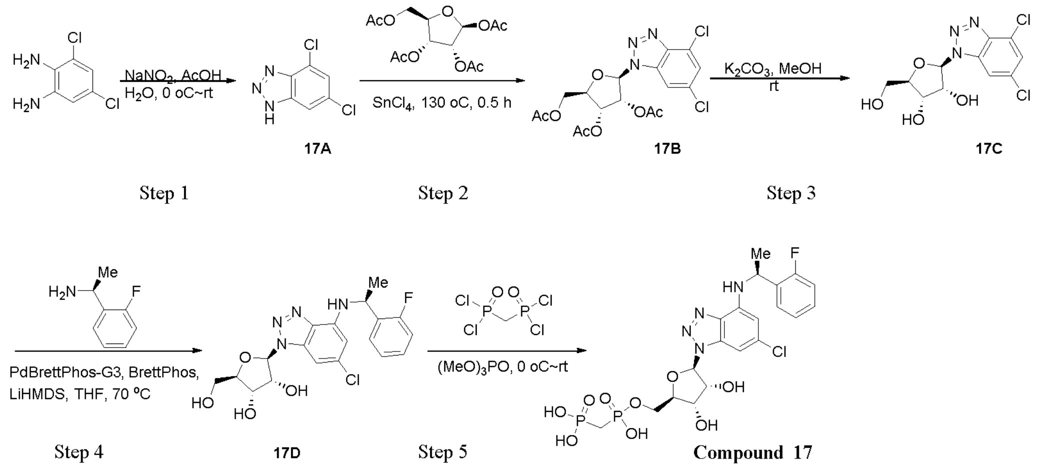


Step 1: 4,6-dichloro-1H-benzo[d][1,2,3]triazole (**17A**)

(2-Amino-3,5-dichlorophenyl)amine (5.0 g, 28.6 mmol) was added into a flask, followed by acetic acid (10 ml) and water (3 ml). Then the mixture was cooled to 0°C, and sodium nitrite solution (2.9 g, 42.9 mmol, 6 ml water) was added dropwise. After the addition, the mixture was stirred at room temperature for another 2 hours. Upon complete depletion of raw materials monitored by LCMS, a product was generated. The solution was adjusted to pH 10, filtered, and the filtrate was extracted with dichloromethane, washed, and concentrated, and then combined with the filter cake to obtain the title compound 17A (4.8 g, 90.4%).
LC-MS (ESI): m/z = 188.0, 190.0 $[M+H]^+$.

Step 2: (2R,3R,4R,5R)-2-(acetoxymethyl)-5-(4,6-dichloro-1H-benzo[d][1,2,3] triazol-1-yl)tetrahydrofuran-3,4-diyl diacetate (**17B**)

Tetraacetyl ribose (2.71 g, 2.10 mmol) was added into a flask, heated to 90°C and stirred for 10 minutes, 17A (0.8 g, 4.26 mmol) and stannum tetrachloride (0.005 ml, 0.043 mmol) were added, and the mixture was warmed to 130°C and stirred under reduced pressure for 20 minutes. Then the mixture was cooled, and upon complete depletion of raw materials monitored by LCMS, a product was generated. Dichloromethane was added, and the reaction was directly passed through a column (PE : EA = 1 : 0-4 : 1), to obtain the title compound 17B (3.6 g, crude).
LC-MS (ESI): m/z = 468.1 $[M+Na]^+$.

Step 3: (2R,3R,4S,SR)-2-(4,6-dichloro-1H-benzo[d][1,2,3]triazol-1-yl)-5-(hydroxymethyl)tetrahydrofuran-3,4-diol (**17C**)

17B (3.6 g, 8.07 mmol) and methanol (80 ml) were added into a flask, followed by potassium carbonate (3.35 g, 24.21 mmol), and the mixture was reacted at room temperature for 1 hour. Upon complete depletion of raw materials monitored by LCMS, a product was generated. The mixture was extracted with dichloromethane, washed, dried and concentrated to directly obtain the title compound 17C (630 mg, 24.4%).
LC-MS (ESI): m/z = 320.1 $[M+H]^+$.

Step 4: (2R,3R,4S,5R)-2-(6-chloro-4-(((S)-1-(2-fluorophenyl)ethyl)amino)-1H-benzo[d][1,2,3]triazol-1-yl)-5-(hydroxymethyl)tetrahydrofuran-3,4-diol (**17D**)

17C (100 mg, 0.31 mmol) and tetrahydrofuran (8 ml) were added into a flask, followed by (S)-1-(2-fluorophenyl)ethylamine (86 mg, 0.62 mmol), methanesulfonic acid (2-dicyclohexylphosphino-3,6-dimethoxy -2',4',6'-triisopropyl-1,1 '-biphenyl)(2'-amino-1,1 '-biphenyl-2-yl)palladium(II) (28 mg, 0.031 mmol), 2-(dicyclohexylphosphino)-3,6-dimethoxy-2'-4'-6'-tri-I-propyl-11'-biphenyl (17 mg, 0.031 mmol) and lithium bistrimethylsilylamide (1.0 M/THF, 0.29 ml, 1.55

mmol). Then under nitrogen protection, the mixture was reacted at 70°C for 4 hours. Upon complete depletion of raw materials monitored by LCMS, a product was generated. The product was cooled, saturated ammonium chloride was added, and the mixture was extracted with ethyl acetate, washed, dried, concentrated and passed through a column (PE : EA = 1 : 0-0 : 1) to obtain the title compound 17D (96 mg, 73.2%).

LC-MS (ESI): m/z = 423.2 $[M+H]^+$.

Step 5: (((((2R,3S,4R,SR)-5-(6-chloro-4-(((S)-1-(2-fluorophenyl)ethyl)amino) -1H-benzo[d][1,2,3]triazol-1-yl)-3,4-dihydroxytetrahydrofuran-2-yl)methoxy) (hydroxy)phosphoryl)methyl)phosphonic acid (Compound 17)

17D (92 mg, 0.22 mmol) and trimethyl phosphate (3 ml) were added into a flask, and methylenebis(phosphonic dichloride) (160 mg, 0.66 mmol) was added under an ice bath. The temperature was naturally warmed to room temperature and the reaction was stirred for 3 hours. Upon complete depletion of raw materials monitored by LCMS, a product was generated. 1 ml of water was added and stirred for 10 minutes, and the reaction solution was subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.36 min, and the preparative liquid was concentrated and lyophilized to obtain the title compound 17 (10 mg, 7.8%).

$^1H$ NMR (400 MHz, $CD_3OD-d_4$) δ 7.44 (t, 1H), 7.28 (d, 1H), 7.17 - 7.10(m, 2H), 7.06 (s, 1H), 6.31 (s, 1H), 6.29 - 6.25(m, 1H), 5.11 (d, 1H), 5.00 (t, 1H), 4.37 (s, 2H), 4.25- 4.16 (m, 1H), 4.05 (s, 2H), 2.25 -2.06(m, 2H), 1.64 (d, 3H).

LC-MS (ESI): m/z = 581.2 $[M+H]^+$.

**Example 18:**

(((((2R,3S,4R,5R)-5-(6-chloro-4-(methoxyamino)-1H-pyrazolo[3,4-d]pyrimidin-1-yl)-3,4-dihydroxytetrahydrofuran-2-yl)methoxy)(hydroxy) phosphoryl)methyl)phosphonic acid (Compound 18)

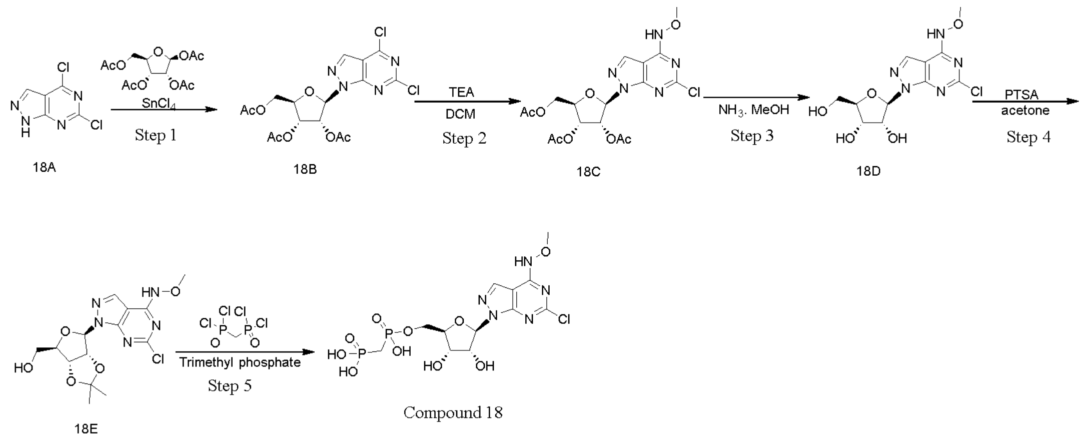


18A 18B 18C 18D

18E Compound 18

Step 1: (2R,3R,4R,5R)-2-(acetoxymethyl)-5-(4,6-dichloro-1H-pyrazolo[3,4-d]pyrimidin-1-yl)tetrahydrofuran-3,4-diyl diacetate (18B)

1, 2, 3, 5-tetraacetyl-β-D-ribofuranose (5.56 g, 17.46 mmol) was heated to 90°C for melting, 18A (3.0 g, 15.87 mmol) and stannum tetrachloride (0.21 g, 0.79 mmol) were added and heated to 130°C under reduced pressure, the mixture was reacted for 15 minutes, after the reaction was completed, the reaction solution was slowly poured to ice water and quenched, extracted with dichloromethane (100 ml × 2), the organic phases were combined, washed with saturated brine, dried over anhydrous sodium sulfate, and filtered, the filtrate was concentrated and then the residue was separated and purified by silica gel column chromatography (petroleum ether : ethyl acetate (v/v) = 5 : 1) to obtain the title compound 18B (4.5 g, 63%).

LC-MS (ESI): m/z = 447.1$[M+H]^+$.

Step 2: (2R,3R,4R,5R)-2-(acetoxymethyl)-5-(6-chloro-4-(methoxyamino)-1H-pyrazolo[3,4-d]pyrimidin-1-yl)tetrahydrofuran-3,4-diyldiacetate (18C)

Compound 18B (1.0 g, 2.24 mmol) was dissolved in dichloromethane (15 ml), methoxylamine hydrochloride (0.37 g, 4.48 mmol) was added, after the addition of triethylamine (1.16 g, 8.96 mmol), the mixture was reacted at room temperature for 3 hours, after the reaction was completed, the reaction solution was directly concentrated, and then the residue was separated and purified by silica gel column chromatography (petroleum ether : ethyl acetate (v/v) = 2 : 1) to obtain the title compound 18C (0.70 g, 68.1%).
LC-MS (ESI): m/z = 458.1 $[M+H]^+$.

Step 3: (2R,3R,4S,5R)-2-(6-chloro-4-(methoxyamino)-1H-pyrazolo[3,4-d]pyrimidin-1-yl)-5-(hydroxymethyl)tetrahydrofuran-3,4-diol (18D)

Compound 18C (0.70 g, 1.53 mmol) was dissolved in methanol (2 ml), ammonia methanol solution (10 ml, 7.0 mol/L) was added, after the addition, the mixture was reacted at room temperature for 15 hours, after the reaction was completed, the reaction solution was directly concentrated to obtain the compound 18D (0.42 g, 82.7%), which product was directly used in the next reaction without purification.
LC-MS (ESI): m/z = 330.1$[M-H]^-$.

Step 4: ((3aR,4R,6R,6aR)-6-(6-chloro-4-(methoxyamino)-1H-pyrazolo[3,4-d]pyrimidin-1-yl)-2,2-dimethyltetrahydrofuro[3,4-d][1,3]dioxol-4-yl)methanol (18E)

Compound 18D (0.42g, 1.27mmol) was dissolved in acetone (5 ml), a catalytic amount of p-toluenesulfonic acid was added, followed by 2,2-dimethoxypropane (0.53g, 5.08mmol), after the addition, the mixture was reacted at room temperature for 2 hours, after the reaction was completed, the reaction was concentrated to remove acetone, water (20ml) was added, extracted with dichloromethane (50ml × 2), washed with saturated brine, dried over anhydrous sodium sulfate, filtered, and the filtrate was concentrated, and the residue was separated and purified by silica gel column chromatography (petroleum ether: ethyl acetate (v/v) = 1 : 1) to obtain the title compound 18E (0.38 g, 80.5%).
LC-MS (ESI): m/z = 372.1 $[M+H]^+$.

Step 5: (((((2R,3S,4R,SR)-5-(6-chloro-4-(methoxyamino)-1H-pyrazolo[3,4-d]pyrimidin-1-yl)-3,4-dihydroxytetrahydrofuran-2-yl)methoxy)(hydroxy) phosphoryl)methyl)phosphonic acid (Compound 18)

Compound 18E (0.10 g, 0.27 mmol) was dissolved in trimethyl phosphate (3 ml), the temperature was decreased to 0°C under an ice bath, methylenebis(phosphonic dichloride) (0.20 g, 0.81 mmol) was dissolved in trimethyl phosphate (0.5 ml), and the mixture was slowly added dropwise into the reaction solution, after the addition, the resulting mixture was naturally warmed to room temperature and reacted for 15 hours, upon completion monitored by LCMS, water (1 ml) was added, followed by 4N hydrochloric acid (1 ml), after the addition, the mixture was stirred for 5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 4.8 min, to obtain the compound 18 (25 mg, 19%).
LC-MS (ESI): m/z = 488.0$[M-H]^-$ .
$^1H$ NMR (400 MHz, DMSO-$d_6$) δ 8.20 (s, 1H), 6.07 (d, 1H), 4.56-4.54 (m, 1H), 4.31-4.29 (m, 1H), 4.13-4.05 (m,2H), 3.94-3.90 (m,1H), 3.82(s, 3H), 2.21-2.11 (m,2H).

**Example 19:**

(((((2R,3S,4R,5R)-5-(6-chloro-4-((2-fluorophenyl)sulfonamido)-1H-pyrazolo [3,4-b]pyridin-1-yl)-3,4-dihydroxytetrahydrofuran-2-yl)methoxy)(hydroxy) phosphoryl)methyl)phosphonic acid (Compound 19)

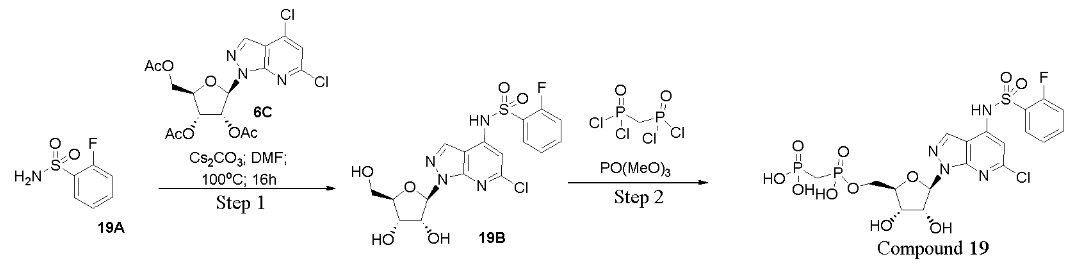


Step 1: N-(6-chloro-1-((2R,3R,4S,SR)-3,4-dihydroxy-5-(hydroxymethyl) tetrahydrofuran-2-yl)-1H-pyrazolo[3,4-b]pyridin-4-yl)-2-fluorobenzenesulfonamide (19B)

Compound 19A (0.70 g, 4.0 mmol) and intermediate 6C (0.90 g, 2.0 mmol), cesium carbonate (3.91 g, 12 mmol) were dissolved in N,N-dimethylformamide (10 ml), the mixture was reacted at 100°C for 16 hours, after the reaction was completed, water (50 ml) was added into the reaction solution to quench the reaction, the aqueous phase was extracted with dichloromethane (150 ml × 2) twice, the organic layers were combined, washed with saturated brine, dried over anhydrous sodium sulfate, filtered, and concentrated, and then the residue was separated and purified by column chromatography (dichloromethane : methanol (v/v) = 10 : 1) to obtain the compound 19B (0.15 g, 16%).
LC-MS (ESI): m/z = 459.10$[M+H]^+$.

Step 2: (((((2R,3S,4R,SR)-5-(6-chloro-4-((2-fluorophenyl)sulfonamido)-1H-pyrazolo[3,4-b]pyridin-1-yl)-3,4-dihydroxytetrahydrofuran-2-yl)methoxy)(hydroxy)phosphoryl)methyl)phosphonic acid (Compound 19)

Compound 19B (0.05 g, 0.11 mmol) was dissolved in trimethyl phosphate (2 ml), the temperature was decreased to 0°C under an ice bath, methylenebis(phosphonic dichloride) (0.11 g, 0.44 mmol) was dissolved in trimethyl phosphate (0.5 ml), and the mixture was slowly added dropwise into the reaction solution, after the addition, the resulting mixture was reacted at 0°C for 3 hours, upon completion monitored by LCMS, water (1 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.0 min, to obtain the compound 19 (25 mg, 37%).
LC-MS (ESI): m/z = 615.0$[M-H]^-$.
$^1H$ NMR (400 MHz, DMSO) δ 8.54 (s, 1H), 8.06 (dd, 1H), 7.79 (dt, 2H), 7.45 (dt, 2H), 6.92 (s, 1H), 6.14 (d, 1H), 4.56 (t, 1H), 4.32 - 4.26 (m, 1H), 4.12 - 4.03 (m, 3H), 3.88 (dd, 3H), 2.18 (t, 2H).

**Example 20:**

(((((2S,4R,SR)-5-(6-chloro-4-(((S)-1-(2-fluorophenyl)ethyl)amino)-1H-pyrazolo[3,4-b]pyridin-1-yl)-4-hydroxy-3-(hydroxymethyl)tetrahydrofuran-2-yl)methoxy)(hydroxy)phosphoryl)methyl)phosphonic acid (Compound 20)

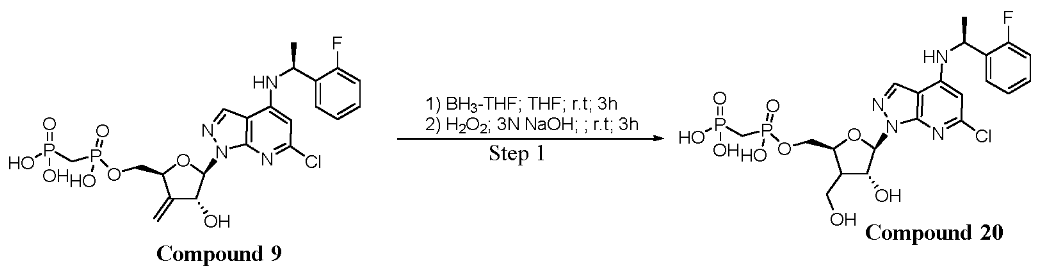

Step 1: (((((2S,4R,5R)-5-(6-chloro-4-(((S)-1-(2-fluorophenyl)ethyl)amino)-1H-pyrazolo[3,4-b]pyridin-1-yl)-4-hydroxy-3-(hydroxymethyl)tetrahydrofuran-2-yl)methoxy)(hydroxy)phosphoryl)methyl)phosphonic acid (Compound 20)

Compound 9 (0.050 g, 0.087 mmol) was dissolved in tetrahydrofuran (5ml) and trimethyl phosphate (2 mL), borane-tetrahydrofuran complex in tetrahydrofuran (1 mL, 1 mmol/mL) was added dropwise at room temperature, after the addition, the mixture was reacted at room temperature for 3 hours, hydrogen peroxide (1 mL) and 3N sodium hydroxide (1 mL) were added, after the addition, the mixture was reacted at room temperature for 3 hours, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.0 min, to obtain the compound 20 (15 mg, 29%).

LC-MS (ESI): m/z = 593.0$[M-H]^-$.

$^1H$ NMR (400 MHz, DMSO) δ 8.38 (s, 1H), 8.21 (d, 1H), 7.40 (t, 1H), 7.32 (dd, 1H), 7.19 (dt, 2H), 6.00 (dd, 2H), 5.07 (s, 1H), 4.73 - 4.63 (m, 1H), 4.49 (t, 1H), 4.38 (d, 1H), 4.31 - 4.13 (m, 2H), 4.05 - 3.94 (m, 2H), 3.87 (s, 1H), 3.75 (d, 1H), 3.68 (s, 2H), 2.39 - 1.86 (m, 3H), 1.58 (d, 3H).

**Example 21**

(((((2R,3 S,4R,SR)-4-amino-5-(6-chloro-4-(((S)-1-(2-fluorophenyl)ethyl) amino)-1H-pyrazolo[3,4-b]pyridin-1-yl)-3-hydroxytetrahydrofuran-2-yl) methoxy)(hydroxy)phosphoryl)methyl)phosphonic acid (Compound 21)

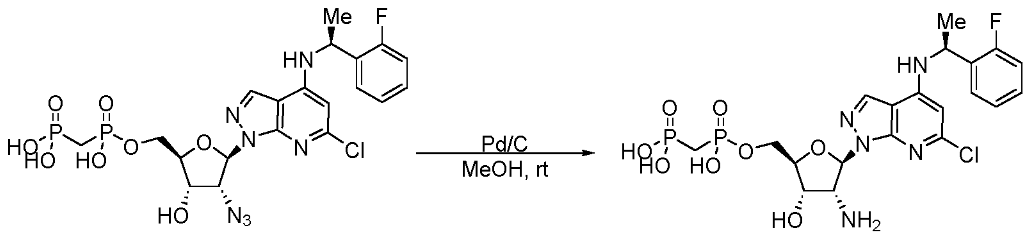


**Compound 13** **Compound 21**

Compound 13 (100 mg, 0.17 mmol), methanol (20 ml), and Pd/C (90 mg) were added into a flask, and the reaction was reduced with hydrogen for 20 minutes at room temperature. Upon complete depletion of raw materials monitored by LCMS, a product was generated. The reaction solution was filtered, concentrated, and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.75 min, and the preparative liquid was concentrated and lyophilized to obtain the title compound 21 as a white solid (40 mg, 40.6%).

$^1H$ NMR (400 MHz, DMSO-$d_6$) δ 8.44 (s, 1H), 8.28 (d, 1H), 7.40 (t, 1H), 7.38 - 7.27 (m, 1H), 7.24 - 7.16 (m, 2H), 6.37 (d, 1H), 6.06 (s, 1H), 5.07 (s, 1H), 4.67 (t, 1H), 4.41 (t, 1H), 4.14 (s, 2H), 3.87 - 3.83 (m, 3H), 1.99 (t, 2H), 1.59 (d, 3H).

LC-MS (ESI): m/z = 580.0 $[M+H]^+$.

**Example 22:**

((((2R,3S,4R,5R)-5-(6-chloro-4-(((S)-1-(2-fluorophenyl)ethyl)amino)-1H-pyrazolo[3,4-b]pyridin-1-yl)-2-ethynyl-3,4-dihydroxytetrahydrofuran-2-yl)methoxy)methyl)phosphonic acid (Compound 22)

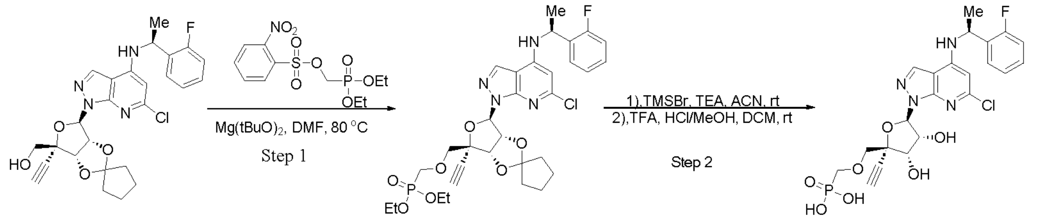


6O 22A Compound 22

Step 1: diethyl((((3a'R,4'R,6'R,6a'S)-4'-(6-chloro-4-(((S)-1-(2-fluorophenyl) ethyl)amino)-1H-pyrazolo[3,4-b]pyridin-1-yl)-6'-ethynyltetrahydrospiro [cyclopentane-1,2'-furo[3,4-d][1,3]dioxol]-6'-yl)methoxy)methyl)phosphonate (22A)

Compound 6O (200 mg, 0.39 mmol) and methyl (diethoxyphosphoryl) 2-nitrobenzenesulfonate (410 mg, 1.17 mmol) were dissolved in dry N,N-dimethylformamide (8 ml), the reaction system was subjected to nitrogen replacement three times, magnesium tert-butoxide (350 mg, 1.95 mmol) was added, after the addition, the mixture was warmed to 80°C and reacted for 48 hours, after the reaction was completed, the reaction was cooled to room temperature and quenched with saturated ammonium chloride solution, then extracted with ethyl acetate (50 ml × 2), the organic layers were combined, washed with saturated brine, dried over anhydrous sodium sulfate, filtered and concentrated, and then the residue was separated and purified by column chromatography (petroleum ether : ethyl acetate (v/v) = 1 : 1-1 : 10) to obtain the compound 22A (55 mg, 21.3%).
LC-MS (ESI): m/z = 663.1[M+H]$^+$.

Step 2: ((((2R,3S,4R,SR)-5-(6-chloro-4-(((S)-1-(2-fluorophenyl)ethyl)amino)-1H-pyrazolo[3,4-b]pyridin-1-yl)-2-ethynyl-3,4-dihydroxytetrahydrofuran-2-yl)methoxy)methyl)phosphonic acid (Compound 22)

Compound 22A (55 mg, 0.083 mmol) was dissolved in acetonitrile (5 ml), triethylamine (250 mg, 2.49 mmol) was added, bromotrimethylsilane (250 mg, 1.66 mmol) was then slowly added dropwise, and after the addition, the mixture was reacted for 2 hours. After the reaction was completed, the mixture was filtered, the filter cake was washed with a small amount of acetonitrile, the filtrate was concentrated and dichloromethane (6 ml) was added, followed by trifluoroacetic acid (0.2 ml) and hydrogen chloride methanol solution (1 ml), after the addition, the mixture was stirred at room temperature for 1 hour, and upon completion monitored by LCMS, the reaction solution was concentrated, and then subjected to preparative HPLC with preparative HPLC separation methods: 1. Instrument: waters 2767 (preparative liquid phase chromatographic instrument), chromatographic column : SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.47 min, and the preparative liquid was lyophilized to obtain the compound 22 (12 mg, 22.4%).
$^1$H NMR (400 MHz, DMSO-$d_6$) δ 8.38 (s, 1H), 8.21 (d, 1H), 7.41 - 7.38 (m, 1H), 7.35 - 7.29 (m, 1H), 7.24 - 7.15 (m, 2H), 6.13 (d, 1H), 6.02 (s, 1H), 5.33 (d, 1H), 5.07 (s, 1H), 4.76 (q, 1H), 4.26 (d, 1H), 3.73 (d, 1H), 3.63 - 3.59 (m, 3H), 3.50 (s, 1H), 1.58 (d, 3H).
LC-MS (ESI): m/z = 541.1 [M+H]$^+$.

**Example 23**

(aminooxy)((((2R,3 S,4R, SR)-5-(6-chloro-4-(((S)-1-(2-fluorophenyl)ethyl) amino)-1H-pyrazolo[3,4-b]pyridin-1-yl)-3,4-dihydroxytetrahydrofuran-2-yl)methoxy)methyl)phosphinic acid (Compound 23)

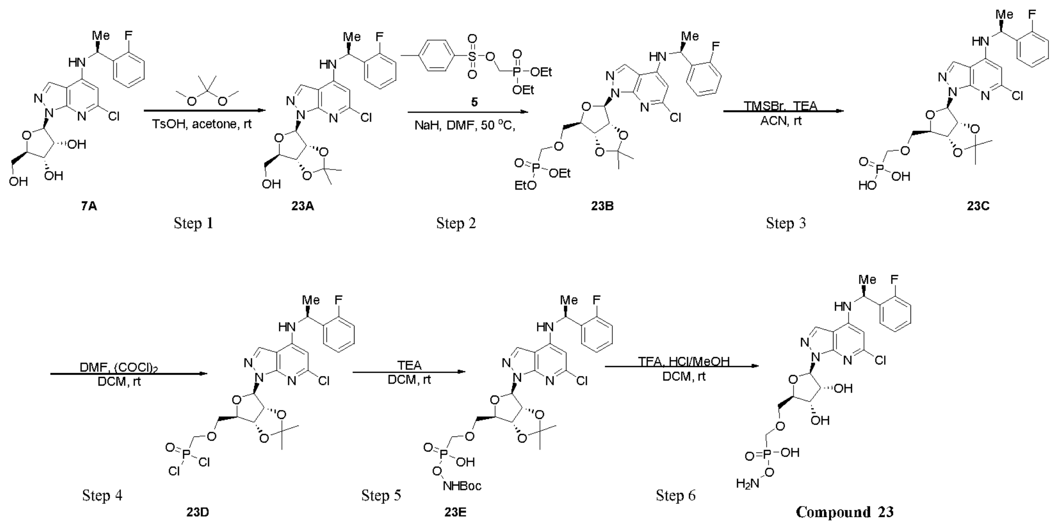


Step 1: ((3aR,4R,6R,6aR)-6-(6-chloro-4-(((S)-1-(2-fluorophenyl)ethyl)amino) -1H-pyrazolo[3,4-b]pyridin-1-yl)-2,2-dimethyltetrahydrofuro[3,4-d][1,3]dioxol-4-yl)methanol (23A)

7A (2.0 g, 4.73 mmol) was dissolved in acetone (20 ml), 2,2-dimethoxypropane (2.46 g, 23.65 mmol) and p-toluenesulfonic acid (0.08 g, 0.47 mmol) were added, after the addition, the mixture was reacted at room temperature for 2 hours, after the reaction was completed, saturated sodium bicarbonate solution (20 ml) was added into the reaction solution, the mixture was extracted with dichloromethane, washed with saturated brine, dried over anhydrous sodium sulfate, concentrated and passed through a column (PE : EA = 1 : 0-1 : 1) to obtain the title compound 23A (2.1 g, 95.9%).

LC-MS (ESI): m/z = 463.2 $[M+H]^+$.

Step 2: diethyl ((((3aR,4R,6R,6aR)-6-(6-chloro-4-(((S)-1-(2-fluorophenyl) ethyl)amino)-1H-pyrazolo[3,4-b]pyridin-1-yl)-2,2-dimethyltetrahydrofuro[3,4-d][1,3]dioxol-4-yl)methoxy)methyl)phosphonate (23B)

23A (2.0 g, 4.32 mmol) was dissolved in dry N,N-dimethylformamide (40 ml), sodium hydride (390 mg, 12.96 mmol) was added and the mixture was reacted at room temperature for 10 minutes; diethyl(tosyloxy)methylphosphonate (4.18 g, 12.96 mmol) was added, after the addition, the mixture was warmed to 50°C and reacted for 16 hours, after the reaction was completed, the mixture was cooled to room temperature and extracted with ethyl acetate (100 ml × 3), the organic layers were combined, washed with saturated brine, dried over anhydrous sodium sulfate, filtered, and concentrated, and then the residue was separated and purified by column chromatography (petroleum ether: ethyl acetate (v/v) = 5 : 1-1 : 10) to obtain the compound 23B (2.0 g, 75.5%).

LC-MS (ESI): m/z = 613.2 $[M+H]^+$.

Step 3: ((((3aR,4R,6R,6aR)-6-(6-chloro-4-(((S)-1-(2-fluorophenyl)ethyl) amino)-1H-pyrazolo[3,4-b]pyridin-1-yl)-2,2-dimethyltetrahydrofuro[3,4-d][1,3]dioxol-4-yl)methoxy)methyl)phosphonic acid (23C)

23B (0.5 g, 0.82 mmol) was dissolved in acetonitrile (10 ml), triethylamine (0.83 g, 8.2 mmol) was added, bromotrimethylsilane (1.62 ml, 12.30 mmol) was then slowly added dropwise, and after the addition, the mixture was reacted for 2 hours. After the reaction was completed, the mixture was concentrated to obtain the target compound 23C (2.1 g, crude).

LC-MS (ESI): m/z = 557.2 $[M+H]^+$.

Step 4: ((((3aR,4R,6R,6aR)-6-(6-chloro-4-(((S)-1-(2-fluorophenyl)ethyl) amino)-1H-pyrazolo[3,4-b]pyridin-1-yl)-2,2-dimethyltetrahydrofuro[3,4-d][1,3]dioxol-4-yl)methoxy)methyl)phosphonic dichloride (23D)

23C (2.0 g, crude ) and 2 drops of a catalytic amount of N,N-dimethylformamide were added into a flask. Oxalyl chloride (0.76 ml, 8.97 mmol) was added dropwise under an ice bath, the temperature was naturally warmed to room temperature and the reaction was stirred for 1 hour. The reaction solution was concentrated to obtain the target compound 23D (2.5 g, crude).

Step 5: (((tert-butoxycarbonyl)amino)oxy)((((3aR,4R,6R,6aR)-6-(6-chloro-4-(((S)-1-(2-fluorophenyl)ethyl)amino)-1H-pyrazolo[3,4-b]pyridin-1-yl)-2,2-dimethyltetrahydrofuro[3,4-d][1,3]dioxol-4-yl)methoxy)methyl)phosphinic acid (23E)

23D (2.5 g, crude) and dichloromethane (10 ml), tert-butyl N-hydroxycarbamate (220 mg, 1.64 mmol) were added into a flask, followed by triethylamine (250 mg, 2.46 mmol). At room temperature, the mixture was stirred for 2 hours. Upon complete depletion of raw materials monitored by LCMS, a product was generated. The reaction solution was slowly poured into water, extracted with dichloromethane, washed, dried, and concentrated to obtain a gray target product 23E (300 mg, crude).

LC-MS (ESI): m/z = 672.2 $[M+H]^+$.

Step 6: (aminooxy)((((2R,3S,4R,SR)-5-(6-chloro-4-(((S)-1-(2-fluorophenyl) ethyl)amino)-1H-pyrazolo[3,4-b]pyridin-1-yl)-3,4-dihydroxytetrahydrofuran-2-yl)methoxy)methyl)phosphinic acid (Compound 23)

23E (300 mg, crude ) and dichloromethane (10 ml) were added into a flask, trifluoroacetic acid (1 ml, 13.42 mmol) and hydrogen chloride methanol solution (1 ml) were added at room temperature. The reaction was stirred and reacted at room temperature for 2 hours. Raw materials were completely depleted as monitored by LCMS. The reaction solution was concentrated to dryness and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.6 min, and the preparative liquid was concentrated and lyophilized to obtain the target compound (25 mg, 10.45%).

$^1$H NMR (400 MHz, DMSO-*d6*) δ 8.37 (s, 1H), 8.26 (d, 1H), 7.40 (t, 1H), 7.32 - 7.28(m, 1H), 7.24 - 7.14 (m, 2H), 6.03 (d, 1H), 6.00 (s, 1H), 5.06 (s, 1H), 4.49 (t, 1H), 4.24 (d, 1H), 4.00 - 3.95 (m, 1H), 3.65 - 3.61 (m, 1H), 3.50 - 3.46 (m, 1H), 3.37 (s, 2H), 3.34 (s, 2H), 1.58 (d, 3H).

**Example 24:**

(((((2R,3S,4R,5R)-5-(6-chloro-4-(methoxyamino)-1H-pyrazolo[3,4-b]pyridin-1-yl)-3,4-dihydroxytetrahydrofuran-2-yl)methoxy)(hydroxy)phosphoryl)methyl) phosphonic acid (Compound 24)

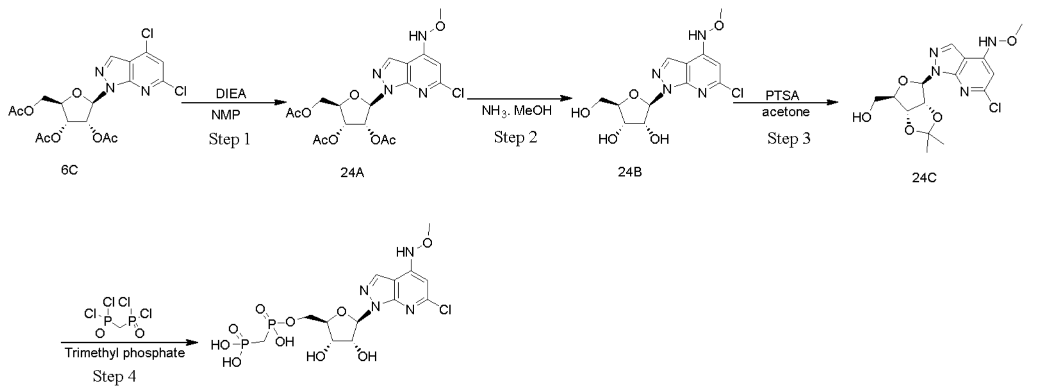

6C 24A 24B 24C

Compound 24

Step 1: (2R,3R,4R,5R)-2-(acetoxymethyl)-5-(6-chloro-4-(methoxyamino)-1H-pyrazolo[3,4-b]pyridin-1-yl)tetrahydrofuran-3,4-diyl diacetate (24A)

Compound 6C (0.5 g, 1.12 mmol) was dissolved in N-methylpyrrolidone (5 ml), methoxylamine hydrochloride (0.18 g, 2.24 mmol) and N,N-diisopropyl ethylamine (0.58 g, 4.48 mmol) were then added, after the addition, the mixture was warmed to 120°C and reacted for 3 hours, after the reaction was completed, the reaction solution was cooled to room temperature, water (20 ml) was added, extracted with ethyl acetate (50 ml × 2), the organic phases were combined, washed with saturated brine, dried over anhydrous sodium sulfate, filtered, the filtrate was concentrated, and then the residue was separated and purified by silica gel column chromatography (petroleum ether : ethyl acetate (v/v) = 4 : 1)

to obtain the title compound 24A (0.30 g, 58.9%).

LC-MS (ESI): m/z = 457.1 $[M+H]^+$.

Step 2: (2R,3R,4S,5R)-2-(6-chloro-4-(methoxyamino)-1H-pyrazolo[3,4-b]pyridin -1-yl)-5-(hydroxymethyl)tetrahydrofuran-3,4-diol (24B)

Compound 24A (0.30 g, 0.65 mmol) was dissolved in methanol (2 ml), ammonia methanol solution (3 ml, 7.0 mol/L) was added, after the addition, the mixture was reacted at room temperature for 15 hours, after the reaction was completed, the reaction solution was directly concentrated to obtain the compound 24B (0.17 g, 80.1%), which product was directly used in the next reaction without purification.

LC-MS (ESI): m/z = 329.1$[M-H]^-$ .

Step 3: ((3aR,4R,6R,6aR)-6-(6-chloro-4-(methoxyamino)-1H-pyrazolo[3,4-b]pyridin-1-yl)-2,2-dimethyltetrahydrofuro[3,4-d][1,3]dioxol-4-yl)methanol (24C)

Compound 24B (0.17 g, 0.52 mmol) was dissolved in acetone (5 ml), a catalytic amount of p-toluenesulfonic acid was added, followed by 2,2-dimethoxypropane (0.26 g, 2.54 mmol), after the addition, the mixture was reacted at room temperature for 2 hours, after the reaction was completed, the reaction was concentrated to remove acetone, water (100 ml) was added, extracted with dichloromethane (25 ml × 2), washed with saturated brine, dried over anhydrous sodium sulfate, filtered, and the filtrate was concentrated, and the residue was separated and purified by silica gel column chromatography (petroleum ether : ethyl acetate (v/v) = 1 : 1) to obtain the title compound 24C (0.1 g, 52.6%).

LC-MS (ESI): m/z = 371.1 $[M+H]^+$.

Step 4: (((((2R,3S,4R,5R)-5-(6-chloro-4-(methoxyamino)-1H-pyrazolo[3,4-b]pyridin-1-yl)-3,4-dihydroxytetrahydrofuran-2-yl)methoxy)(hydroxy)phosphoryl) methyl)phosphonic acid (Compound 24)

Compound 24C (0.10 g, 0.27 mmol) was dissolved in trimethyl phosphate (3 ml), the temperature was decreased to 0°C under an ice bath, methylenebis(phosphonic dichloride) (0.20 g, 0.81 mmol) was dissolved in trimethyl phosphate (0.5 ml), and the mixture was slowly added dropwise into the reaction solution, after the addition, the mixture was naturally warmed to room temperature and reacted for 15 hours, upon completion monitored by LCMS, water (1 ml) was added, followed by 4N hydrochloric acid (1 ml), after the addition, the mixture was stirred for 5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 5.2 min, to obtain the compound 24 (21 mg, 16.1%).

LC-MS (ESI): m/z = 487.0$[M-H]^-$.

$^1$H NMR (400 MHz, DMSO-*d6*) δ 11.04 (s, 1H), 8.10 (s, 1H), 6.41 (s, 1H), 6.14 (d, 1H), 4.58-4.55 (m, 1H), 4.31-4.28 (m, 1H), 4.12-4.04 (m, 2H), 3.93-3.89 (m, 1H), 3.77 (s, 3H), 2.23-2.13 (m, 2H).

**Example 25:**

(((((2R,3S,4R,5R)-5-(6-chloro-4-((cyclopentyloxy)amino)-1H-pyrazolo[3,4-d]pyrimidin-1-yl)-3,4-dihydroxytetrahydrofuran-2-yl)methoxy)(hydroxy) phosphoryl)methyl)phosphonic acid (Compound 25)

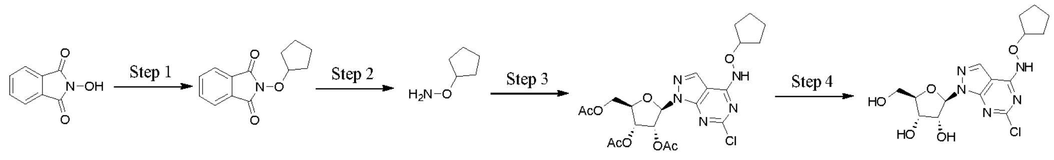


25A 25B 25C 25D

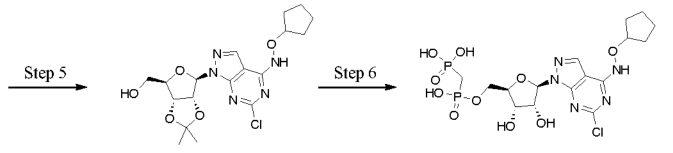


25E Compound 25

Step 1: 2-(cyclopentyloxy)isoindoline-1,3-dione (25A)

The known compound 2-hydroxylisoindoline-1,3-dione (3.4 g, 20.8 mmol) and triphenylphosphine (6.55 g, 25.0 mmol) were added into ultra-dried tetrahydrofuran (34 mL), under nitrogen protection, the mixture was cooled to 0°C, diisopropyl azodicarboxylate (5.05 g, 25.0 mmol) was added dropwise, and then stirred for 30 min, cyclopentanol (2.0 g, 20.8 mmol) was added dropwise, the mixture was stirred at room temperature for 16 hours, water (50 mL) was added, extracted with EA (50 mL), the EA phase was concentrated to dryness, and the residue was purified by column chromatography, to obtain 1.4 g of 25A (yield = 29%).
LCMS m/z = 242.1 $[M+1]^+$.

Step 2: O-cyclopentylhydroxylamine (25B)

2-(Cyclopentyloxy)isoindoline-1 3-dione (25A) (1.4 g, 6.0 mmol) was added into dichloromethane (14 mL), hydrazine hydrate (0.73 g, 12 mmol) was then added, the mixture was stirred at room temperature for 16 h, a large amount of white solids was precipitated, filtered, the filter cake was washed with dichloromethane, water was added into the mother liquor, and the resulting mixture was stirred, liquid separation was then performed, 10 ml of hydrogen chloride/1,4-dioxane solution was added into the dichloromethane phase, the mixture was stirred for 5 minutes, and concentrated to dryness, to obtain 0.8 g of 25B (yield = 96%).
LCMS m/z = 102.2 $[M+1]^+$.

Step 3: (2R,3R,4R,5R)-2-(acetoxymethyl)-5-(6-chloro-4-((cyclopentyloxy) amino)-1H-pyrazolo[3,4-d]pyrimidin-1-yl)tetrahydrofuran-3,4-diyl diacetate (25C)

18B (0.5 g, 1.11 mmol) and o-cyclopentylhydroxylamine hydrochloride (0.14 g, 1.33 mmol) were added into dichloromethane, triethylamine (0.5 g, 4.44 mmol) was added, the mixture was stirred at room temperature for 16 h, water (10 mL) was added, extracted with dichloromethane (10 mL), the dichloromethane phase was concentrated to dryness, and the residue was purified by column chromatography (PE : EA = 2 : 1) to obtain 0.3 g of 25C (yield = 70%).
LCMS m/z = 512.2 $[M+1]^+$.

Step 4: (2R,3R,4S,5R)-2-(6-chloro-4-((cyclopentyloxy)amino)-1H-pyrazolo [3,4-d]pyrimidin-1-yl)-5-(hydroxymethyl)tetrahydrofuran-3,4-diol (25D)

Compound 25C (0.3 g, 0.58 mmol) was added into ammonia/methanol solution (3 mL), the mixture was stirred at room temperature overnight, and directly concentrated to dryness, to obtain 220 mg of 25D (yield = 100%).
LCMS m/z = 386.2 $[M+1]^+$.

Step 5: ((3aR,4R,6R,6aR)-6-(6-chloro-4-((cyclopentyloxy)amino)-1H-pyrazolo[3,4-d]pyrimidin-1-yl)-2,2-dimethyltetrahydrofuro[3,4-d][1,3]dioxol-4-yl)methanol (25E)

Compound 25D (220 mg, 0.57 mmol) and 2,2-dimethoxypropane (307 mg, 2.85 mmol) were added into acetone (4.4 mL), followed by a catalytic amount of p-toluenesulfonic acid, and the mixture was stirred at room temperature for 3 hours. Water (20 mL) was added, and the mixture was extracted with ethyl acetate (20 mL), then concentrated to dryness, and purified by column chromatography (PE : EA = 2 : 1) to obtain 200 mg of 25E (yield = 82%).
LCMS m/z = 426.2 $[M+1]^+$.

Step 6: (((((2R,3S,4R,5R)-5-(6-chloro-4-((cyclopentyloxy)amino)-1H-pyrazolo[3,4-d]pyrimidin-1-yl)-3,4-dihydroxytetrahydrofuran-2-yl)methoxy)(hydroxy)phosphoryl)methyl)phosphonic acid (Compound 25)

Compound 25E (200 mg, 0.47 mmol) was added into trimethyl phosphate (2 mL), the mixture was cooled to 0°C, methylene bis(dichlorophosphine) (106 mg, 1.41 mmol) was added, the mixture was stirred at room temperature for 2 hours, 1 mL of 6 N hydrochloric acid solution was added into the reaction system, the resulting mixture was stirred for 1 h, and separated and purified by a preparative liquid chromatography column (liquid phase preparative conditions: C18 reversed phase preparative column; mobile phase: deionized water containing 0.1% trifluoroacetic acid (A) and acetonitrile containing 0.1% trifluoroacetic acid (B); gradient elution: B = 5%-70%; elution time: 15 min; flow rate: 12 mL/min; column temperature: 30°C, retention time: 4.56 min), to obtain 100 mg (compound 25) (yield = 39%).
$^1$HNMR (400 MHz, DMSO-*d6*) δ 8.08 (s, 1H), 7.00-5.50 (m, 7H), 4.56-4.50 (m, 2H), 4.30-4.28 (t, 1H), 4.14-4.06 (m, 2H), 3.95-3.90 (m, 1H), 2.24-2.14 (t, 2H), 1.95-1.88 (m, 2H), 1.81-1.70 (m, 4H), 1.58-1.54 (m, 2H).
LCMS m/z = 544.1 $[M+1]^+$.

**Example 26:**

(((((2R,3S,4R,5R)-5-(6-chloro-4-(isopropoxyamino)-1H-pyrazolo[3,4-b]pyridin-1-yl)-3,4-dihydroxytetrahydrofuran-2-yl)methoxy)(hydroxy) phosphoryl)methyl)phosphonic acid (Compound 26)

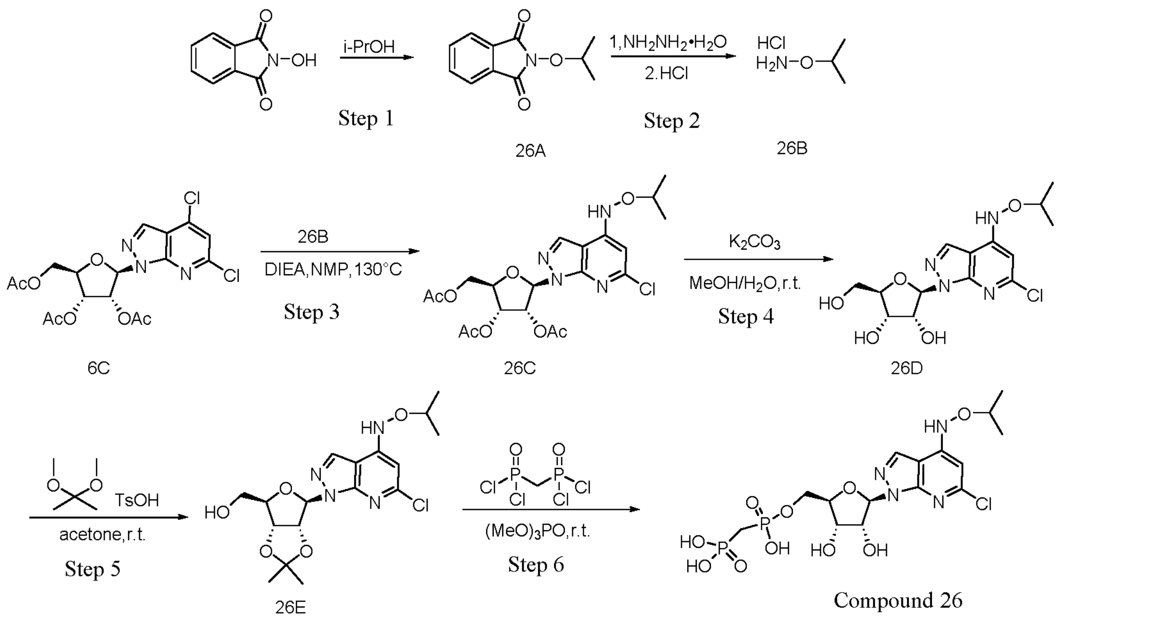


Step 1: 2-isopropoxyisoindoline-1,3-dione (26A)

2-Hydroxylisoindoline-1,3-dione (1 g, 6.13 mmol), isopropanol (0.47 ml, 6.13 mmol) and triphenylphosphine (1.93 g, 7.36 mmol) were dissolved in anhydrous tetrahydrofuran (40 ml), the mixture was stirred under an ice-water bath, diisopropyl azodicarboxylate (1.487 g, 7.36 mmol) was added dropwise, then the ice-water bath was removed, the mixture was stirred at room temperature overnight, after the reaction was completed, the mixture was concentrated, and the resulting residue was purified by silica gel column chromatography (eluent: EA : PE = 0%-80%), to obtain the product 26A (1.12 g, 89.0%), as a colorless oil.
$^1$H NMR (400 MHz, DMSO) δ 7.87 (s, 4H), 4.48-4.43 (m, 1H), 1.30 (d, 6H).

Step 2: O-isopropylhydroxylamine hydrochloride (26B)

Compound 26A (1.12 g, 5.46 mmol) was dissolved in dichloromethane (10 ml), 80% hydrazine hydrate solution (0.66 ml, 10.93 mmol) was added, the mixture was stirred at room temperature overnight, after the reaction was completed, the mixture was filtered, the filtrate was washed with water (10 ml) and saturated brine (10 ml), 4 M hydrogen chloride in dioxane (1.4 ml) was added into the filtrate, and the mixture was concentrated, to obtain the compound 26B (320 mg,

52.8%), as a light yellow solid.
$^1$H NMR (400 MHz, DMSO-*d6*) δ 11.00 (s, 3H), 4.36 (s, 1H), 1.23 (s, 6H).

Step 3: (2R,3R,4R,5R)-2-(acetoxymethyl)-5-(6-chloro-4-(isopropoxyamino)-1H-pyrazolo[3,4-b]pyridin-1-yl)tetrahydrofuran-3,4-diyl diacetate (26C)

Compound 6C (500 mg, 1.12 mmol), compound 26B (187 mg, 1.68 mmol) and N,N-diisopropyl ethylamine (434 mg, 3.36 mmol) were dissolved in N-methylpyrrolidone (8 ml), refluxed and reacted at 130°C for 3h, after the reaction was completed, water (10 ml) and ethyl acetate (20 ml) were added, organic phases were separated, the aqueous phase was extracted with ethyl acetate (10 ml × 2), the organic phases were combined, dried over anhydrous sodium sulfate, concentrated, and the resulting residue was purified by silica gel column chromatography (eluent: ethyl acetate :petroleum ether = 0%-50%), to obtain the compound 26C (253 mg, 46.6%), as a white solid.
LCMS m/z = 485.1 $[M+1]^+$.

Step 4: (2R,3R,4S,5R)-2-(6-chloro-4-(isopropoxyamino)-1H-pyrazolo[3,4-b]pyridin-1-yl)-5-(hydroxymethyl)tetrahydrofuran-3,4-diol (26D)

Compound 26C (253 mg, 1.523 mmol) and potassium carbonate (220 mg, 1.57 mmol) were dissolved in methanol (5 ml), stirred at room temperature for 1h, after the reaction was completed, the mixture was adjusted to pH 6-7 with 1 M hydrochloric acid, extracted with ethyl acetate (10 ml × 4), the organic phases were combined, dried over anhydrous sodium sulfate, and concentrated, to obtain the compound 26D (184 mg, 98.6%), as a light yellow solid.
LCMS m/z = 359.2 $[M+1]^+$.

Step 5: ((3aR,4R,6R,6aR)-6-(6-chloro-4-(isopropoxyamino)-1H-pyrazolo[3,4-b]pyridin-1-yl)-2,2-dimethyltetrahydrofuro[3,4-d][1,3]dioxol-4-yl)methanol (26E)

Compound 26D (184 mg, 0.514 mmol), 2,2-dimethoxypropane (267 mg, 2.57 mmol) and p-toluenesulfonic acid (10 mg, 0.0514 mmol) were dissolved in acetone (5 ml), stirred at room temperature overnight, after the reaction was completed, saturated sodium bicarbonate solution was added to adjust the solution to pH 6-7, the mixture was concentrated, and the resulting residue was purified by silica gel column chromatography (eluent: ethyl acetate : petroleum ether = 0%-50%), to obtain the compound 26E (126 mg, 61.9%).
LCMS m/z = 399.2 $[M+1]^+$.

Step 6: (((((2R,3S,4R,5R)-5-(6-chloro-4-(isopropoxyamino)-1H-pyrazolo[3,4-b]pyridin-1-yl)-3,4-dihydroxytetrahydrofuran-2-yl)methoxy)(hydroxy)phosphoryl) methyl)phosphonic acid (Compound 26)

Compound 26E (126 mg, 0.317 mmol) was dissolved in trimethyl phosphate (1 ml), stirred under an ice-water bath at 0°C, methylenebis(phosphonic dichloride) (237 mg, 0.950 mmol) was dissolved in trimethyl phosphate (2 ml) and the mixture was added dropwise into the reaction flask, the mixture was then warmed to room temperature and stirred overnight, 6M hydrochloric acid (3 ml) was added, and the resulting mixture was stirred at room temperature for 6h, after the reaction was completed, the mixture was concentrated, and the residue was separated and purified by a preparative liquid chromatography column with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 10.0 min, to obtain the compound 26 (20 mg, 12.9%), as a light yellow solid.
LCMS m/z = 517.0 $[M+1]^+$.
$^1$H NMR (400 MHz, DMSO) δ 10.84 (s, 1H), 8.12 (s, 1H), 6.41 (s, 1H), 6.13 (d, 1H), 4.57 (t, 1H), 4.29 (t, 1H), 4.08 (dt, 3H), 3.88 (d, 2H), 2.16 (t, 2H), 1.27 (d, 6H).

**Example 27:**

(((((2R,3S,4R,5R)-5-(6-chloro-4-(isopropoxyamino)-1H-pyrazolo[3,4-d]pyrimidin-1-yl)-3,4-dihydroxytetrahydrofuran-2-yl)methoxy)(hydroxy) phosphoryl)methyl)phosphonic acid (Compound 27)

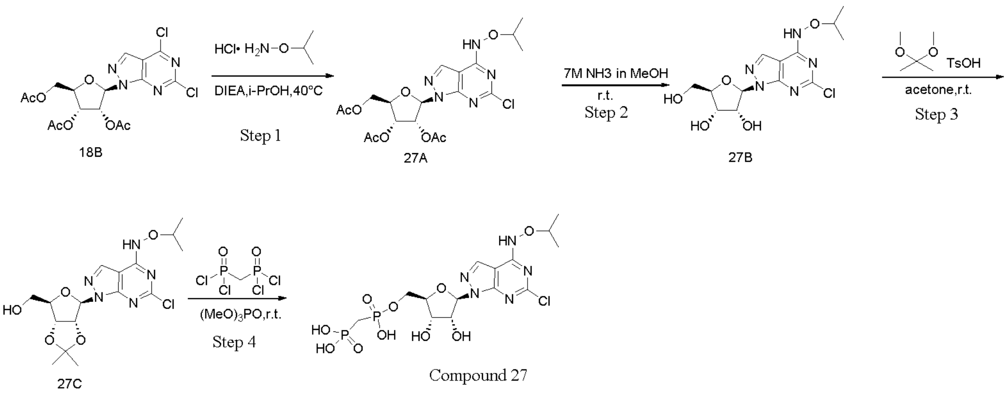


Step 1: (2R,3R,4R,5R)-2-(acetoxymethyl)-5-(6-chloro-4-(isopropoxyamino)-1H-pyrazolo[3,4-d]pyrimidin-1-yl)tetrahydrofuran-3,4-diyl diacetate (27A)

Substrate 18B (600 mg, 1.34 mmol), compound 26B (223 mg, 2.01 mmol) and diisopropylethylamine (519 mg, 4.03 mmol) were dissolved in isopropanol (14 ml), refluxed at 40°C for 5 h, after the reaction was completed, the mixture was concentrated, and the resulting residue was purified by silica gel column chromatography (eluent: EA : PE = 0%-50%), to obtain the product 27A (527 mg, 80.9%).

$^1$H NMR (400 MHz, $CDCl_3$) δ 8.14 (s, 1H), 6.52 (d, 1H), 5.94 (dd, 1H), 5.78 (t, 1H), 4.47 - 4.38 (m, 2H), 4.32 - 4.14 (m, 2H), 2.16 - 2.05 (m, 9H), 1.42 - 1.33 (m, 6H).

Step 2: (2R,3R,4S,5R)-2-(6-chloro-4-(isopropoxyamino)-1H-pyrazolo[3,4-d]pyrimidin-1-yl)-5-(hydroxymethyl)tetrahydrofuran-3,4-diol (27B)

Compound 27A (523 mg, 1.08 mmol) was dissolved in 7M ammonia methanol solution (10 ml), stirred at room temperature for 5.5 h, after the reaction was completed, the mixture was concentrated, to obtain the compound 27B (380 mg, 100%), as a yellow oil.

LCMS m/z = 360.1[M+1]$^+$.

Step 3: ((3aR,4R,6R,6aR)-6-(6-chloro-4-(isopropoxyamino)-1H-pyrazolo[3,4-d]pyrimidin-1-yl)-2,2-dimethyltetrahydrofuro[3,4-d][1,3]dioxol-4-yl)methanol (27C)

Compound 27B (380 mg, 1.08 mmol), 2,2-dimethoxypropane (550 mg, 5.40 mmol) and p-toluenesulfonic acid (20 mg, 0.108 mmol) were dissolved in acetone (10 ml), stirred at room temperature overnight, after the reaction was completed, saturated sodium bicarbonate solution was added to adjust the solution to pH 6-7, the mixture was concentrated, and the resulting residue was purified by silica gel column chromatography (eluent: EA : PE = 0%-50%), to obtain the product 27C (323 mg, 76.9%).

LCMS m/z = 400.1 [M+1]$^+$.

Step 4: (((((2R,3S,4R,5R)-5-(6-chloro-4-(isopropoxyamino)-1H-pyrazolo[3,4-d]pyrimidin-1-yl)-3,4-dihydroxytetrahydrofuran-2-yl)methoxy)(hydroxy) phosphoryl)methyl)phosphonic acid (Compound 27)

Compound 27C (323 mg, 0.809 mmol) was dissolved in trimethyl phosphate (2 ml), stirred under an ice-water bath at 0°C, methylenebis(phosphonic dichloride) (607 mg, 2.43 mmol) was dissolved in trimethyl phosphate (2 ml) and the mixture was added dropwise into the reaction flask, the mixture was then warmed to room temperature and stirred overnight, 6M hydrochloric acid (3 ml) was added, and the resulting mixture stirred at room temperature for 6h, after the reaction was completed, the mixture was concentrated, and the residue was separated and purified by a preparative liquid chromatography column with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 12.0 min, to obtain the compound 27 (162 mg, 38.6%).

LCMS m/z = 518.1 [M+1]$^+$.

$^1$H NMR (400 MHz, DMSO) δ 8.17 (s, 1H), 6.07 (d, 1H), 4.55 (t, 1H), 4.29 (t, 1H), 4.19 (dq, 1H), 4.10 (ddd, 2H),

3.96 - 3.87 (m, 1H), 2.19 (t, 2H), 1.27 (t, 6H).

**Example 28:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 28)

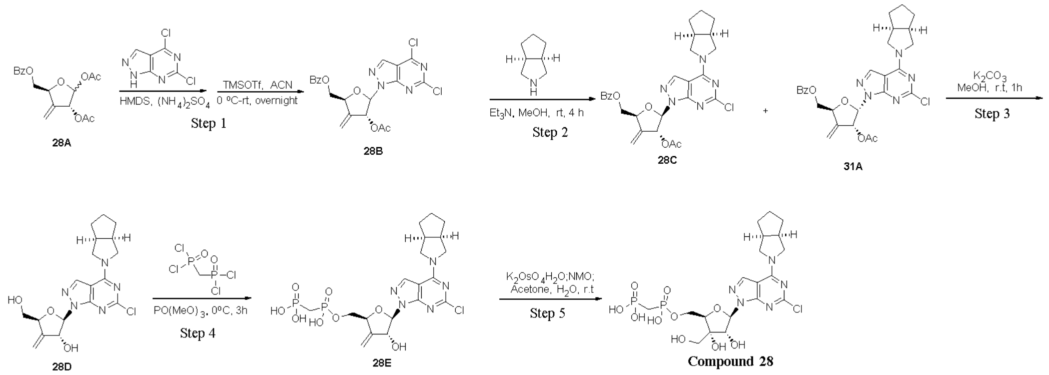


Step 1: ((2S,4R)-4-acetoxy-5-(4,6-dichloro-1H-pyrazolo[3,4-d]pyrimidin-1-yl)-3-methylenetetrahydrofuran-2-yl)methyl benzoate (28B)

The compound 4,6-dichloro-1H-pyrazolo[3,4-d]pyrimidine (3.68 g, 19.46 mmol) was dissolved in hexamethyldisilazane (50 ml), ammonium sulfate (30 mg, 0.2 mmol) was added, the mixture was refluxed for 3 hours, concentrated under reduced pressure to remove hexamethyldisilazane, the crude was dissolved in acetonitrile (50 ml), 28A (5 g, 14.97 mmol) was added, and the mixture was cooled to 0 °C, trimethylsilyl trifluoromethanesulfonate (333 mg, 14.97 mmol) was added dropwise, stirred at room temperature for 18 h, after the reaction was completed, ethyl acetate (50 ml) was added for dilution, the mixture was washed with aqueous sodium bicarbonate solution (20 ml) and saturated brine (20 ml), the organic phase was dried over anhydrous sodium sulfate, and concentrated, and the resulting residue was purified by silica gel column chromatography (eluent: EA : PE = 0%-50%), to obtain the product (4.5 g, 65%).

Step 2: ((2S,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-methylenetetrahydrofuran-2-yl)methyl benzoate (28C)

Compound 28B (4.5 g, 9.72 mmol) was dissolved in methanol (50 ml), triethylamine (2.45 g, 24.3 mmol) and cis-7-azabicyclo[3.3.0]octane (1.29 g, 11.66 mmol) were added, after the addition, the mixture was reacted at room temperature for 3 hours, and after the reaction was completed, the mixture was concentrated under reduced pressure to obtain a mixture of 28C and 31A.

LC-MS (ESI): m/z = 538.2 $[M+1]^+$.

Purification of compounds 28C and 31A

The above mixture was used for purification, and after separation, compound 28C (retention time: 0.794 s, 1.23 g) and compound 31A (retention time: 1.366 s, 0.23 g) were obtained.

Purification conditions:

instrument: Waters 150 MGM; column: DAICEL CHIRALPAK AD;
mobile phase: A for $CO_2$ and B for EtOH (0.1%$NH_3$•$H_2O$); gradient: B 40%; flow rate: 120 mL /min; back pressure: 100 bar;
column temperature: 35°C; wavelength: 220 nm; cycle: 13 min;

Step 3: (2R,3R,5S)-2-(6-chloro-4-((3aR,6aS)-hexahydrocyclopenta[c]pyrrol-2(1H)-yl)-1H-pyrazolo[3,4-d]pyrimidin-1-yl)-5-(hydroxymethyl)-4-methylenetetrahydrofuran-3-ol(28D)

Compound 28C (300 mg, 0.56 mmol) was dissolved in methanol (3 ml), potassium carbonate (309 mg, 2.24 mmol) was added, after the addition, the mixture was reacted at room temperature for 2 hours, water (3 ml) was added

to quench the reaction, the mixture was extracted with ethyl acetate (5 ml × 3), dried over anhydrous sodium sulfate, concentrated and the resulting residue was purified by silica gel column chromatography (eluent: EA : PE = 0%-70%), to obtain the product 28D (295 mg, 98%).

LC-MS (ESI): m/z = 392.1 $[M+1]^+$.

Step 4: (((((2S,4R,5R)-5-(6-chloro-4-((3aR,6aS)-hexahydrocyclopenta[c] pyrrol-2(1H)-yl)-1H-pyrazolo[3,4-d]pyrimidin-1-yl)-4-hydroxy-3-methylenetetrahydrofuran-2-yl)methoxy)(hydroxy)phosphoryl)methyl)phosphonic acid (28E)

Compound 28D (295 mg, 0.57 mmol) was dissolved in trimethyl phosphate (3 ml), the temperature was decreased to 0°C under an ice bath, methylenebis(phosphonic dichloride) (570 mg, 2.28 mmol) was dissolved in trimethyl phosphate (2 ml), and the mixture was slowly added dropwise into the reaction solution, after the addition, the mixture was reacted at 0°C for 3 hours, upon completion monitored by LCMS, water (2 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 28E (100 mg, 32%).

LC-MS (ESI): m/z = 550.1$[M-H]^-$ .

Step 5: (((((2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl) phosphonic acid (Compound 28)

Compound 28E (100 mg, 0.18 mmol) and N-methylmorpholine oxide (63 mg, 0.54 mmol) were dissolved in acetone (3 ml) and water (1 mL), potassium osmate(VI) dihydrate (6 mg, 0.018 mmol) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 28 (20 mg, 20%).

LC-MS (ESI): m/z = 584.0 $[M+1]^+$.

$^1H$ NMR (400 MHz, $D_2O$) δ 8.07 (d, 1H), 6.12 (d, 1H), 4.95 (s, 1H), 4.32 (s, 1H), 4.15 (s, 2H), 4.02 - 3.63 (m, 4H), 3.62 - 3.46 (m, 1H), 3.39 (s, 1H), 2.87 (s, 1H), 2.76 (s, 1H), 2.30 (t, 2H), 1.93 - 1.80 (m, 2H), 1.78 - 1.69 (m, 1H), 1.64 (m, 1H), 1.57 - 1.36 (m, 2H).

**Example 29:**

(((((2R,3S,4R,5R)-5-(6-chloro-4-((3aR,6aS)-hexahydrocyclopenta[c]pyrrol-2(1H)-yl)-1H-pyrazolo[3,4-b]pyridin-1-yl)-3,4-dihydroxy-3-(hydroxymethyl)tetrahydrofuran-2-yl)methoxy)(hydroxy)phosphoryl)methyl) phosphonic acid (Compound 29)

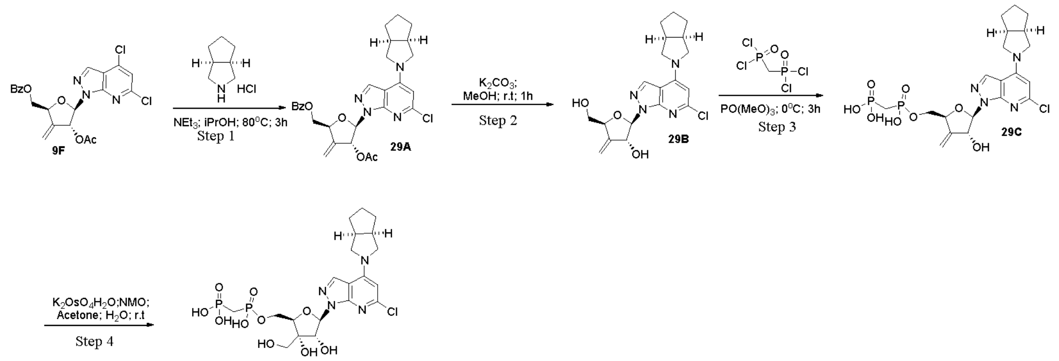

Compound 29

Step 1: ((2S,4R,5R)-4-acetoxy-5-(6-chloro-4-((3aR,6aS)-hexahydrocyclopenta [c]pyrrol-2(1H)-yl)-1H-pyrazolo[3,4-b]pyridin-1-yl)-3-methylenetetrahydrofuran-2-yl)methyl benzoate (29A)

Compound 9F (1.2 g, 2.6 mmol) was dissolved in isopropanol (20 ml), triethylamine (1.05 g, 10.4 mmol) and cis-7-azabicyclo[3.3.0]octane hydrochloride (0.58 g, 5.2 mmol) were added, after the addition, the mixture was reacted at 80°C for 3 hours, after the reaction was completed, the mixture was concentrated under reduced pressure, and the crude 29A was directly used in the next reaction.

LC-MS (ESI): m/z = 537.3[M+H]$^+$.

Step 2: (2R,3R,5S)-2-(6-chloro-4-((3aR,6aS)-hexahydrocyclopenta[c]pyrrol-2(1H)-yl)-1H-pyrazolo[3,4-b]pyridin-1-yl)-5-(hydroxymethyl)-4-methylenetetrahydrofuran-3-ol (29B)

Crude 29A was dissolved in methanol (20 ml), potassium carbonate (1.49 g, 10.8 mmol) was added, after the addition, the mixture was reacted at room temperature for 2 hours, water (20 ml) was added, solids were precipitated, filtered, the filter cake was washed with water (2 mL) once, and the filter cake was azeotropically dried by distilling off toluene and water, and spun to dryness under reduced pressure to obtain the title compound 29B (0.65 g, two-step yield: 64%).

LC-MS (ESI): m/z = 391.1[M+H]$^+$.

Step 3: (((((2S,4R,5R)-5-(6-chloro-4-((3aR,6aS)-hexahydrocyclopenta[c] pyrrol-2(1H)-yl)-1H-pyrazolo[3,4-b]pyridin-1-yl)-4-hydroxy-3-methylenetetrahydrofuran-2-yl)methoxy)(hydroxy)phosphoryl)methyl)phosphonic acid (29C)

Compound 29B (0.35 g, 0.9 mmol) was dissolved in trimethyl phosphate (3 ml), the temperature was decreased to 0°C under an ice bath, methylenebis(phosphonic dichloride) (0.67 g, 2.7 mmol) was dissolved in trimethyl phosphate (1.5 ml), and the mixture was slowly added dropwise into the reaction solution, after the addition, the mixture was reacted at 0°C for 3 hours, upon completion monitored by LCMS, water (1 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 29C (80 mg, 16%).

LC-MS (ESI): m/z = 547.0[M-H]$^-$ .

Step 4: (((((2R,3S,4R,5R)-5-(6-chloro-4-((3aR,6aS)-hexahydrocyclopenta [c]pyrrol-2(1H)-yl)-1H-pyrazolo[3,4-b]pyridin-1-yl)-3,4-dihydroxy-3-(hydroxymethyl)tetrahydrofuran-2-yl)methoxy)(hydroxy)phosphoryl) methyl)phosphonic acid (Compound 29)

Compound 29C (0.080 g, 0.15 mmol) and N-methylmorpholine oxide (0.053 g, 0.45 mmol) were dissolved in acetone (3 ml) and water (1 mL), potassium osmate(VI) dihydrate (0.006 g, 0.015 mmol) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic

instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 29 (45 mg, 51%).

LC-MS (ESI): m/z = 581.0[M-H]$^-$ .

$^1$H NMR (400 MHz, MeOD) δ 8.24 (s, 1H), 6.33 (d, 1H), 6.14 (s, 1H), 4.92 (d, 1H), 4.44 - 4.20 (m, 3H), 3.83 (q, 4H), 3.48 (s, 2H), 2.90 (s, 2H), 2.58-2.34 (m, 2H), 2.01-1.82 (m, 3H), 1.78 - 1.56 (m, 3H).

**Example 30:**

(((((2R,3S,4R,5R)-5-(4-((benzyloxy)amino)-6-chloro-1H-pyrazolo[3,4-d]pyrimidin-1-yl)-3,4-dihydroxytetrahydrofuran-2-yl)methoxy)(hydroxy) phosphoryl)methyl)phosphonic acid (Compound 30)

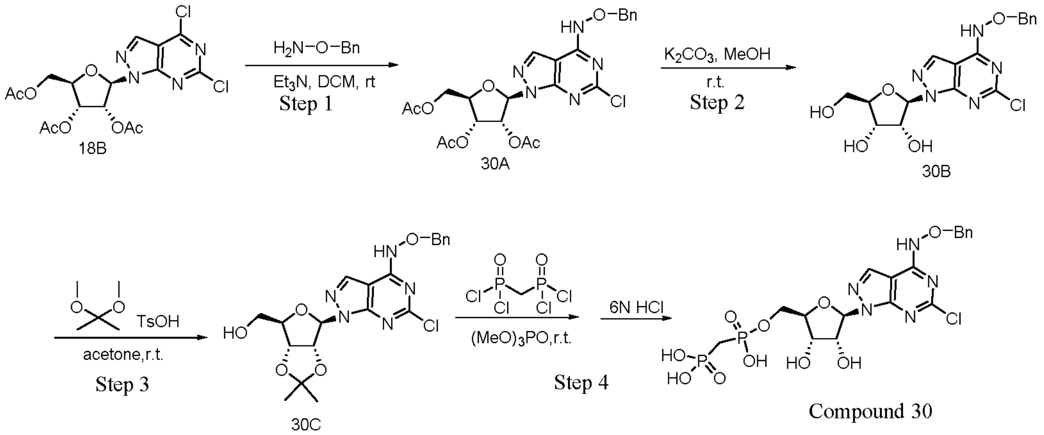


Step 1: (2R,3R,4R,5R)-2-(acetoxymethyl)-5-(4-((benzyloxy)amino)-6-chloro-1H-pyrazolo[3,4-d]pyrimidin-1-yl)tetrahydrofuran-3,4-diyl diacetate (30A)

Compound 18B (600 mg, 1.34 mmol) was dissolved in dichloromethane (6 ml), o-benzylhydroxylamine (248 mg, 2.01 mmol) and triethylamine (135 mg, 2.01 mmol) were added, the mixture was stirred at room temperature for 2 h, after the reaction was completed, the mixture was concentrated, and the resulting residue was purified by silica gel column chromatography (eluent: EA : PE = 0%-50%), to obtain the product 30A (548 mg, 77%).

LCMS m/z = 534.2 [M+1]$^+$.

Step 2: (2R,3R,4S,5R)-2-(4-((benzyloxy)amino)-6-chloro-1H-pyrazolo[3,4-d]pyrimidin-1-yl)-5-(hydroxymethyl)tetrahydrofuran-3,4-diol (30B)

Compound 30A (548 mg, 1.03 mmol) was dissolved in methanol (6 ml), potassium carbonate (426 mg, 3.09 mmol) was added, the mixture was stirred at room temperature for 2 h, and after the reaction was completed, the mixture was concentrated, to obtain the compound 30B (400 mg, 95%).

LCMS m/z = 408.1 [M+1]$^+$.

Step 3: ((3aR,4R,6R,6aR)-6-(4-((benzyloxy)amino)-6-chloro-1H-pyrazolo [3,4-d]pyrimidin-1-yl)-2,2-dimethyltetrahydrofuro[3,4-d][1,3]dioxol-4-yl)methanol (30C)

Compound 30B (167 mg, 0.41 mmol), 2,2-dimethoxypropane (213 mg, 2.05 mmol) and p-toluenesulfonic acid (7.79 mg, 0.041 mmol) were dissolved in acetone (2 ml), stirred at room temperature overnight, after the reaction was completed, saturated sodium bicarbonate solution was added to adjust the solution to pH 6-7, the mixture was concentrated, and the resulting residue was purified by silica gel column chromatography (eluent: EA : PE = 0%-50%), to obtain the product 30C (137 mg, 75%).

LCMS m/z = 448.2 [M+1]$^+$.

Step 4: (((((2R,3S,4R,5R)-5-(4-((benzyloxy)amino)-6-chloro-1H-pyrazolo [3,4-d]pyrimidin-1-yl)-3,4-dihydroxytetrahydrofuran-2-yl)methoxy)(hydroxy) phosphoryl)methyl)phosphonic acid (Compound 30)

Compound 30C (137 mg, 0.31 mmol) was dissolved in trimethyl phosphate (2 ml), stirred under an ice-water bath at 0°C, methylenebis(phosphonic dichloride) (77.5 mg, 0.93 mmol) was dissolved in trimethyl phosphate (2 ml) and added dropwise into the reaction flask, the mixture was then warmed to room temperature and stirred overnight, 6M hydrochloric acid (3 ml) was added, and the mixture was stirred at room temperature for 6 h, after the reaction was completed, the mixture was concentrated, and the residue was separated and purified by a preparative liquid chromatography column with reparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 12.0 min, to obtain the product 30 (58 mg, 33%).

LCMS m/z = 566.20 $[M+1]^+$.

$^1H$ NMR (400 MHz, DMSO) δ 8.10 (s, 1H), 7.49 (d, 2H), 7.41 (m, 3H), 6.05 (m, 1H), 5.02 (d, 2H), 4.54 (s, 1H), 4.29 (s, 1H), 4.07 (s, 2H), 3.88 (s, 1H), 2.15 (t, 2H).

**Example 31:**

(((((2R,3S,4R,5S)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 31)

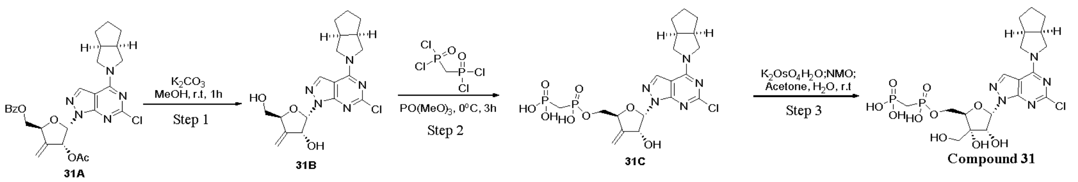

31A 31B 31C Compound 31

Step 1: (2S,3R,5S)-2-(6-chloro-4-((3aR,6aS)-hexahydrocyclopenta[c]pyrrol-2(1H)-yl)-1H-pyrazolo[3,4-d]pyrimidin-1-yl)-5-(hydroxymethyl)-4-methylenetetrahydrofuran-3-ol (31B)

Compound 31A (300 mg, 0.56 mmol) was dissolved in methanol (3 ml), potassium carbonate (309 mg, 2.24 mmol) was added, after the addition, the mixture was reacted at room temperature for 2 hours, water (3 ml) was added to quench the reaction, the mixture was extracted with ethyl acetate (5 ml × 3), dried over anhydrous sodium sulfate, concentrated and the resulting residue was purified by silica gel column chromatography (eluent: EA : PE = 0%-70%), to obtain the product 31B (290 mg, 98%).

LC-MS (ESI): m/z = 392.1 $[M+1]^+$.

Step 2: (((((2S,4R,5S)-5-(6-chloro-4-((3aR,6aS)-hexahydrocyclopenta [c]pyrrol-2(1H)-yl)-1H-pyrazolo[3,4-d]pyrimidin-1-yl)-4-hydroxy-3-methylenetetrahydrofuran-2-yl)methoxy)(hydroxy)phosphoryl)methyl)phosphonic acid (31C)

Compound 31B (290 mg, 0.57 mmol) was dissolved in trimethyl phosphate (3 ml), the temperature was decreased to 0°C under an ice bath, methylenebis(phosphonic dichloride) (570 mg, 2.28 mmol) was dissolved in trimethyl phosphate (2 ml), and the mixture was slowly added dropwise into the reaction solution, after the addition, the mixture was reacted at 0°C for 3 hours, upon completion monitored by LCMS, water (2 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 31C (80 mg, 26%).

LC-MS (ESI): m/z = $550.1[M+H]^-$ .

Step 3: (((((2R,3S,4R,5S)-5-(6-chloro-4-((3aR,6aS)-hexahydrocyclopenta [c]pyrrol-2(1H)-yl)-1H-pyrazolo[3,4-d]pyrimidin-1-yl)-3,4-dihydroxy-3-(hydroxymethyl)tetrahydrofuran-2-yl)methoxy)(hydroxy)phosphoryl) methyl)phosphonic acid (Compound 31)

Compound 31C (80 mg, 0.15 mmol) and N-methylmorpholine oxide (53 mg, 0.45 mmol) were dissolved in acetone (3 ml) and water (1 mL), potassium osmate(VI) dihydrate (0.006 g, 0.015) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 31 (20 mg, 20%).
LC-MS (ESI): m/z = 584.0 $[M+1]^+$.
$^1H$ NMR (400 MHz, $D_2O$): 8.12 (d, 1H), 6.18 (d, 1H), 4.98-4.95 (m, 1H), 4.52-4.50 (m, 1H), 4.26-4.15 (m, 2H), 3.94-3.75 (m, 4H), 3.58-3.46 (m, 2H), 2.90-2.77 (m, 2H), 2.41-2.31 (m, 2H), 1.91-1.48 (m, 6H).

**Example 32:**

(((((2R,3S,4R,5R)-5-(6-chloro-4-((3aR,6aS)-hexahydrocyclopenta[c]pyrrol-2(1H)-yl)-1H-pyrazolo[3,4-b]pyridin-1-yl)-3-(difluoromethyl)-3,4-dihydroxytetrahydrofuran-2-yl)methoxy)(hydroxy)phosphoryl)methyl)phosphonic acid (Compound 32)

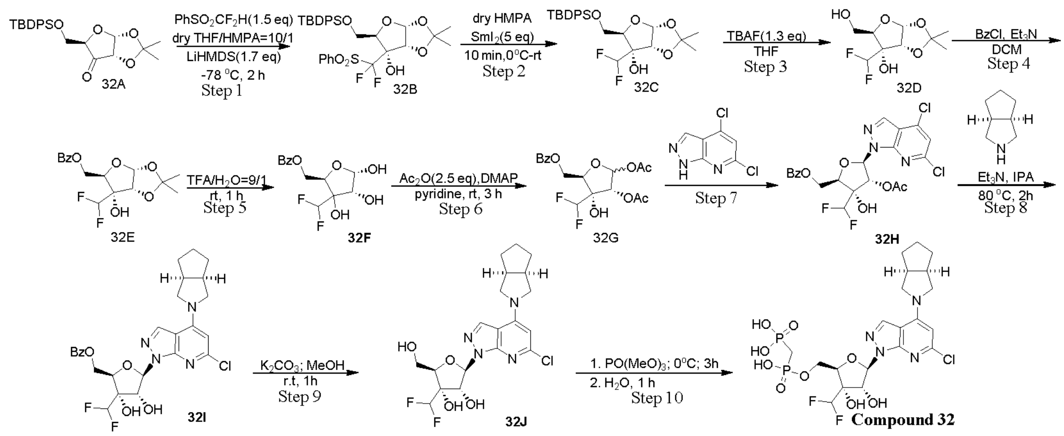

32A 32B 32C 32D 32E 32F 32G 32H 32I 32J Compound 32

Step 1: (3aR,5R,6R,6aR)-5-(((tert-butyldiphenylsilyl)oxy)methyl)-6-(difluoro (phenylsulfonyl)methyl)-2,2-dimethyltetrahydrofuro[2,3-d][1,3]dioxol-6-ol (32B)

32A (20 g, 46.95 mmol) was dissolved in dry tetrahydrofuran (200 ml), [(difluoromethyl)sulfonyl]benzene (13.52 g, 70.43 mmol) and hexamethylphosphoric triamide (20 ml) were added, the reaction system was subjected to nitrogen replacement three times, the reaction system was cooled to - 78°C, lithium bistrimethylsilylamide (80 ml, 80 mmol) was slowly added dropwise, the mixture was reacted at -78°C for 2 h, after the reaction was completed, the mixture was quenched with saturated ammonium chloride solution (100 ml), extracted with ethyl acetate (3 × 100 ml), the organic phases were combined, dried over anhydrous sodium sulfate, filtered, and concentrated, and the residue was subjected to fast separation and purification by column chromatography (eluent: EA : PE = 0%-30%) to obtain the compound 32B (21.56 g, 75%).
$^1H$ NMR (400 MHz, $CDCl_3$) :7.93 (d, 2H), 7.77 - 7.70 (m, 1H), 7.66 - 7.62 (m, 4H), 7.61 - 7.55 (m, 2H), 7.41 - 7.37 (m, 3H), 7.37 - 7.31 (m, 3H), 6.06 (d, 1H), 5.23 (d, 1H), 4.36 (dd, 1H), 4.00 (s, 1H), 3.97 - 3.88 (m, 1H), 3.50 (s, 1H), 1.61 (s, 3H), 1.45 (s, 3H), 1.01 (s, 9H).
$^{19}F$ NMR (376 MHz, $CDCl_3$): -101.22, -101.87, -103.37, -104.03.

Step 2: (3aR,5R,6R,6aR)-5-(((tert-butyldiphenylsilyl)oxy)methyl)-6-(difluoromethyl)-2,2-dimethyltetrahydrofuro[2,3-d][1,3]dioxol-6-ol (32C)

32B (21.56 g, 34.84 mmol) was dissolved in dry hexamethylphosphoric triamide (20 ml), the reaction system was subjected to nitrogen replacement three times, the reaction system was placed in an ice bath and cooled, samarium(II) iodide (174.2 mmol) dissolved in tetrahydrofuran (0.1 M) was added, the mixture was transferred to room temperature and reacted for 10 min, after the reaction was completed, the mixture was quenched with saturated aqueous ammonium chloride solution (500 ml), extracted with ethyl acetate (3 × 500 ml), the organic phases were combined, dried over anhydrous sodium sulfate, filtered, and concentrated, and then the residue was subjected to fast separation and purification by column chromatography (eluent: EA : PE = 0%-30%) to obtain the compound 32C (13.21 g, 78%).
$^1$H NMR (400 MHz, CDCl3) :7.68 (m, 4H), 7.46 - 7.35 (m, 6H), 6.19 - 5.90 (m, 1H), 5.92 (d, 1H), 4.65 (d, 1H), 4.09 (dd, 5.1, 1H), 3.94 (d, 2H), 3.03 (s, 1H), 1.58 (s, 3H), 1.41 (s, 3H), 1.06 (s, 9H).
19F NMR (376 MHz, $CDCl_3$): -127.34, -128.12, -129.08, -129.86.

Step 3: (3aR,5R,6R,6aR)-6-(difluoromethyl)-5-(hydroxymethyl)-2,2-dimethyltetrahydrofuro[2,3-d][1,3]dioxol-6-ol (32D)

Compound 32C (13.21 g, 27.64 mmol) was dissolved in tetrahydrofuran (130 ml), tetrabutylammonium fluoride (9.40 g, 35.93 mmol) was added, the mixture was reacted at room temperature for 1 hour, after the reaction was completed, water (50 ml) was added into the reaction solution to quench the reaction, the aqueous phase was extracted with ethyl acetate (100 ml × 3), the organic layers were combined, dried over anhydrous sodium sulfate, filtered, and the filtrate was concentrated to obtain the compound 32D (13.21 g, crude), which was directly used in the next step.
$^1$H NMR (400 MHz, $CDCl_3$) :6.14 - 5.84 (m, 1H), 5.90 (d, 1H), 4.62 (d, 1H), 4.06 (dd, 1H), 4.00 (dd, 1H), 3.93 (dd, 1H), 3.03 (s, 1H), 1.60 (s, 3H), 1.41 (s, 3H).
$^{19}$F NMR (376 MHz, $CDCl_3$) : -127.56, -128.34, -128.63, -129.41.

Step 4: ((3aR,5R,6R,6aR)-6-(difluoromethyl)-6-hydroxy-2,2-dimethyltetrahydrofuro[2,3-d][1,3]dioxol-5-yl)methyl benzoate (32E)

The compound from the previous step, 32D crude (13.21 g, 27.64 mmol), and triethylamine (8.39 g, 82.92 mmol) were dissolved in dichloromethane (130 ml), benzoyl chloride (4.66 g, 33.17 mmol) was added, after the addition, the mixture was reacted at room temperature for 2 hours, after the reaction was completed, water (50 ml) was added into the reaction solution to quench the reaction, the aqueous phase was extracted with dichloromethane (80 ml × 3), the organic layers were combined, washed with saturated brine, dried over anhydrous sodium sulfate, filtered, and concentrated, and then the residue was separated and purified by column chromatography (eluent: EA : PE = 0%-20%) to obtain the compound 32E (8 g, two-step yield 84%).
$^1$H NMR (400 MHz, $CDCl_3$): 8.06 (m, 2H), 7.60 - 7.54 (m, 1H), 7.44 (m, 2H), 6.10 - 5.81 (m, 1H), 5.95 (t, 1H), 4.71 (m, 1H), 4.65 (d, 1H), 4.57 (m, 1H), 4.36 - 4.28 (m, 1H), 3.19 (s, 1H), 1.62 (s, 3H), 1.42 (s, 3H).
$^{19}$F NMR (376 MHz, $CDCl_3$): -127.54, -128.32, -128.88, -129.67.

Step 5: ((2R,3S,4R,5S)-3-(difluoromethyl)-3,4,5-trihydroxytetrahydrofuran-2-yl)methyl benzoate (32F)

Compound 32E (8 g, 23.23 mmol) was added into a 100 mL reaction flask, trifluoroacetic acid (9 ml)/water (1 ml) was added, the mixture was reacted at room temperature for 1 h, after the reaction was completed, saturated aqueous sodium bicarbonate solution was added to adjust the solution to pH = 7, the aqueous phase was extracted with ethyl acetate, the organic layers were combined, washed with saturated brine, dried over anhydrous sodium sulfate, filtered, and concentrated, and then the residue was separated and purified by column chromatography (eluent: EA : PE = 0%-50%) to obtain the compound 32F (6.72 g, 95%).
$^1$H NMR (400 MHz, CDCl3) :8.08 - 7.98 (m, 2H), 7.59 (t, 1H), 7.46 (t, 2H), 5.96 (t, 1H), 5.54 (d, 1H), 4.65 (t, 1H), 4.55 - 4.46 (m, 2H), 4.35 (d, 1H).
$^{19}$F NMR (376 MHz, CDCl3): -127.38, -128.16, -128.69, -129.47.

Step 6: (3R,4R,5R)-5-((benzoyloxy)methyl)-4-(difluoromethyl)-4-hydroxytetrahydrofuran-2,3-diyl diacetate (32G)

Compound 32F (1.0 g, 3.29 mmol) was dissolved in pyridine (10 ml), the temperature was decreased to 0°C under an ice bath, acetic anhydride (840 mg, 8.23 mmol) and 4-dimethylaminopyridine (40 mg, 0.33 mmol) were added, the mixture was transferred to room temperature and reacted for 3 h, after the reaction was completed, ethyl acetate (50 ml) was added for dilution, the resulting mixture was washed with 0.1 N hydrochloric acid (100 ml) to remove pyridine, concentrated under reduced pressure and subjected to fast separation and purification (eluent: EA : PE = 0%-30%) to

obtain the compound 32G (1.2 g, 94%).

$^1H$ NMR (400 MHz, $CDCl_3$): 8.10 (d, 2H), 7.60 (t, 1H), 7.48 (t, 2H), 6.24 (d, 1H), 6.10 (t, 1H), 5.69 (d, 1H), 4.78 (dd, 1H), 4.54 (t, 1H), 4.39 (m, 1H), 2.17 (s, 3H), 1.93 (s, 3H).

$^{19}F$ NMR (376 MHz, $CDCl_3$): -126.61, -127.40, -129.45, -130.23.

Step 7: ((2R,3R,4R,5R)-4-acetoxy-5-(4,6-dichloro-1H-pyrazolo[3,4-b]pyridin-1-yl)-3-(difluoromethyl)-3-hydroxytetrahydrofuran-2-yl)methyl benzoate (32H)

Compound 32G (0.6 g, 1.55 mmol) was added into a 25 mL reaction flask, heated to 90°C, 4,6-dichloro-1H-pyrazolo[3,4-b]pyridine (0.38 g, 2.02 mmol) was added, after the addition, two drops of stannum tetrachloride were added dropwise, then the mixture was warmed to 130°C and reacted under reduced pressure for 30 minutes, cooled to room temperature, and the residue was separated and purified by silica gel column chromatography (petroleum ether : ethyl acetate (v/v) = 10 : 1-5 : 1) to obtain the title compound 32H (0.6 g, 75%).

LC-MS (ESI): m/z = 474.1 $[M-42+H]^+$.

Step 8: ((2R,3S,4R,5R)-5-(6-chloro-4-((3aR,6aS)-hexahydrocyclopenta [c]pyrrol-2(1H)-yl)-1H-pyrazolo[3,4-b]pyridin-1-yl)-3-(difluoromethyl)-3,4-dihydroxytetrahydrofuran-2-yl)methyl benzoate (32I)

Compound 32H (600 mg, 1.17 mmol) was dissolved in isopropanol (6 ml), triethylamine (355 mg, 3.51 mmol) and cis-7-azabicyclo[3.3.0]octane hydrochloride (195 mg, 1.76 mmol) were added, the mixture was reacted in an oil bath pan at 80 °C for 3 h, and after the reaction was completed, the mixture was concentrated under reduced pressure and subjected to fast separation and purification (eluent: EA : PE = 0%-50%) to obtain the compound 32I (300 mg, 47%).

LC-MS (ESI): m/z = 549.2 $[M+H]^+$.

Step 9: (2R,3S,4R,5R)-5-(6-chloro-4-((3aR,6aS)-hexahydrocyclopenta [c]pyrrol-2(1H)-yl)-1H-pyrazolo[3,4-b]pyridin-1-yl)-3-(difluoromethyl)-2-(hydroxymethyl)tetrahydrofuran-3,4-diol (32J)

Compound 32I (300 mg, 0.55 mmol) was dissolved in methanol (3 ml), potassium carbonate (228 mg, 1.65 mmol) was added, after the addition, the mixture was reacted at room temperature for 2 h, water (3 ml) was added to quench the reaction, the mixture was extracted with ethyl acetate (5 ml × 3), dried over anhydrous sodium sulfate, filtered, and concentrated, and then the residue was subjected to fast separation and purification by column chromatography (eluent: EA : PE = 0%-70%), to obtain the product 32J (200 mg, 82%).

LC-MS (ESI): m/z = 445.2 $[M+H]^+$.

Step 10: (((((2R,3S,4R,5R)-5-(6-chloro-4-((3aR,6aS)-hexahydrocyclopenta [c]pyrrol-2(1H)-yl)-1H-pyrazolo[3,4-b]pyridin-1-yl)-3-(difluoromethyl)-3,4-dihydroxytetrahydrofuran-2-yl)methoxy)(hydroxy)phosphoryl)methyl)phosphonic acid (Compound 32)

Compound 32J (200 mg, 0.45 mmol) was dissolved in trimethyl phosphate (2 ml), the temperature was decreased to 0°C under an ice bath, methylenebis(phosphonic dichloride) (338 mg, 1.35 mmol) was dissolved in trimethyl phosphate (2 ml), and the mixture was slowly added dropwise into the reaction solution, after the addition, the mixture was reacted at 0°C for 3 h, upon completion monitored by LCMS, water (2 ml) was added, after the addition, the mixture was stirred for 1 h, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 32 (162 mg, 60%).

LC-MS (ESI): m/z = 603.2$[M+H]^+$.

$^1H$ NMR (400 MHz, $D_2O$): 8.21 (s, 1H), 6.51 - 6.08 (t, 1H), 6.17 (d, 1H), 5.24 (d, 1H), 4.34 (s, 1H), 4.10 (s, 2H), 3.71 (s, 2H), 3.37 (s, 2H), 2.81 (s, 2H), 2.24 (m, 2H), 1.67 (m, 6H).

$^{19}F$ NMR (376 MHz, $D_2O$): -125.50, -126.30, -129.85, -130.62

**Example 33:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3-(difluoromethyl)-3,4-dihydroxytetrahydrofuran-2-yl)methoxy)(hydroxy)phosphoryl)methyl)phosphonic acid (Compound 33)

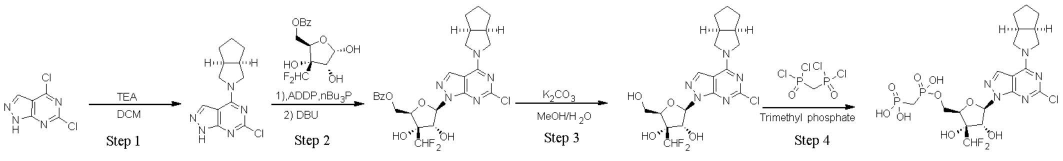


18A 33A 33B 33C Compound 33

Step 1: 6-chloro-4-((3aR,6aS)-hexahydrocyclopenta[c]pyrrol-2(1H)-yl)-1H-pyrazolo[3,4-d]pyrimidine (33A)

4,6-Dichloro-1H-pyrazolo[3,4-C]pyrimidine (2.0 g, 10.58 mmol) was dissolved in dichloromethane (30 ml), followed by cis-7-azabicyclo[3.3.0]octane hydrochloride (1.88 g, 12.72 mmol) and triethylamine (3.22 g, 31.74 mmol), after the addition, the mixture was reacted at room temperature for 1 hour, after the reaction was completed, water was added, the mixture was extracted with dichloromethane (50 ml × 2) twice, the organic phases were combined, washed with saturated brine, dried over anhydrous sodium sulfate, and filtered, and the filtrate was concentrated and then crystallized with dichloromethane and petroleum ether to obtain the compound 33A (2.4 g, 86%).

LC-MS (ESI): m/z = 264.1 $[M+H]^+$.

Step 2: ((2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-3-(difluoromethyl)-3,4-dihydroxytetrahydrofuran-2-yl)methyl benzoate (33B)

Under nitrogen protection, compound 32F (1.0 g, 3.29 mmol) was dissolved in anhydrous acetonitrile (20 ml), the mixture was cooled to 0°C, 1,1'-(azodicarbonyl)dipiperidine (1.24 g, 4.94 mmol) was dissolved in anhydrous acetonitrile (5 ml), and the mixture was slowly added dropwise into the reaction solution with the temperature being controlled at 0°C, and then the mixture was stirred for 10 min, tri-n-butylphosphine (1.07 g, 5.26 mmol) was then slowly added dropwise into the reaction solution, after the addition, the mixture was warmed to room temperature and reacted for 10 min, and then the mixture was warmed to 35°C and reacted for 1 hour; another reaction flask was taken, under nitrogen protection, compound 33A (1.65 g, 6.25 mmol) was dissolved in anhydrous acetonitrile (15 ml), DBU (0.95 g, 6.25 mmol) was added, after the addition, the mixture was stirred at room temperature for 30 min, and then this reaction solution was slowly added dropwise into another reaction solution, after the addition, the mixture was warmed to 35°C and reacted for 2 hours, after the reaction was completed, the mixture was cooled to room temperature, saturated ammonium chloride solution was added to quench the reaction, the mixture was extracted with ethyl acetate (50 ml × 2), the organic phases were combined, washed with saturated brine, dried over anhydrous sodium sulfate, filtered, and concentrated, and then the residue was separated and purified by silica gel column chromatography (petroleum ether : ethyl acetate (v/v) = 2 : 1) to obtain the title compound 33B (0.5 g, 27.6%).

LC-MS (ESI): m/z = 550.1 $[M+H]^+$.

Step 3: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-(difluoromethyl)-2-(hydroxymethyl)tetrahydrofuran-3,4-diol (33C)

Compound 33B (0.5 g, 0.91 mmol) was dissolved in methanol (10 ml), potassium carbonate (0.25 g, 1.82 mmol) was dissolved in water (4 ml) and the mixture was added into the reaction solution, after the addition, the mixture was stirred at room temperature for 1 hour, after the reaction was completed, the reaction solution was concentrated, water was added into the concentrated residue, the resulting mixture was extracted with dichloromethane (50 ml × 2) twice, the organic phases were combined, washed with saturated brine, dried over anhydrous sodium sulfate, and filtered, and the filtrate was concentrated, and then the residue was separated and purified by silica gel column chromatography (petroleum ether : ethyl acetate (v/v) = 1 : 1) to obtain the title compound 33C (0.27g, 66.5%).

LC-MS (ESI): m/z = 446.1 $[M+H]^+$.

Step 4: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-(difluoromethyl)-3,4-dihydroxytetrahydrofuran-2-yl)methoxy)(hydroxy)phosphoryl)methyl)phosphonic acid (Compound 33)

Compound 33C (0.26 g, 0.58 mmol) was dissolved in trimethyl phosphate (3 ml), the temperature was decreased to 0°C under an ice bath, methylenebis(phosphonic dichloride) (0.43 g, 1.74 mmol) was dissolved in trimethyl phosphate (2 ml), and the mixture was slowly added dropwise into the reaction solution, after the addition, the temperature was controlled at 0°C and the mixture was reacted for 2 hours, after the reaction was completed, water (1 ml) was added, and the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.2 min, to obtain the compound 33 (180 mg, 51.4%).

LCMS m/z = 602.1 $[M-H]^-$ .

$^1H$ NMR (400 MHz, $D_2O$): 8.13 (d, 1H), 6.34 (t, 1H), 6.13 (d, 1H), 5.24 (d, 1H), 4.37 (s, 1H), 4.16 (s, 2H), 3.93-3.73 (m, 2H), 3.56-3.42 (m, 2H), 2.89-2.78 (m, 2H), 2.38-2.29 (m, 2H), 1.91-1.48 (m, 6H).

**Example 34:**

(((((2R,3S,4R,SR)-5-(6-chloro-4-(((S)-1-(2-fluorophenyl)ethyl)amino)-2H-pyrazolo[3,4-d]pyrimidin-2-yl)-3,4-dihydroxytetrahydrofuran-2-yl)methoxy)(hydroxy)phosphoryl)methyl)phosphonic acid (Compound 34)

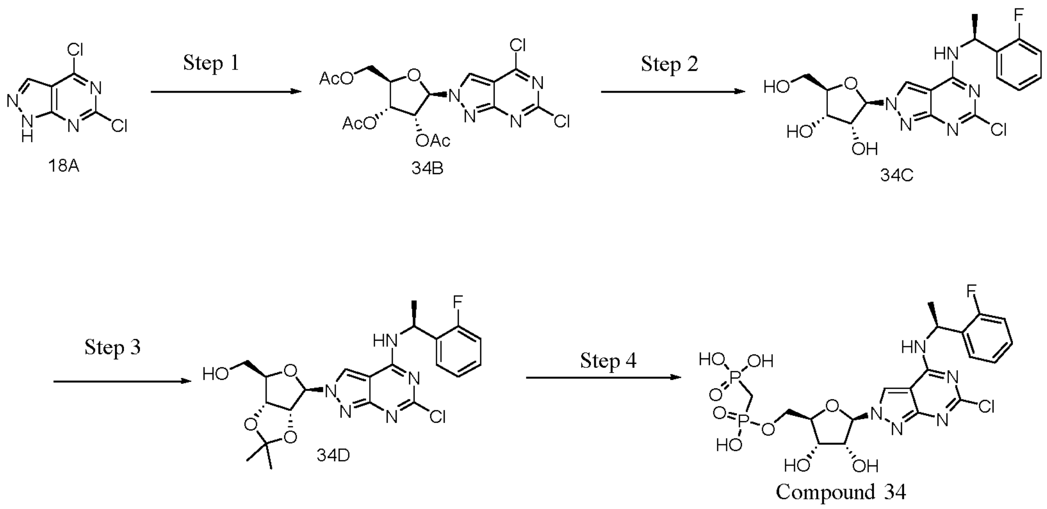


18A

34B

34C

34D

Compound 34

Step 1: (2R,3R,4R,5R)-2-(acetoxymethyl)-5-(4,6-dichloro-2H-pyrazolo[3,4-d]pyrimidin-2-yl)tetrahydrofuran-3,4-diyl diacetate (34B)

The compound 4,6-dichloro-1H-pyrazolo[3,4-d]pyrimidine 18A (8.0 g, 42.55 mmol) was dissolved in hexamethyldisilazane (40 mL), and ammonium sulfate (0.8 g) was added, and the mixture was reacted at 120°C for 5 hours under nitrogen. After concentration, acetonitrile (80 mL) and (2S,3R,4R,5R)-5-(acetoxymethyl)tetrahydrofuran-2, 3, 4-triyl triacetate (14.9 g, 46.81 mmol) were added, trimethylsilyl trifluoromethanesulfonate (14.2 g, 63.83 mmol) was added dropwise at room temperature, the mixture was reacted at room temperature for 12 hours, after concentration, saturated aqueous sodium bicarbonate solution (100 mL) was added, the mixture was extracted with ethyl acetate (100 mL × 3), washed with saturated brine (200 mL × 1), dried over anhydrous sodium sulfate, and concentrated, and the residue was purified by silica gel column chromatography (petroleum ether : ethyl acetate (v/v) = 100 : 40) to obtain the product 34B (6.5 g, yield 26%).

LCMS m/z = 447.1$[M+1]^+$.

Step 2: (2R,3R,4S,5R)-2-(6-chloro-4-(((S)-1-(2-fluorophenyl)ethyl)amino)-2H-pyrazolo[3,4-d]pyrimidin-2-yl)-5-(hydroxymethyl)tetrahydrofuran-3,4-diol (34C)

Compound 34B (0.8 g, 1.8 mmol) was dissolved in methanol (30 mL), (S)-1-(2-fluorophenyl)ethylamine hydrochloride (315 mg, 1.8 mmol) and triethylamine (545 mg, 5.4 mmol) were added, the mixture was reacted at room temperature for 2 hours, and ammonia methanol solution (7 N, 60 mL) was added, the resulting mixture was reacted at room temperature for 12 hours, and concentrated to obtain the crude product 34C (1.6 g, yield 100%).

LCMS m/z = 424.1 $[M+1]^+$.

Step 3: ((3aR,4R,6R,6aR)-6-(6-chloro-4-(((S)-1-(2-fluorophenyl)ethyl)amino) -2H-pyrazolo[3,4-d]pyrimidin-2-yl)-2,2-dimethyltetrahydrofuro[3,4-d][1,3]dioxol-4-yl)methanol (34D)

Compound 34C (1.6 g, 1.8 mmol) was added into acetone (40 mL), followed by 2,2-dimethoxypropane (0.56 g, 5.4 mmol) and p-toluenesulfonic acid (0310 mg, 1.8 mmol), the mixture was reacted at room temperature for 12 hours, and concentrated, and the residue was purified by silica gel column chromatography (methanol : dichloromethane (v/v) = 5 : 100) to obtain the product 34D (400 mg, yield 48%).

LCMS m/z = 464.1$[M+1]^+$.

Step 4: (((((2R,3S,4R,SR)-5-(6-chloro-4-(((S)-1-(2-fluorophenyl)ethyl)amino) -2H-pyrazolo[3,4-d]pyrimidin-2-yl)-3,4-dihydroxytetrahydrofuran-2-yl)methoxy)(hydroxy)phosphoryl)methyl)phosphonic acid (Compound 34)

Compound 34D (400 mg, 0.86mmol) was added into trimethyl phosphate (3 mL), followed by methylenediphosphonic dichloride (639 mg, 2.58 mmol), and the mixture was reacted at room temperature for 1h. Hydrochloric acid (6N, 2 mL) was added, and the mixture was reacted at room temperature for 0.5 hours, and directly separated and purified by a preparative liquid chromatography column (liquid phase preparative conditions: C18 reversed phase preparative column, mobile phase: deionized water containing 0.1% trifluoroacetic acid (A) and acetonitrile (B), gradient elution, B content = 5%-50%, elution time: 15 min, flow rate: 15 mL/min, and column temperature: 30°C) to obtain the title compound 34 (40 mg, yield 8%, retention time being about 7.2 min).

MS M/Z (ESI): m/z = 582.1 $[M+1]^+$.

$^1$H NMR (400 MHz, DMSO-d6) δ 9.57 (d, 1H), 8.97 (s, 1H), 7.69 - 7.46 (m, 1H), 7.32 - 7.25 (m, 1H), 7.20 - 7.12 (m, 2H), 5.88 (d, 1H), 5.67 - 5.56 (m, 1H), 4.33 - 4.28 (m, 1H), 4.27 - 4.05 (m, 4H), 2.45 - 2.27 (m, 2H), 1.54 (d, 3H).

**Example 35:**

(((((2R,3S,4R,5R)-5-(6-chloro-4-(5,5-difluorohexahydrocyclopenta[c]pyrrol-2(1H)-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 35)

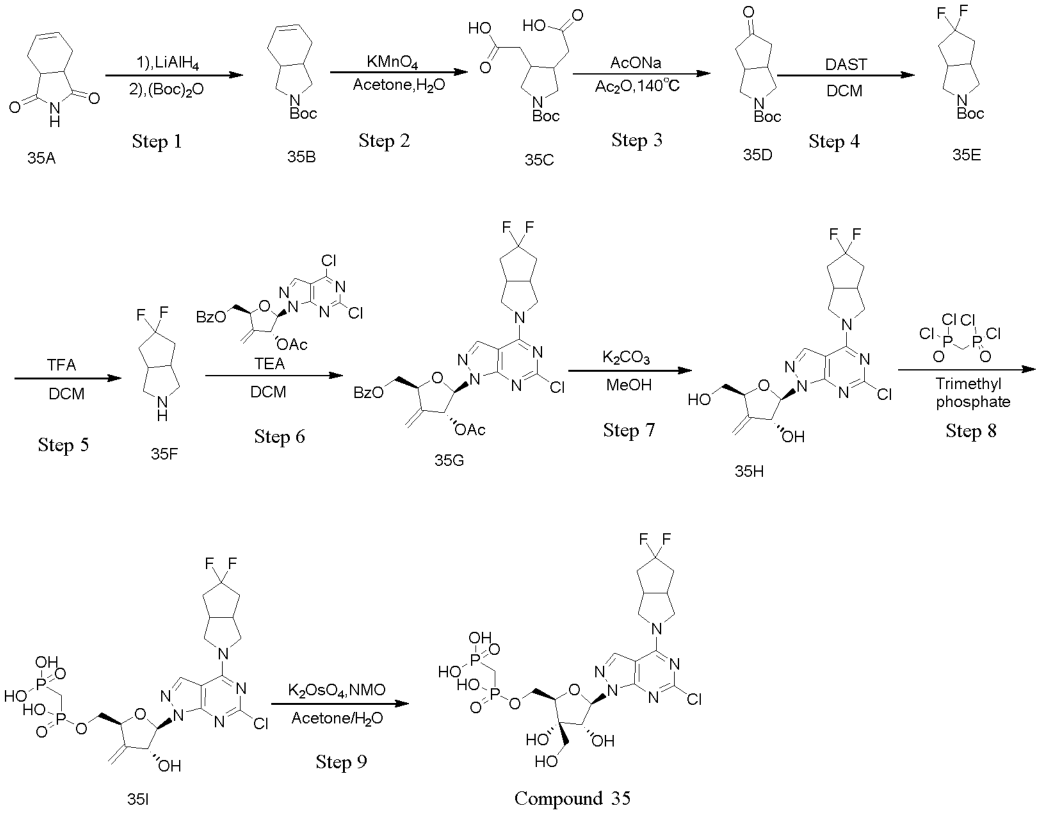


Step 1: tert-butyl 1,3,3a,4,7,7a-hexahydro-2H-isoindole-2-carboxylate (35B)

At room temperature, compound 35A (20.0 g, 132.3 mmol) was dissolved in tetrahydrofuran (200 mL), at T<35°C, lithium aluminum hydride (12.6 g, 330.8mmol) was added in portions, and after the addition, the mixture was warmed to 60°C and reacted for 2 hours. The reaction solution was cooled to room temperature, at T<35°C, water (24 mL) was added dropwise to the reaction solution, di-tert-butyl dicarbonate (28.9 g, 132.3 mmol) was added, the mixture was reacted at room temperature for 2 hours, the reaction solution was filtered, the filtrate was concentrated to dryness, and the residue was purified by column chromatography (petroleum ether : ethyl acetate (v : v) = 10 : 1-1 : 1), to obtain the compound 35B (16.0 g, 54%).

LC-MS (ESI): m/z = 168.2 $[M+H-tBu]^+$.

Step 2: 2,2'-(1-(tert-butoxycarbonyl)pyrrolidine-3,4-diyl)diacetic acid (35C)

At room temperature, potassium permanganate (14.2 g, 89.6mmol) was dissolved in water (300 mL), the compound 35B (8.0 g, 35.8 mmol) was dissolved in acetone (150 mL), and added dropwise to the reaction solution at T<30°C, and the mixture was reacted at room temperature for 3 hours. At T<30°C, the reaction solution was adjusted to about pH 4-5 with 3N hydrochloric acid, saturated sodium thiosulfate solution was added until the system becomes colorless and transparent, the mixture was extracted with ethyl acetate (200 mL × 2), and the organic phase was concentrated to dryness, to obtain the crude compound 35C (9.0 g), which was directly used in the next step without purification.

LC-MS (ESI): m/z = 286.1 $[M-H]^-$.

Step 3: tert-butyl 5-oxohexahydrocyclopenta[c]pyrrole-2(1H)-carboxylate (35D)

At room temperature, the compound 35C (9.0 g, 31.3mmol) was dissolved in acetic anhydride (100 mL), the mixture was warmed to 130°C and reacted for 1 hour, anhydrous sodium acetate (3.1 g, 37.6 mmol) was added, and the mixture was reacted at 130°C for 2 hours. The reaction solution was concentrated to dryness, dissolved in ethyl acetate (100 mL), washed with water (50 mL × 1), the organic phase was concentrated to dryness, and the residue was purified by column chromatography (petroleum ether : ethyl acetate (v : v) = 10 : 1-3 : 1), to obtain the compound 35D (2.5 g, 35%).

LC-MS (ESI): m/z = 170.1$[M+H-tBu]^+$.

Step 4: tert-butyl 5,5-difluorohexahydrocyclopenta[c]pyrrole-2(1H)-carboxylate (35E)

At room temperature, compound 35D (2.5 g, 11.1mmol) was dissolved in dichloromethane (100 mL), diethylaminosulfur trifluoride (8.1 g, 50.0mmol) was added, and the mixture was reacted at room temperature for 16 hours. The reaction solution was poured into saturated aqueous sodium bicarbonate solution (100 mL), extracted with dichloromethane (100 mL × 1), the organic phase was concentrated to dryness, and the residue was purified by column chromatography (petroleum ether : ethyl acetate (v : v) = 10 : 1-5 : 1), to obtain the compound 35E (0.75 g, 27%).
LC-MS (ESI): m/z = 248.2$[M+H]^+$.

Step 5: 5,5-difluorooctahydrocyclopenta[c]pyrrole (35F)

Compound 35E (0.75 g, 3.03 mmol) was dissolved in a mixed solvent of dichloromethane (10 ml) and trifluoroacetic acid (2 ml), the mixture was reacted at room temperature for 3 hours, and after the reaction was completed, the mixture was directly concentrated to obtain the compound 35F (0.79 g, 100%).
LC-MS (ESI): m/z = 148.2$[M+H]^+$.

Step 6: ((2S,4R,5R)-4-acetoxy-5-(6-chloro-4-(5,5-difluorohexahydrocyclopenta[c]pyrrol-2(1H)-yl)-1H-pyrazolo[3,4-d]pyrimidin-1-yl)-3-methylenetetrahydrofuran-2-yl)methyl benzoate (35G)

Compound 28B (0.7 g, 1.51 mmol) was dissolved in dichloromethane (10 ml), compound 35F (0.47, 1.81 mmol) was then added, the temperature was decreased to 0°C, triethylamine (0.46 g, 4.53 mmol) was then added, after the addition, the mixture was reacted at room temperature for 1 hour, after the reaction was completed, the reaction solution was directly concentrated, and after concentration, the residue was separated and purified by silica gel column chromatography (petroleum ether : ethyl acetate (v/v) = 1 : 2) to obtain the compound 35G (0.5 g, 57.6%).
LC-MS (ESI): m/z = 574.2$[M+H]^+$.

Step 7: (2R,3R,5S)-2-(6-chloro-4-(5,5-difluorohexahydrocyclopenta[c]pyrrol-2(1H)-yl)-1H-pyrazolo[3,4-d]pyrimidin-1-yl)-5-(hydroxymethyl)-4-methylenetetrahydrofuran-3-ol (35H)

Compound 35G (0.5 g, 0.87 mmol) was dissolved in methanol (10 ml), potassium carbonate (0.36 g, 2.61 mmol) was dissolved in water (4 ml) and the mixture was added into the reaction solution, after the addition, the mixture was stirred at room temperature for 3 hour, after the reaction was completed, the reaction solution was concentrated, water was added into the concentrated residue, the mixture was extracted with dichloromethane (50 ml × 2) twice, the organic phases were combined, washed with saturated brine, dried over anhydrous sodium sulfate, and filtered, and the filtrate was concentrated, and then the residue was separated and purified by silica gel column chromatography (petroleum ether : ethyl acetate (v/v) = 1 : 3) to obtain the title compound 35H (0.34 g, 91.3%).
LC-MS (ESI): m/z = 428.1$[M+H]^+$.

Step 8: (((((2S,4R,5R)-5-(6-chloro-4-(5,5-difluorohexahydrocyclopenta [c]pyrrol-2(1H)-yl)-1H-pyrazolo[3,4-d]pyrimidin-1-yl)-4-hydroxy-3-methylenetetrahydrofuran-2-yl)methoxy)(hydroxy)phosphoryl)methyl)phosphonic acid (35I)

Compound 35H (0.34 g, 0.79 mmol) was dissolved in trimethyl phosphate (3 ml), the temperature was decreased to 0°C under an ice bath, methylenebis(phosphonic dichloride) (0.59 g, 2.37 mmol) was dissolved in trimethyl phosphate (2 ml), and the mixure was slowly added dropwise into the reaction solution, after the addition, the temperature was controlled at 0°C and the mixture was reacted for 2 hours, after the reaction was completed, water (2 ml) was added, and the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.5 min, to obtain the compound 351 (0.26 g, 56.1%).
LC-MS (ESI): m/z = 584.1$[M-H]^-$.

Step 9: (((((2R,3S,4R,5R)-5-(6-chloro-4-(5,5-difluorohexahydrocyclopenta [c]pyrrol-2(1H)-yl)-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl) phosphonic acid (Compound 35)

Compound 35I (0.26 g, 0.44 mmol) was dissolved in a mixed solvent of acetone (6 ml) and water (2 ml), N-methylmorpholine oxide (0.25 g, 2.2 mmol) was added, a catalytic amount of potassium osmate(VI) dihydrate (16 mg) was added, after the addition, the mixture was reacted at room temperature for 15 hours, after the reaction was completed, the mixture was concentrated to remove acetone and the reaction system was directly subjected to preparative HPLC with preparative HPLC separation methods: 1. Instrument: waters 2767 (preparative liquid phase chromatographic instrument); chromatographic column: SunFire@ PrepC18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.4 min, to obtain the compound 35 (0.13 g, 46.9%).

LC-MS (ESI): m/z = 618.1[M-H]$^-$.

$^1$H NMR (400 MHz, $D_2O$): 8.09 (d, 1H), 6.12 (d, 1H), 4.95 (d, 1H), 4.33-4.31 (m, 1H), 4.18-4.11 (m, 2H), 4.07-3.15 (m, 6H), 3.14-3.02 (m, 2H), 2.53-2.11 (m, 6H).

**Example 36:**

(((((2R,4R,5R)-5-(6-chloro-4-(tetrahydro-1H-furo[3,4-c]pyrrol-5(3H)-yl)-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 36)

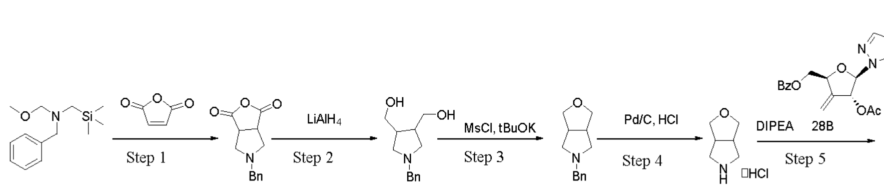


36A 36B 36C 36D 36E

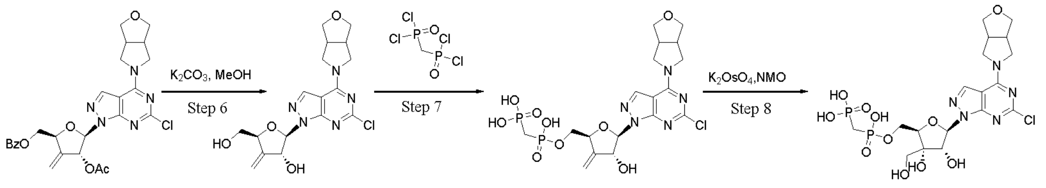


36F 36G 36H **Compound 36**

Step 1: 5-benzyltetrahydro-1H-furo[3,4-c]pyrrole-1,3(3aH)-dione (36B)

At room temperature, compound 36A (10.0 g, 42.1 mmol) and maleic anhydride (4.5 g, 46.3 mmol) were dissolved in dichloromethane (200 mL), trifluoroacetic acid (0.5 g, 4.2 mmol) was added, the mixture was reacted at room temperature for 2 hours, and the reaction solution was concentrated to dryness, to obtain the title compound (crude, 9.7 g), which was directly used in the next step.

Step 2: (1-benzylpyrrolidine-3,4-diyl)dimethanol (36C)

At room temperature, compound 36B (9.7 g, 42.0 mmol) was dissolved in tetrahydrofuran (200 mL), at T < 35°C, lithium aluminum hydride (3.5 g, 92.3 mmol) was added in portions, and the mixture was reacted at room temperature for 2 hours. At T < 35°C, water (6.6 mL) was added dropwise into the reaction solution, the mixture was filtered, the solid was rinsed with ethyl acetate (200 mL × 1), the organic phase was combined, concentrated to dryness, and the residue was purified by column chromatography (dichloromethane : methanol (v/v) = 30/1), to obtain the title compound (3.8 g, yield 41%).

LC-MS (ESI): m/z = 222.3 [M+H]$^+$.

Step 3: 5-benzylhexahydro-1H-furo[3,4-c]pyrrole (36D)

At room temperature, compound 36C (3.8 g, 17.2 mmol) was dissolved in tetrahydrofuran (100 mL), potassium

tert-butoxide (4.1 g, 36.1 mmol) was added, the mixture was reacted for 30 minutes, methylsulfonyl chloride (2.2 g, 18.9 mmol) was added dropwise into the reaction solution, the mixture was reacted for 10 minutes, potassium tert-butoxide (1.9 g, 17.2 mmol) was added, the resulting mixture was reacted at room temperature for 30 minutes, the mixture was warmed to 60°C and reacted for 2 hours, the reaction solution was poured into saturated aqueous ammonium chloride solution (200 mL), the mixture was extracted with ethyl acetate (100 mL × 1), the organic phase was concentrated to dryness, and the residue was purified by column chromatography (petroleum ether : ethyl acetate (v/v) = 1/1), to obtain the title compound (2 g, yield 57%).

LC-MS (ESI): m/z = 204.3 $[M+H]^+$.

Step 4: hexahydro-1H-furo[3,4-c]pyrrole hydrochloride (36E)

At room temperature, compound 36D (2.0 g, 9.8 mmol) was dissolved in tetrahydrofuran (20 mL), 4N hydrogen chloride in dioxane (0.7 mL, 28 mmol) was added, the mixture was concentrated to dryness, methanol (40 mL) was added, followed by 10% Pd/C (0.1 g), the reaction system was subjected to hydrogen replacement, the mixture was reacted at room temperature for 16 hours, the reaction solution was filtered and concentrated to dryness, to obtain the title compound (1.2 g, yield 81%).

Step 5: ((2S,4R,5R)-4-acetoxy-5-(6-chloro-4-(tetrahydro-1H-furo[3,4-c]pyrrol -5(3H)-yl)-1H-pyrazolo[3,4-d]pyrimidin-1-yl)-3-methylenetetrahydrofuran-2-yl)methyl benzoate (36F)

28B (750 mg, 1.62 mmol) and 36E (290 mg, 1.94 mmol) were added into dichloromethane, followed by N,N-diisopropyl ethylamine (840 mg, 6.48 mmol), and the mixture was reacted at room temperature for 2h. Upon complete depletion of raw materials monitored by TLC, the reaction solution was directly added into silica gel, and concentrated, and the residue was separated by column chromatography (EA : PE(v/v) = 1/1), to obtain the title compound (500 mg, 57%).

LC-MS (ESI): m/z = 540.2 $[M+H]^+$.

Step 6: (2R,3R,5S)-2-(6-chloro-4-(tetrahydro-1H-furo[3,4-c]pyrrol-5(3H)-yl)-1H-pyrazolo[3,4-d]pyrimidin-1-yl)-5-(hydroxymethyl)-4-methylenetetrahydrofuran-3-ol (36G)

36F (500 mg, 0.93mmol) and potassium carbonate (390 mg, 2.79 mmol) were added into methanol (10 ml), the mixture was reacted at room temperature for 3h, upon completion monitored by LC-MS, the reaction solution was directly added into silica gel, and concentrated under reduced pressure, and the residue was separated and purified by silica gel column chromatography (DCM : MeOH(v/v) = 10/1), to obtain the title compound (220 mg, 60%).

LC-MS (ESI): m/z = 394.1 $[M+H]^+$.

Step 7: (((((2S,4R,5R)-5-(6-chloro-4-(tetrahydro-1H-furo[3,4-c]pyrrol-5(3H)-yl)-1H-pyrazolo[3,4-d]pyrimidin-1-yl)-4-hydroxy-3-methylenetetrahydrofuran-2-yl)methoxy)(hydroxy)phosphoryl)methyl)phosphonic acid (36H)

36G (220 mg, 0.56 mmol) was added into trimethyl phosphate (4 ml), methylenebis(phosphonic dichloride) (420 mg, 1.68mmol) was dissolved in trimethyl phosphate (1ml), the mixture was added dropwise into the reaction system under an ice bath, the mixture was kept at 0°C and reacted for 3h, upon complete depletion of raw materials monitored by LC-MS, water (0.5 ml) was added under an ice bath, and the resulting mixture was reacted at 0°C for another 5 h, and the reaction was monitored by LC-MS, and then the reaction mixture was separated and purified by HPLC to obtain the title compound (100 mg, 32%). Preparative HPLC separation methods: 1. Instrument: waters 2767 (preparative liquid phase chromatographic instrument); chromatographic column: SunFire@ Prep C18 (19 mm × 250 mm); 2. The sample was dissolved in DMF and filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 40%; c. flow rate: 12 mL/min; d. elution time: 20 min.

LC-MS (ESI): m/z = 552.1 $[M+H]^+$.

Step 8: (((((2R,4R,5R)-5-(6-chloro-4-(tetrahydro-1H-furo[3,4-c]pyrrol-5(3H)-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 36)

36H (100 mg, 0.18mmol), potassium osmate(VI) dihydrate (10 mg) and N-methylmorpholine oxide (110 mg, 0.9 mmol) were added into a mixed system of acetone (3 ml) and water (1 ml). The mixture was reacted at room temperature overnight, upon completion monitored by LC-MS, the reaction solution was filtered, the filtrate was concentrated under reduced pressure to remove acetone, and then the residue was separated and purified by HPLC to obtain the title compound (18 mg, 17%). Preparative HPLC separation methods: 1. Instrument: waters 2767 (preparative liquid phase chromatographic instrument); chromatographic column: SunFire@ Prep C18 (19 mm × 250 mm); 2. The sample was dissolved in DMF and filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 40%; c. flow rate: 12 mL/min; d. elution time: 20 min.

$^1$H NMR (400 MHz, MeOD) δ 8.22 (s, 1H), 6.26 (d, 1H), 4.95 (d, 1H), 4.40-4.35 (m, 1H), 4.30-4.24 (m, 2H), 4.20-4.10 (m, 1H), 4.07 - 3.92 (m, 3H), 3.85-3.73 (m, 6H), 2.52 - 2.33 (m, 2H), 1.33-1.28 (m, 2H).

LC-MS (ESI): m/z = 586.1[M+H]$^+$.

**Example 37:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-(methoxymethyl) tetrahydrofuran-2-yl)methoxy)(hydroxy)phosphoryl)methyl)phosphonic acid (Compound 37)

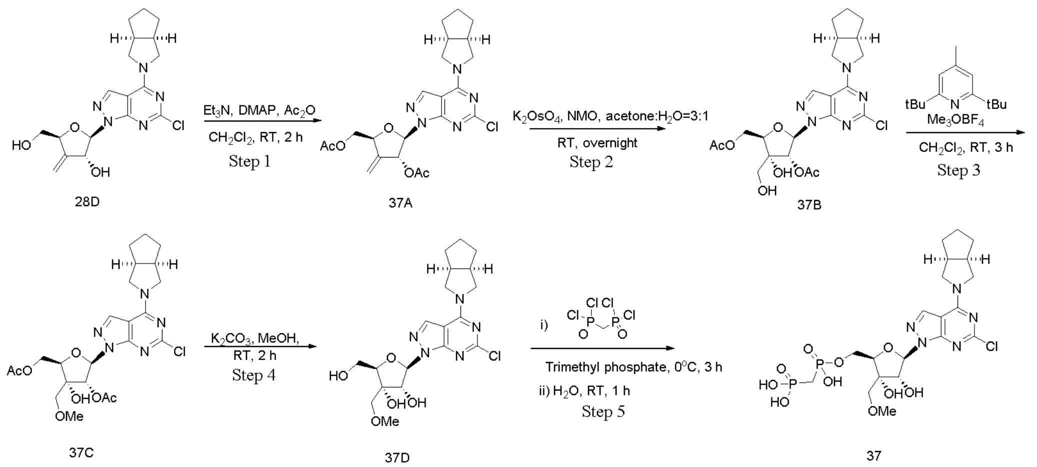

28D 37A 37B 37C 37D 37

Step 1: ((2S,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-methylenetetrahydrofuran-2-yl)methyl acetate (37A)

Compound 28D (2.00 g, 5.10 mmol) was dissolved in dry dichloromethane (80 ml), triethylamine (1.55 g, 15.3 mmol), 4-dimethylaminopyridine (312 mg, 2.55 mmol) and acetic anhydride (1.04 g, 10.2 mmol) were added, after the addition, the mixture was reacted at room temperature for 2 hours, upon completion monitored by TLC, saturated aqueous ammonium chloride solution (100 ml) was added, the mixture was extracted with dichloromethane (30 ml × 3), the organic phases were combined, dried over anhydrous sodium sulfate, and filtered, and the filtrate was concentrated, and then the residue was separated and purified by silica gel column chromatography (petroleum ether : ethyl acetate (v/v) = 4 : 1) to obtain 37A (2.24 g, 92.4 %), as a white foam solid.

Step 2: ((2R,3R,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-hydroxy-3-(hydroxymethyl)tetrahydrofuran-2-yl)methyl acetate (37B)

Compound 37A (2.28 g, 4.79 mmol) was dissolved in acetone : water = 3 : 1 (80 ml), N-methyl morpholine-N-oxide (2.81 g, 23.9 mmol) and potassium osmate(VI) tetrahydrate (353 mg, 0.958 mmol) were added, after the addition, the mixture was reacted at room temperature for 15 hours, upon completion monitored by TLC, saturated aqueous sodium thiosulfate solution (10 mL) and water 100 (mL) were added, the mixture was extracted with ethyl acetate (30 ml × 8), the organic phases were combined and dried over anhydrous sodium sulfate, filtered and concentrated, and then purified by column chromatography (petroleum ether : ethyl acetate (v/v) = 1 : 2) to obtain the compound 37B (1.84 g, 75.5%), as a white foam.

LC-MS (ESI): m/z = 510.2 [M+H]$^+$.

Step 3: ((2R,3R,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-hydroxy-3-(methoxymethyl)tetrahydrofuran-2-yl)methyl acetate (37C)

Compound 37B (1.00 g, 1.96 mmol) was dissolved in dry dichloromethane (50 ml), 2, 6-ditertbutyl -4-methylpyridine (1.59 g, 9.80 mmol) was added at room temperature, after 10 minutes, trimethyloxyonium tetrafluoroborate (580 mg, 3.92 mmol) was added in portions, and after the addition, the mixture was reacted at room temperature for 3 hours. Upon completion monitored by TLC, the mixture was dried with suction. The crude was purified by silica gel column chromatography (petroleum ether : ethyl acetate (v/v) = 1 : 1) to obtain the compound 37C (720 mg, 70. 1%) as a white foam solid.

LC-MS (ESI): m/z = 524.2 $[M+H]^+$.

Step 4: (2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-2-(hydroxymethyl)-3-(methoxymethyl)tetrahydrofuran-3,4-diol (37D)

Compound 37C (720 mg, 1.37 mmol) was dissolved in methanol (30 ml), potassium carbonate (570 mg, 4.12 mmol) was added at room temperature, and after the addition, the mixture was reacted at room temperature for 1 hour. Upon completion monitored by TLC, saturated aqueous ammonium chloride solution (50 mL) and water (50 mL) were added, and the mixture was extracted with ethyl acetate (10 ml × 8), the organic phases were combined, dried over anhydrous sodium sulfate, filtered and concentrated, and then purified by column chromatography (petroleum ether: ethyl acetate (v/v) = 1 : 2 to 1 : 10) to obtain the compound 37D (510 mg, 84.2%).

Step 5: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(methoxymethyl)tetrahydrofuran-2-yl)methoxy)(hydroxy)phosphoryl)methyl) phosphonic acid (Compound 37)

Compound 37D (260 mg, 0.592 mmol) was dissolved in trimethyl phosphate (3 ml), the temperature was decreased to 0°C under an ice bath, methylenebis(phosphonic dichloride) (393 mg, 2.37 mmol) was dissolved in trimethyl phosphate (0.5 ml), and the mixture was slowly added dropwise into the reaction solution, after the addition, the mixture was reacted at 0°C for 3 hours, upon completion monitored by LCMS, water (1 ml) was added, after the addition, the mixture was stirred for 0.5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@PrepC18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 10 min, retention time: 3.93 min, to obtain the compound 37 (45 mg, 12.7%).

LC-MS (ESI): m/z = 580.0 $[M-OH]^+$.

$^1$H NMR (400 MHz, $CD_3OD$) δ 8.20 (s, 1H), 6.28 (d, 1H), 4.74 - 4.51 (m, 3H), 4.30 - 4.11 (m, 2H), 4.06 (dd, 2H), 4.00 - 3.90 (m, 1H), 3.68 - 3.56 (m, 2H), 3.36 (s, 3H), 3.03 - 2.92 (m, 1H), 2.88 - 2.78 (m, 1H), 2.71 (t, 2H), 2.04 - 1.81 (m, 3H), 1.77 - 1.52 (m, 3H).

**Example 38:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-methyltetrahydrofuran-2-yl)methoxy)(hydroxy)phosphoryl)methyl)phosphonic acid (Compound 38)

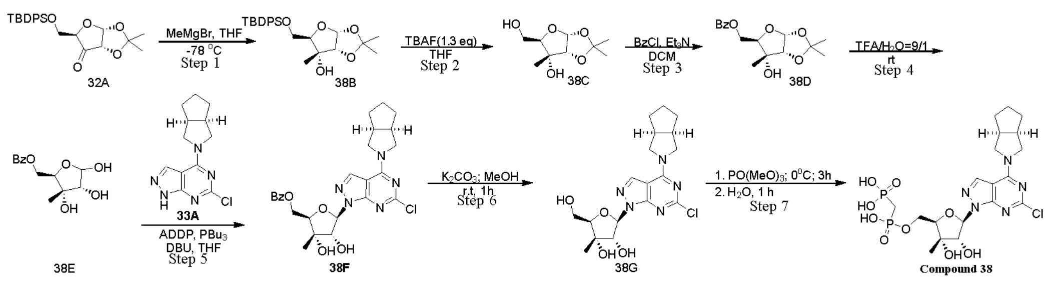

Step 1: (3aR,5R,6R,6aR)-5-(((tert-butyldiphenylsilyl)oxy)methyl)-2,2,6-trimethyltetrahydrofuro[2,3-d][1,3]dioxol-6-ol (38B)

32A (1 g, 2.35 mmol) was dissolved in dry tetrahydrofuran (10 ml), the reaction system was subjected to nitrogen replacement three times, cooled to -78 °C, methylmagnesium bromide (7.05 mmol) was slowly added dropwise, the mixture was reacted at -78 °C for 2 h, after the reaction was completed, the mixture was quenched with saturated ammonium chloride solution (100 ml), extracted with ethyl acetate (3 × 100 ml), the organic phases were combined, dried over anhydrous sodium sulfate, filtered, and concentrated, and the residue was subjected to fast separation and purification by column chromatography (eluent: EA : PE = 0%-30%) to obtain the compound 38B (1 g, 96%).

$^1$H NMR (400 MHz, $CDCl_3$) δ 7.71 - 7.68 (m, 4H), 7.46 - 7.33 (m, 6H), 5.76 (d, 1H), 4.07 (d, 1H), 3.89 (dd, 1H), 3.83 (s, 1H), 3.82 (d, 1H), 1.56 (s, 3H), 1.34 (s, 3H), 1.11 (s, 3H), 1.06 (s, 9H).

Step 2: (3aR,5R,6R,6aR)-5-(hydroxymethyl)-2,2,6-tnmethyltetrahydrofuro[2,3-d][1,3]dioxol-6-ol (38C)

Compound 38B (1 g, 2.26 mmol) was dissolved in tetrahydrofuran (10 ml), tetrabutylammonium fluoride (0.89 g, 3.39 mmol) was added, the mixture was reacted at room temperature for 1 hour, after the reaction was completed, water (10 ml) was added into the reaction solution to quench the reaction, the aqueous phase was extracted with ethyl acetate (20 ml × 3), the organic layers were combined, dried over anhydrous sodium sulfate, filtered, and concentrated to obtain the compound 38C (1 g, crude), which was directly used in the next step.

$^1$H NMR (400 MHz, $CDCl_3$) δ 5.78 (d, 1H), 4.12 (d, 1H), 3.90 (dd, 1H), 3.81 (dd, 1H), 3.73 (dd, 1H), 1.59 (s, 3H), 1.36 (s, 3H), 1.16 (s, 3H).

Step 3: ((3aR,5R,6R,6aR)-6-hydroxy-2,2,6-trimethyltetrahydrofuro[2,3-d][1,3] dioxol-5-yl)methyl benzoate (38D)

The compound from the previous step, 38C crude, and triethylamine (0.7 g, 6.78 mmol) were dissolved in dichloromethane (10 ml), benzoyl chloride (0.48 g, 3.39 mmol) was added, after the addition, the mixture was reacted at room temperature for 2 hours, after the reaction was completed, water (10 ml) was added into the reaction solution to quench the reaction, the aqueous phase was extracted with dichloromethane (10 ml × 3), the organic layers were combined, washed with saturated brine, dried over anhydrous sodium sulfate, filtered, and concentrated, and then the residue was separated and purified by column chromatography (eluent: EA : PE = 0%-20%) to obtain the compound 38D (0.66 g, two-step yield 95%).

$^1$H NMR (400 MHz, $CDCl_3$) δ 8.14 - 7.99 (m, 2H), 7.59 - 7.48 (m, 1H), 7.43 (t, 2H), 5.83 (d, 1H), 4.57 (dd, 1H), 4.38 (dd, 1H), 4.15 (d, 1H), 4.11 (dd, 1H), 2.76 (s, 1H), 1.60 (s, 3H), 1.37 (s, 3H), 1.25 (s, 3H).

Step 4: ((2R,3S,4R)-3,4,5-trihydroxy-3-methyltetrahydrofuran-2-yl)methyl benzoate (38E)

Compound 38D (3 g, 9.74 mmol) was added into a 100 mL reaction flask, trifluoroacetic acid (9 ml)/water (1 ml) was added, the mixture was reacted at room temperature for 1 h, after the reaction was completed, saturated aqueous sodium bicarbonate solution was added to adjust the solution to pH = 7, the aqueous phase was extracted with ethyl acetate, the organic layers were combined, washed with saturated brine, dried over anhydrous sodium sulfate, filtered, and concentrated, and then the residue was separated and purified by column chromatography (eluent: EA : PE = 0%-50%) to obtain the compound 38E (2.45 g, 94%).

$^1$H NMR (400 MHz, DMSO) δ 8.14 - 7.89 (m, 2H), 7.54 (t, 1H), 7.42 (t, 2H), 5.38 (d, 1H), 4.50 (dd, 1H), 4.37 (dd, 1H), 4.26 (dd, 1H), 3.84 (d, 1H), 1.33 (s, 3H).

Step 5: ((2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-3,4-dihydroxy-3-methyltetrahydrofuran-2-yl)methyl benzoate (38F)

Under nitrogen protection, compound 38E (1 g, 3.73 mmol) was dissolved in anhydrous tetrahydrofuran (20 ml), the mixture was cooled to 0°C, 1,1'-(azodicarbonyl)dipiperidine (1.41 g, 5.6 mmol) was dissolved in anhydrous acetonitrile (5 ml), and the mixture was slowly added dropwise into the reaction solution with the temperature being controlled at 0°C, and then the mixture was stirred for 10 min, tri-n-butylphosphine (1.13 g, 5.6 mmol) was then slowly added dropwise into the reaction solution, after the addition, the mixture was warmed to room temperature and reacted for 10 min, and then the mixture was warmed to 35°C and reacted for 1 hour; another reaction flask was taken, under nitrogen protection, compound 33A (1.96 g, 7.46 mmol) was dissolved in anhydrous acetonitrile (15 ml), DBU (1.13 g, 7.46 mmol) was added, after the addition, the mixture was stirred at room temperature for 30 min, and then this reaction solution was slowly added dropwise into another reaction solution, after the addition, the mixture was warmed to 35°C and reacted for 2 hours, after the reaction was completed, the mixture was cooled to room temperature, saturated

ammonium chloride solution was added to quench the reaction, the mixture was extracted with ethyl acetate (50 ml × 2), the organic phases were combined, washed with saturated brine, dried over anhydrous sodium sulfate, filtered, and concentrated, and then the residue was separated and purified by silica gel column chromatography (petroleum ether : ethyl acetate (v/v) = 2 : 1) to obtain the target compound 38F (0.18 g, 9%).

LC-MS (ESI): m/z = 514.3 $[M+H]^+$.

Step 6: (2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-2-(hydroxymethyl)-3-methyltetrahydrofuran-3,4-diol (38G)

Compound 38F (180 mg, 0.35 mmol) was dissolved in methanol (3 ml), potassium carbonate (193 mg, 1.4 mmol) was added, after the addition, the mixture was reacted at room temperature for 2 h, water (3 ml) was added to quench the reaction, the mixture was extracted with ethyl acetate (5 ml × 3), dried over anhydrous sodium sulfate, filtered, and concentrated, and then the residue was subjected to fast separation and purification by column chromatography (eluent: EA : PE = 0%-70%), to obtain the product 38G (140 mg, 98%).

LC-MS (ESI): m/z = 410.2 $[M+H]^+$.

Step 7: (((((2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-3,4-dihydroxy-3-methyltetrahydrofuran-2-yl)methoxy)(hydroxy)phosphoryl)methyl)phosphonic acid (Compound 38)

Compound 38G (140 mg, 0.45 mmol) was dissolved in trimethyl phosphate (2 ml), the temperature was decreased to 0°C under an ice bath, methylenebis(phosphonic dichloride) (338 mg, 1.35 mmol) was dissolved in trimethyl phosphate (2 ml), and the mixture was slowly added dropwise into the reaction solution, after the addition, the mixture was reacted at 0°C for 3 h, upon completion monitored by LCMS, water (2 ml) was added, after the addition, the mixture was stirred for 1 h, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 38 (90 mg, 46%).

LC-MS (ESI): m/z = 566.0$[M-H]^-$.

$^1H$ NMR (400 MHz, DMSO) δ 8.85 (s, 1H), 5.76 (d, 1H), 4.18 - 4.12 (m, 2H), 4.07 (d, 2H), 4.03 (d, 2H), 3.90 - 3.82 (m, 1H), 3.61 (dd, 1H), 3.47 (dd, 1H), 2.95 - 2.83 (m, 1H), 2.73 (dd, 1H), 2.23 (t, 2H), 1.91 - 1.79 (m, 2H), 1.78 - 1.71 (m, 1H), 1.67 - 1.56 (m, 2H), 1.55 - 1.45 (m, 1H), 1.27 (s, 3H).

**Example 39:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-vinyltetrahydrofuran-2-yl)methoxy)(hydroxy)phosphoryl)methyl)phosphonic acid (Compound 39)

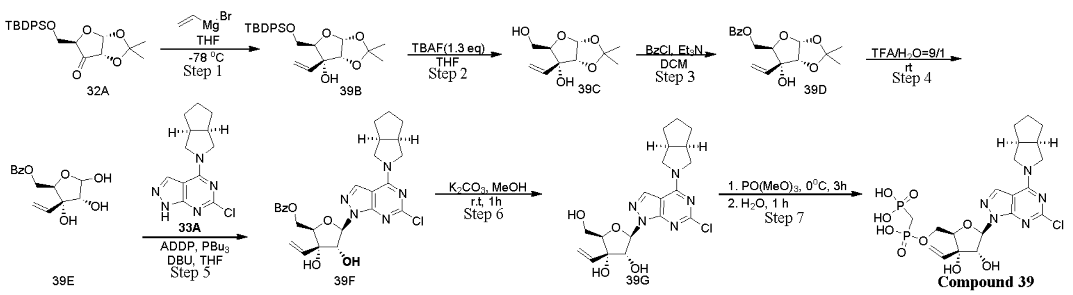


Step 1: (3aR,5R,6R,6aR)-5-(((tert-butyldiphenylsilyl)oxy)methyl)-2,2-dimethyl-6-vinyltetrahydrofuro[2,3-d][1,3]dioxol-6-ol (39B)

Compound 32A (5 g, 11.72 mmol) was dissolved in dry tetrahydrofuran (50 ml), the reaction system was subjected to nitrogen replacement three times, and cooled to -78°C, vinylmagnesium bromide (35.16 mmol) was slowly

added dropwise, the mixture was reacted at -78°C for 2 h, after the reaction was completed, the mixture was quenched with saturated ammonium chloride solution (100 ml), and extracted with ethyl acetate (3 × 100 ml), the organic phases were combined, dried over anhydrous sodium sulfate, filtered, and concentrated, and the residue was subjected to fast separation and purification by column chromatography (eluent: EA : PE = 0%-30%) to obtain the compound 39B (4.5 g, 84%).

$^1$H NMR (400 MHz, $CDCl_3$) δ 7.71 - 7.63 (m, 4H), 7.45 - 7.31 (m, 6H), 5.83 (d, 1H), 5.73 (dd, 1H), 5.47 (dd, 1H), 5.22 (dd, 1H), 4.21 (d, 1H), 4.04 (t, 1H), 3.77 - 3.72 (m, 2H), 1.60 (s, 3H), 1.36 (s, 3H), 1.05 (s, 9H).

Step 2: (3aR,5R,6R,6aR)-5-(hydroxymethyl)-2,2-dimethyl-6-vinyltetrahydrofuro[2,3-d][1,3]dioxol-6-ol (39C)

Compound 39B (4.5 g, 9.90 mmol) was dissolved in tetrahydrofuran (50 ml), tetrabutylammonium fluoride (3.36 g, 12.87 mmol) was added, the mixture was reacted at room temperature for 1 hour, after the reaction was completed, water (10 ml) was added into the reaction solution to quench the reaction, the aqueous phase was extracted with ethyl acetate (20 ml × 3), the organic layers were combined, dried over anhydrous sodium sulfate, filtered, and concentrated to obtain the compound 39C (5.13 g, crude), which was directly used in the next step.

Step 3: ((3aR,5R,6R,6aR)-6-hydroxy-2,2-dimethyl-6-vinyltetrahydrofuro[2,3-d][1,3]dioxol-5-yl)methyl benzoate (39D)

The compound from the previous step, 39C crude, and triethylamine (3.01 g, 29.7 mmol) were dissolved in dichloromethane (40 ml), benzoyl chloride (2.09 g, 14.85 mmol) was added, after the addition, the mixture was reacted at room temperature for 2 hours, after the reaction was completed, water (10 ml) was added into the reaction solution to quench the reaction, the aqueous phase was extracted with dichloromethane (10 ml × 3), the organic layers were combined, washed with saturated brine, dried over anhydrous sodium sulfate, filtered, and concentrated, and then the residue was separated and purified by column chromatography (eluent: EA : PE = 0%-20%) to obtain the compound 39D (2.54 g, two-step yield 80%).

$^1$H NMR (400 MHz, $CDCl_3$) δ 8.18 - 7.95 (m, 2H), 7.54 (d, 1H), 7.42 (t, 2H), 5.90 (d, 1H), 5.81 (dd, 1H), 5.59 (dd, 1H), 5.35 (dd, 1H), 4.45 (dd, 1H), 4.33 - 4.18 (m, 3H), 1.62 (s, 3H), 1.37 (s, 3H).

Step 4: ((2R,3S,4R)-3,4,5-trihydroxy-3-vinyltetrahydrofuran-2-yl)methyl benzoate (39E)

Compound 39D (2.54 g, 7.98 mmol) was added into a 100 mL reaction flask, trifluoroacetic acid (9 ml)/water (1 ml) was added, the mixture was reacted at room temperature for 1 h, after the reaction was completed, saturated aqueous sodium bicarbonate solution was added to adjust the solution to pH = 7, the aqueous phase was extracted with ethyl acetate, the organic layers were combined, washed with saturated brine, dried over anhydrous sodium sulfate, filtered, and concentrated, and then the residue was separated and purified by column chromatography (eluent: EA : PE = 0%-50%) to obtain the compound 39E (2.1 g, 95%).

$^1$H NMR (400 MHz, $CDCl_3$) δ8.03 - 7.97 (m, 2H), 7.57 (t, 1H), 7.48 - 7.40 (m, 2H), 5.91 (dd, 1H), 5.58 (dd, 1H), 5.45 (d, 1H), 5.37 (d, 1H), 4.50 - 4.40 (m, 2H), 4.22 (dd, 1H), 4.13 (d, 1H), 3.23 (t, 3H).

Step 5: ((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-vinyltetrahydrofuran-2-yl)methyl benzoate (39F)

Under nitrogen protection, compound 39E (1 g, 3.57 mmol) was dissolved in anhydrous tetrahydrofuran (20 ml), the mixture was cooled to 0°C, 1,1'-(azodicarbonyl)dipiperidine (1.80 g, 7.14 mmol) was dissolved in anhydrous acetonitrile (5 ml), and the mixture was slowly added dropwise into the reaction solution with the temperature being controlled at 0°C, and then the mixture was stirred for 10 min, tri-n-butylphosphine (1.44 g, 7.14 mmol) was then slowly added dropwise into the reaction solution, after the addition, the mixture was warmed to room temperature and reacted for 10 min, and then the mixture was warmed to 35°C and reacted for 1 hour; another reaction flask was taken, under nitrogen protection, compound 33A (1.41 g, 5.35 mmol) was dissolved in anhydrous acetonitrile (15 ml), DBU (0.82 g, 5.35 mmol) was added, after the addition, the mixture was stirred at room temperature for 30 min, and then this reaction solution was slowly added dropwise into another reaction solution, after the addition, the mixture was warmed to 35°C and reacted for 2 hours, after the reaction was completed, the mixture was cooled to room temperature, saturated ammonium chloride solution was added to quench the reaction, the mixture was extracted with ethyl acetate (50 ml × 2), the organic phases were combined, washed with saturated brine, dried over anhydrous sodium sulfate, filtered, and concentrated, and then the residue was separated and purified by silica gel column chromatography (petroleum ether : ethyl acetate (v/v) = 2 : 1) to obtain the target compound 39F (100 mg, 5%).

LC-MS (ESI): m/z = 526.2 $[M+H]^+$.

Step 6: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-2-(hydroxymethyl)-3-vinyltetrahydrofuran-3,4-diol (39G)

Compound 39F (50 mg, 0.35 mmol) was dissolved in methanol (3 ml), potassium carbonate (193 mg, 1.4 mmol) was added, after the addition, the mixture was reacted at room temperature for 2 h, water (3 ml) was added to quench the reaction, the mixture was extracted with ethyl acetate (5 ml × 3), dried over anhydrous sodium sulfate, filtered, and concentrated, and then the residue was subjected to fast separation and purification by column chromatography (eluent: EA : PE = 0%-70%), to obtain the product 39G (40 mg, 98%).

LC-MS (ESI): m/z = 422.1 $[M+H]^+$.

Step 7: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-vinyltetrahydrofuran-2-yl)methoxy)(hydroxy)phosphoryl)methyl)phosphonic acid (Compound 39)

Compound 39G (40 mg, 0.095 mmol) was dissolved in trimethyl phosphate (0.5 ml), the temperature was decreased to 0°C under an ice bath, methylenebis(phosphonic dichloride) (72 mg, 0.285 mmol) was dissolved in trimethyl phosphate (0.5 ml), and the mixture was slowly added dropwise into the reaction solution, after the addition, the mixture was reacted at 0°C for 3 h, upon completion monitored by LCMS, water (1 ml) was added, after the addition, the mixture was stirred for 1 h, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 39 (14 mg, 25%).

LC-MS (ESI): m/z = 578.0 $[M-H]^-$.

$^1$H NMR (400 MHz, $D_2O$) δ 8.21 (d, 1H), 6.21 (d, 1H), 6.10 (dd, 1H), 5.59 (d, 1H), 5.49 (d, 1H), 5.11 (d, 1H), 4.25 (s, 1H), 4.09 - 3.90 (m, 3H), 3.83 (dd, 1H), 3.62 (s, 1H), 3.50 (d, 1H), 2.92 (s, 1H), 2.80 (s, 1H), 2.25 (t, 2H), 1.88 (dd, 2H), 1.77 (d, 1H), 1.71 - 1.61 (m, 1H), 1.51 (s, 2H).

## Example 40:

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3-ethynyl-3,4-dihydroxytetrahydrofuran-2-yl)methoxy)(hydroxy)phosphoryl)methyl)phosphonic acid (Compound 40)

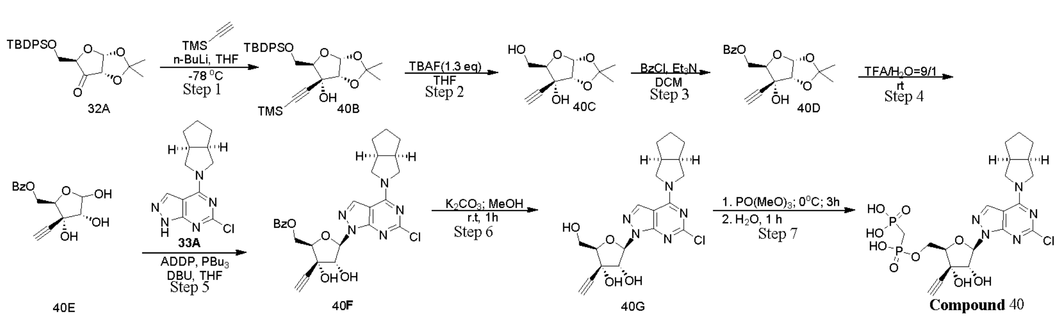


Step 1: (3aR,5R,6R,6aR)-5-(((tert-butyldiphenylsilyl)oxy)methyl)-2,2-dimethyl-6-((trimethylsilyl)ethynyl)tetrahydrofuro[2,3-d][1,3]dioxol-6-ol (40B)

Trimethylsilylacetylene (2.88 g, 29.3 mmol) was dissolved in dry tetrahydrofuran (30 ml), the reaction system was subjected to nitrogen replacement three times, the reaction system was cooled to -78 °C, n-Butyllithium (25.78 ml) was slowly added dropwise, the mixture was reacted at -78 °C for 30 min, compound 32A (5 g, 11.72 mmol) dissolved in tetrahydrofuran (10 ml) was added dropwise into the system, the mixture was reacted at -78°C for another 2 h, after the reaction was completed, the reaction was quenched with saturated ammonium chloride solution (100 ml), and extracted with ethyl acetate (3 × 100 ml), the organic phases were combined, dried over anhydrous sodium sulfate, filtered, and concentrated, and then the residue was subjected to fast separation and purification by column chromatography (eluent: EA : PE = 0%-30%) to obtain the compound 40B (5.72 g, 93%).

$^1$H NMR (400 MHz, $CDCl_3$) δ 7.69 (m, 4H), 7.48 - 7.30 (m, 6H), 5.85 (d, 1H), 4.54 (d, 1H), 4.05 (d, 1H), 4.02 (d, 1H), 3.98 (dd, 1H), 1.59 (s, 3H), 1.37 (s, 3H), 1.08 (s, 9H), 0.11 (s, 9H).

Step 2: (3aR,5R,6R,6aR)-6-ethynyl-5-(hydroxymethyl)-2,2-dimethyltetrahydrofuro[2,3-d][1,3]dioxol-6-ol (40C)

Compound 40B (5.72 g, 10.90 mmol) was dissolved in tetrahydrofuran (50 ml), tetrabutylammonium fluoride (14.17 ml, 14.17 mmol) was added, the mixture was reacted at room temperature for 1 hour, after the reaction was completed, water (10 ml) was added into the reaction solution to quench the reaction, the aqueous phase was extracted with ethyl acetate (20 ml × 3), the organic layers were combined, dried over anhydrous sodium sulfate, filtered, and concentrated to obtain the compound 40C (2.3 g, crude), which was directly used in the next step.
$^1$H NMR (400 MHz, $CDCl_3$) δ 5.90 (d, 1H), 4.56 (d, 1H), 4.02 - 3.94 (m, 3H), 2.63 (s, 1H), 1.60 (s, 3H), 1.39 (s, 3H).

Step 3: ((3aR,5R,6R,6aR)-6-ethynyl-6-hydroxy-2,2-dimethyltetrahydrofuro [2,3-d][1,3]dioxol-5-yl)methyl benzoate (40D)

The compound from the previous step, 40C crude, and triethylamine (3.31 g, 32.7 mmol) were dissolved in dichloromethane (30 ml), benzoyl chloride (2.30 g, 16.35 mmol) was added, after the addition, the mixture was reacted at room temperature for 2 hours, after the reaction was completed, water (20 ml) was added into the reaction solution to quench the reaction, the aqueous phase was extracted with dichloromethane (20 ml × 3), the organic layers were combined, washed with saturated brine, dried over anhydrous sodium sulfate, filtered, and concentrated, and then the residue was separated and purified by column chromatography (eluent: EA : PE = 0%-20%) to obtain the compound 40D (3.4 g, crude), which was directly used in the next step.
$^1$H NMR (400 MHz, $CDCl_3$) δ 8.13 - 8.00 (m, 2H), 7.55 (d, 1H), 7.43 (t, 2H), 5.94 (d, 1H), 4.74 (dd, 1H), 4.62 - 4.57 (m, 2H), 4.21 (dd, 1H), 2.66 (s, 1H), 1.60 (s, 3H), 1.39 (s, 3H).

Step 4: ((2R,3S,4R)-3-ethynyl-3,4,5-trihydroxytetrahydrofuran-2-yl)methyl benzoate (40E)

Compound 40D crude was added into a 100 mL reaction flask, trifluoroacetic acid (9 ml)/water (1 ml) was added, the mixture was reacted at room temperature for 1 h, after the reaction was completed, saturated aqueous sodium bicarbonate solution was added to adjust the solution to pH = 7, the aqueous phase was extracted with ethyl acetate, the organic layers were combined, washed with saturated brine, dried over anhydrous sodium sulfate, filtered, and concentrated, and then the residue was separated and purified by column chromatography (eluent: EA : PE = 0%-50%) to obtain the compound 40E (2.5 g, three-step yield 83%).
$^1$H NMR (400 MHz, MeOD) δ 8.11 - 8.00 (m, 2H), 7.62 (t, 1H), 7.48 (t, 2H), 5.40 (d, 1H), 4.58 (d, 1H), 4.50 (d, 1H), 4.43 (d, 1H), 4.15 (d, 1H), 3.09 (s, 1H).

Step 5: ((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-ethynyl-3,4-dihydroxytetrahydrofuran-2-yl)methyl benzoate (40F)

Under nitrogen protection, compound 40E (1 g, 3.59 mmol) was dissolved in anhydrous tetrahydrofuran (20 ml), the mixture was cooled to 0°C, 1,1'-(azodicarbonyl)dipiperidine (1.36 g, 5.38 mmol) was dissolved in anhydrous acetonitrile (5 ml), and the mixture was slowly added dropwise into the reaction solution with the temperature being controlled at 0°C, and then the mixture was stirred for 10 min, tri-n-butylphosphine (1.09 g, 5.38 mmol) was then slowly added dropwise into the reaction solution, after the addition, the mixture was warmed to room temperature and reacted for 10 min, and then the mixture was warmed to 35°C and reacted for 1 hour; another reaction flask was taken, under nitrogen protection, compound 33A (1.89 g, 7.18 mmol) was dissolved in anhydrous acetonitrile (15 ml), DBU (1.09 g, 7.18 mmol) was added, after the addition, the mixture was stirred at room temperature for 30 min, and then this reaction solution was slowly added dropwise into another reaction solution, after the addition, the mixture was warmed to 35°C and reacted for 2 hours, after the reaction was completed, the mixture was cooled to room temperature, saturated ammonium chloride solution was added to quench the reaction, the mixture was extracted with ethyl acetate (50 ml × 2), the organic phases were combined, washed with saturated brine, dried over anhydrous sodium sulfate, filtered, and concentrated, and then the residue was separated and purified by silica gel column chromatography (petroleum ether : ethyl acetate (v/v) = 2 : 1) to obtain the target compound 40F (0.18 g, 9%).
LC-MS (ESI): m/z = 524.2 $[M+H]^+$.

Step 6: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-ethynyl-2-(hydroxymethyl)tetrahydrofuran-3,4-diol (40G)

Compound 40F (180 mg, 0.35 mmol) was dissolved in methanol (3 ml), potassium carbonate (145 mg, 1.05

mmol) was added, after the addition, the mixture was reacted at room temperature for 2 h, water (3 ml) was added to quench the reaction, the mixture was extracted with ethyl acetate (5 ml × 3), dried over anhydrous sodium sulfate, filtered, and concentrated, and then the residue was subjected to fast separation and purification by column chromatography (eluent: EA : PE = 0%-70%), to obtain the product 40G (140 mg, 97%).

LC-MS (ESI): m/z = 420.1 $[M+H]^+$.

Step 7: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-ethynyl-3,4-dihydroxytetrahydrofuran-2-yl)methoxy)(hydroxy)phosphoryl)methyl)phosphonic acid (Compound 40)

Compound 40G (140 mg, 0.36 mmol) was dissolved in trimethyl phosphate (2 ml), the temperature was decreased to 0°C under an ice bath, methylenebis(phosphonic dichloride) (270 mg, 1.08 mmol) was dissolved in trimethyl phosphate (2 ml), and the mixture was slowly added dropwise into the reaction solution, after the addition, the mixture was reacted at 0°C for 3, upon completion monitored by LCMS, water (2 ml) was added, after the addition, the mixture was stirred for 1 h, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 40 (72 mg, 35%).

LC-MS (ESI): m/z = 576.0$[M-H]^-$.

$^1H$ NMR (400 MHz, DMSO) δ 8.88 (d, 1H), 5.80 (dd, 1H), 4.68 (dd, 2H), 4.37 - 4.28 (m, 1H), 4.27 - 4.17 (m, 2H), 4.04 (dd, 1H), 3.87 (dd, 1H), 3.66 - 3.55 (m, 2H), 3.47 (dd, 1H), 2.96 - 2.81 (m, 1H), 2.74 (d, 1H), 2.28 - 2.14 (m, 2H), 1.91 - 1.79 (m, 2H), 1.76 (dd, 1H), 1.67 - 1.56 (m, 2H), 1.53 (dd, 1H).

**Example 41:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-(trifluoromethyl) tetrahydrofuran-2-yl)methoxy)(hydroxy)phosphoryl)methyl)phosphonic acid (Compound 41)

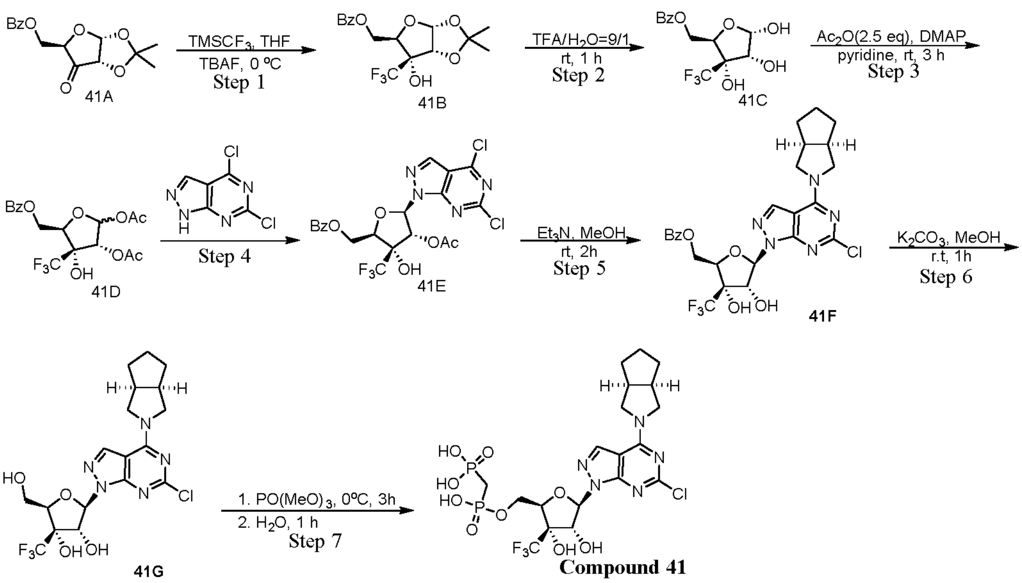


Step 1: ((3aR,5R,6R,6aR)-6-hydroxy-2,2-dimethyl-6-(trifluoromethyl) tetrahydrofuro[2,3-d][1,3]dioxol-5-yl)methyl benzoate (41B)

41A (1 g, 3.42 mmol) was dissolved in dry tetrahydrofuran (10 ml), the reaction system was subjected to nitrogen replacement three times, and cooled to 0 °C, trimethylsilyltrifluoromethane (1.02 g, 7.18 mmol), and tetrabutylammonium

fluoride (1.07 g, 4.10 mmol) were slowly added dropwise, after the reaction was completed, the mixture was quenched with water (5 ml), and extracted with ethyl acetate (3 × 5 ml), the organic phases were combined, dried over anhydrous sodium sulfate, filtered, and concentrated, and then the residue was subjected to fast separation and purification by column chromatography (eluent: EA : PE = 0%-30%) to obtain the compound 41B (0.8 g, 65%).

$^1H$ NMR (400 MHz, $CDCl_3$) δ 8.16 - 8.05 (m, 2H), 7.66 - 7.55 (m, 1H), 7.51 - 7.38 (m, 2H), 5.99 (d, 1H), 4.76 (dd, 1H), 4.62 (d, 1H), 4.55 - 4.47 (m, 1H), 4.42 - 4.31 (m, 1H), 1.63 (s, 3H), 1.43 (s, 3H).

$^{19}F$ NMR (376 MHz, $CDCl_3$) δ -73.95.

Step 2: ((2R,3S,4R,5S)-3,4,5-trihydroxy-3-(trifluoromethyl)tetrahydrofuran-2-yl)methyl benzoate (41C)

Compound 41B (3.14 g, 8.67 mmol) was added into a 100 mL reaction flask, trifluoroacetic acid (9 ml)/water (1 ml) was added, the mixture was reacted at room temperature for 1 h, after the reaction was completed, saturated aqueous sodium bicarbonate solution was added to adjust the solution to pH = 7, the aqueous phase was extracted with ethyl acetate, the organic layers were combined, washed with saturated brine, dried over anhydrous sodium sulfate, filtered, and concentrated, and then the residue was separated and purified by column chromatography (eluent: EA : PE = 0%-50%) to obtain the compound 41C (2.65 g, 95%).

$^1H$ NMR (400 MHz, $CDCl_3$) δ8.09 - 7.97 (m, 2H), 7.57 (t, 1H), 7.44 (dd, 2H), 5.61 (d, 1H), 4.74 (dd, 1H), 4.52 (d, 2H), 4.35 (d, 1H).

$^{19}F$ NMR (376 MHz, $CDCl_3$) δ-74.89.

Step 3: (3R,4R,5R)-5-((benzoyloxy)methyl)-4-hydroxy-4-(trifluoromethyl) tetrahydrofuran-2,3-diyl diacetate (41D)

Compound 41C (500 mg, 1.55 mmol) was dissolved in pyridine (5 ml), the temperature was decreased to 0°C under an ice bath, acetic anhydride (396 mg, 3.88 mmol) and 4-dimethylaminopyridine (19 mg, 0.16 mmol) were added, the mixture was transferred to room temperature and reacted for 3 h, after the reaction was completed, ethyl acetate (50 ml) was added for dilution, the mixture was washed with 0.1 N hydrochloric acid (20 ml) to remove pyridine, concentrated under reduced pressure and subjected to fast separation and purification (eluent: EA : PE = 0%-30%) to obtain the compound 41D (550 g, 87%).

$^1H$ NMR (400 MHz, $CDCl_3$) δ 8.08 - 8.02 (m, 2H), 7.58 (t, 1H), 7.45 (t, 2H), 6.54 (d, 1H), 5.53 (d, 1H), 4.74 - 4.67 (m, 2H), 4.46 (dd, 1H), 3.64 (dd, 1H), 2.17 (s, 3H), 2.11 (s, 3H).

Step 4: ((2R,3R,4R,5R)-4-acetoxy-5-(4,6-dichloro-1H-pyrazolo[3,4-d]pyrimidin-1-yl)-3-hydroxy-3-(trifluoromethyl)tetrahydrofuran-2-yl)methyl benzoate (41E)

Compound 41D (550 mg, 1.35 mmol) was added into a 25 mL reaction flask, heated to 90°C, 4,6-dichloro-1H-pyrazolo[3,4-C]pyrimidine (383 mg, 2.03 mmol) was added, after the addition, two drops of stannum tetrachloride were added dropwise, then the mixture was warmed to 130°C, reacted under reduced pressure for 30 minutes, and cooled to room temperature, and the residue was separated and purified by silica gel column chromatography (petroleum ether : ethyl acetate (v/v) = 10 : 1-5 : 1) to obtain the title compound 41E (293 mg, 41%).

LC-MS (ESI): m/z = 535.0$[M+H]^+$.

Step 5: ((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(trifluoromethyl)tetrahydrofuran-2-yl)methyl benzoate (41F)

Compound 41E (293 mg, 0.55 mmol) was dissolved in methanol (3 ml), triethylamine (167 mg, 1.65 mmol) and cis-7-azabicyclo[3.3.0]octane hydrochloride (122 mg, 1.1 mmol) were added, the mixture was reacted at room temperature for 2 h, and after the reaction was completed, the mixture was concentrated under reduced pressure to obtain the crude compound 41F, which was directly used in the next step.

LC-MS (ESI): m/z = 568.2$[M+H]^+$.

Step 6: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-2-(hydroxymethyl)-3-(trifluoromethyl)tetrahydrofuran-3,4-diol (41G)

The compound from the previous step, 41F crude was dissolved in methanol (3 ml), potassium carbonate (228 mg, 1.65 mmol) was added, after the addition, the mixture was reacted at room temperature for 2 h, water (3 ml) was added to quench the reaction, the mixture was extracted with ethyl acetate (5 ml × 3), dried over anhydrous sodium sulfate, filtered, and concentrated, and then the residue was subjected to fast separation and purification by column chromatography (eluent: EA : PE = 0%-70%), to obtain the compound 41G (158 mg, 62%).

LC-MS (ESI): m/z = 464.1[M+H]+.

Step 7: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(trifluoromethyl)tetrahydrofuran-2-yl)methoxy)(hydroxy)phosphoryl)methyl) phosphonic acid (Compound 41)

Compound 41G (158 mg, 0.34 mmol) was dissolved in trimethyl phosphate (2 ml), the temperature was decreased to 0°C under an ice bath, methylenebis(phosphonic dichloride) (255 mg, 1.02 mmol) was dissolved in trimethyl phosphate (1 ml), and the mixture was slowly added dropwise into the reaction solution, after the addition, the mixture was reacted at 0°C for 3 h, upon completion monitored by LCMS, water (1 ml) was added, after the addition, the mixture was stirred for 1 h, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 41 (100 mg, 47%).

LC-MS (ESI): m/z = 622.1[M+H]+.

$^1$H NMR (400 MHz, $D_2O$) δ 8.15 (d, 1H), 6.14 (d, 1H), 5.30 (d, 1H), 4.44 (d, 1H), 4.26 (s, 1H), 4.17 (s, 1H), 4.04 - 3.86 (m, 1H), 3.85 - 3.73 (m, 1H), 3.65 - 3.52 (m, 1H), 3.47 (d, 1H), 2.91 (s, 1H), 2.79 (s, 1H), 2.23 (t, 2H), 1.98 - 1.81 (m, 2H), 1.77 (d, 1H), 1.70 - 1.62 (m, 1H), 1.51 (dd, 2H).

$^{19}$F NMR (376 MHz, $D_2O$) δ -73.67.

**Example 42:**

(((((2R,3S,4R,SR)-5-(6-chloro-4-(((1S,5R)-5-hydroxyadamantan-2-yl)amino)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 42)

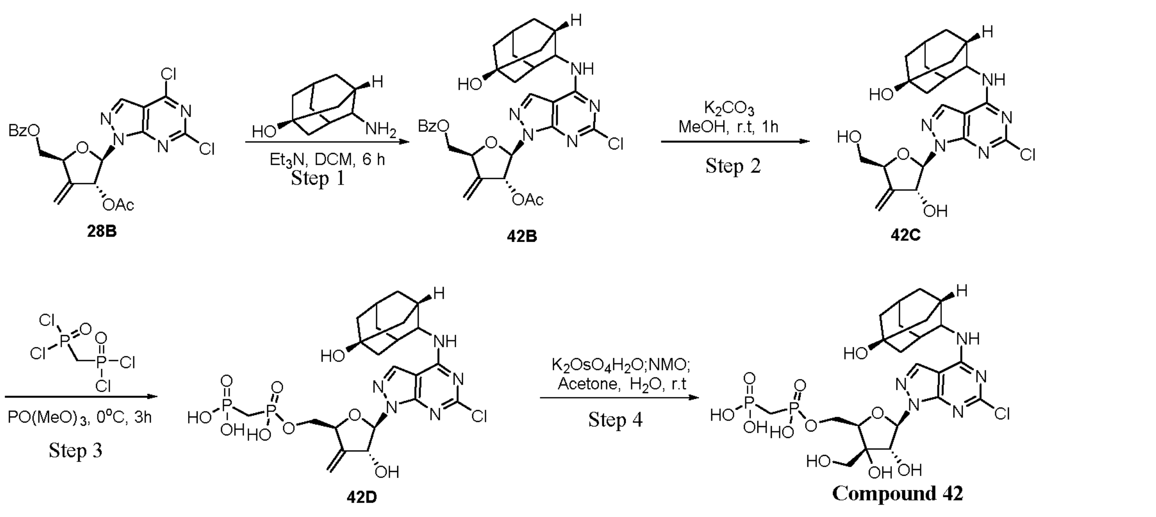

28B 42B 42C 42D Compound 42

Step 1: ((2S,4R,5R)-4-acetoxy-5-(6-chloro-4-(((1S,5R)-5-hydroxyadamantan-2-yl)amino)-1H-pyrazolo[3,4-d]pyrimidin-1-yl)-3-methylenetetrahydrofuran-2-yl)methyl benzoate (42B)

Compound 28B (3 g, 6.48 mmol) was dissolved in dichloromethane (30 ml), triethylamine (1.97 g, 19.44 mmol) and (1R, 3S)-4-aminoadamantan-1-ol hydrochloride (2.64 g, 12.96 mmol) were added, after the addition, the mixture was reacted at room temperature for 6 hours, after the reaction was completed, the mixture was concentrated and the resulting residue was purified by silica gel column chromatography (eluent: EA : PE = 0%-70%) to obtain the target compound 42B (2.32 g, 58%).

LC-MS (ESI): m/z = 594.2 [M+1]+.

Step 2: (2R,3R,5S)-2-(6-chloro-4-(((1S,5R)-5-hydroxyadamantan-2-yl)amino) - 1H-pyrazolo[3,4-d]pyrimidin-1-yl)-5-(hydroxymethyl)4-methylenetetrahydrofuran-3-ol (42C)

Compound 42B (2.32 mg, 3.91 mmol) was dissolved in methanol (25 ml), potassium carbonate (1.62 g, 11.73 mmol) was added, after the addition, the mixture was reacted at room temperature for 4 hours, water (3 ml) was added to quench the reaction, the mixture was extracted with ethyl acetate (30 ml × 3), dried over anhydrous sodium sulfate, concentrated and the resulting residue was purified by silica gel column chromatography (eluent: MeOH : EA = 0%-10%), to obtain the product 42C (1.72 g, 98%).

LC-MS (ESI): m/z = 448.1 $[M+1]^+$.

Step 3: (((((2S,4R,5R)-5-(6-chloro-4-(((1S,5R)-5-hydroxyadamantan-2-yl)amino) -1H-pyrazolo[3,4-d]pyrimidin-1-yl)-4-hydroxy-3-methylenetetrahydrofuran-2-yl)methoxy)(hydroxy)phosphoryl)methyl)phosphonic acid (42D)

Compound 42C (500 mg, 1.12 mmol) was dissolved in trimethyl phosphate (4 ml), the temperature was decreased to 0°C under an ice bath, methylenebis(phosphonic dichloride) (840 mg, 3.36 mmol) was dissolved in trimethyl phosphate (2 ml), and the mixture was slowly added dropwise into the reaction solution, after the addition, the mixture was reacted at 0°C for 3 hours, upon completion monitored by LCMS, water (2 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 42D (400 mg, 59%).

LC-MS (ESI): m/z = 604.0$[M-H]^-$.

Step 4: (((((2R,3S,4R,SR)-5-(6-chloro-4-(((1S,SR)-5-hydroxyadamantan-2-yl)amino)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 42)

Compound 42D (400 mg, 0.66 mmol) and N-methylmorpholine oxide (232 mg, 1.98 mmol) were dissolved in acetone (3 ml) and water (1 mL), potassium osmate(VI) dihydrate (22 mg, 0.066) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 42 (150 mg, 36%).

LC-MS (ESI): m/z = 640.0 $[M+1]^+$.

$^1H$ NMR (400 MHz, D2O) δ = 8.24 (s, 1H), 6.15 (d, 1H), 4.98 (d, 1H), 4.82 (d, 1H), 4.36 - 4.29 (m, 1H), 4.13 (dd, 3H), 3.88 (t, 2H), 2.42 - 2.36 (m, 2H), 2.30 (d, 1H), 2.27 - 2.21 (m, 1H), 2.18 - 2.11 (m, 1H), 1.93 (d, 2H), 1.79 (s, 2H), 1.73 (s, 3H), 1.63 (d, 2H).

**Example 43:**

(((((2R,3S,4R,5R)-5-(6-chloro-4-(((1R,5R)-5-hydroxyadamantan-2-yl)amino)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 43)

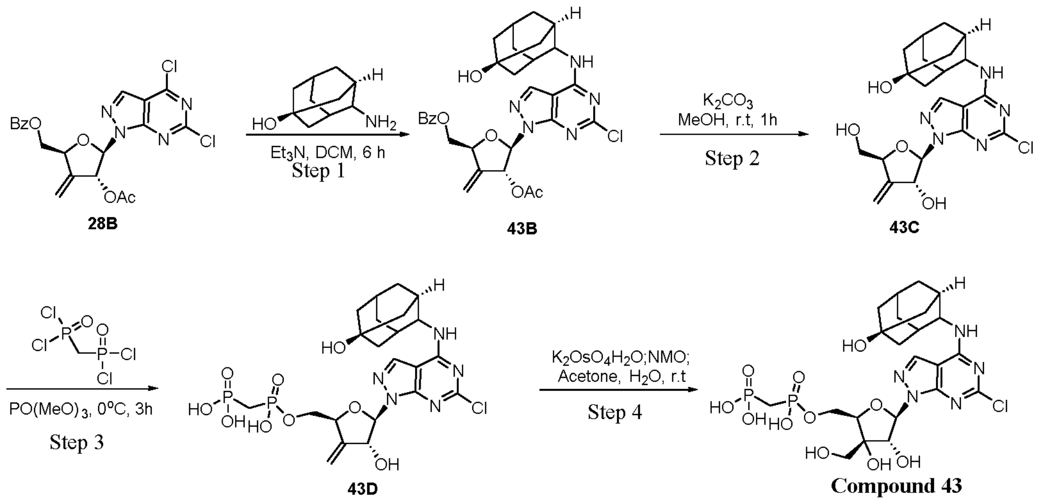

28B 43B 43C
43D Compound 43

Step 1: ((2S,4R,5R)-4-acetoxy-5-(6-chloro-4-(((1R,5R)-5-hydroxyadamantan-2-yl)amino)-1H-pyrazolo[3,4-d]pyrimidin-1-yl)-3-methylenetetrahydrofuran-2-yl)methyl benzoate (43B)

Compound 28B (3 g, 6.48 mmol) was dissolved in dichloromethane (30 ml), triethylamine (1.97 g, 19.44 mmol) and (1R, 3R)-4-aminoadamantan-1-ol hydrochloride (2.64 g, 12.96 mmol) were added, after the addition, the mixture was reacted at room temperature for 6 hours, after the reaction was completed, the mixture was concentrated and the resulting residue was purified by silica gel column chromatography (eluent: EA : PE = 0%-70%) to obtain the target compound 43B (2.2 g, 55%).
LC-MS (ESI): m/z = 594.2 $[M+1]^+$.

Step 2: (2R,3R,5S)-2-(6-chloro-4-(((1R,5R)-5-hydroxyadamantan-2-yl)amino)-1H-pyrazolo[3,4-d]pyrimidin-1-yl)-5-(hydroxymethyl)-4-methylenetetrahydrofuran-3-ol (43C)

Compound 43B (2.2 mg, 3.70 mmol) was dissolved in methanol (25 ml), potassium carbonate (1.54 g, 11.11 mmol) was added, after the addition, the mixture was reacted at room temperature for 4 hours, water (3 ml) was added to quench the reaction, the mixture was extracted with ethyl acetate (20 ml × 3), dried over anhydrous sodium sulfate, and concentrated and the resulting residue was purified by silica gel column chromatography (eluent: MeOH : EA = 0%-10%), to obtain the product 43C (1.62 g, 98%).
LC-MS (ESI): m/z = 448.1 $[M+1]^+$.

Step 3: (((((2S,4R,5R)-5-(6-chloro-4-(((1R,5R)-5-hydroxyadamantan-2-yl)amino) -1H-pyrazolo[3,4-d]pyrimidin-1-yl)-4-hydroxy-3-methylenetetrahydrofuran-2-yl)methoxy)(hydroxy)phosphoryl)methyl)phosphonic acid (43D)

Compound 43C (500 mg, 1.12 mmol) was dissolved in trimethyl phosphate (4 ml), the temperature was decreased to 0°C under an ice bath, methylenebis(phosphonic dichloride) (840 mg, 3.36 mmol) was dissolved in trimethyl phosphate (2 ml), and the mixture was slowly added dropwise into the reaction solution, after the addition, the mixture was reacted at 0°C for 3 hours, upon completion monitored by LCMS, water (2 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 43D (350 mg, 52%).
LC-MS (ESI): m/z = 604.0$[M-H]^-$.

Step 4: (((((2R,3S,4R,5R)-5-(6-chloro-4-(((1R,5R)-5-hydroxyadamantan-2-yl)amino)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 43)

Compound 43D (350 mg, 0.58 mmol) and N-methylmorpholine oxide (204 mg, 1.74 mmol) were dissolved in

acetone (3 ml) and water (1 mL), potassium osmate(VI) dihydrate (19 mg, 0.058) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 43 (150 mg, 40%).

LC-MS (ESI): m/z = 640.0 $[M+1]^+$.

1H NMR (400 MHz, D2O) δ = 8.23 (s, 1H), 6.14 (d, 1H), 4.98 (s, 1H), 4.82 (s, 1H), 4.41 - 4.35 (m, 1H), 4.21 (s, 1H), 4.17 - 4.08 (m, 2H), 3.88 (t, 2H), 2.29 (t, 5H), 2.14 (s, 1H), 1.91 (s, 4H), 1.77 (s, 3H), 1.55 - 1.47 (m, 2H).

**Example 44:**

(((((2S,3R,4R,5R)-5-(6-chloro-4-((3aR,6aS)-hexahydrocyclopenta[c]pyrrol-2(1H)-yl)-1H-pyrazolo[3,4-d]pyrimidin-1-yl)-4-hydroxy-3-(hydroxymethyl) tetrahydrofuran-2-yl)methoxy)(hydroxy)phosphoryl)methyl)phosphonic acid (Compound 44)

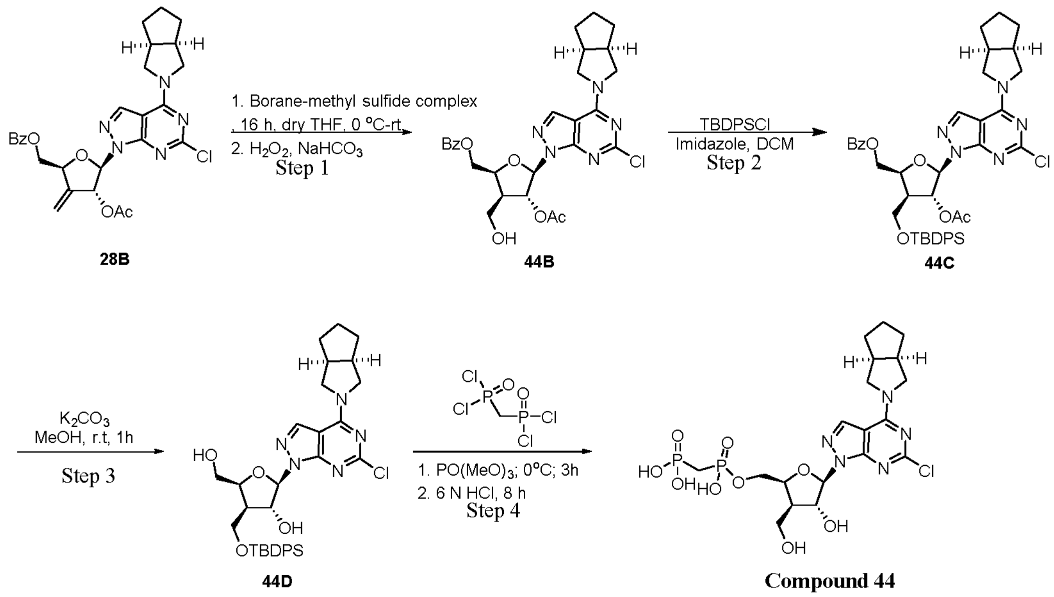


Step 1: ((2S,3S,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-(hydroxymethyl) tetrahydrofuran-2-yl)methyl benzoate (44B)

Compound 28B (2 g, 3.72 mmol) was dissolved in anhydrous tetrahydrofuran (20 ml), nitrogen replacement was carried out three times, dimethyl sulfide borane complex (11.15 mmol) was added dropwise under an ice bath, the mixture was stirred at room temperature for 16 h, 30% hydrogen peroxide (5 ml) and aqueous saturated sodium bicarbonate solution (1 ml) were added dropwise under an ice bath, after the addition, the mixture was reacted at room temperature for 2 hours, after the reaction was completed, water was added to quench the reaction, the reaction solution was extracted with ethyl acetate (20 ml × 3), the organic phases were combined, concentrated and the resulting residue was purified by silica gel column chromatography (eluent: EA : PE = 0%-70%), to obtain the target compound 44B (0.93 g, 45%).

LC-MS (ESI): m/z = 556.2 $[M+1]^+$.

Step 2: ((2S,3S,4R,5R)-4-acetoxy-3-(((tert-butyldiphenylsilyl)oxy)methyl)-5-(6-chloro-4-((3aR,6aS)-hexahydrocyclopenta[c]pyrrol-2(1H)-yl)-1H-pyrazolo[3,4-d]pyrimidin-1-yl)tetrahydrofuran-2-yl)methyl benzoate (44C)

Compound 44B (0.93 g, 1.67 mol) was dissolved in dichloromethane (10 ml), imidazole (341 mg, 5.02 mol)

and tert-butylchlorodiphenylsilane (689 mg, 2.51 mol) were added, the mixture were reacted at room temperature for 3 h, after the reaction was completed, water (10 ml) was added to quench the reaction, organic phases were separated, the aqueous phase was extracted with dichloromethane (10 ml × 2), the organic phases were combined, dried over anhydrous sodium sulfate, concentrated and the resulting residue was purified by silica gel column chromatography (eluent: EA : PE = 0%-70%), to obtain the target compound 44C (648 mg, 98%).

$^1H$ NMR (400 MHz, $CDCl_3$) δ 8.09 - 7.92 (m, 2H), 7.83 (s, 1H), 7.72-7.66 (m, 4H), 7.53 (t, 1H), 7.46 - 7.30 (m, 8H), 6.35 (d, 1H), 5.95 (t, 1H), 4.79 - 4.64 (m, 2H), 4.55 (dd, 1H), 4.10 (d, 2H), 4.02 - 3.90 (m, 2H), 3.56 (s, 2H), 2.90 (s, 1H), 2.82 (dt, 2H), 1.99 (s, 3H), 1.94 (s, 1H), 1.82 (dt, 1H), 1.74 - 1.67 (m, 1H), 1.59-1.52 (m, 2H), 1.26 (s, 1H), 1.08 (s, 9H).

Step 3: (2R,3R,4R,5S)-4-(((tert-butyldiphenylsilyl)oxy)methyl)-2-(6-chloro-4-((3aR,6aS)-hexahydrocyclopenta[c]pyrrol-2(1H)-yl)-1H-pyrazolo[3,4-d]pyrimidin-1-yl)-5-(hydroxymethyl)tetrahydrofuran-3-ol (44D)

Compound 44C (1.2 g, 1.51 mmol) was dissolved in methanol (10 ml), potassium carbonate (626 mg, 4.53 mmol) was added, after the addition, the mixture was reacted at room temperature for 2 hours, water (3 ml) was added to quench the reaction, the mixture was extracted with ethyl acetate (10 ml × 3), dried over anhydrous sodium sulfate, and concentrated and the resulting residue was purified by silica gel column chromatography (eluent: EA : PE = 0%-70%), to obtain the target compound 44D (648 mg, 98%).

LC-MS (ESI): m/z = 648.2 $[M+1]^+$.

Step 4: (((((2S,3R,4R,5R)-5-(6-chloro-4-((3aR,6aS)-hexahydrocyclopenta[c] pyrrol-2(1H)-yl)-1H-pyrazolo[3,4-d]pyrimidin-1-yl)-4-hydroxy-3-(hydroxymethyl)tetrahydrofuran-2-yl)methoxy)(hydroxy)phosphoryl)methyl) phosphonic acid (Compound 44)

Compound 44D (350 mg, 0.54 mmol) was dissolved in trimethyl phosphate (3 ml), the temperature was decreased to 0°C under an ice bath, methylenebis(phosphonic dichloride) (405 mg, 1.62 mmol) was dissolved in trimethyl phosphate (2 ml), and the mixture was slowly added dropwise into the reaction solution, after the addition, the mixture was reacted at 0°C for 3 hours, upon completion monitored by LCMS, 6 N HCl (2 ml) was added, after the addition, the mixture was stirred for 8 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 44 (180 mg, 58%).

LC-MS (ESI): m/z = 568.0$[M+H]^+$.

$^1H$ NMR (400 MHz, $D_2O$) δ 8.11 (d, 1H), 6.04 (d, 1H), 4.96 (dd, 1H), 4.61 (td, 1H), 4.24 - 4.04 (m, 2H), 3.95 (dd, 2H), 3.90 - 3.70 (m, 2H), 3.62 - 3.49 (m, 1H), 3.50 - 3.36 (m, 1H), 2.94-2.87 (m, 1H), 2.37 - 2.19 (m, 2H), 1.95 - 1.81 (m, 2H), 1.76 (dt, 1H), 1.70 - 1.60 (m, 1H), 1.59 - 1.39 (m, 2H).

**Example 45:**

(((((2R,3S,4R,5R)-5-(6-chloro-4-((3aR,6aS)-hexahydrocyclopenta[c]pyrrol-2(1H)-yl)-1H-pyrazolo[3,4-b]pyridin-1-yl)-3-ethynyl-3,4-dihydroxytetrahydrofuran-2-yl)methoxy)(hydroxy)phosphoryl)methyl)phosphonic acid (Compound 45)

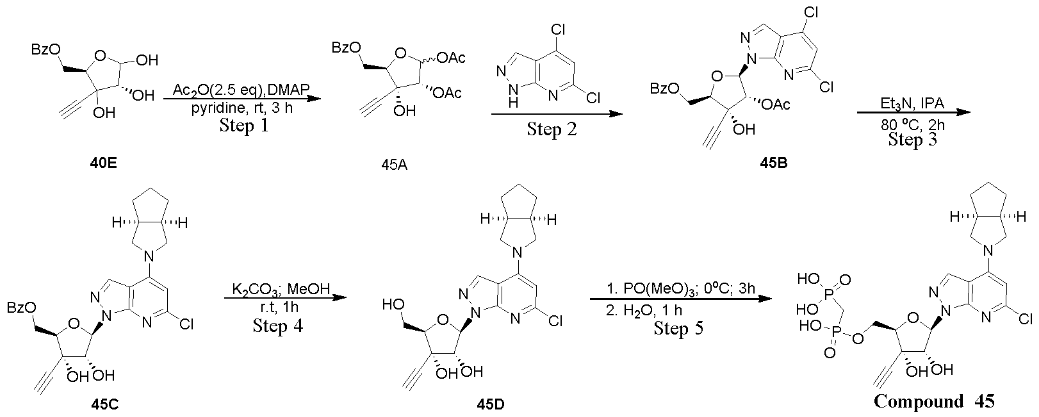


40E 45A 45B 45C 45D Compound 45

Step 1: (3R,4R,5R)-5-((benzoyloxy)methyl)-4-ethynyl-4-hydroxytetrahydrofuran-2,3-diyl diacetate (45A)

Compound 40E (1.0 g, 3.29 mmol) was dissolved in pyridine (10 ml), the temperature was decreased to 0°C under an ice bath, acetic anhydride (840 mg, 8.23 mmol) and 4-dimethylaminopyridine (40 mg, 0.33 mmol) were added, the mixture transferred to room temperature and reacted for 3 h, after the reaction was completed, ethyl acetate (50 ml) was added for dilution, the mixture was washed with 0.1 N hydrochloric acid (100 ml) to remove pyridine, concentrated under reduced pressure and subjected to fast separation and purification (eluent: EA : PE = 0%-30%) to obtain the compound 45A (1.2 g, 94%).

$^1$H NMR (400 MHz, $CDCl_3$) δ 8.11 (d, 2H), 7.57 (t, 1H), 7.45 (t, 2H), 6.19 (d, 1H), 5.50 (d, 1H), 4.74 (dd, 1H), 4.57 (dd, 1H), 4.45 (dd, 1H), 2.75 (s, 1H), 2.18 (s, 3H), 2.02 (s, 3H).

$^1$H NMR (400 MHz, $CDCl_3$) δ 8.05 (d, 2H), 7.57 (t, 1H), 7.45 (t, 2H), 6.51 (d, 1H), 5.44 (d, 1H), 4.65 (dd, 1H), 4.61 - 4.54 (m, 2H), 2.68 (s, 1H), 2.18 (s, 3H), 2.11 (s, 3H).

Step 2: ((2R,3R,4R,5R)-4-acetoxy-5-(4,6-dichloro-1H-pyrazolo[3,4-b]pyridin-1-yl)-3-ethynyl-3-hydroxytetrahydrofuran-2-yl)methyl benzoate (45B)

Compound 45A (1.4 g, 3.31 mmol) was added into a 25 mL reaction flask, heated to 90°C, 4,6-dichloro-1H-pyrazolo[3,4-b]pyridine (0.38 g, 4.97 mmol) was added, after the addition, two drops of stannum tetrachloride were added dropwise, then the mixture was warmed to 130°C, reacted under reduced pressure for 30 minutes, and cooled to room temperature, and the residue was separated and purified by silica gel column chromatography (petroleum ether : ethyl acetate (v/v) = 10 : 1-5 : 1) to obtain the title compound 45B (300 mg, 15%).

LC-MS (ESI): m/z = 490.1 $[M+H]^+$.

Step 3: ((2R,3S,4R,SR)-5-(6-chloro-4-((3aR,6aS)-hexahydrocyclopenta [c]pyrrol-2(1H)-yl)-1H-pyrazolo[3,4-b]pyridin-1-yl)-3-ethynyl-3,4-dihydroxytetrahydrofuran-2-yl)methyl benzoate (45C)

Compound 45B (300 mg, 0.61 mmol) was dissolved in isopropanol (6 ml), triethylamine (370 mg, 3.66 mmol) and cis-7-azabicyclo[3.3.0]octane hydrochloride (203 mg, 1.83 mmol) were added, the mixture was reacted in an oil bath pan at 80 °C for 3 h, and after the reaction was completed, the mixture was concentrated under reduced pressure and subjected to fast separation and purification (eluent: EA : PE = 0%-50%) to obtain the compound 45C (167 mg, 52%).

LC-MS (ESI): m/z = 523.1 $[M+H]^+$.

Step 4: (2R,3S,4R,5R)-5-(6-chloro-4-((3aR,6aS)-hexahydrocyclopenta[c] pyrrol-2(1H)-yl)-1H-pyrazolo[3,4-b]pyridin-1-yl)-3-ethynyl-2-(hydroxymethyl) tetrahydrofuran-3,4-diol (45D)

Compound 45C (167 mg, 0.32 mmol) was dissolved in methanol (3 ml), potassium carbonate (133 mg, 0.96 mmol) was added, after the addition, the mixture was reacted at room temperature for 2 h, water (3 ml) was added to quench the reaction, the mixture was extracted with ethyl acetate (5 ml × 3), dried over anhydrous sodium sulfate, filtered, and concentrated, and then the residue was subjected to fast separation and purification by column chromatography (eluent: EA : PE = 0%-70%), to obtain the product 45D (130 mg, 97%).

LC-MS (ESI): m/z = 419.2 $[M+H]^+$.

Step 5: (((((2R,3S,4R,5R)-5-(6-chloro-4-((3aR,6aS)-hexahydrocyclopenta[c] pyrrol-2(1H)-yl)-1H-pyrazolo[3,4-b]pyridin-1-yl)-3-ethynyl-3,4-dihydroxytetrahydrofuran-2-yl)methoxy)(hydroxy)phosphoryl)methyl)phosphonic acid (Compound 45)

Compound 45D (130 mg, 0.31 mmol) was dissolved in trimethyl phosphate (2 ml), the temperature was decreased to 0°C under an ice bath, methylenebis(phosphonic dichloride) (233 mg, 0.93 mmol) was dissolved in trimethyl phosphate (2 ml), and the mixture was slowly added dropwise into the reaction solution, after the addition, the mixture was reacted at 0°C for 3 h, upon completion monitored by LCMS, water (2 ml) was added, after the addition, the mixture was stirred for 1 h, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 45 (50 mg, 28%).

LC-MS (ESI): m/z = 577.1[M+H]$^+$.

$^1$H NMR (400 MHz, $D_2O$) δ 8.81 (s, 1H), 6.35 (s, 1H), 5.96 (d, 1H), 4.96 (s, 1H), 4.46 (s, 1H), 4.27 (d, 2H), 4.19 (s, 1H), 3.91 (s, 1H), 3.76 (s, 1H), 3.56 (s, 1H), 3.15 (s, 1H), 2.95 (d, 2H), 2.32-2.20 (m, 2H), 1.91 (s, 2H), 1.86 - 1.76 (m, 1H), 1.72 - 1.64 (m, 1H), 1.56 (s, 2H).

**Example 46:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3-ethyl-3,4-dihydroxytetrahydrofuran-2-yl)methoxy)(hydroxy)phosphoryl)methyl)phosphonic acid (Compound 46)

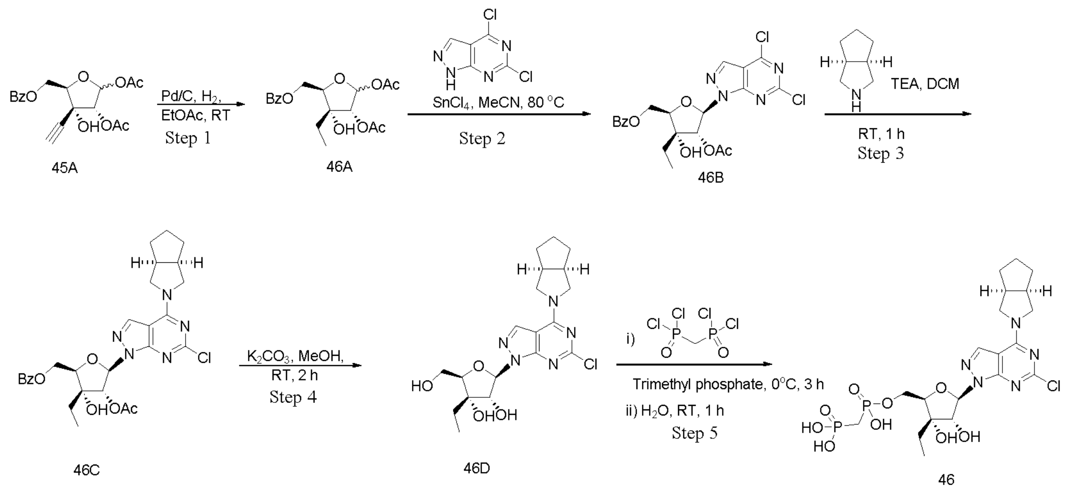


Step 1: (3R,4R,5R)-5-((benzoyloxy)methyl)-4-ethyl-4-hydroxytetrahydrofuran-2,3-diyldiacetate (46A)

Compound 45A (700 mg, 1.93 mmoL) was dissolved in ethyl acetate (10 mL), Pd/C (21 mg, 0.193 mmoL) was added, the gas was drained from the flask and a hydrogen balloon was inserted, the mixture was reacted at room temperature for 2 hours, upon completion monitored by TLC, the mixture was filtered through Celite, washed with ethyl acetate, and the organic phase was drained, and the resulting residue was directly used in the next reaction.

Step 2: ((2R,3R,4R,5R)-4-acetoxy-5-(4,6-dichloro-1H-pyrazolo[3,4-d]pyrimidin-1-yl)-3-ethyl-3-hydroxytetrahydrofuran-2-yl)methyl benzoate (46B)

Compound 46A (730 mg, 2.17 mmoL) and 4,6-dichloro-1H-pyrazolo[3,4-d]pyrimidine (452 mg, 2.39 mmoL) were dissolved in dry acetonitrile (30 mL), stannum tetrachloride (1.13 g, 4.35 mmoL) was added dropwise while stirring, after the addition, the mixture was warmed to 80°C and reacted for 2 hours, upon completion monitored by TLC, saturated aqueous sodium bicarbonate solution (10 mL) and water (100 mL) were added, the mixture was extracted with ethyl acetate (30 mL × 8), the organic phases were combined and dried over anhydrous sodium sulfate, filtered and concentrated, and then purified by column chromatography (petroleum ether : ethyl acetate (v/v) = 2 : 1) to obtain the compound 46B (65 mg, 6.7% over 2 steps), as a white foam.

Step 3: ((2R,3R,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-ethyl-3-hydroxytetrahydrofuran-2-yl)methyl benzoate (46C)

Compound 46B (65 mg, 0.131 mmoL) was dissolved in dry dichloromethane (50 mL), triethylamine (67 mg, 0.657 mmoL) and cis-7-azabicyclo[3.3.0]octane (29 mg, 0.197 mmoL) were added at room temperature, and after the addition, the mixture was reacted at room temperature for 2 hours. Upon completion monitored by TLC, the mixture was dried with suction. The crude was purified by silica gel column chromatography (petroleum ether : ethyl acetate (v/v) = 2 : 1) to obtain the compound 46C (70 mg, 93.5%) as a white foam solid.

LC-MS (ESI):1.07 min, 570.2 $[M+H]^+$.

Step 4: (2R,3S,4R,5R)-5-(6-chloro-4-((3aR,6aS)-hexahydrocyclopenta [c]pyrrol-2(1H)-yl)-1H-pyrazolo[3,4-d]pyrimidin-1-yl)-3-ethyl-2-(hydroxymethyl) tetrahydrofuran-3,4-diol (46D)

Compound 46C (70 mg, 0.123 mmoL) was dissolved in methanol (3 mL), potassium carbonate (67.9 mg, 0.491 mmoL) was added at room temperature, and after the addition, the mixture was reacted at room temperature for 1 hour. Upon completion monitored by TLC, saturated aqueous ammonium chloride solution (2 mL) and water (5 mL) were added, and the mixture was extracted with ethyl acetate (2 mL × 8), the organic phases were combined, dried over anhydrous sodium sulfate, filtered and concentrated, and then purified by column chromatography (petroleum ether: ethyl acetate (v/v) = 1 : 2 to 1 : 10) to obtain the compound 46D (45 mg, 86.5%), as a white foam.

Step 5: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-ethyl-3,4-dihydroxytetrahydrofuran-2-yl)methoxy)(hydroxy)phosphoryl)methyl)phosphonic acid (Compound 46)

Compound 46D (45 mg, 0.106 mmoL) was dissolved in trimethyl phosphate (0.5 mL), the temperature was decreased to 0°C under an ice bath, methylenebis(phosphonic dichloride) (106 mg, 0.425 mmoL) was dissolved in trimethyl phosphate (0.5 mL), and the mixture was slowly added dropwise into the reaction solution, after the addition, the mixture was reacted at 0°C for 3 hours, upon completion monitored by LCMS, water (0.3 mL) was added, after the addition, the mixture was stirred for 0.5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 10 min, to obtain the compound 46 (27 mg, 43.7%).

LC-MS (ESI): 0.64 min, 580.1 $[M-H]^-$;

$^1H$ NMR (400 MHz, Deuterium Oxide) δ 8.26 (d, 1H), 6.23 (d, 1H), 5.01 (d, 1H), 4.36 (t, 1H), 4.23 - 3.97 (m, 3H), 3.94 - 3.85 (m, 1H), 3.70 (s, 1H), 3.57 (dd, 1H), 3.00 (s, 1H), 2.88 (s, 1H), 2.32 (t, 2H), 1.95 (dt, 3H), 1.88- 1.71 (m, 3H), 1.71- 1.52 (m, 2H),, 1.12 (t, 3H).

**Example 47:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3-cyclopropyl-3,4-dihydroxytetrahydrofuran-2-yl)methoxy)(hydroxy)phosphoryl)methyl)phosphonic acid (Compound 47)

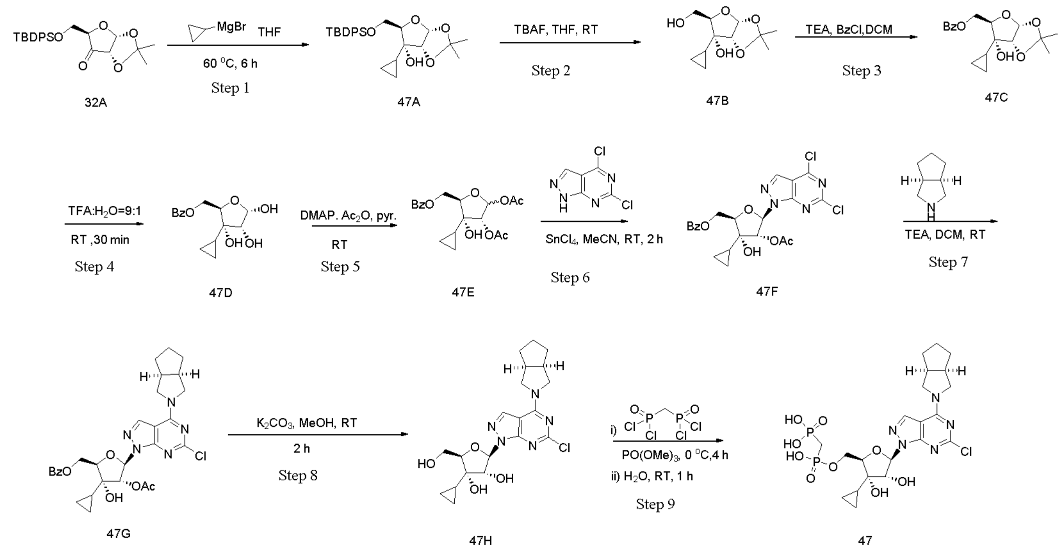


Step 1: (3aR,5R,6R,6aR)-5-(((tert-butyldiphenylsilyl)oxy)methyl)-6-cyclopropyl-2,2-dimethyltetrahydrofuro[2,3-d][1,3]dioxol-6-ol (47A)

Compound 32A (6.0 g, 14.1 mmoL) was dissolved in dry tetrahydrofuran (200 mL), cyclopropylmagnesium bromide (28.1 mL, 1 M in dry THF, 28.1 mmoL) was added dropwise under an ice-water bath, and then the mixture was warmed to 60°C and reacted for about 6 hours. Upon completion monitored by TLC, saturated aqueous ammonium chloride solution (100 mL) and water (300 mL) were added, and the mixture was extracted with ethyl acetate (100 mL × 3), the organic phases were combined, dried over anhydrous sodium sulfate, filtered and concentrated, and then the resulting residue (7 g) was directly used in the next reaction.

Step 2: (3aR,5R,6R,6aR)-6-cyclopropyl-5-(hydroxymethyl)-2,2-dimethyltetrahydrofuro[2,3-d][1,3]dioxol-6-ol (47B)

Compound 47A (7.0 g, 14.9 mmoL) was dissolved in dry tetrahydrofuran (200 mL), tetrabutylammonium fluoride in tetrahydrofuran (19.4 mL, 1 M in dry THF, 19.4 mmoL) was added dropwise at room temperature, and then the mixture was reacted at room temperature for about 2 hours. Upon completion monitored by TLC, saturated aqueous ammonium chloride solution (100 mL) and water (200 mL) were added, and the mixture was extracted with ethyl acetate (100 mL × 5), the organic phases were combined, dried over anhydrous sodium sulfate, filtered and concentrated, and then the crude was purified by silica gel column chromatography (petroleum ether : ethyl acetate (v/v) = 3 : 7) to obtain the compound 47B (2.64 g, 81.5% over 2 steps), as a colorless oil.

Step 3: ((3aR,5R,6R,6aR)-6-cyclopropyl-6-hydroxy-2,2-dimethyltetrahydrofuro[2,3-d][1,3]dioxol-5-yl)methyl benzoate (47C)

Compound 47B (2.64 g, 11.5 mmoL) was dissolved in dry dichloromethane (150 mL), triethylamine (3.49 g, 34.4 mmoL) and benzoyl chloride (2.10 g, 14.9 mmoL) were added at room temperature, and after the addition, the mixture was reacted at room temperature for 2 hours. Upon completion monitored by TLC, saturated aqueous ammonium chloride solution (100 mL) and water (200 mL) were added, and the mixture was extracted with dichloromethane (100 mL × 5), the organic phases were combined, dried over anhydrous sodium sulfate, filtered and concentrated, and then the crude was purified by silica gel column chromatography (petroleum ether : ethyl acetate (v/v) = 5 : 1) to obtain the compound 47C (3.64 g, 95.0%) as a white foam solid.

Step 4: ((2R,3S,4R,5S)-3-cyclopropyl-3,4,5-trihydroxytetrahydrofuran-2-yl)methyl benzoate (47D)

Compound 47C (3.50 g, 10.5 mmoL) was dissolved in a mixed solvent of trifluoroacetic acid (45 mL) and water (5 mL), and the mixture was reacted at room temperature for 30 minutes. Saturated aqueous sodium bicarbonate solution was added to adjust the solution to a neutral PH, the mixture was extracted with dichloromethane (50 mL × 10), the organic phases were combined and dried over anhydrous sodium sulfate, filtered and concentrated, and the resulting crude was purified by silica gel column chromatography (petroleum ether : ethyl acetate (v/v) = 1 : 1) to obtain the compound 47D (2.80 g, 90.9%), as a colorless oil.

Step 5: (3R,4R,5R)-5-((benzoyloxy)methyl)-4-cyclopropyl-4-hydroxytetrahydrofuran-2,3-diyl diacetate (47E)

Compound 47D (2.80 g, 9.51 mmoL) was dissolved in pyridine (30 mL), 4-dimethylaminopyridine (233 mg, 1.91 mmoL) and acetic anhydride (2.43 g, 23.8 mmoL) were added at room temperature, and after the addition, the mixture was reacted at room temperature for 2 hours. Upon completion monitored by TLC, dichloromethane (150 mL) was added, the organic phase was washed with 1 N diluted aqueous hydrochloric acid solution (50 mL × 3) to remove excess pyridine, and the organic phase was dried over anhydrous sodium sulfate, filtered and concentrated. The crude was purified by silica gel column chromatography (petroleum ether : ethyl acetate (v/v) = 4 : 1) to obtain the compound 47E (2.60 g, 72.2%) as a white foam solid.

Step 6: ((2R,3R,4R,SR)-4-acetoxy-3-cyclopropyl-5-(4,6-dichloro-1H-pyrazolo[3,4-d]pyrimidin-1-yl)-3-hydroxytetrahydrofuran-2-yl)methyl benzoate (47F)

Compound 47E (1.5 g, 3.97 mmoL) and 4,6-dichloro-1H-pyrazolo[3,4-d]pyrimidine (825 mg, 4.37 mmoL) were dissolved in dry acetonitrile (50 mL), stannum tetrachloride (2.07 g, 7.94 mmoL) was added dropwise while stirring, after the addition, the mixture was reacted at room temperature for 4 hours, upon completion monitored by TLC, saturated aqueous sodium bicarbonate solution (20 mL) and water (100 mL) were added, the mixture was extracted with ethyl acetate (30 mL × 8), the organic phases were combined and dried over anhydrous sodium sulfate, filtered and concentrated, and then purified by column chromatography (petroleum ether : ethyl acetate (v/v) = 2 : 1) to obtain the compound 47F (140 mg, 7.0%), as a white foam.

Step 7: ((2R,3R,4R,5R)-4-acetoxy-5-(6-chloro-4-((3aR,6aS)-hexahydrocyclopenta [c]pyrrol-2(1H)-yl)-1H-pyrazolo[3,4-d]pyrimidin-1-yl)-3-cyclopropyl-3-hydroxytetrahydrofuran-2-yl)methyl benzoate (47G)

Compound 47F (140 mg, 0.276 mmoL) was dissolved in dry dichloromethane (10 mL), triethylamine (140 mg, 1.37 mmoL) and cis-7-azabicyclo[3.3.0]octane (61.3 mg, 0.414 mmoL) were added at room temperature, and after the addition, the mixture was reacted at room temperature for 2 hours. Upon completion monitored by TLC, the mixture was dried with suction. The crude was purified by silica gel column chromatography (petroleum ether : ethyl acetate (v/v) = 2 : 1) to obtain the compound 47G (130 mg, 81.1%) as a white foam solid.

LC-MS (ESI): 582.2 $[M+H]^+$.

Step 8: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-cyclopropyl-2-(hydroxymethyl) tetrahydrofuran-3,4-diol (47H)

Compound 47G (130 mg, 0.224 mmoL) was dissolved in methanol (8 mL), potassium carbonate (155 mg, 1.12 mmoL) was added at room temperature, and after the addition, the mixture was reacted at room temperature for 2 hours. Upon completion monitored by TLC, saturated aqueous ammonium chloride solution (2 mL) and water (5 mL) were added, and the mixture was extracted with ethyl acetate (2 mL × 8), the organic phases were combined, dried over anhydrous sodium sulfate, filtered and concentrated, and then purified by column chromatography (petroleum ether: ethyl acetate (v/v) = 1 : 2 to 1 : 10) to obtain the compound 47H (89 mg, 91.2%).

LC-MS (ESI): 436.2 $[M+H]^+$.

Step 9: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3-cyclopropyl-3,4-dihydroxytetrahydrofuran-2-yl)methoxy)(hydroxy)phosphoryl)methyl)phosphonic acid (Compound 47)

Compound 47H (89 mg, 0.204 mmoL) was dissolved in trimethyl phosphate (1 mL), the temperature was decreased to 0°C under an ice bath, methylenebis(phosphonic dichloride) (153 mg, 0.612 mmoL) was dissolved in trimethyl phosphate (0.5 mL), and the mixture was slowly added dropwise into the reaction solution, after the addition, the mixture was reacted at 0°C for 3 hours, upon completion monitored by LCMS, water (0.5 mL) was added, after the addition, the mixture was stirred for 0.5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 10 min, to obtain the compound 47 (40 mg, 33.0%).

LC-MS (ESI): 0.59 min, 592.1 $[M-H]^-$;

$^1$H NMR (400 MHz, Deuterium Oxide) δ 8.25 (d, 1H), 6.20 (d, 1H), 5.03 (d, 1H), 4.42 - 4.25 (m, 3H), 4.13- 3.95 (m, 1H), 3.89 (td, 1H), 3.68 (td, 1H), 3.61 - 3.48 (m, 1H), 3.00 (s, 1H), 2.87 (d, 1H), 2.48 - 2.27 (m, 2H), 2.05 - 1.91 (m, 2H), 1.89 - 1.69 (m, 2H), 1.68 - 1.51 (m, 2H), 1.24 - 1.13 (m, 1H), 0.78 - 0.50 (m, 4H).

**Example 48:**

(((((2R,3S,4R,5R)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-(1H-1,2,3-triazol-4-yl)tetrahydrofuran-2-yl)methoxy)(hydroxy)phosphoryl)methyl)phosphonic acid (Compound 48)

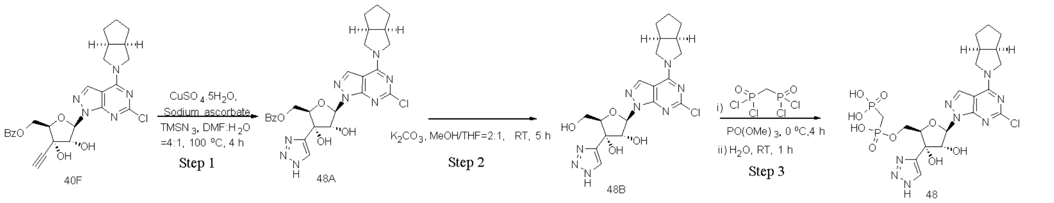


Step 1: ((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(1H-1,2,3-triazol-4-yl)tetrahydrofuran-2-yl)methyl benzoate (48A)

Under nitrogen protection, compound 40F (315 mg, 0.601 mmoL), anhydrous copper sulphate (31 mg, 0.124 mmoL), sodium ascorbate (49 mg, 0.247 mmoL) and azidotrimethylsilane (107 mg, 0.928 mmoL) were dissolved in a mixed solvent of N,N-dimethylformamide/water (4: 1, v/v) (20 mL), and the mixture was reacted at 100°C for 4 hours. Upon completion monitored by TLC, water (50 mL) was added, the mixture was extracted with dichloromethane (20 mL × 5), the organic phases were combined, dried over anhydrous sodium sulfate, filtered and concentrated, and the resulting crude was purified by silica gel column chromatography (dichloromethane: methanol (v/v) = 10 : 1) to obtain the compound 48A (300 mg, 88.0%).
LC-MS (ESI): 567.2 [M+H]$^+$.

Step 2: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-2-(hydroxymethyl)-3-(1H-1,2,3-triazol-4-yl)tetrahydrofuran-3,4-diol (48B)

Compound 48A (150 mg, 0.265 mmoL) was dissolved in a mixed solvent of methanol (6 mL) and tetrahydrofuran (3 mL), potassium carbonate (110 mg, 0.794 mmoL) was added at room temperature, and after the addition, the mixture was reacted at room temperature for 5 hours. Upon completion monitored by TLC, saturated aqueous ammonium chloride solution (3 mL) and water (5 mL) were added, and the mixture was extracted with ethyl acetate (2 mL × 8), the organic phases were combined, dried over anhydrous sodium sulfate, filtered and concentrated, and then purified by column chromatography (dichloromethane: methanol (v/v) = 10 : 1) to obtain the compound 48B (90 mg, 73.5%), as a brownish yellow solid.
LC-MS (ESI):463.1 [M+H]$^+$.

Step 3: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(1H-1,2,3-triazol-4-yl)tetrahydrofuran-2-yl)methoxy)(hydroxy)phosphoryl)methyl) phosphonic acid (Compound 48)

Compound 48B (90 mg, 0.194 mmoL) was dissolved in trimethyl phosphate (1 mL), the temperature was decreased to 0°C under an ice bath, methylenebis(phosphonic dichloride) (146 mg, 0.583 mmoL) was dissolved in trimethyl phosphate (1 mL), and the mixture was slowly added dropwise into the reaction solution, after the addition, the mixture was reacted at 0°C for 3 hours, upon completion monitored by LCMS, water (0.5 mL) was added, after the addition, the mixture was stirred for 0.5 hours, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 10 min, to obtain the compound

48 (38 mg, 31.4%).

LC-MS (ESI): 619.0 [M-H]⁻;

$^1$H NMR (400 MHz, D2O) δ 8.39 (s, 1H), 6.42 (s, 1H), 5.80 (s, 1H), 4.92 - 4.63 (m, 2H, overlapped), 4.26 - 4.05 (m, 1H), 3.99 - 3.71 (m, 4H), 3.62 (d, 1H), 3.03 (s, 1H), 2.90 (s, 1H), 2.07 - 1.57 (m, 8H).

**Example 49**

(((((2R,3S,4R,5R)-5-(4-(2-azabicyclo[2.2.2]octan-2-yl)-6-chloro-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 49)

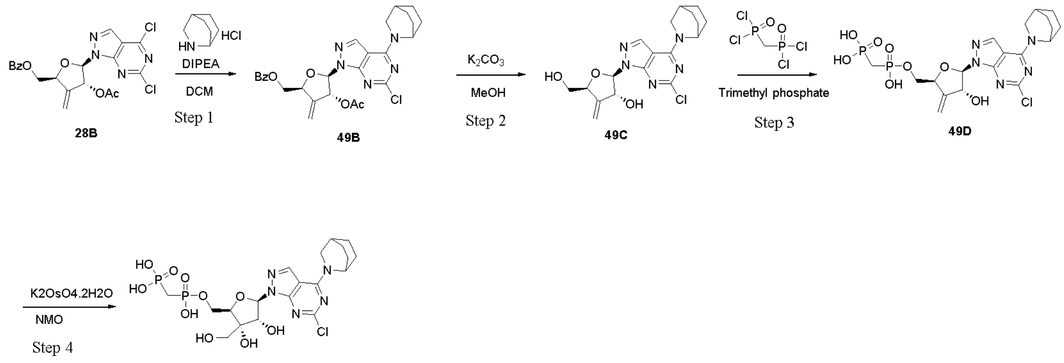


28B, 49B, 49C, 49D, Compound 49

Step 1: ((2S,4R,5R)-5-(4-(2-azabicyclo[2.2.2]octan-2-yl)-6-chloro-1H-pyrazolo[3,4-d]pyrimidin-1-yl)-4-acetoxy-3-methylenetetrahydrofuran-2-yl)methyl benzoate (49B)

28B (1 g, 2.16 mmol) and 2-azabicyclo[2.2.2]octane hydrochloride (410 mg, 2.79 mmol) were added into dichloromethane, followed by N,N-diisopropyl ethylamine (1.12 g, 8.64 mmol), and the mixture was reacted at room temperature for 2h. Upon complete depletion of raw materials monitored by TLC, the reaction solution was directly added to silica gel, concentrated, and then the crude product was separated and purified by silica gel column chromatography (eluent: EA/PE = 0-30%), to obtain the title compound (600 mg, 51.6%).

LC-MS (ESI): m/z = 538.2 [M+H]⁺.

Step 2: (2R,3R,5S)-2-(4-(2-azabicyclo[2.2.2]octan-2-yl)-6-chloro-1H-pyrazolo[3,4-d]pyrimidin-1-yl)-5-(hydroxymethyl)-4-methylenetetrahydrofuran-3-ol (49C)

49B (600 mg, 1.12 mmol), and potassium carbonate (460 mg, 3.33 mmol) were added into methanol (10 ml), the mixture was reacted at room temperature for 3h, upon completion monitored by LC-MS, the mixture was concentrated to remove methanol, water (20 mL) was added and the reaction was extracted with ethyl acetate (20 mL × 2), the organic phases were combined, washed with saturated brine, dried over anhydrous sodium sulfate, filtered, and concentrated, and the crude product was separated and purified by silica gel column chromatography (eluent: DCM : MeOH = 0-5%), to obtain the title compound (430 mg, 98%).

LC-MS (ESI): m/z = 392.1 [M+H]⁺.

Step 3: (((((2S,4R,5R)-5-(4-(2-azabicyclo[2.2.2]octan-2-yl)-6-chloro-1H-pyrazolo[3,4-d]pyrimidin-1-yl)-4-hydroxy-3-methylenetetrahydrofuran-2-yl)methoxy)(hydroxy)phosphoryl)methyl)phosphonic acid (49D)

49C (430 mg, 1.1 mmol) was added into trimethyl phosphate (5 ml), methylenebis(phosphonic dichloride) (830 mg, 3.31mmol) was dissolved in trimethyl phosphate (1 ml), and the mixture was added dropwise into the reaction system under an ice bath, the mixture was kept at 0°C and reacted for 3 h, upon complete depletion of raw materials monitored by LC-MS, water (1 ml) was added under an ice bath, the mixture was reacted at 0°C for another 1 h, and separated and purified by C18 reversed phase column chromatography, with mobile phases A and B: mobile phase A: acetonitrile; mobile phase B: water (containing 0.1%TFA), (A/B = 40%/60%), to obtain the title compound (200 mg, 33%).

LC-MS (ESI): m/z = 550.1 [M+H]⁺.

Step 4: (((((2R,3S,4R,5R)-5-(4-(2-azabicyclo[2.2.2]octan-2-yl)-6-chloro-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 49)

49D (200 mg, 0.36 mmol), potassium osmate (VI) dihydrate (10 mg) and N-methylmorpholine oxide (210 mg, 1.79 mmol) were added into a mixed system of acetone (6 ml) and water (2 ml). The mixture was reacted at room temperature overnight, upon completion monitored by LC-MS, the reaction solution was filtered, the filtrate was concentrated under reduced pressure to remove acetone, and then the residue was separated and purified by HPLC to obtain the title compound (18 mg, 8%). Preparative HPLC separation methods: 1. Instrument: waters 2767 (preparative liquid phase chromatographic instrument); chromatographic column: SunFire@ Prep C18 (19 mm × 250 mm); 2. The sample was dissolved in DMF and filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 40%; c. flow rate: 12 mL/min; d. elution time: 20 min.

$^1$H NMR (400 MHz, $D_2O$) δ 8.25 (s, 1H), 6.15 (d, 1H), 4.97 (d, 1H), 4.35 - 4.28 (m, 1H), 4.11 (s, 2H), 3.91 - 3.57 (m, 4H), 2.31-2.17 (m, 2H), 2.16 - 1.90 (m, 2H), 1.90-1.66 (m, , 8H).

LC-MS (ESI): m/z = 584.1[M+H]$^+$.

**Example 50**

(((((2R,3S,4R,5R)-5-(4-(adamantan-1-ylamino)-6-chloro-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 50)

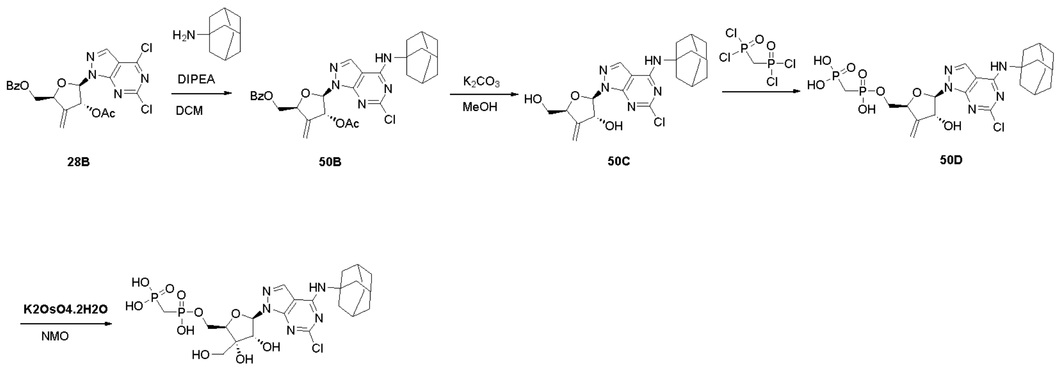

28B 50B 50C 50D

Compound 50

Step 1: ((2S,4R,5R)-4-acetoxy-5-(4-(adamantan-1-ylamino)-6-chloro-1H-pyrazolo[3,4-d]pyrimidin-1-yl)-3-methylenetetrahydrofuran-2-yl)methyl benzoate (50B)

28B (1 g, 2.16 mmol) and amantadine (650 mg, 4.30 mmol) were added into dichloromethane, followed by N,N-diisopropyl ethylamine (0.83 g, 6.46 mmol), and the mixture was reacted at room temperature for 16h. Upon complete depletion of raw materials monitored by TLC, the reaction solution was directly added to silica gel, concentrated, and then the crude product was separated and purified by silica gel column chromatography (eluent: EA/PE = 0-30%), to obtain the title compound (730 mg, 58%).

LC-MS (ESI): m/z = 612.2 [M+H]$^+$.

Step 2: (2R,3R,5S)-2-(4-(adamantan-1-ylamino)-6-chloro-1H-pyrazolo[3,4-d]pyrimidin-1-yl)-5-(hydroxymethyl)-4-methylenetetrahydrofuran-3-ol (50C)

50B (730 mg, 1.26 mmol) and potassium carbonate (520 mg, 3.76 mmol) were added into methanol (10 ml), the mixture was reacted at room temperature for 3 h, upon completion monitored by LC-MS, a large amount of white solids was precipitated, water (20 ml) was added, the mixture was filtered, the solids were directly washed with water, concentrated under reduced pressure and dried, to obtain the title compound (500 mg, 91%).

LC-MS (ESI): m/z = 432.2 [M+H]$^+$.

Step 3: (((((2S,4R,5R)-5-(4-(adamantan-1-ylamino)-6-chloro-1H-pyrazolo [3,4-d]pyrimidin-1-yl)-4-hydroxy-3-methylenetetrahydrofuran-2-yl)methoxy)(hydroxy)phosphoryl)methyl)phosphonic acid (50D)

50C (500 mg, 1.16 mmol) was added into trimethyl phosphate (10 ml), methylenebis(phosphonic dichloride) (870 mg, 3.48mmol) was dissolved in trimethyl phosphate (2 ml), and the mixture was added dropwise into the reaction system under an ice bath, the mixture was kept at 0°C and reacted for 3 h, upon complete depletion of raw materials monitored by LC-MS, water (0.5 ml) was added under an ice bath, the mixture was reacted at 0°C for another 1 h, and separated and purified by $C_{18}$ reversed phase column chromatography, with mobile phases A and B: mobile phase A: acetonitrile; mobile phase B: water (containing 0.1%TFA), (A/B = 40%/60%), to obtain the title compound (400 mg, 58%).

LC-MS (ESI): m/z = 590.1 $[M+H]^+$.

Step 4: (((((2R,3S,4R,5R)-5-(4-(adamantan-1-ylamino)-6-chloro-1H-pyrazolo [3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 50)

50D (400 mg, 0.68 mmol), potassium osmate (VI) dihydrate (10 mg) and N-methylmorpholine oxide (400 mg, 3.4 mmol) were added into a mixed system of acetone (6 ml) and water (2 ml). The mixture was reacted at room temperature overnight, upon completion monitored by LC-MS, the reaction solution was filtered, the filtrate was concentrated under reduced pressure to remove acetone, and then the residue was separated and purified by HPLC to obtain the title compound (45 mg, 10.6%). Preparative HPLC separation methods: 1. Instrument: waters 2767 (preparative liquid phase chromatographic instrument); chromatographic column: SunFire@ Prep C18 (19 mm × 250 mm); 2. The sample was dissolved in DMF and filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 40%; c. flow rate: 12 mL/min; d. elution time: 20 min.

$^1$HNMR(400 MHz, DMSO) δ 8.38 (s, 1H), 8.09 (s, 1H), 6.00 (d, 1H), 4.75 (d, 1H), 4.27-4.22 (m, 1H), 4.07-4.04 (m, 1H), 3.99-3.92 (m 1H), 3.54 (dd, 2H), 2.22 - 2.07 (m, 11H), 1.70-1.67 (m, 6H)

LC-MS (ESI): m/z = 624.1$[M+H]^+$.

**Example 51**

(((((2R,4R,5R)-5-(4-(8-azabicyclo[3.2.1]octan-8-yl)-6-chloro-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 51)

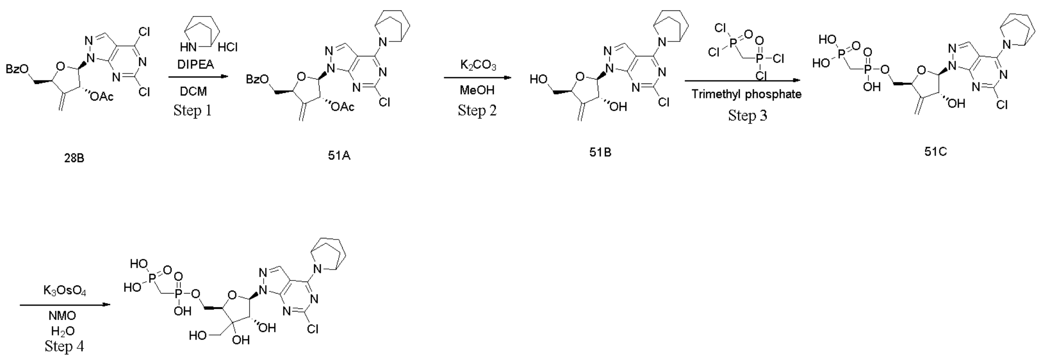


28B 51A 51B 51C

Compound 51

Step 1: ((2S,4R,5R)-5-(4-(8-azabicyclo[3.2.1]octan-8-yl)-6-chloro-1H-pyrazolo[3,4-d]pyrimidin-1-yl)-4-acetoxy-3-methylenetetrahydrofuran-2-yl)methyl benzoate (51A)

28B (1.0 g, 2.1 mmol) was dissolved in dichloromethane (10 mL), 8-azabicyclo[3.2.1]octane hydrochloride (0.41 g, 2.7 mmol) and diisopropyl ethylamine (1.1 g, 8.4 mmol) were then added, after the addition, the mixture was reacted at room temperature for 2 hours, after the reaction was completed, water was added, the mixture was extracted with dichloromethane (50 mL × 2) twice, the organic phases were combined, washed with saturated brine, dried over anhydrous sodium sulfate, filtered, and concentrated, and the residue was separated and purified by silica gel column chromatography (petroleum ether: ethyl acetate (v/v) = 2 : 1) to obtain the title compound 51A (0.8 g, 69%).

LC-MS (ESI): m/z = 538.2 $[M+H]^+$.

Step 2: (2R,3R,5S)-2-(4-(8-azabicyclo[3.2.1]octan-8-yl)-6-chloro-1H-pyrazolo[3,4-d]pyrimidin-1-yl)-5-(hydroxymethyl)-4-methylenetetrahydrofuran-3-ol (51B)

Compound 51A (0.8 g, 1.49 mmol) was dissolved in methanol (8 mL), potassium carbonate (0.62 g, 4.47 mmol) was added, after the addition, the mixture was stirred at room temperature for 2 hours, the mixture was concentrated to remove methanol, water (20 mL) was added, the mixture was extracted with ethyl acetate (20 mL × 2), the organic phases were combined, washed with saturated brine, dried over anhydrous sodium sulfate, filtered, and concentrated to obtain the title compound 51B (0.58 g, 100%).
LC-MS (ESI): m/z = 392.1 $[M+H]^+$.

Step 3: (((((2S,4R,5R)-5-(4-(8-azabicyclo[3.2. 1]octan-8-yl)-6-chloro-1H-pyrazolo[3,4-d]pyrimidin-1-yl)-4-hydroxy-3-methylenetetrahydrofuran-2-yl)methoxy)(hydroxy)phosphoryl)methyl)phosphonic acid (51C)

Compound 51B (0.58 g, 1.48 mmol) was dissolved in trimethyl phosphate (3 mL), the temperature was decreased to 0°C under an ice bath, methylenebis(phosphonic dichloride) (1.15 g, 4.45 mmol) was dissolved in trimethyl phosphate (3 mL) and the mixture was slowly added dropwise into the reaction solution, after the addition, the temperature was controlled at 0°C and the mixture was reacted for 2 hours, and after the reaction was completed, water (1 ml) was added, and the mixture was directly purified by C18 reversed phase column chromatography, with mobile phases A and B: mobile phase A: acetonitrile; mobile phase B: water (containing 0.1%TFA), (A/B = 40%/60%), to obtain the compound 51C (140 mg, 20%).
LC-MS (ESI): m/z = 550.1 $[M+H]^+$.

Step 4: (((((2R,4R,5R)-5-(4-(8-azabicyclo[3.2. 1]octan-8-yl)-6-chloro-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 51)

Compound 51C (0.14 g, 0.25 mmol) was dissolved in a mixed solvent of acetone (6 ml) and water (2 ml), N-methylmorpholine oxide (0.15 g, 1.27 mmol) was added, a catalytic amount of potassium osmate(VI) dihydrate (5 mg) was added, after the addition, the mixture was reacted at room temperature for 15 hours, after the reaction was completed, the mixture was concentrated to remove acetone and the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 51 (0.04 g, 27%).
LCMS m/z = 582.0 $[M-H]^-$.
$^1H$ NMR (400 MHz, $D_2O$): 8.25(s, 1H), 6.16-6.15 (d, 1H), 4.98-4.96 (d, 1H), 4.91(s, 1H), 4.70(d, 1H), 4.33-4.30 (t, 1H), 4.13-4.10 (t, 2H), 3.90-3.81 (m, 2H), 2.29-1.56 (m, 12H).

**Example 52**

(((((2R,3 S,4R,SR)-5-(6-chloro-4-((3aR,6aS)-hexahydrocyclopenta[c]pyrrol-2(1H)-yl)-1H-pyrazolo[3,4-d]pyrimidin-1-yl)-3,4-dihydroxy-3-(prop-1-yn-1-yl)tetrahydrofuran-2-yl)methoxy)(hydroxy)phosphoryl)methyl)phosphonic acid (Compound 52)

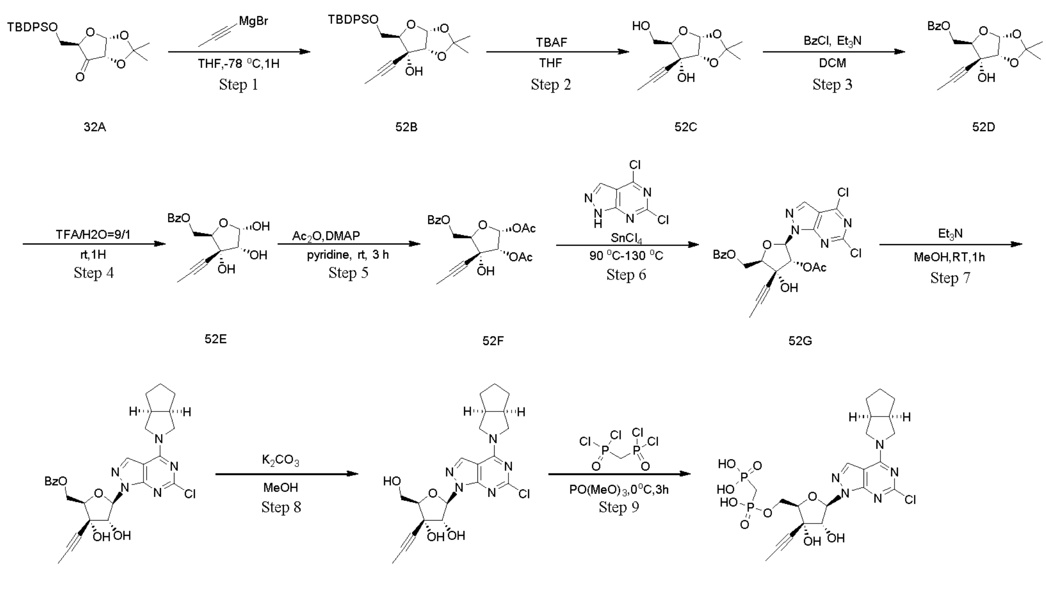


32A 52B 52C 52D

52E 52F 52G

52H 52I Compound 52

Step 1: (3aR,5R,6R,6aR)-5-(((tert-butyldiphenylsilyl)oxy)methyl)-2,2-dimethyl-6-(prop-1-yn-1-yl)tetrahydrofuro[2,3-d][1,3]dioxol-6-ol (52B)

32A (5.0 g, 11.78 mmol) was dissolved in anhydrous tetrahydrofuran (50 mL), under nitrogen protection, the mixture was cooled to -78°C with dry-ice ethanol, 1-propynyl magnesium bromide in tetrahydrofuran (12.96 mL, 12.96 mmol) was added dropwise, and then the mixture was reacted at this temperature for 3 hours. After the reaction was completed, aqueous ammonium chloride solution was added to quench the reaction, the reaction mixture was extracted with ethyl acetate (100 mL × 2), the organic phases were combined, washed with saturated brine, dried over anhydrous sodium sulfate, filtered, and spun to dryness to obtain the crude 52B (5.65 g, 103.22%), which was directly used in the next step.

Step 2: (3aR,5R,6R,6aR)-5-(hydroxymethyl)-2,2-dimethyl-6-(prop-1-yn-1-yl)tetrahydrofuro[2,3-d][1,3]dioxol-6-ol (52C)

Compound 52B (7.0 g, 15.06 mmol) was dissolved in THF (100 mL), tetrabutylammonium fluoride in tetrahydrofuran (19.58 mL, 19.58 mmol) was added, and the mixture was stirred at room temperature for 3 hours. After the reaction was completed, the mixture was spun to remove tetrahydrofuran, ethyl acetate (50 mL) was added, the mixture was washed with water and saturated brine, dried over anhydrous sodium sulfate, filtered, the filtrate was concentrated, and then the residue was separated and purified by silica gel column chromatography (petroleum ether: ethyl acetate (v/v) = 1 : 1) to obtain 52C (2.82 g, 82.76%).

Step 3: ((3aR,5R,6R,6aR)-6-hydroxy-2,2-dimethyl-6-(prop-1-yn-1-yl)tetrahydrofuro[2,3-d][1,3]dioxol-5-yl)methyl benzoate (52D)

52C (2.82 g, 12.46 mmol) was dissolved in dichloromethane (28 mL), under nitrogen protection, the reaction was cooled to 0°C, triethylamine (3.78 g, 37.38 mmol) was added, benzoyl chloride (2.28 g, 16.20 mmol) was added dropwise, and then the mixture was reacted at this temperature for 3 hours. After the reaction was completed, water was added to quench the reaction, the liquid separation was conducted, the aqueous phase was extracted with dichloromethane (20 mL) once, the organic phases were combined, washed with brine, dried, filtered, and the filtrate was concentrated, and then the residue was separated and purified by silica gel column chromatography (petroleum ether: ethyl acetate (v/v) = 5 : 1) to obtain 52D (3.27 g, 79.44%).

Step 4: ((2R,3S,4R,5S)-3,4,5-trihydroxy-3-(prop-1-yn-1-yl)tetrahydrofuran-2-yl)methyl benzoate (52E)

52D (3.27g, 9.90mmol) was added into a single-necked flask, trifluoroacetic acid (9 mL) and water (1mL) were added, and the mixture was stirred at room temperature for 3 hours. After the reaction, the mixture was spun to remove the majority of trifluoroacetic acid, ethyl acetate (20mL) was added, the mixture was washed with saturated aqueous sodium bicarbonate solution until the aqueous phase was basic, then dried over anhydrous sodium sulfate, filtered, the

filtrate was concentrated, and then the residue was separated and purified by silica gel column chromatography (petroleum ether: ethyl acetate (v/v) = 1 : 1) to obtain 52E (1.37 g, 47.67%).

Step 5: (2R,3R,4R,SR)-5-((benzoyloxy)methyl)-4-hydroxy-4-(prop-1-yn-1-yl)tetrahydrofuran-2,3-diyl diacetate (52F)

52E (1.37 g, 4.72 mmol) was dissolved in pyridine (13 mL), DMAP (0.058 g, 0.47 mmol) was added, acetic anhydride (0.96g, 9.44mmol) was added dropwise, and the mixture was reacted at room temperature for 3 hours. After the reaction was completed, the mixture was spun to remove most of the pyridine, ethyl acetate (20mL) was added, the mixture was washed with 1M hydrochloric acid, until the pyridine was completely removed, then the organic phase was washed with saturated brine, dried over anhydrous sodium sulfate, spun to dryness, filtered, the filtrate was concentrated, and then the residue was separated and purified by silica gel column chromatography (petroleum ether : ethyl acetate (v/v) = 2 : 1) to obtain 52F (1.2 g, 67.91%).

Step 6: ((2R,3R,4R,5R)-4-acetoxy-5-(4,6-dichloro-1H-pyrazolo[3,4-d]pyrimidin-1-yl)-3-hydroxy-3-(prop-1-yn-1-yl)tetrahydrofuran-2-yl)methyl benzoate (52G)

52F (1.2 g, 3.31 mmol) was added to a single-necked flask, heated to 90°C while stirring to a molten state, 4,6-dichloro-1H-pyrazolo[3,4-d]pyrimidine (0.94 g, 4.96 mmol) and 1 drop of stannum tetrachloride were added, the mixture was then warmed to 130°C, stirred for 30 minutes under reduced pressure with a water pump, after the reaction was completed, the mixture was cooled to room temperature, dichloromethane and a small amount of methanol were added, ultrasonic treatment was performed to dissolve the majority of the reactants, and the mixture was directly mixed with silica gel, and then separated and purified by silica gel column chromatography (petroleum ether: ethyl acetate (v/v) = 2 : 1) to obtain 52G (0.5 g, 29.89%).

Step 7: ((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(prop-1-yn-1-yl)tetrahydrofuran-2-yl)methyl benzoate (52H)

52G (0.5 g, 0.99 mmol) was dissolved in methanol (5 mL), triethylamine (0.30 g, 2.97 mmol) and octahydrocyclopenta[c]pyrrole hydrochloride (0.219 g, 1.48 mmol) were added, the mixture was stirred at room temperature for 3 hours, after the reaction was completed, the mixture was spun to dryness, and separated and purified by silica gel column chromatography (petroleum ether : ethyl acetate (v/v) = 2 : 1) to obtain 52H (0.35 g, 60.95%).

Step 8: (2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-2-(hydroxymethyl)-3-(prop-1-yn-1-yl)tetrahydrofuran-3,4-diol (52I)

52H (0.35 g, 0.6 mmol) was dissolved in methanol (5 mL), potassium carbonate (0.25 g, 1.80 mmol) was added, the mixture was stirred at room temperature for 3 hours, after the reaction was completed, ethyl acetate (10 mL) was added, the mixture was washed with water and saturated brine, dried over anhydrous sodium sulfate, spun to dryness, and separated and purified by silica gel column chromatography (petroleum ether : ethyl acetate (v/v) = 1 : 1) to obtain 52I (0.19 g, 72.98%).

Step 9: (((((2R,3S,4R,5R)-5-(6-chloro-4-((3aR,6aS)-hexahydrocyclopenta[c] pyrrol-2(1H)-yl)-1H-pyrazolo[3,4-d]pyrimidin-1-yl)-3,4-dihydroxy-3-(prop-1-yn-1-yl)tetrahydrofuran-2-yl)methoxy)(hydroxy)phosphoryl)methyl)phosphonic acid (Compound 52)

Compound 52I (0.19 g, 0.44 mmol) was dissolved in trimethyl phosphate (2 mL), the temperature was decreased to 0°C under an ice bath, methylenebis(phosphonic dichloride) (0.33 g, 1.32 mmol) was dissolved in trimethyl phosphate (2 mL), and the mixture was slowly added dropwise into the reaction solution, after the addition, the temperature was controlled at 0°C and the mixture was reacted for 2 hours, after the reaction was completed, water (1 mL) was added, and the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.2 min, to obtain the compound 52 (94 mg, 36.09%).

LCMS m/z = 592.1 $[M+H]^-$.

$^1H$ NMR (400 MHz, DMSO-d6) δ 8.30 (s, 1H), 5.98 (d, 1H), 4.82 (t, 1H), 4.28 (td, 1H), 4.13 - 4.02 (m, 2H), 4.02 - 3.93 (m, 1H), 3.91 - 3.81 (m, 1H), 3.61 (dd, 1H), 3.48 (dd, 1H), 2.93 - 2.85 (m, 1H), 2.80 - 2.69 (m, 1H), 2.25 - 2.00 (m,

2H), 1.93 - 1.70 (m, 6H), 1.67 - 1.47 (m, 3H).

**Example 53:**

(((((2R,3 S,4R,SR)-5-(6-chloro-4-((R/S)-2-(2-fluorophenyl)piperidin-1-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 53)

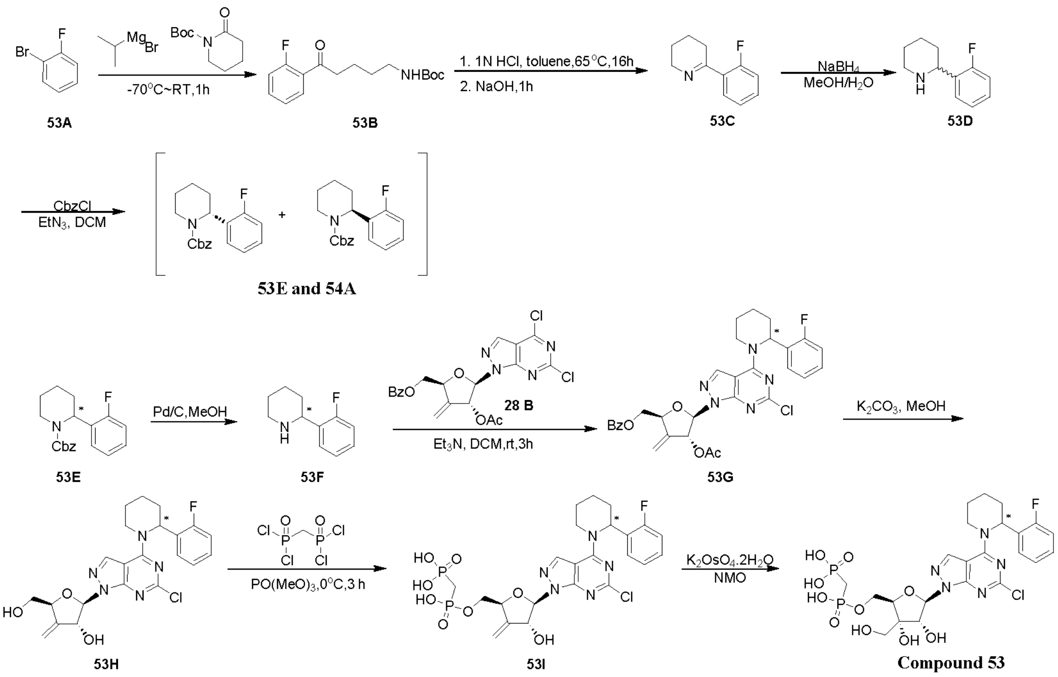


Step 1: tert-butyl (5-(2-fluorophenyl)-5-oxopentyl)carbamate (53B)

53A (10 g, 57.14 mmol) was dissolved in dry tetrahydrofuran (100 ml), the reaction system was subjected to nitrogen replacement three times, the reaction system was cooled to -78°C, isopropylmagnesium bromide (62.86 mmol) was slowly added dropwise, and then the mixture was warmed to 0°C and reacted for 1 h. The reaction system was cooled to -78°C, tert-butyl 2-oxopiperidine-1-carboxylate (62.86 mmol) dissolved in tetrahydrofuran was added dropwise, the mixture was then naturally warmed to room temperature, and reacted for 2 h, after the reaction was completed, saturated ammonium chloride solution (100 ml) was added to quench the reaction, the mixture was extracted with ethyl acetate (3 × 100 ml), the organic phases were combined, dried over anhydrous sodium sulfate, filtered and concentrated to obtain the crude compound 53B, which was directly used in the next step.
LC-MS (ESI): m/z = 196.2 [M-100+1].

Step 2: 6-(2-fluorophenyl)-2,3,4,5-tetrahydropyridine (53C)

The crude from the previous step was dissolved in toluene (80 ml), concentrated hydrochloric acid (10 ml) was added, the reaction system was placed in a 65 °C oil bath pan and stirred for 16 h, after the reaction was completed, the reaction system was naturally cooled to room temperature, 2 M aqueous sodium hydroxide solution was used to adjust the reaction system to pH = 14, the mixture was extracted with ethyl acetate (3 × 100 ml), the organic phases were combined, washed with saturated brine, dried over anhydrous sodium sulfate, filtered and concentrated to obtain the crude compound 53C, which was directly used in the next step.
LC-MS (ESI): m/z = 178.1 $[M+1]^+$.

Step 3: 2-(2-fluorophenyl)piperidine (53D)

The crude from the previous step was dissolved in a mixed solution of methanol/water = 4/1 (100 ml), the reaction system was cooled under an ice bath, sodium borohydride (4.76 g, 125.71 mmol) was added in portions, the

mixture was naturally warmed to room temperature, and reacted at room temperature for 1 h. After the reaction was completed, the reaction was quenched with 1 M aqueous hydrochloric acid solution at 0°C, extracted with ethyl acetate (100 ml × 3), dried over anhydrous sodium sulfate, concentrated and the resulting residue was purified by silica gel column chromatography (eluent: EA : PE = 0%-50%), to obtain the compound 53D (4.09 g, 40%).

LC-MS (ESI): m/z = 180.1 $[M+1]^+$.

Step 4: benzyl -2-(2-fluorophenyl)piperidine-1-carboxylate (53E and 54A)

53D (4.09 g, 22.86 mmol) was dissolved in dichloromethane (40 ml), triethylamine (6.93 g, 68.58 mmol) was added, the reaction system was placed in an ice bath and cooled, benzyl chloroformate (5.85 g, 34.29 mmol) was added dropwise, and the mixture was naturally warmed to room temperature and stirred at room temperature for 2 h. After the reaction was completed, water (40 ml) was added to quench the reaction, the reaction was extracted with dichloromethane (40 ml × 2), dried over anhydrous sodium sulfate, and concentrated, and the resulting residue was purified by silica gel column chromatography (eluent: EA : PE = 0%-20%), to obtain the compound 53E and 54A (6.80 g, 95%).

LC-MS (ESI): m/z = 314.1 [M+1].

Resolution of compounds 53E and 54A

Benzyl 2-(2-fluorophenyl)piperidine-1-carboxylate (compounds 53E and 54A) (5 g) was taken for resolution, and separated to obtain the compound 53E (retention time: 1.257s, 2.31 g, ee% = 100%), and compound 54A (retention time: 1.553 s, 2.34 g, ee% = 100%). Resolution conditions: instrument: Waters 150 MGM; column: DAICEL CHIRALPAK AD; mobile phase: A for $CO_2$ and B for EtOH (0.1%NH3•H2O); gradient: B 40%; flow rate: 120 mL /min; back pressure: 100 bar; column temperature: 35°C; wavelength: 220 nm; cycle: 13 min;

Step 5: (R/S)-2-(2-fluorophenyl)piperidine (53F)

Compound 53E (2 g, 6.48 mmol) was dissolved in methanol (20 ml), the reaction system was subjected to nitrogen replacement three times, Pd/C (20 mg) was added, the reaction system was subjected to hydrogen replacement three times, the mixture was reacted at room temperature overnight, after the reaction was completed, the mixture was filtered, the filter cake was washed with methanol, and the filtrate was spun to dryness to obtain the compound 53F (1.12 g, 98%).

LC-MS (ESI): m/z = 180.2 $[M+1]^+$.

Step 6: ((2S,4R,5R)-4-acetoxy-5-(6-chloro-4-((R/S)-2-(2-fluorophenyl) piperidin-1-yl)-1H-pyrazolo[3,4-d]pyrimidin-1-yl)-3-methylenetetrahydrofuran-2-yl)methyl benzoate (53G)

Compound 53F (1.12 g, 6.26 mmol) was dissolved in dichloromethane (10 ml), triethylamine (1.90 g, 18.77 mmol) and compound 28B (2.42 g, 5.22 mmol) were added, after the addition, the mixture was reacted at room temperature for 6 hours, after the reaction was completed, the mixture was concentrated and the resulting residue was purified by silica gel column chromatography (eluent: EA : PE = 0%-30%) to obtain the target compound 53G (1.83 g, 58%).

LC-MS (ESI): m/z = 606.2 $[M+1]^+$.

Step 7: (2R,3R,5S)-2-(6-chloro-4-((R/S)-2-(2-fluorophenyl)piperidin-1-yl)-1H -pyrazolo[3,4-d]pyrimidin-1-yl)-5-(hydroxymethyl)-4-methylenetetrahydrofuran-3-ol (53H)

Compound 53G (1.83 g, 3.02 mmol) was dissolved in methanol (20 ml), potassium carbonate (1.25 g, 9.06 mmol) was added, after the addition, the mixture was reacted at room temperature for 4 hours, water (20 ml) was added to quench the reaction, the mixture was extracted with ethyl acetate (30 ml × 3), dried over anhydrous sodium sulfate, concentrated and the resulting residue was purified by silica gel column chromatography (eluent: MeOH : EA = 0%-10%), to obtain the product 53H (1.36 g, 98%).

LC-MS (ESI): m/z = 460.1 $[M+1]^+$.

Step 8: (((((2S,4R,SR)-5-(6-chloro-4-((R/S)-2-(2-fluorophenyl)piperidin-1-yl)-1H-pyrazolo[3,4-d]pyrimidin-1-yl)-4-hydroxy-3-methylenetetrahydrofuran-2-yl)methoxy)(hydroxy)phosphoryl)methyl)phosphonic acid (53I)

Compound 53H (200 mg, 0.43 mmol) was dissolved in trimethyl phosphate (2 ml), the temperature was decreased to 0°C under an ice bath, methylenebis(phosphonic dichloride) (435 mg, 1.74 mmol) was dissolved in trimethyl phosphate (2 ml), and the mixture was slowly added dropwise into the reaction solution, after the addition, the mixture

was reacted at 0°C for 3 hours, upon completion monitored by LCMS, water (2 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 53I (156 mg, 59%).

LC-MS (ESI): m/z = 618.2 $[M+1]^+$.

Step 9: (((((2R,3S,4R,5R)-5-(6-chloro-4-((R/S)-2-(2-fluorophenyl)piperidin-1-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 53)

Compound 53I (156 mg, 0.25 mmol) and N-methylmorpholine oxide (103 mg, 0.76 mmol) were dissolved in acetone (3 ml) and water (1 mL), potassium osmate(VI) dihydrate (10 mg) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 42 (60 mg, 36%).

LC-MS (ESI): m/z = 652.2 $[M+1]^+$.

$^1H$ NMR (400 MHz, $D_2O$) δ 7.99 (s, 1H), 7.09 (t, 4H), 6.13 (d, 1H), 5.90 (s, 1H), 4.88 (d, 1H), 4.51 (s, 1H), 4.28 (s, 1H), 4.09 (s, 2H), 3.82 (q, 2H), 3.49 (d, 1H), 2.22 (dd, 4H), 1.76 (d, 2H), 1.53 (d, , 2H).

$^{19}F$ NMR (377 MHz, $D_2O$) δ -114.54.

Example 54:

(((((2R,3 S,4R,SR)-5-(6-chloro-4-((S/R)-2-(2-fluorophenyl)piperidin-1-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl) phosphonic acid (Compound 54)

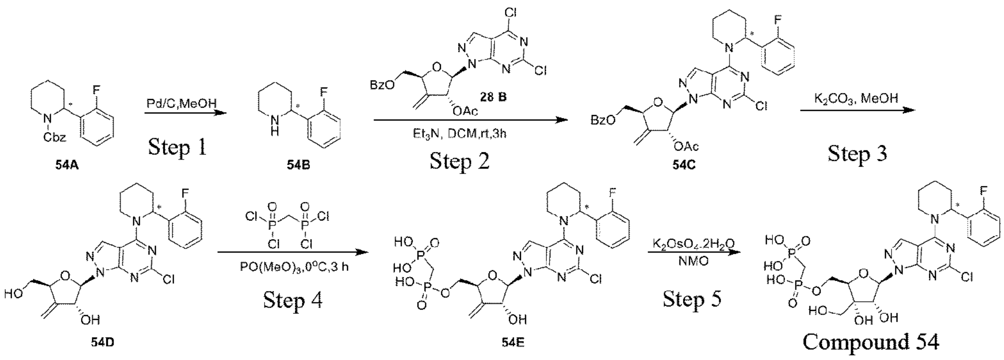


Step 1: (S/R)-2-(2-fluorophenyl)piperidine (54B)

Compound 54A (2 g, 6.48 mmol) was dissolved in methanol (20 ml), the reaction system was subjected to nitrogen replacement three times, Pd/C (20 mg) was added, the reaction system was subjected to hydrogen replacement three times, the mixture was reacted at room temperature overnight, after the reaction was completed, the mixture was filtered, the filter cake was washed with methanol, and the filtrate was spun to dryness to obtain the compound 54B (1.11 g, 98%).

LC-MS (ESI): m/z = 180.2 $[M+1]^+$.

Step 2: ((2S,4R,5R)-4-acetoxy-5-(6-chloro-4-((S/R)-2-(2-fluorophenyl)piperidin-1-yl)-1H-pyrazolo[3,4-d]pyrimidin-1-yl)-3-methylenetetrahydrofuran-2-yl)methyl benzoate (54C)

Compound 54B (1.11 g, 6.26 mmol) was dissolved in dichloromethane (10 ml), triethylamine (1.90 g, 18.77 mmol) and compound 28B (2.42 g, 5.22 mmol) were added, after the addition, the mixture was reacted at room temperature for 6 hours, after the reaction was completed, the mixture was concentrated and the resulting residue was purified by silica gel column chromatography (eluent: EA : PE = 0%-30%) to obtain the target compound 54C (1.81 g, 48%).
LC-MS (ESI): m/z = 606.2 $[M+1]^+$.

Step 3: (2R,3R,SS)-2-(6-chloro-4-((S/R)-2-(2-fluorophenyl)piperidin-1-yl)-1H-pyrazolo[3,4-d]pyrimidin-1-yl)-5-(hydroxymethyl)-4-methylenetetrahydrofuran-3-ol (54D)

Compound 54C (1.81 g, 2.99 mmol) was dissolved in methanol (20 ml), potassium carbonate (1.25 g, 9.06 mmol) was added, after the addition, the mixture was reacted at room temperature for 4 hours, water (20 ml) was added to quench the reaction, the mixture was extracted with ethyl acetate (30 ml × 3), dried over anhydrous sodium sulfate, concentrated and the resulting residue was purified by silica gel column chromatography (eluent: MeOH : EA = 0%-10%), to obtain the product 54D (1.35 g, 98%).
LC-MS (ESI): m/z = 460.1 $[M+1]^+$.

Step 4: (((((2S,4R,SR)-5-(6-chloro-4-((S/R)-2-(2-fluorophenyl)piperidin-1-yl)-1H-pyrazolo[3,4-d]pyrimidin-1-yl)-4-hydroxy-3-methylenetetrahydrofuran-2-yl)methoxy)(hydroxy)phosphoryl)methyl)phosphonic acid (54E)

Compound 54D (200 mg, 0.43 mmol) was dissolved in trimethyl phosphate (2 ml), the temperature was decreased to 0°C under an ice bath, methylenebis(phosphonic dichloride) (435 mg, 1.74 mmol) was dissolved in trimethyl phosphate (2 ml), and the mixture was slowly added dropwise into the reaction solution, after the addition, the mixture was reacted at 0°C for 3 hours, upon completion monitored by LCMS, water (2 ml) was added, after the addition, the mixture was stirred for 1 hour, and after the reaction was completed, the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.0 min, to obtain the compound 54E (155 mg, 59%).
LC-MS (ESI): m/z = 618.2$[M+1]^+$.

Step 5: (((((2R,3S,4R,5R)-5-(6-chloro-4-((S/R)-2-(2-fluorophenyl)piperidin-1-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl) phosphonic acid (Compound 54)

Compound 54E (156 mg, 0.25 mmol) and N-methylmorpholine oxide (103 mg, 0.76 mmol) were dissolved in acetone (3 ml) and water (1 mL), potassium osmate(VI) dihydrate (10 mg) was added at room temperature, after the addition, the mixture was reacted at room temperature for 16 hours, after the reaction was completed, sodium sulfite was added to quench the reaction, and the reaction solution was concentrated and then subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 54 (57 mg, 35%).
LC-MS (ESI): m/z = 652.2 $[M+1]^+$.
$^1H$ NMR (400 MHz, $D_2O$) δ 8.09 (s, 1H), 7.31 (s, 1H), 7.19 (dd, 2H), 7.10 (t, 1H), 6.16 (d, 1H), 5.97 (s, 1H), 4.95 (d, 1H), 4.58 (s, 1H), 4.30 (t, 1H), 4.15 - 4.00 (m, 2H), 3.85 (q, 2H), 3.62 (s, 1H), 2.25 (s, 2H), 2.19 (d, 1H), 2.15 (s, 1H), 1.90 (s, 1H), 1.78 (s, 1H), 1.66 (s, 2H).
$^{19}F$ NMR (376 MHz, $D_2O$) δ -114.64.

**Example 55:**

(((((2R,3S,4R,5R)-5-(6-chloro-4-(4,4-dimethyl-1,4-azasilinan-1-yl)-1H-pyrazolo[3,4-d]pyrimidin-1-yl)-3,4-dihydroxy-3-(hydroxymethyl)tetrahydrofuran-2-yl)methoxy)(hydroxy)phosphoryl)methyl)phosphonic acid (Compound 55)

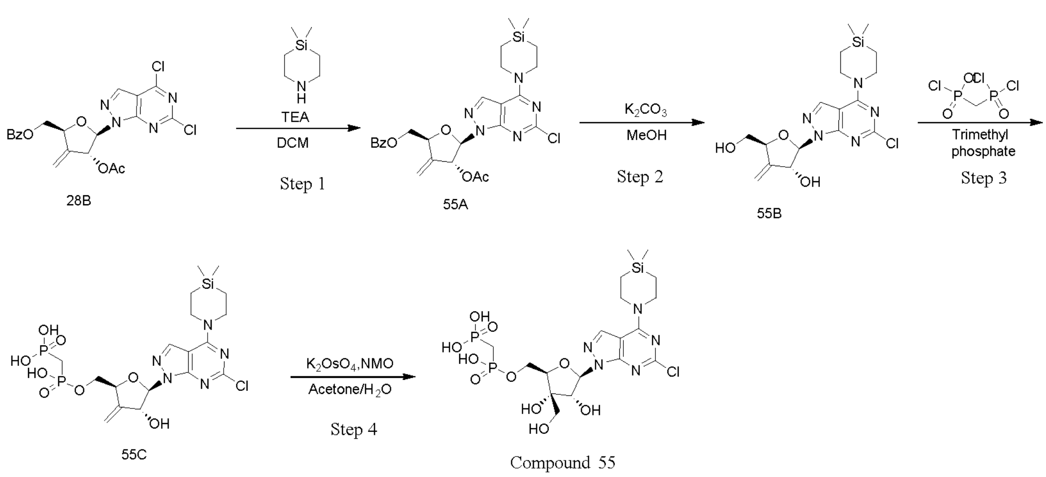

Compound 55

Step 1: ((2S,4R,5R)-4-acetoxy-5-(6-chloro-4-(4,4-dimethyl-1,4-azasilinan-1-yl)-1H-pyrazolo[3,4-d]pyrimidin-1-yl)-3-methylenetetrahydrofuran-2-yl)methylbenzoate (55A)

Compound 28B (1.5 g, 3.24 mmol) was dissolved in dichloromethane (15 ml), 4,4-dimethyl-[1,4]silapiperidine hydrochloride (0.64, 3.86 mmol) was then added, the temperature was decreased to 0°C, triethylamine (0.99 g, 9.74 mmol) was then added, after the addition, the mixture was reacted at room temperature for 1 hour, after the reaction was completed, the reaction solution was directly concentrated, and after concentration, the residue was separated and purified by silica gel column chromatography (petroleum ether : ethyl acetate (v/v) = 4 : 1) to obtain the compound 55A (0.7 g, 38.8%).

LC-MS (ESI): m/z = 556.1 $[M+H]^+$.

Step 2: (2R,3R,5S)-2-(6-chloro-4-(4,4-dimethyl-1,4-azasilinan-1-yl)-1H-pyrazolo[3,4-d]pyrimidin-1-yl)-5-(hydroxymethyl)-4-methylenetetrahydrofuran-3-ol (55B)

Compound 55A (0.7 g, 1.26 mmol) was dissolved in methanol (10 ml), potassium carbonate (3.78 g, 2.61 mmol) was added, after the addition, the mixture was stirred at room temperature for 3 hour, after the reaction was completed, the reaction solution was concentrated, water was added into the concentrated residue, the mixture was extracted with dichloromethane (50 ml × 2) twice, the organic phases were combined, washed with saturated brine, dried over anhydrous sodium sulfate, and filtered, and the filtrate was concentrated, and then the residue was separated and purified by silica gel column chromatography (petroleum ether : ethyl acetate (v/v) = 3 : 1) to obtain the title compound 55B (0.46 g, 89.0%).

LC-MS (ESI): m/z = 410.1 $[M+H]^+$.

Step 3: (((((2S,4R,5R)-5-(6-chloro-4-(4,4-dimethyl-1,4-azasilinan-1-yl)-1H-pyrazolo [3,4-d]pyrimidin-1-yl)-4-hydroxy-3-methylenetetrahydrofuran-2-yl)methoxy)(hydr-oxy)phosphoryl)methyl)phosphonic acid (55C)

Compound 55B (0.46 g, 1.12 mmol) was dissolved in trimethyl phosphate (5 ml), the temperature was decreased to 0°C under an ice bath, methylenebis(phosphonic dichloride) (0.84 g, 3.36 mmol) was dissolved in trimethyl phosphate (5 ml), and the mixture was slowly added dropwise into the reaction solution, after the addition, the temperature was controlled at 0°C and the mixture was reacted for 2 hours, after the reaction was completed, water (5 ml) was added, and the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.9 min, to obtain the compound 55C (0.31 g, 48.7%).

LC-MS (ESI): m/z = 568.1 $[M+H]^+$.

Step 4: (((((2R,3S,4R,5R)-5-(6-chloro-4-(4,4-dimethyl-1,4-azasilinan-1-yl)-1H-pyrazolo[3,4-d]pyrimidin-1-yl)-3,4-dihydroxy-3-(hydroxymethyl) tetrahydrofuran-2-yl)methoxy)(hydroxy)phosphoryl)methyl)phosphonic acid (Compound 55)

Compound 55C (0.31 g, 0.2 mmol) was dissolved in a mixed solvent of acetone (10 ml) and water (3 ml), N-methylmorpholine oxide (0.19 g, 1.65 mmol) was added, a catalytic amount of potassium osmate(VI) dihydrate (6 mg) was added, after the addition, the mixture was reacted at room temperature for 15 hours, upon completion monitored by LCMS, the mixture was concentrated to remove acetone solvent and the reaction system was directly subjected to preparative HPLC with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 6.5 min, to obtain the compound 55 (170 mg, 51.3%).

LC-MS (ESI): m/z = 600.1$[M-H]^-$.

$^1$H NMR (400 MHz, $D_2O$): 8.13 (s, 1H), 6.11 (d, 1H), 4.93 (d, 1H), 4.29-4.27 (m, 1H), 4.10-4.07 (m, 2H), 3.94-3.78 (m, 6H), 2.31-2.21 (m, 2H), 0.82 (d, 4H), 0.00 (s, 6H).

**Example 56:**

2-(((2R,3S,4R,5R)-5-(6-chloro-4-((3aR,6aS)-hexahydrocyclopenta[c]pyrrol-2(1H)-yl)-1H-pyrazolo[3,4-b]pyridin-1-yl)-3,4-dihydroxy-3-(hydroxymethyl) tetrahydrofuran-2-yl)methoxy)-2-(4-(2-oxotetrahydropyrimidin-1(2H)-yl)benzyl) malonic acid (Compound 56)

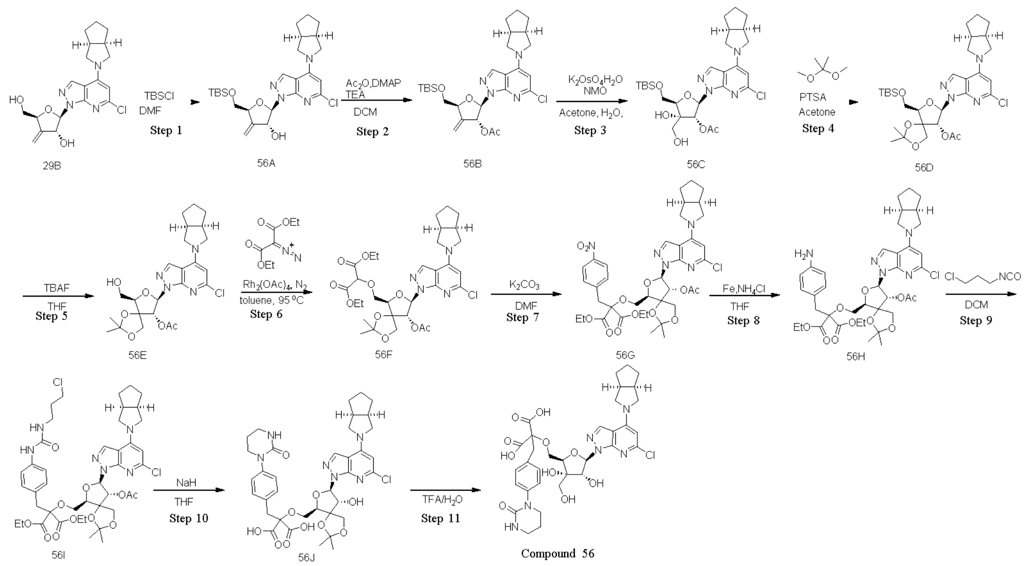


Step 1: (2R,3R,5S)-5-(((tert-butyldimethylsilyl)oxy)methyl)-2-(6-chloro-4-((3aR,6aS)-hexahydro cyclopenta[c]pyrrol-2(1H)-yl)-1H-pyrazolo[3,4-b]pyridin-1-yl)-4-methylenetetrahydrofuran-3-ol (56A)

Compound 29B (5.0 g, 12.79 mmol) was dissolved in N,N-dimethylformamide (50 ml), imidazole (4.35 g, 63.96 mmol) was added, after the addition, under nitrogen protection, the temperature was decreased by 0-5°C, tertbutyldimethylsilyl chloride (2.12 g, 14.07 mmol) was added in portions, after the addition, the mixture was naturally warmed to room temperature and reacted for 2 hours, after the reaction was completed, water was added to quench the reaction, the mixture was extracted with ethyl acetate (100 ml × 2), the organic layers were combined, washed with saturated brine, dried and concentrated, and the residue was separated and purified by column chromatography (petroleum ether: ethyl acetate (v/v) = 3 : 1) to obtain the compound 56A (5.3 g, 82%).

Step 2: (2R,3R,5S)-5-(((tert-butyldimethylsilyl)oxy)methyl)-2-(6-chloro-4-((3aR,6aS)-hexahydrocyc lopenta[c]pyrrol-2(1H)-yl)-1H-pyrazolo[3,4-b]pyridin-1-yl)-4-methylenetetrahydrofuran-3-yl acetate (56B)

Compound 56A (5.3 g, 10.49 mmol) was dissolved in dichloromethane (50 ml), triethylamine (3.18 g, 31.47 mmol) and a catalytic amount of 4-dimethylaminopyridine were added, after the addition, under nitrogen protection, the temperature was decreased by 0-5°C, acetic anhydride (2.14 g, 20.98 mmol) was added, after the addition, the mixture was naturally warmed to room temperature and reacted for 2 hours, after the reaction was completed, water was added to quench the reaction, the mixture was extracted with dichloromethane (100 ml × 2), the organic layers were combined, washed with saturated brine, dried and concentrated, and then the residue was separated and purified by column chromatography (petroleum ether : ethyl acetate (v/v) = 5 : 1) to obtain the compound 56B (5.6 g, 97%).

Step 3: (2R,3R,4R,5R)-5-(((tert-butyldimethylsilyl)oxy)methyl)-2-(6-chloro-4-((3aR,6aS)-hexahydro-cyclopenta[c]pyrrol-2(1H)-yl)-1H-pyrazolo[3,4-b]pyridin-1-yl)-4-hydroxy-4-(hydroxymethyl)tetrah ydrofuran-3-yl acetate (56C)

Compound 56B (5.6 g, 10.23 mmol) was dissolved in a mixed solvent of acetone (120 ml) and water (30 ml), N-methylmorpholine oxide (3.53 g, 30.69 mmol) was added, a catalytic amount of potassium osmate(VI) dihydrate (110 mg) was added, after the addition, the mixture was reacted at room temperature for 15 hours, after the reaction was completed, the mixture was concentrated to remove acetone, water (50 ml) was added, the mixture was extracted with ethyl acetate (100 ml × 2) twice, the organic phases were combined, washed with saturated brine and saturated aqueous sodium bicarbonate solution, dried over anhydrous sodium sulfate, filtered, the filtrate was concentrated, and then the residue was separated and purified by column chromatography (petroleum ether : ethyl acetate (v/v) = 3 : 1) to obtain the compound 56C (4.6 g, 77.3%).

LC-MS (ESI): m/z = 581.2 $[M+H]^+$.

Step 4: (6R,8R,9R)-6-(((tert-butyldimethylsilyl)oxy)methyl)-8-(6-chloro-4-((3aR,6aS)-hexahy-drocyclopenta[c]pyrrol-2(1H)-yl)-1H-pyrazolo[3,4-b]pyridin-1-yl)-2,2-dimethyl-1,3,7-trioxaspiro[4.4]nonan-9-yl acetate (56D)

Compound 56C (4.6 g, 7.91 mmol) was dissolved in acetone (50 ml), a catalytic amount of p-toluenesulfonic acid was added, 2,2-dimethoxypropane (8.24 g, 79.1mmol) was then added, after the addition, the mixture was reacted at room temperature for 24 hours, after the reaction was completed, the reaction was concentrated to remove acetone, water (50 ml) was added, the mixture was extracted with ethyl acetate (100 ml × 2), washed with saturated brine, dried over anhydrous sodium sulfate, filtered, the filtrate was concentrated, and then the residue was separated and purified by column chromatography (petroleum ether : ethyl acetate (v/v) = 6 : 1) to obtain the compound 56D (4.0 g, 81%).

Step 5: (6R,8R,9R)-8-(6-chloro-4-((3aR,6aS)-hexahydrocyclopenta[c]pyrrol-2(1H)-yl)-1H-pyrazo-lo[3,4-b]pyridin-1-yl)-6-(hydroxymethyl)-2,2-dimethyl-1,3,7-trioxaspiro[4.4]nonan-9-yl acetate (56E)

Compound 56D (4.0 g, 6.44 mmol) was dissolved in tetrahydrofuran (40 ml), tetrabutylammonium fluoride (7.08 ml, 7.08 mmol, 1.0 mol/L tetrahydrofuran solution) was added, after the addition, the mixture was reacted at room temperature for 2 hours, and after the reaction was completed, the reaction solution was directly concentrated. After concentration, the residue was separated and purified by column chromatography (petroleum ether : ethyl acetate (v/v) = 1 : 1) to obtain the compound 56E (3.1 g, 95%).

LC-MS (ESI): m/z = 507.1 $[M+H]^+$.

Step 6: Diethyl 2-(((6R,8R,9R)-9-acetoxy-8-(6-chloro-4-((3aR,6aS)-hexahydrocyclopenta [c]pyrrol-2(1H)-yl)-1H-pyrazo-lo[3,4-b]pyridin-1-yl)-2,2-dimethyl-1,3,7-trioxaspiro[4.4]nonan-6-yl)methoxy)malonate (56F)

Compound 56E (3.1 g, 6.11 mmol) was dissolved in toluene (30 ml), diethyl diazomalonate (2.27 g, 12.2 mmol) and rhodium(II) acetate dimmer (0.14 g, 0.32 mmol) were added, after the addition, under nitrogen protection, the mixture was warmed to 100°C and reacted for 1 hour, after the reaction was completed, the reaction solution was directly concentrated, and after concentration, the residue was separated and purified by column chromatography (petroleum ether : ethyl acetate (v/v) = 3 : 1) to obtain the compound 56F (3.0 g, 74%).

Step 7: Diethyl 2-(((6R,8R,9R)-9-acetoxy-8-(6-chloro-4-((3aR,6aS)-hexahydrocyclopenta [c]pyrrol-2(1H)-yl)-1H-pyrazo-lo[3,4-b]pyridin-1-yl)-2,2-dimethyl-1,3,7-trioxaspiro[4.4]nonan-6-yl)methoxy)-2-(4-nitrobenzyl)malonate (56G)

Compound 56F (3.0 g, 4.51 mmol) was dissolved in dry N,N-dimethylformamide (30 ml), the reaction system was subjected to nitrogen replacement three times, potassium carbonate (1.87 g, 13.53 mmol) was added, after the

addition, the mixture was stirred at room temperature for 30 min, p-nitrobenzyl bromide (1.95 g, 9.02 mmol) was then added, after the addition, the mixture was stirred at room temperature for 16 hours, after the reaction was completed, saturated ammonium chloride was added to quench the reaction, the mixture was extracted with ethyl acetate (100 ml × 2), the organic layers were combined, washed with saturated brine, dried over anhydrous sodium sulfate, filtered, and concentrated, and then the residue was separated and purified by column chromatography (petroleum ether : ethyl acetate (v/v) = 3 : 1) to obtain the compound 56G (2.6 g, 72%).

Step 8: Diethyl 2-(((6R,8R,9R)-9-acetoxy-8-(6-chloro-4-((3aR,6aS)-hexahydrocyclopenta [c]pyrrol-2(1H)-yl)-1H-pyrazolo[3,4-b]pyridin-1-yl)-2,2-dimethyl-1,3,7-trioxaspiro[4.4]nonan-6-yl)methoxy)-2-(4-aminobenzyl)malonate (56H)

Compound 56G (2.6 g, 3.25 mmol) was dissolved in a mixed solvent of ethanol (50 mL) and water (10 ml), and iron powder (900 mg, 16.15 mmol) and ammonium chloride (171 mg, 3.25 mmol) were added. After the addition, the mixture was warmed to 50°C and reacted for 4 hours, after the reaction was completed, the mixture was filtered, the filtrate was concentrated, and the residue was separated and purified by column chromatography (ethyl acetate : petroleum ether (v/v) = 1 : 1) to obtain the compound 56H (2.1 g, 84%).

LC-MS (ESI): m/z = 770.3 $[M+H]^+$.

Step 9: Diethyl 2-(((6R,8R,9R)-9-acetoxy-8-(6-chloro-4-((3aR,6aS)-hexahydrocyclopenta [c]pyrrol-2(1H)-yl)-1H-pyrazolo[3,4-b]pyridin-1-yl)-2,2-dimethyl-1,3,7-trioxaspiro[4.4]nonan-6-yl)methoxy)-2-(4-(3-(3-chloropropyl)ureido)benzyl)malonate (56I)

Compound 56H (2.1 g, 2.73 mmol) was dissolved in dichloromethane (20 ml), 3-chloropropyl isocyanate (1.31 g, 10.95 mmol) was added, after the addition, the temperature was kept at 25°C and the mixture was reacted for 24 hours, after the reaction was completed, the reaction solution was directly concentrated, and after concentration, the residue was separated and purified by column chromatography (ethyl acetate : petroleum ether (v/v) = 1 : 1) to obtain the compound 56I (1.9 g, 78.2%).

Step 10: 2-(((6R,8R,9R)-8-(6-chloro-4-((3aR,6aS)-hexahydrocyclopenta[c] pyrrol-2(1H)-yl)-1H-pyrazolo[3,4-b]pyridin-1-yl)-9-hydroxy-2,2-dimethyl-1,3,7-trioxaspiro[4.4]nonan-6-yl)methoxy)-2-(4-(2-oxotetrahydropyrimidin-1 (2H)-yl)benzyl)malonic acid (56J)

Compound 56I (0.12 g, 0.13 mmol) was dissolved in ultra-dried tetrahydrofuran (3 ml), the reaction system was subjected to nitrogen replacement three times, the temperature was cooled to 0°C, sodium hydride (10 mg, 60% content, 0.26 mmol) was added, after the addition, the temperature was warmed to 25°C and the mixture was reacted for 3 hours, after the reaction was completed, the mixture was cooled to 0°C, water (1 ml) was added, after the addition, the mixture was stirred at room temperature for 4 hours, and after the reaction was completed, the reaction system was directly subjected to preparative HPLC for purification with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 15.5 min, to obtain the compound 56J (10 mg, 10%).

LC-MS (ESI): m/z = 755.2 $[M+H]^+$.

Step 11: 2-(((2R,3R,4R,5R)-5-(6-chloro-4-(hexahydrocyclopenta[c]pyrrol-2(1H)-yl)-1H-pyrazolo[3,4-b]pyridin-1-yl)-3,4-dihydroxy-3-(hydroxymethyl) tetrahydrofuran-2-yl)methoxy)-2-(4-(2-oxotetrahydropyrimidin-1(2H)-yl)benzyl)malonic acid (Compound 56)

Compound 56J (10 mg, 0.013 mmol) was dissolved in a mixed solvent of trifluoroacetic acid (0.5 ml) and water (0.5 ml), the mixture was stirred at room temperature for 15 hours, and after the reaction was completed, the reaction system was directly subjected to preparative HPLC for purification with preparative HPLC separation methods: 1. Instrument: waters 2767 (preparative liquid phase chromatographic instrument); SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 8.6 min, to obtain the compound 56 (5 mg, 53%).

LC-MS (ESI): m/z = 713.2$[M-H]^-$.

$^1$H NMR (400 MHz, $CD_3OD$): 8.24 (s, 1H), 7.32 (d, 2H), 7.16 (d, 2H), 6.22 (s, 1H), 6.18 (d, 1H), 4.21-4.12 (m,

4H), 4.04-4.01 (m, 4H), 3.87-3.77 (m, 4H), 3.71-3.68 (m, 2H), 3.37-3.35 (m, 2H), 2.91-2.91 (m, 2H), 2.06-1.60 (m, 8H).

**Example 57:**

2-(((2R,3 S/R,4R,SR)-5-(4-amino-6-chloro-1H-pyrazolo[3,4-d]pyrimidin-1-yl)-3,4-dihydroxy-3-(hydroxymethyl)tetrahydrofuran-2-yl)methoxy)-2-(4-(2-oxotetrahydrop-yrimidin-1(2H)-yl)benzyl)malonic acid (Compound 57)

2-(((2R,3R/S,4R,5R)-5-(4-amino-6-chloro-1H-pyrazolo[3,4-d]pyrimidin-1-yl)-3,4-dihydroxy-3-(hydroxymethyl)tetrahydrofuran-2-yl)methoxy)-2-(4-(2-oxotetrahydrop-yrimidin-1(2H)-yl)benzyl)malonic acid (Compound 58)

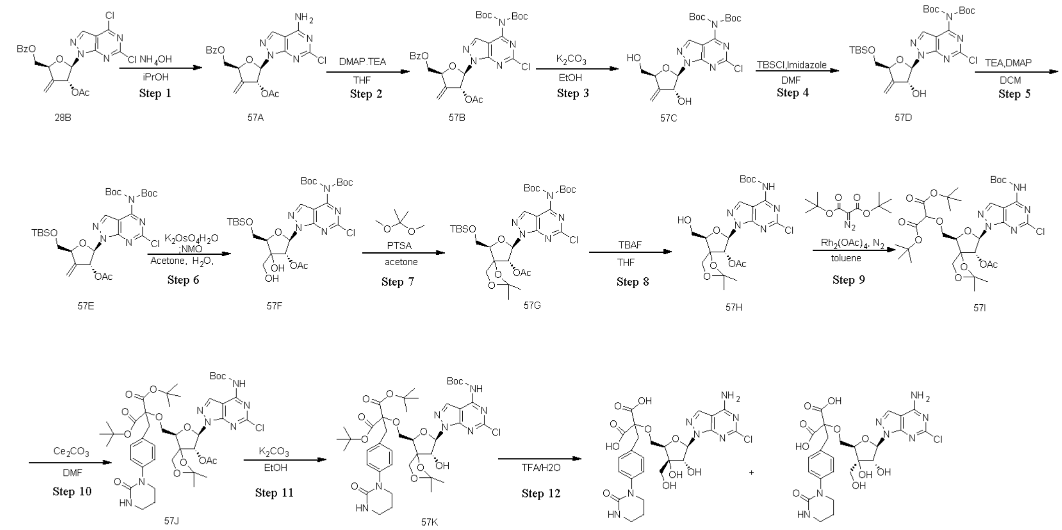

Compound 57 and Compound 58

Step 1: ((2S,4R,5R)-4-acetoxy-5-(4-amino-6-chloro-1H-pyrazolo[3,4-d]pyrimidin-1-yl)-3-methylenetetrahydrofuran-2-yl)methyl benzoate (57A)

Compound 28B (18.0 g, 38.85 mmol) was dissolved in a mixed solvent of dichloromethane (100 ml) and isopropanol (100 ml), aqueous ammonia (14.60 g, 116.55 mmol, 28%) was added at room temperature, after the addition, the mixture was reacted at room temperature for 16 hours, after the reaction was completed, the mixture was concentrated to remove dichloromethane and then water was added, then the mixture was extracted with ethyl acetate (150 ml × 2), the organic phases were combined, washed with saturated brine, dried over anhydrous sodium sulfate, filtered, the filtrate was concentrated, and then the residue was separated and purified by silica gel column chromatography (petroleum ether : ethyl acetate (v/v) = 4 : 1) to obtain the title compound 57A (15.1 g, 87.5%).
LC-MS (ESI): m/z = 444.1 $[M+H]^+$.

Step 2: ((2S,4R,5R)-4-acetoxy-5-(4-(bis(tert-butoxycarbonyl)amino)-6-chloro-1H-pyrazolo[3,4-d]pyrimidin-1-yl)-3-methylenetetrahydrofuran-2-yl)methyl-benzoate (57B)

Compound 57A (15.0 g, 33.80 mmol) was dissolved in tetrahydrofuran (150 ml), and triethylamine (10.26 g, 101.43 mmol), 4-dimethylaminopyridine (0.41 g, 3.38 mmol), and di-tert-butyl dicarbonate (22.13 g, 101.43 mmol) were successively added, after the addition, the mixture was reacted at room temperature for 5 hours, after the reaction was completed, the mixture was directly concentrated, and after concentration, the residue was separated and purified by silica gel column chromatography (petroleum ether : ethyl acetate (v/v) = 5 : 1) to obtain the title compound 57B (19.2 g, 88.2%).

Step 3: Tert-butyl (tert-butoxycarbonyl)(6-chloro-1-((2R,3R,5S)-3-hydroxy-5-(hydroxyl-methyl)-4-methylenetetrahydrofuran-2-yl)-1H-pyrazolo[3,4-d]pyrimidin-4-yl)carbam-ate (57C)

Compound 57B (19.2 g, 29.81 mmol) was dissolved in a mixed solvent of dichloromethane (100 ml) and methanol (190 ml), solid potassium carbonate (12.36 g, 89.43 mmol) was added, an equivalent amount of water was

added, after the addition, the mixture was reacted at room temperature for 1 hour, after the reaction was completed, water was added, then the mixture was extracted with dichloromethane (150 ml × 2), the organic phases were combined, washed with saturated brine, dried over anhydrous sodium sulfate, filtered, the filtrate was concentrated, and then the residue was separated and purified by silica gel column chromatography (petroleum ether : ethyl acetate (v/v) = 2 : 1) to obtain the title compound 57C (3.8 g, 32%).

LC-MS (ESI): m/z = 498.1 $[M+H]^+$.

Step 4: tert-butyl (tert-butoxycarbonyl)(1-((2R,3R,SS)-5-(((tertbutyldimethylsilyl)oxy)-methyl)-3-hydroxy-4-methylenetetrahydrofuran-2-yl)-6-chloro-1H-pyrazolo[3,4-d]pyrimidin-4-yl)carbamate (57D)

Compound 57C (3.8 g, 9.55 mmol) was dissolved in N,N-dimethylformamide (38 ml), imidazole (3.25 g, 47.75 mmol) was added, after the addition, under nitrogen protection, the temperature was decreased by 0-5°C, tertbutyldimethylsilyl chloride (2.59 g, 17.19 mmol) was added in portions, after the addition, the mixture was reacted at 0-5°C for 1 hour, after the reaction was completed, water was added to quench the reaction, the mixture was extracted with ethyl acetate (100 ml × 2), the organic layers were combined, washed with saturated brine, dried and concentrated to obtain the compound 57D (crude, 4.8 g, 98%), which was directly used in the next reaction without purification.

Step 5: (2R,3R,5S)-2-(4-(bis(tert-butoxycarbonyl)amino)-6-chloro-1H-pyrazolo[3,4-d]pyrimidin-1-yl)-5-(((tert-butyldimethylsilyl)oxy)methyl)-4-methylenetetrahydro-furan-3-yl acetate (57E)

Compound 57D (4.8 g, 9.37 mmol) was dissolved in dichloromethane (50 ml), triethylamine (2.84 g, 28.14 mmol) and a catalytic amount of 4-dimethylaminopyridine were added, after the addition, under nitrogen protection, the temperature was decreased by 0-5°C, acetic anhydride (1.91 g, 18.79 mmol) was added, after the addition, the mixture was naturally warmed to room temperature and reacted for 2 hours, after the reaction was completed, water was added to quench the reaction, the mixture was extracted with dichloromethane (100 ml × 2), the organic layers were combined, washed with saturated brine, dried and concentrated, and then the residue was separated and purified by column chromatography (petroleum ether: ethyl acetate (v/v) = 5 : 1) to obtain the compound 57E (4.2 g, 80.9%).

Step 6: (2R,3R,5R)-2-(4-(bis(tert-butoxycarbonyl)amino)-6-chloro-1H-pyrazolo[3,4-d]pyrimidin-1-yl)-5-(((tert-butyldimethylsilyl)oxy)methyl)-4-hydroxy-4-(hydroxyl-methyl)tetrahydrofuran-3-yl acetate (57F)

Compound 57E (4.2 g, 7.58 mmol) was dissolved in a mixed solvent of acetone (80 ml) and water (20 ml), N-methylmorpholine oxide (3.07 g, 22.74 mmol) was added, a catalytic amount of potassium osmate(VI) dihydrate (84 mg) was added, after the addition, the mixture was reacted at room temperature for 15 hours, after the reaction was completed, the mixture was concentrated to remove acetone, water (50 ml) was added, the mixture was extracted with ethyl acetate (100ml × 2) twice, the organic phases were combined, washed with saturated brine and saturated aqueous sodium bicarbonate solution, dried over anhydrous sodium sulfate, filtered, the filtrate was concentrated, and then the residue was separated and purified by column chromatography (petroleum ether : ethyl acetate (v/v) = 3 : 1) to obtain the compound 57F (3.8 g, 85%).

Step 7: (5R,6R,8R,9R)-8-(4-(bis(tert-butoxycarbonyl)amino)-6-chloro-1H-pyrazolo[3,4-d]pyrimidin-1-yl)-6-(((tert-butyldimethylsilyl)oxy)methyl)-2,2-dimethyl-1,3,7-trioxaspiro[4.4]nonan-9-yl acetate (57G)

Compound 57F (3.8 g, 6.46 mmol) was dissolved in acetone (50 ml), a catalytic amount of p-toluenesulfonic acid was added, followed by 2,2-dimethoxypropane (6.73 g, 64.6 mmol), after the addition, the mixture was warmed to 25°C and reacted for 16 hours, after the reaction was completed, the mixture was concentrated to remove acetone, water (50 ml) was added, the mixture was extracted with ethyl acetate (100 ml × 2), washed with saturated brine, dried over anhydrous sodium sulfate, filtered, the filtrate was concentrated, and then the residue was separated and purified by column chromatography (petroleum ether: ethyl acetate (v/v) = 5 : 1) to obtain the compound 57G (3.4 g, 83%).

Step 8: (5R,6R,8R,9R)-8-(4-((tert-butoxycarbonyl)amino)-6-chloro-1H-pyrazolo[3,4-d]pyrimidin-1-yl)-6-(hydroxymethyl)-2,2-dimethyl-1,3,7-trioxaspiro[4.4]nonan-9-yl acetate (57H)

Compound 57G (3.4 g, 5.41 mmol) was dissolved in tetrahydrofuran (40 ml), tetrabutylammonium fluoride (8.1 ml, 8.1 mmol, 1.0 mol/L tetrahydrofuran solution) was added, after the addition, the mixture was reacted at room temperature for 2 hours, and after the reaction was completed, the reaction solution was directly concentrated. After concentration, the residue was separated and purified by column chromatography (ethyl acetate : petroleum ether (v/v) = 1 : 1) to obtain the compound 57H (2.2 g, 79%).

LC-MS (ESI): m/z = 514.1 $[M+H]^+$.

Step 9: Di-tert-butyl 2-(((5R,6R,8R,9R)-9-acetoxy-8-(4-((tert-butoxycarbonyl) amino)-6-chloro-1H-pyrazolo[3,4-d]pyrimidin-1-yl)-2,2-dimethyl-1,3,7-trioxaspiro[4.4]nonan-6-yl)methoxy)malonate (57I)

Compound 57H (0.9 g, 1.75 mmol) was dissolved in toluene (15 ml), tert-butyl diazomalate (0.85 g, 3.50 mmol) and rhodium(II) acetate dimmer (88 mg, 0.088 mmol) were added, after the addition, under nitrogen protection, the mixture was warmed to 100°C and reacted for 1 hour, after the reaction was completed, the reaction solution was directly concentrated, and after concentration, the residue was separated and purified by column chromatography (petroleum ether : ethyl acetate (v/v) = 4 : 1) to obtain the compound 57I (0.8 g, 62.7%).

Step 10: Di-tert-butyl 2-(((5R,6R,8R,9R)-9-acetoxy-8-(4-((tert-butoxycarbonyl)amino)-6-chloro-1H-pyrazolo[3,4-d]pyrimidin-1-yl)-2,2-dimethyl-1,3,7-trioxaspiro[4.4] nonan-6-yl)methoxy)-2-(4-(2-oxotetrahydropyrimidin-1 (2H)-yl)benzyl)malonate (57J)

Compound 57I (0.8 g, 1.1 mmol) was dissolved in dry N,N-dimethylformamide (10 ml), the reaction system was subjected to nitrogen replacement three times, cesium carbonate (1.06 g, 3.25 mmol) was added, after the addition, the mixture was stirred at room temperature for 30 min, 1-(4-(chloromethyl)phenyl)tetrahydropyrimidine -2(1H)-one (0.49 g, 2.2 mmol) and potassium iodide (91 mg, 0.55 mmol) were added, after the addition, the mixture was stirred at room temperature for 16 hours, after the reaction was completed, saturated ammonium chloride was added to quench the reaction, the mixture was extracted with ethyl acetate (100 ml × 2), the organic layers were combined, washed with saturated brine, dried over anhydrous sodium sulfate and concentrated, and then the residue was separated and purified by column chromatography (ethyl acetate : petroleum ether (v/v) = 10 : 1) to obtain the compound 57J (0.9 g, 89%).

Step 11: Di-tert-butyl 2-(((5S,6R,8R,9R)-8-(4-((tert-butoxycarbonyl)amino)-6-chloro-1H-pyrazolo[3,4-d]pyrimidin-1-yl)-9-hydroxy-2,2-dimethyl-1,3,7-trioxaspiro[4.4]nonan-6-yl)methoxy)-2-(4-(2-oxotetrahydropyrimidin-1 (2H)-yl)benzyl)malonate (57K)

Compound 57J (0.9 g, 0.98 mmol) was dissolved in ethanol (10 ml), potassium carbonate (0.41 g, 2.97 mmol) was added, after the addition, the mixture was stirred at room temperature for 30 min, after the reaction was completed, saturated ammonium chloride was added to quench the reaction, the mixture was extracted with ethyl acetate (50 ml × 2), the organic layers were combined, washed with saturated brine, dried over anhydrous sodium sulfate, and after concentration, the residue was separated and purified by column chromatography (dichloromethane : methanol (v/v) = 10 : 1) to obtain the compound 57K (0.8 g, 93.3%).

LC-MS (ESI): m/z = 874.3 $[M+H]^+$.

Step 12: 2-(((2R,3S/R,4R,5R)-5-(4-amino-6-chloro-1H-pyrazolo[3,4-d]pyrimidin-1-yl)-3,4-dihydroxy-3-(hydroxymethyl)tetrahydrofuran-2-yl)methoxy) -2-(4-(2-oxotetrahydrop-yrimidin-1(2H)-yl)benzyl)malonic acid (Compounds 57 and 58)

Compound 57K (0.80 g, 0.91 mmol) was dissolved in a mixed solvent of trifluoroacetic acid (8 ml) and water (8 ml), the mixture was stirred at room temperature for 5 hours, and after the reaction was completed, the reaction system was directly subjected to preparative HPLC for purification with preparative HPLC separation methods: 1. Instrument: waters 2767 (preparative liquid phase chromatographic instrument); chromatographic column: SunFire@ Prep C18 (19 mm × 250 mm); 2. The sample was filtered with a 0.45 μm filter to prepare a sample solution; 3. Preparative chromatography conditions: a. composition of mobile phases A and B: mobile phase A: acetonitrile; mobile phase B: water (containing 0.1% TFA); b. gradient elution: mobile phase A: 5% to 50%; c. flow rate: 12 mL/min; d. elution time: 20 min, retention time: 7.8 min, to obtain the compound 57 (80 mg, 14.0%), retention time: 8.5 min, to obtain the compound 58 (50 mg, 8.8%).

Compound 57: LC-MS (ESI): m/z = 620.1$[M-H]^-$.

Compound 57: $^1H$ NMR (400 MHz, $CD_3OD$): 8.10 (s, 1H), 7.39 (d, 2H), 7.25 (d, 2H), 6.21 (d, 1H), 4.94 (d, 1H), 4.74 (s, 2H), 4.65 (s, 1H), 4.28-4.25 (m, 1H), 4.01-3.77 (m, 4H), 3.69-3.66 (m, 2H), 3.39-3.36 (m, 2H), 2.09-2.03 (m, 2H).

Compound 58: LC-MS (ESI): m/z = 620.1$[M-H]^-$.

Compound 58: $^1H$ NMR (400 MHz, $CD_3OD$): 8.09 (s, 1H), 7.39 (d, 2H), 7.25 (d, 2H), 6.19 (d, 1H), 4.75 (s, 2H), 4.67 (s, 1H), 4.18-4.17 (m, 1H), 4.07-3.84 (m, 4H), 3.69-3.66 (m, 2H), 3.39-3.36 (m, 2H), 2.09-2.03 (m, 2H).

**Biological test examples**

**1. Test method for CD73 enzyme activity in vitro**

Human recombinant protein CD73 (Aero, Cat. No. CD3-H52H7) was prepared into a 0.04 ng/μL protein solution, and substrate AMP (Sigma, Cat. No. A1752-1G) was prepared into a 100 μM substrate solution. 5 μL of compounds at different concentrations were added into a 384-well white transparent bottom plate, 5 μL of 0.04 ng/μL recombinant CD73 enzyme solution was added, and the mixture was incubated at room temperature for 15 minutes. 10 μL of 100 μM substrate solution was added, the mixture was incubated at 37°C for 40 minutes, 5 μL of Malachite Green Reagent A (R&D, Cat. No. DY996) was added, the mixture was incubated at room temperature for 10 minutes, and finally, 5 μL of Malachite Green Reagent B was added, the mixture was incubated at room temperature for 20 minutes, and a microplate reader PHERAstar FSX instrument was used to detect the absorbance at 620 nm. $IC_{50}$ values were calculated using the origin9.2 software. The calculation formula of the inhibition rate was shown in Formula 1, wherein $OD_{sample}$ is the readout for the compound, $OD_{negative}$ is the readout for the negative control without substrate, and $OD_{positive}$ is the readout for the vehicle control.

$$\text{Inhibition rate\%} = (1-(OD_{sample} - OD_{negative})/(OD_{positive} - OD_{negative})) \times 100\%$$

(Formula 1)

Test results: the $IC_{50}$ values of the compound of the present invention against CD73 enzyme activity in vitro is less than 100nM, preferably, the $IC_{50}$ values of the compound is less than 50nM, more preferably, the $IC_{50}$ values of the compound is less than 10nM, and most preferably, the $IC_{50}$ values of the compound is less than 1nM. Specific $IC_{50}$ values for some examples were shown in Table 1.

Table 1 CD73 enzyme activity in vitro

| Compound | $IC_{50}$ (nM) |
|---|---|
| 12 | 0.4 |
| 18 | 1.8 |
| 24 | 5.3 |
| 25 | 0.4 |
| 26 | 2.9 |
| 27 | 1.7 |
| 28 | 0.7 |
| 29 | 2.6 |
| 30 | 0.2 |
| 31 | 0.7 |
| 32 | 0.9 |
| 33 | 1.2 |
| 38 | 0.3 |
| 40 | 0.06 |

Conclusion: the compound of the present invention has a good inhibitory effect on CD73.

**2. In vivo drug efficacy test**

B16F10 xenograft tumor model in vivo

B16F10 cells were purchased from ATCC, the cell medium was DMEM + 10% FBS + 1% double antibody, and

the cells were cultured in an incubator at 37°C under 5% $CO_2$. After the cells had grown to a number sufficient for inoculation, the cells were harvested. The cell-containing culture solution was transferred to a 50 mL sterile centrifuge tube, and centrifuged at 1500 rpm for 3 minutes. The centrifuge tube was taken out after centrifugation, and the supernatant was discarded. The cells were resuspended in a pre-cooled PBS and centrifuged at 1500 rpm for 3 minutes, and after centrifugation, the supernatant was discarded; the process was repeated twice. Finally, the cells were resuspended in a pre-cooled PBS. After counting, the cells were diluted to $1 \times 10^6$ cells/mL with a pre-cooled PBS. The prepared cell suspension was placed on ice, and the cells were inoculated subcutaneously in the right axilla with a 1 mL pre-cooled syringe, 100 μL for each animal. The second day of inoculation was recorded as Day 0, and compound 28 (the vehicle was phosphate buffer saline) was given by intragastric administration every day after randomization, and animals in both the group with the anti-PD-1 antibody (anti-PD-1; BioXcell, Cat. CD279) and the group with combined drugs were injected intraperitoneally with the anti-PD-1 antibody (twice a week). The long and short diameters of the tumors were measured with an electronic vernier caliper, and the body weight of the animals was recorded. The data were processed according to Formula (1), Formula (2) and Formula (3), respectively, to calculate the tumor volume and tumor growth inhibition rate, wherein a is the long diameter of the tumor and b is the short diameter of the tumor. T is the tumor volume of the treated group at the end of the experiment, and C is the tumor volume of the vehicle (vehicle) group at the end of the experiment; $T_0$ is the tumor volume of the treated group at the beginning of the experiment, and $C_0$ is the tumor volume of the vehicle group at the beginning of the experiment.

$$\text{Tumor volume (mm}^3) = (a \times b^2)/ 2 \qquad \text{Formula (1)}$$

$$T/C\ (\%) = (T - T_0)/(C - C_0) \times 100 \qquad \text{Formula (2)}$$

$$\text{Tumor growth inhibition rate (TGI\%)} = 100 - T/C\ (\%) \qquad \text{Formula (3)}$$

Results: As shown in Figure 1, compared with the vehicle control group, there was no significant difference in the body weight of the animals in all the treated groups, and there was no significant decrease in the body weight of the animals, indicating that the animals had good tolerance, and the example compound had no obvious toxic and side effects. As shown in Table 2 and Figure 2 below, the tumor growth inhibition rate of Compound 28 combined with the anti-PD-1 antibody reached 91.61%, which was significantly better than the anti-tumor efficacy of AB680 combined with the anti-PD- antibody.

Table 2 Tumor growth inhibition rate of B16F10 xenograft tumor in vivo

| Group | TGI% (day 15) |
|---|---|
| Vehicle | - |
| AB680 30 mg/kg | 57.96 |
| Compound 28 100 mg/kg | 50.12 |
| Anti-PD-1 10 mg/kg | 68.51 |
| AB680 30 mg/kg+anti-PD-1 10 mg/kg | 69.25 |
| Compound 28 30 mg/kg+anti-PD-1 10 mg/kg | 91.61 |
| Notes: the AB680 structure CAS number is 2105904-82-1. | |

**3. Pharmacokinetic test among animals of varying genera**

3.1 Pharmacokinetic test in rats

Test objective: A single dose of each test compound was administered to SD rats intravenously, and the concentrations of the test compounds in plasma of rats were measured to evaluate the pharmacokinetic characteristics of the test compounds in rats.

Test subject: the example compound of the present invention.

Experimental animals: male SD rats, about 220 g, 6-8 weeks old, 3 rats/compound, purchased from Chengdu Ddossy Experimental Animals Co., Ltd.

Experimental method: on the day of the test, 6 SD rats were randomly grouped according to their body weight; the animals were fasted with water available for 12 to 14 h one day before the administration, and were fed 4 h after the administration; and the administration was performed according to Table 3.

Table 3 Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | | 1 | 0.2 | 5 | Plasma | Intravenously |

Vehicle for intravenous administration: 5% DMA + 5% Solutol + 90% Saline
before and after the administration, 0.1 ml of blood was taken from the orbits of the rats under isoflurane anesthesia, and placed in an EDTAK2 centrifuge tube. Centrifugation was performed at 5000 rpm at 4°C for 10 min, and plasma was collected. Time points for blood collection: 0, 5 min, 15 min, 30 min, 1 h, 2 h, 4 h, 6 h, 8 h, and 24 h. Before analysis and detection, all samples were stored at -80°C.

**3.2 Pharmacokinetic test in mice**

**Test objective:** A single dose of each test compound was administered to BALB/c mice intravenously, and the concentrations of the test compounds in plasma of mice were measured to evaluate the pharmacokinetic characteristics of the test compounds in mice.
Test subject: the example compound of the present invention.
**Experimental animals:** male BALB/c mice, about 22 g, 6-8 weeks old, 9 mice/compound, purchased from Chengdu Ddossy Experimental Animals Co., Ltd.
**Experimental method:** on the day of the test, 9 BALB/c mice were randomly grouped according to their body weights. the animals were fasted with water available for 12 to 14 h one day before the administration, and were fed 4 h after the administration; and the administration was performed according to Table 4.

Table 4 Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 9 | | 1 | 0.2 | 5 | Plasma | Intravenously |

Vehicle for intravenous administration: 5% DMA + 5% Solutol + 90% Saline
before and after the administration, 0.06 ml of blood was taken from the orbits of the mice under isoflurane anesthesia, and placed in an EDTAK2 centrifuge tube. Centrifugation was performed at 5000 rpm at 4°C for 10 min, and plasma was collected. Time points for blood collection: 0, 5 min, 15 min, 30 min, 1 h, 2 h, 4 h, 6 h, 8 h, and 24 h. Before analysis and detection, all samples were stored at -80°C.

**3.3 Pharmacokinetic test in beagle dogs**

**Test objective:** a single dose of test compounds was administered to beagle dogs intravenously, and the concentrations of the test compounds in plasma of the beagle dogs were measured to evaluate pharmacokinetic characteristics of the test compounds in the beagle dogs.
**Experimental animals:** male beagle dogs, about 8-11 kg, 6 beagle dogs/compound, purchased from Beijing Marshall Biotechnology Co., Ltd.
**Experimental method:** on the day of the test, 6 beagle dogs were randomly grouped according to their body weights. the animals were fasted with water available for 12 to 14 h one day before the administration, and were fed 4 h after the administration; and the administration was performed according to Table 5.

Table 5 Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | | 0.5 | 0.5 | 1 | Plasma | Intravenously |

Vehicle for intravenous administration: 5% DMA + 5% Solutol + 90% Saline
before and after the administration, 1 ml of blood was taken from the limb veins, and placed in an EDTAK2 centrifuge tube. Centrifugation was performed at 5000 rpm at 4°C for 10 min, and plasma was collected. Time points for blood collection: 0, 5 min, 15 min, 30 min, 1 h, 2 h, 4 h, 6 h, 8 h, 10 h, 12 h, 24 h, 48 h, 72 h, 96 h. Before analysis and detection, all samples were stored at -80°C.

### 3.4 Pharmacokinetic test in cynomolgus monkeys

**Test objective:** a single dose of test compounds was administered to cynomolgus monkeys intravenously, and the concentrations of the test compounds in plasma of the cynomolgus monkeys were measured to evaluate pharmacokinetic characteristics of the test compounds in the cynomolgus monkeys.
Test subject: the example compound of the present invention.
**Experimental animals:** male cynomolgus monkeys, about 3-5 kg, 6 cynomolgus monkeys/compound, purchased from Suzhou Xishan Zhongke Laboratory Animal Co., Ltd.
**Experimental method:** on the day of the test, 6 cynomolgus monkeys were randomly grouped according to their body weights. the animals were fasted with water available for 12 to 14 h one day before the administration, and were fed 4 h after the administration; and the administration was performed according to Table 6.

Table 6 Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | | 0.5 | 0.5 | | Plasma | Intravenously |

Vehicle for intravenous administration: 5% DMA + 5% Solutol + 90% Saline
before and after the administration, 1 ml of blood was taken from the limb veins, and placed in an EDTAK2 centrifuge tube. Centrifugation was performed at 5000 rpm at 4°C for 10 min, and plasma was collected. Time points for blood collection: 0, 5 min, 15 min, 30 min, 1 h, 2 h, 4 h, 6 h, 8 h, 10 h, 12 h, 24 h, 48 h, 72 h, and 96 h before analysis and detection, all samples being stored at -80°C.

The compounds of the present invention have excellent pharmacokinetic characteristics.

**4. CYP450 enzyme inhibition test**

In this test, after the mixed human liver microsomes were incubated with compounds at different concentrations (0.05-50 μM) and corresponding probe drugs, the changes of CYP enzyme activity were measured, and the $IC_{50}$ value was calculated to evaluate the inhibition potential of the compound on each CYP enzyme.

**5. Test for hERG potassium ion channel**

Test platform: electrophysiological manual patch-clamp system

Cell line: Chinese hamster ovary (CHO) cell lines stably expressing hERG potassium ion channel

Test method: In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

Data processing: Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / I\text{o})] \times 100\%$$

wherein, Inhibition % represents the percentage of inhibition of hERG potassium current by the compound, and I and Io represent the amplitude of hERG potassium current after and before dosing, respectively.

Compound $IC_{50}$ was calculated using GraphPad Prism 5 software by fitting according to the following equation:

$$Y = \text{Bottom} + (\text{Top-Bottom})/(1+10\text{^}((\text{LogIC50-X})*\text{HillSlope}))$$

wherein, X represents the Log value of the tested concentration of the test sample, Y represents the inhibition percentage at the corresponding concentration, and Bottom and Top represent the minimum and maximum inhibition percentage, respectively.

The compounds of the present invention had low toxic and side effects.

## Claims

1. A compound of formula (I), and a stereoisomer, a pharmaceutically acceptable salt, a solvate, a co-crystal or a deuterated product thereof,

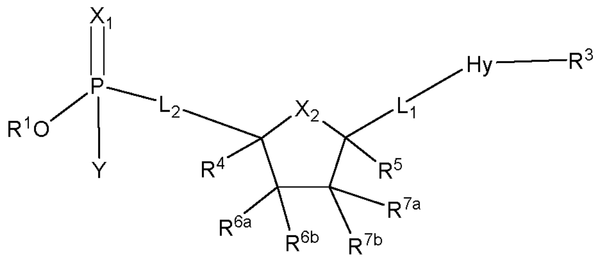

(I)

**characterized in that**

$X_1$ is selected from O or S;
$X_2$ is selected from $CR^{x2a}R^{x2b}$, O, S or $NR^{x2c}$;
Y is selected from $OR^2$, $-C(R^{y1}R^{y2})-P(=O)(OR^2)OR^2$, $-C(R^{y1}R^{y2})-P(=O)(OR^2)NHR^{y3}$, $-C(R^{y1}R^{y2})-P(=O)(OR^2)SR^{y3}$, $-C(R^{y1}R^{y2})-R^{y4}$, $-C(R^{y1}R^{y2})-P(=S)(OR^2)OR^2$, $-C(R^{y1}R^{y2})-P(=S)(OR^2)NHR^{y3}$ or $-C(R^{y1}R^{y2})-P(=S)(OR^2)SR^{y3}$;
$R^1$ and $R^2$ are each independently selected from H, deuterium, amino, $C_{1-6}$alkyl, $C_{2-6}$alkenyl, $C_{2-6}$alkynyl, $C_{3-10}$cycloalkyl, 3- to 10-membered heterocycloalkyl, $C_{6-10}$aryl, 5- to 10-membered heteroaryl, $-C(R^{2a}R^{2b})-O-C(O)-OR^{2c}$, $-C_{0-6}alkyl-S(O)_rR^{2c}$, $-C_{0-6}alkyl-C(O)R^{2c}$, $-C_{0-6}alkyl-C(O)OR^{2c}$ or $-C_{0-6}alkyl-C(O)NR^{2c}$, wherein the alkyl, cycloalkyl, heterocycloalkyl, aryl or heteroaryl is optionally further substituted with 1 or more groups selected from $R^x$;
r is selected from 1 or 2;
$R^{y1}$, $R^{y2}$, $R^{y3}$, $R^{x2a}$, $R^{x2b}$ and $R^{x2c}$ are each independently selected from H, deuterium, hydroxyl, cyano, $C_{1-6}$alkyl, deuterated $C_{1-6}$alkyl or halo $C_{1-6}$alkyl;
$R^{y4}$ is selected from 4- to 10-membered heterocycloalkyl, 5- to 10-membered heteroaryl, $C_{3-10}$cycloalkyl or $C_{6-10}$aryl, wherein the heterocycloalkyl, heteroaryl, cycloalkyl or aryl is optionally further substituted with 1 or more groups selected from $R^x$;
$R^{2a}$ and $R^{2b}$ are each independently selected from H, deuterium, hydroxyl, cyano, halogen, $C_{1-6}$alkyl, deuterated $C_{1-6}$alkyl, halo $C_{1-6}$alkyl, $C_{3-10}$cycloalkyl, 3-to 10-membered heterocycloalkyl, $C_{6-10}$aryl or 5- to 10-membered heteroaryl;
each $R^{2c}$ is independently selected from H, deuterium, $C_{1-6}$alkyl, deuterated $C_{1-6}$alkyl, halo $C_{1-6}$alkyl, $C_{3-10}$cycloalkyl, 3- to 10-membered heterocycloalkyl, $C_{6-10}$aryl or 5- to 10-membered heteroaryl, wherein the alkyl, cycloalkyl, heterocycloalkyl, aryl or heteroaryl is optionally further substituted with 1 or more groups selected from $R^x$;
$L_1$ is selected from a bond, S, O, NH, $N(C_{1-6}alkyl)$ or $C_{1-6}$alkyl, wherein the alkyl is optionally further substituted with 1 or more groups selected from $R^x$;
$L_2$ is selected from $-(CR^{L2a}R^{L2b})n-$, *-Z-$C_{6-10}$aryl-, *-Z-(5- to 10-membered heteroaryl)-, *-$C_{6-10}$aryl-Z-, *-(5- to 10-membered heteroaryl)-Z-, $C_{2-6}$alkenyl or $C_{2-6}$alkynyl, wherein the carbon atom in the $-(CR^{L2a}R^{L2b})n-$ is optionally replaced with 1 or more groups selected from N, O, S, S(O) or $S(O)_2$, the $L_2$ is optionally further substituted with 1 or more groups selected from $R^x$, and * denotes the site where the $L_2$ is connected to P atom;
$R^{L2a}$ and $R^{L2b}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl, or $-C_{1-6}alkyl-O-C_{1-6}alkyl$, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl;
or, $R^{L2a}$ and $R^{L2b}$ on the same carbon atom together with the carbon atom to which they are attached form $C_{3-6}$cycloalkyl or 4- to 6-membered heterocycloalkyl;
each n is independently selected from 1, 2, 3, 4, 5, 6, 7, 8, 9 or 10;
each Z is independently selected from a bond, -O- or $C_{1-6}$alkyl, wherein the alkyl is optionally further substituted with 1 or more groups selected from $R^x$;
$R_4$, $R_5$, $R^{6a}$, $R^{6b}$, $R^{7a}$, and $R^{7b}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino or alkyl is optionally further substituted with 1 or more groups selected from deuterium, halogen, hydroxyl, cyano or $C_{1-6}$alkoxy;
or, $R^{6a}$ and $R^{6b}$, or $R^{7a}$ and $R^{7b}$ together form $C_{2-6}$alkenyl, $C_{3-6}$cycloalkyl or 4-to 6-membered heterocycloalkyl, wherein the alkenyl is optionally further substituted with 1 or more groups selected from deuterium, halogen, hydroxyl or cyano;

or, $R^4$, $R^5$ and $X_2$, or $R^4$, $R^{6a}$ and $X_2$, or $R^{L2a}$, $R^5$ and $X_2$, or $R^4$, $R^{7a}$ and $X_2$ together form $C_{5-7}$cycloalkyl or 5- to 7-membered heterocycloalkyl;

or, $R^4$ and $R^{6a}$, or $R^{6b}$ and $R^{7b}$, or $R^4$ and $R^{L2a}$, or $R^4$ and $X_2$ together form $C_{3-7}$cycloalkyl or 3- to 7-membered heterocycloalkyl;

or, $R^{x2a}$ and $R^4$, or $R^{x2a}$ and $R^5$, or $R^{6a}$ and $R^4$, or $R^{6b}$ and $R^{7b}$, or $R^{7a}$and $R^5$ together form one chemical bond, provided that at most one group forms one chemical bond;

Hy is selected from 5- to 15-membered heteroaryl or 5- to 15-membered heterocycloalkyl, wherein the heteroaryl or heterocycloalkyl is optionally further substituted with 1 or more groups selected from $R^{hy}$;

$R^3$ is selected from $L_3$-$R^{3a}$, 5- to 10-membered heterocycloalkyl, $C_{5-10}$cycloalkyl, 5- to 10-membered heteroaryl or $C_{6-10}$aryl, wherein the heterocycloalkyl, cycloalkyl, heteroaryl or aryl is optionally further substituted with 1 or more groups selected from $R^y$;

$L_3$ is selected from **-N($R^{L3}$)$C_{1-6}$alkyl-, -N($R^{L3}$)-, **-N($R^{L3}$)-O-, **-N($R^{L3}$)S(O)-, **-N($R^{L3}$)$S(O)_2$-, **-N($R^{L3}$)S(O)-$C_{1-6}$alkyl-, **-N($R^{L3}$)$S(O)_2$-$C_{1-6}$alkyl- or **-N($R^{L3}$)-N=CH-, wherein ** denotes the site where $L_3$ is connected to Hy;

$R^{3a}$ is selected from H, $C_{1-6}$alkyl, benzyl, 5- to 15-membered carbocyclyl or 5-15 heterocyclyl, wherein the alkyl, benzyl, carbocyclyl or heterocyclyl is optionally further substituted with 1 or more groups selected from $R^y$;

$R^{L3}$ is each independently selected from H, deuterium, $C_{1-6}$alkyl, halo $C_{1-6}$alkyl or deuterated $C_{1-6}$alkyl;

or, $R^{L3}$ and $R^{hy}$ together with the atom to which they are attached form 5- to 8-membered heterocycloalkyl;

$R^{hy}$ and $R^X$ are each independently selected from deuterium, halogen, amino, hydroxyl, cyano, =O, $SF_5$, azido, $C_{1-6}$alkyl, $C_{1-6}$alkoxy, $C_{2-6}$alkenyl or $C_{2-6}$alkynyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, -O-$C_{1-6}$alkyl-$C_{6-10}$aryl or -O-$C_{1-6}$alkyl-(5- to 10-membered heteroaryl);

$R^y$ is selected from deuterium, halogen, amino, hydroxyl, cyano, =O, $SF_5$, azido, -P(=O)$(C_{1-6}alkyl)_2$, -P(=S)$(C_{1-6}alkyl)_2$,

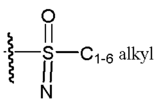 , 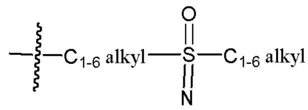 ,

-Si$(C_{1-6}alkyl)_3$, -N=S(=O)$(C_{1-6}alkyl)_2$, -$C_{1-6}$alkyl-S(=O)(=N), -$C_{1-6}$alkyl, $C_{1-6}$alkoxy, $C_{2-6}$alkenyl or $C_{2-6}$alkynyl, wherein the alkyl, alkoxy, amino, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy.

2. The compound, and the stereoisomer, pharmaceutically acceptable salt, solvate, co-crystal or deuterated product thereof according to claim 1, **characterized in that**

$L_2$ is selected from *-O-$(CR^{L2a}R^{L2b})$n'-, *-$(CR^{L2a}R^{L2b})$n'-O-, -$(CR^{L2a}R^{L2b})$n-,-O-, *-O-$(CR^{L2a}R^{L2b})$n'-O-$(CR^{L2a}R^{L2b})$m-, *-$(CR^{L2a}R^{L2b})$n'-O-$(CR^{L2a}R^{L2b})$m-, *-O-$(CR^{L2a}R^{L2b})$n'-O-, *-O-N($R^{L2c}$)-, *-(O)$C_{1-6}$alkyl, *-Z-$C_{6-10}$aryl, *-Z-(5- to 10-membered heteroaryl), $C_{2-6}$alkenyl or $C_{2-6}$alkynyl, wherein the alkyl, aryl, heteroaryl, alkenyl or alkynyl is optionally further substituted with 1 or more groups selected from $R^x$;

$R^{L2a}$, $R^{L2b}$, and $R^{L2c}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl or -$C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl;

or, $R^{L2a}$ and $R^{L2b}$ on the same carbon atom together with the carbon atom to which they are attached form $C_{3-6}$cycloalkyl or 4- to 6-membered heterocycloalkyl;

m, n, and n' are each independently selected from 1, 2, 3, 4 or 5.

3. The compound, and the stereoisomer, pharmaceutically acceptable salt, solvate, co-crystal or deuterated product thereof according to claim 1, **characterized in that**

$R^3$ is selected from $L_3$-$R^{3a}$;

$R^{3a}$ is selected from H, $C_{1-6}$alkyl, benzyl, 5- to 15-membered carbocyclyl or 5-15 heterocyclyl, wherein the alkyl,

benzyl, carbocyclyl or heterocyclyl is further substituted with 1 or more groups selected from $R^y$;
when $R^{3a}$ is substituted with two or more $R^y$, $R^y$ is selected from deuterium, halogen, amino, hydroxyl, cyano, =O, $SF_5$, azido, -P(=O)($C_{1-6}$alkyl)2, -P(=S)($C_{1-6}$alkyl)$_2$,

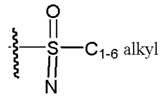 , 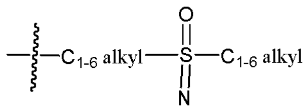 ,

-Si($C_{1-6}$alkyl)$_3$, -N=S(=O)($C_{1-6}$alkyl)$_2$, -$C_{1-6}$alkyl-S(=O)(=N), -$C_{1-6}$alkyl, $C_{1-6}$alkoxy, $C_{2-6}$alkenyl or $C_{2-6}$alkynyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy, provided that, when $R^{3a}$ is substituted with two $R^y$, $R^y$ is not a combination of F and $SF_5$;
when $R^{3a}$ is substituted with one $R^y$, $R^y$ is selected from azido, -P(=O)($C_{1-6}$alkyl)$_2$, -P(=S)($C_{1-6}$alkyl)$_2$,

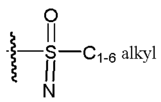 , 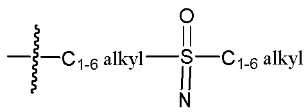 ,

-Si($C_{1-6}$alkyl)$_3$,-N=S(=O)($C_{1-6}$alkyl)$_2$ or -$C_{1-6}$alkyl-S(=O)(=N), wherein the alkyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy.

**4.** The compound, and the stereoisomer, pharmaceutically acceptable salt, solvate, co-crystal or deuterated product thereof according to claim 1, **characterized in that**

$R^4$, $R_5$, $R^{6a}$, $R^{6b}$, $R^{7a}$, and $R^{7b}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1 or more groups selected from deuterium, halogen, hydroxyl or cyano;
or, $R^{6a}$ and $R^{6b}$, or $R^{7a}$ and $R^{7b}$ together form $C_{2-6}$alkenyl, $C_{3-6}$cycloalkyl or 4-to 6-membered heterocycloalkyl, wherein the alkenyl is optionally further substituted with 1 or more groups selected from deuterium, halogen, hydroxyl or cyano;
or, $R^4$, $R^5$ and $X_2$, or $R^4$, $R^{6a}$ and $X_2$, or $R^4$, $R^{7a}$ and $X_2$ together form $C_{5-7}$cycloalkyl or 5- to 7-membered heterocycloalkyl;
or, $R^4$ and $R^{6a}$, or $R^{6b}$ and $R^{7b}$, or $R^4$ and $X_2$ together with the atom to which they are attached form $C_{3-7}$cycloalkyl, or 3- to 7-membered heterocycloalkyl;
or, $R^{x2a}$ and $R^4$, or $R^{x2a}$ and $R^5$, or $R^{6a}$ and $R^4$, or $R^{6b}$ and $R^{7b}$, or $R^{7a}$and $R^5$ together form one chemical bond, provided that at most one group forms one chemical bond;
provided that

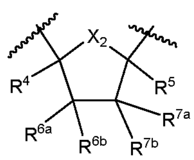

is not selected from the following structures:

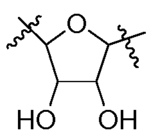 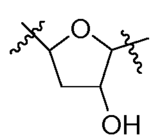 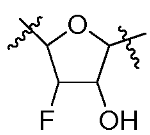 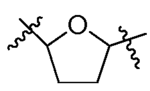 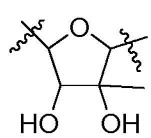

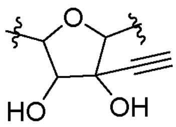 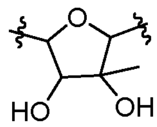.

5. The compound, and the stereoisomer, pharmaceutically acceptable salt, solvate, co-crystal or deuterated product thereof according to claim 1, **characterized in that**

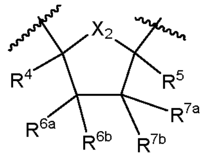

is selected from

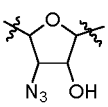 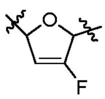 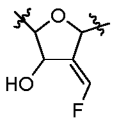 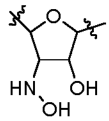 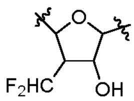 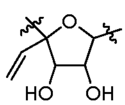 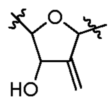 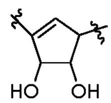

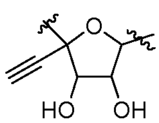 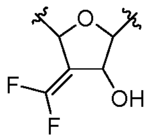 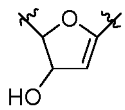 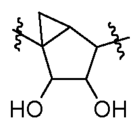 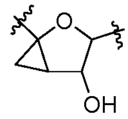 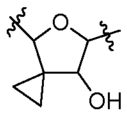

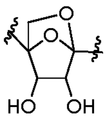 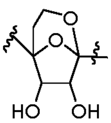 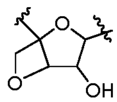 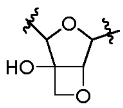 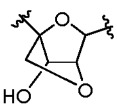 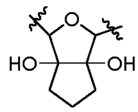 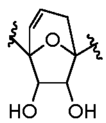.

6. The compound, and the stereoisomer, pharmaceutically acceptable salt, solvate, co-crystal or deuterated product thereof according to claim 1, **characterized in that**

$R^{L2a}$, $R^5$ and $X_2$ together with the atom to which they are attached form $C_{5-7}$cycloalkyl, or 5- to 7-membered heterocycloalkyl;
or, $R^4$ and $R^{L2a}$ together with the atom to which they are attached form $C_{3-7}$cycloalkyl, or 3- to 7-membered heterocycloalkyl;
further,
$R^{L2a}$, $R^5$ and $X_2$ together with the atom to which they are attached form 6-membered heterocycloalkyl, or 7-membered heterocycloalkyl;
or, $R^4$ and $R^{L2a}$ together with the atom to which they are attached form 3-membered cycloalkyl, 4-membered cycloalkyl, or 5-membered cycloalkyl.

7. The compound, and the stereoisomer, pharmaceutically acceptable salt, solvate, co-crystal or deuterated product thereof according to claim 1, **characterized in that** the compound has a structure of formula (II):

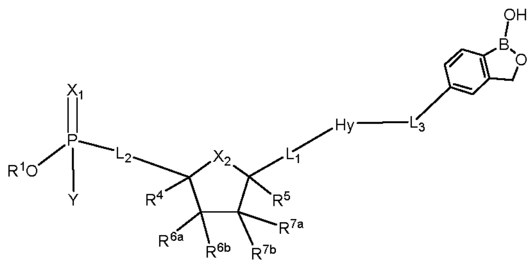

(II).

**8.** The compound, and the stereoisomer, pharmaceutically acceptable salt, solvate, co-crystal or deuterated product according to claim 1, **characterized in that** the compound has a structure of formula (III), (IV), (V), or (VI):

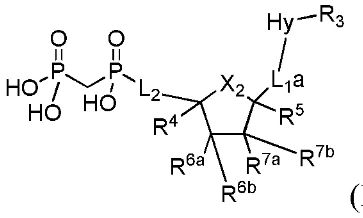

(III)

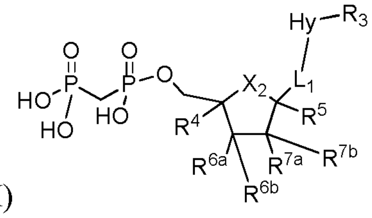

(IV)

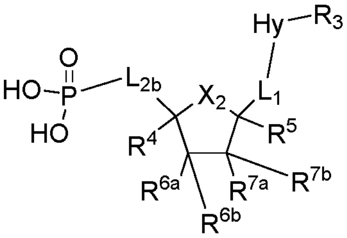

(V)

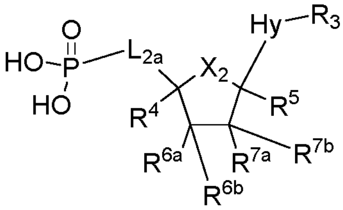

(VI)

$L_1$ is selected from S, O, NH, $N(CH_3)$ or $-CH_2-$;

$L_{1a}$ is selected from a bond, S, O, NH, $N(CH_3)$ or $-CH_2-$;

$L_2$ is selected from $*-O-(CR^{L2a}R^{L2b})_2-$, $*-(CR^{L2a}R^{L2b})n'-O-$, -O-, $*-O-(CR^{L2a}R^{L2b})n'-O-(CR^{L2a}R^{L2b})_m-$, $*-(CR^{L2a}R^{L2b})n'-O-(CR^{L2a}R^{L2b})m-$, $*-O-(CR^{L2a}R^{L2b})n'-O-$, $*-O-N(R^{L2c})-$, $*-C(O)C_{1-6}$alkyl-, $*-Z-C_{6-10}$aryl-, *-Z-(5- to 10-membered heteroaryl)-, $C_{2-6}$alkenyl or $C_{2-6}$alkynyl, wherein the alkyl, aryl, heteroaryl, alkenyl or alkynyl is optionally further substituted with 1 to 3 groups selected from $R^x$, and * denotes the site where the $L_2$ is connected to P atom;

$L_{2a}$ is selected from $*-O-(CR^{L2a}R^{L2b})_2-$, $*-(CR^{L2a}R^{L2b})n'-O-$, $*-O-(CR^{L2a}R^{L2b})n'-O-(CR^{L2a}R^{L2b})_m-$, $*-(CR^{L2a}R^{L2b})_2-O-(CR^{L2a}R^{L2b})_m-$, $*-O-(CR^{L2a}R^{L2b})n'-O-$, $*-O-N(R^{L2c})-$, $*-C(O)C_{1-6}$alkyl-, $*-Z-C_{6-10}$aryl-, *-Z-(5- to 10-membered heteroaryl)-, $C_{2-6}$alkenyl or $C_{2-6}$alkynyl, wherein the alkyl, aryl, heteroaryl, alkenyl or alkynyl is optionally further substituted with 1 to 3 groups selected from $R^x$, and * denotes the site where the $L_2$ is connected to P atom;

$L_{2b}$ is selected from $*-O-(CR^{L2a}R^{L2b})_2-$, $*-(CR^{L2a}R^{L2b})n'-O-$, -O-, $*-O-(CR^{L2a}R^{L2b})n'-O-(CR^{L2a}R^{L2b})m-$, $*-(CR^{L2a}R^{L2b})n'-O-(CR^{L2a}R^{L2b})m-$, $*-O-(CR^{L2a}R^{L2b})n'-O-$, $*-O-N(R^{L2c})-$, $*-C(O)C_{1-6}$alkyl-, $*-Z-C_{6-10}$aryl-, *-Z-(5- to 10-membered heteroaryl)-, $C_{2-6}$alkenyl or $C_{2-6}$alkynyl, wherein the alkyl, aryl, heteroaryl, alkenyl or alkynyl is optionally further substituted with 1 to 3 groups selected from $R^x$, and * denotes the site where the $L_2$ is connected to P atom;

$R^{L2a}$, $R^{L2b}$, and $R^{L2c}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl or $-C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, 5- to 10-membered heteroaryl, $C_{6-10}$aryl, 4- to 10-membered heterocycloalkyl or $C_{3-10}$cycloalkyl;

or, $R^{L2a}$ and $R^{L2b}$ on the same carbon atom together with the carbon atom to which they are attached form $C_{3-6}$cycloalkyl, or 4- to 6-membered heterocycloalkyl;

$X_2$ is selected from $CH_2$ or O;

m, n, and n' are each independently selected from 1, 2 or 3;
provided that, in the compound of formula (V), when $L_{2b}$ is selected from *-($CR^{L2a}R^{L2b}$)-O-($CR^{L2a}R^{L2b}$)-, $R^{L2a}$ and $R^{L2b}$ are not both selected from H.

**9.** The compound, and the stereoisomer, pharmaceutically acceptable salt, solvate, co-crystal or deuterated product according to claim 8, **characterized in that** the compound has a structure of formula (III-2), (III-3), (IV-2), (IV-3), (V-2), (V-3), (VI-2), or (VI-3):

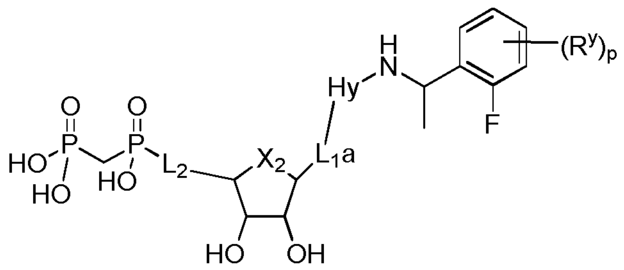

(III-2)

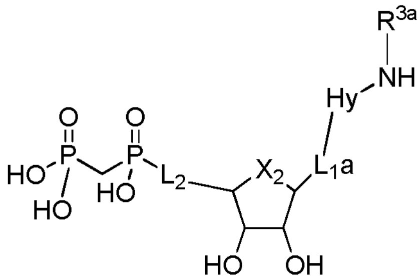

(III-3)

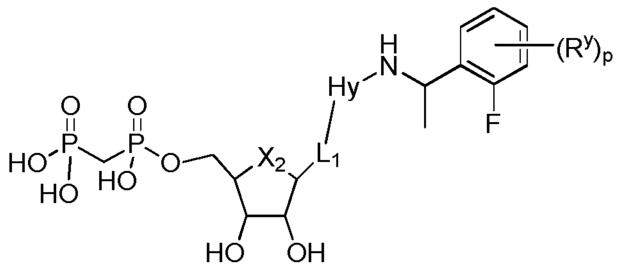

(IV-2)

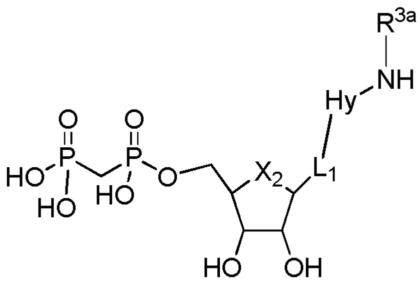

(IV-3)

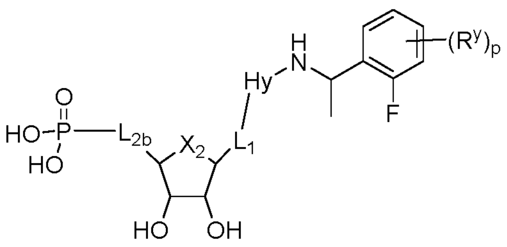

(V-2)

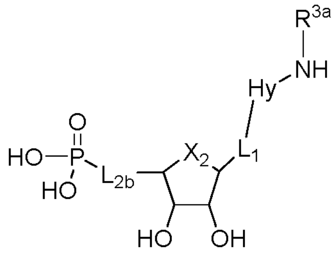

(V-3)

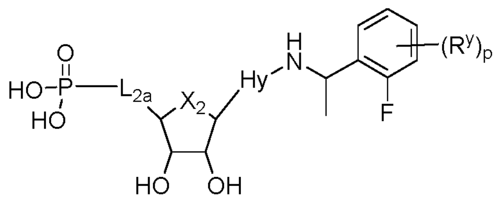

(VI-2)

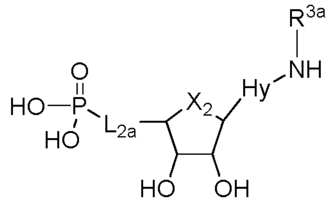

(VI-3)

p is selected from 0, 1 or 2;

$R^{3a}$ is selected from 5- to 6-membered monocyclic cycloalkyl, 5- to 6-membered monocyclic heterocycloalkyl, 8- to 10-membered bicyclic cycloalkyl, 8-to 10-membered bicyclic heterocycloalkyl, 11- to 15-membered tricyclic cycloalkyl or 11- to 15-membered tricyclic heterocycloalkyl, wherein the cycloalkyl or heterocycloalkyl is optionally further substituted with 1 to 3 groups selected from $R^y$.

**10.** The compound, and the stereoisomer, pharmaceutically acceptable salt, solvate, co-crystal or deuterated product thereof according to claim 1, **characterized in that**

Hy is selected from 6-membered heteroaryl, a 5-membered heteroaryl fused 5-membered heteroaryl, a 5-membered heteroaryl fused phenyl, a 5-membered heteroaryl fused 5-membered heterocycloalkyl, a 5-membered heteroaryl fused 6-membered heterocycloalkyl, a 5-membered heterocycloalkyl fused 5-membered heteroaryl, a 5-membered heterocycloalkyl fused 6-membered heteroaryl, a 5-membered heterocycloalkyl fused phenyl, a 5-membered heterocycloalkyl fused 5-membered heterocycloalkyl, a 5-membered heterocycloalkyl fused 6-membered heterocycloalkyl, a 6-membered heteroaryl fused 5-membered heteroaryl, a 6-membered heteroaryl fused 6-membered heteroaryl, a 6-membered heteroaryl fused phenyl, a 6-membered heteroaryl fused 5-membered heterocycloalkyl, a 6-membered heteroaryl fused 6-membered heterocycloalkyl, a 6-membered heterocycloalkyl fused 5-membered heteroaryl, a 6-membered heterocycloalkyl fused 6-membered heteroaryl, a 6-membered heterocycloalkyl fused phenyl, a 6-membered heterocycloalkyl fused 5-membered heterocycloalkyl, a 6-membered heterocycloalkyl fused 6-membered heterocycloalkyl or

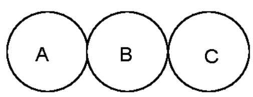

;

A ring is selected from 5- to 6-membered heteroaryl, 5- to 6-membered heterocycloalkyl or phenyl;

B ring is selected from 5- to 6-membered heteroaryl, 4- to 6-membered heterocycloalkyl or 4-6cycloalkyl;

C ring is selected from 5- to 6-membered heteroaryl, 5- to 6-membered heterocycloalkyl or phenyl;

the heteroaryl, aryl, phenyl or heterocycloalkyl is optionally further substituted with 1 or more groups selected from $R^{hy}$;

each $R^{hy}$ is substituted with a group independently selected from H, deuterium, halogen, amino, hydroxyl, cyano, $C_{1-6}$alkyl, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy.

**11.** The compound, and the stereoisomer, pharmaceutically acceptable salt, solvate, co-crystal or deuterated product thereof according to claim 1, **characterized in that**

Hy is selected from:

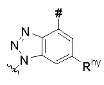 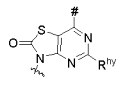 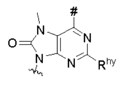 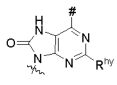 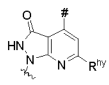 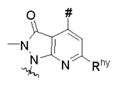 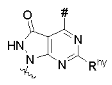 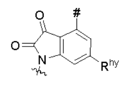

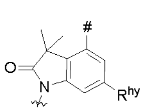 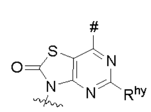 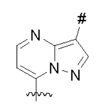 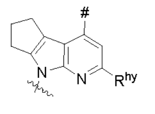 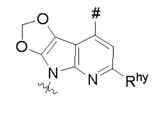 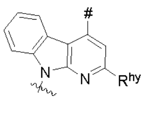 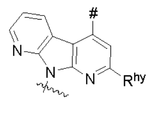 ;

each $R^{hy}$ is independently selected from deuterium, halogen, amino, hydroxyl, cyano, $SF_5$, azido, $C_{1-4}$alkyl, $C_{1-4}$alkoxy, $C_{2-4}$alkenyl or $C_{2-4}$alkynyl, wherein the alkyl, alkoxy, alkenyl or alkynyl is optionally further substituted with 1 to 3 groups selected from deuterium, halogen, amino, hydroxyl, cyano, deuterated $C_{1-4}$alkyl, halo $C_{1-4}$alkyl, hydroxyl $C_{1-4}$alkyl, $C_{1-4}$alkoxy, deuterated $C_{1-4}$alkoxy, halo $C_{1-4}$alkoxy or hydroxyl $C_{1-4}$alkoxy.

**12.** The compound, and the stereoisomer, pharmaceutically acceptable salt, solvate, co-crystal or deuterated product thereof according to claim 1, **characterized in that**
Y is selected from $-C(R^{y1}R^{y2})-R^{y4}$, $-C(R^{y1}R^{y2})-P(=S)(OR^2)OR^2$, $-C(R^{y1}R^{y2})-P(=S)(OR^2)NR^{y3}$ or $-C(R^{y1}R^{y2})-P(=S)(OR^2)SR^{y3}$.

**13.** The compound, and the stereoisomer, pharmaceutically acceptable salt, solvate, co-crystal or deuterated product thereof according to claim 1, **characterized in that** the compound has a structure of formula (VII):

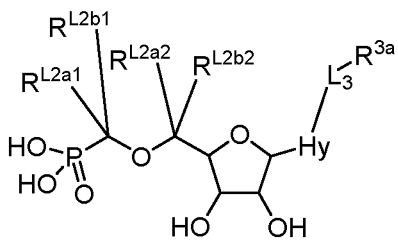
(VII)

$R^{L2a1}$, $R^{L2b1}$, $R^{L2a2}$, and $R^{L2b2}$ are each independently selected from H, deuterium, halogen, amino, hydroxyl, cyano, $C_{1-6}$alkyl, $C_{1-6}$alkoxy, $C_{2-6}$alkenyl, $C_{2-6}$alkynyl or $-C_{1-6}$alkyl-O-$C_{1-6}$alkyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy or hydroxyl $C_{1-6}$alkoxy;
or, $R^{L2a1}$ and $R^{L2b1}$ on the same carbon atom together with the carbon atom to which they are attached form $C_{3-6}$cycloalkyl, or 4- to 6-membered heterocycloalkyl;
or, $R^{L2a2}$ and $R^{L2b2}$ on the same carbon atom together with the carbon atom to which they are attached form $C_{3-6}$cycloalkyl, or 4- to 6-membered heterocycloalkyl;
provided that, at least three of $R^{L2a1}$, $R^{L2b1}$, $R^{L2a2}$ and $R^{L2b2}$ are not selected from H;
$L_3$ is selected from $**-N(R^{L3})C_{1-6}$alkyl- or $-N(R^{L3})-$;
$R^{3a}$ is selected from 5- to 15-membered carbocyclyl or 5- 15 heterocyclyl, wherein the carbocyclyl or heterocyclyl is optionally further substituted with 1 or more groups selected from $R^y$.

**14.** The compound, and the stereoisomer, pharmaceutically acceptable salt, solvate, co-crystal or deuterated product thereof according to claim 1, **characterized in that** the compound has a structure of formula (VIII) as follows:

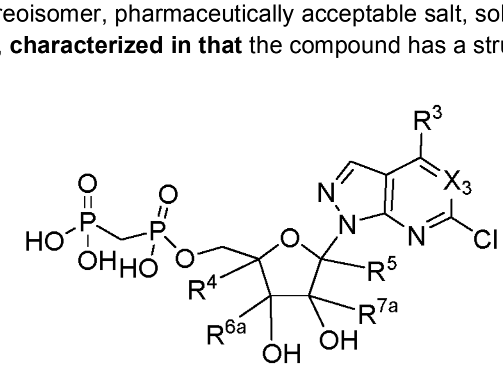
(VIII)

$X_3$ is selected from CH or N;
$R^4$, $R_5$, $R^{6a}$, and $R^{7a}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino or alkyl is optionally further substituted with 1, 2, or 3 groups selected from deuterium, halogen, hydroxyl, cyano or $C_{1-6}$alkoxy;
$R^3$ is selected from -NH-O-$R^{3a}$, 3- to 6-membered monocyclic cycloalkyl, 4-to 6-membered monocyclic heterocycloalkyl, 5- to 10-membered spiro ring, 4- to 10-membered fused ring, or 5- to 9-membered bridged ring, wherein the cycloalkyl, heterocycle cycloalkyl, spiro ring, bridged ring or fused ring is optionally further substituted with 1, 2, or 3 groups selected from $R^y$;
$R^{3a}$ is selected from $C_{1-6}$alkyl, benzyl, 3- to 6-membered monocyclic cycloalkyl, 4- to 6-membered monocyclic heterocycloalkyl, 5- to 12-membered spiro ring, 4- to 10-membered fused ring, or 5- to 9-membered bridged ring, wherein the cycloalkyl, heterocycloalkyl, spiro ring, bridged ring or fused ring is optionally further substituted with 1, 2, or 3 groups selected from $R^y$;
$R^y$ is selected from deuterium, halogen, amino, hydroxyl, cyano, =O, $SF_5$ or azido;
provided that, when $R^3$ is substituted or unsubstituted 3- to 6-membered monocyclic cycloalkyl, 4- to 6-membered monocyclic heterocycloalkyl, 5- to 12-membered spiro ring, 4- to 10-membered fused ring, or 5- to 9-membered bridged ring, $R^4$, $R_5$, $R^{6a}$, and $R^{7a}$ are not H or deuterium at the same time.

**15.** The compound, and the stereoisomer, pharmaceutically acceptable salt, solvate, co-crystal or deuterated product thereof according to claim 14,

**characterized in that**, $R_4$, $R_5$, $R^{6a}$, and $R^{7a}$ are each independently selected from H, deuterium, hydroxyl, cyano, halogen, azido, amino, $C_{1-6}$alkyl, $C_{3-6}$cycloalkyl, $C_{2-6}$alkenyl or $C_{2-6}$alkynyl, wherein the amino or alkyl is optionally further substituted with 1, 2, or 3 groups selected from deuterium, halogen, hydroxyl, cyano or $C_{1-6}$alkoxy, provided that $R_4$, $R_5$, $R^{6a}$, and $R^{7a}$ are not H or deuterium at the same time;
$R^3$ is selected from 3- to 6-membered monocyclic cycloalkyl, 4- to 6-membered monocyclic heterocycloalkyl, 5- to 10-membered spiro ring, 4- to 10-membered fused ring or 5- to 9-membered bridged ring, wherein the cycloalkyl, heterocycle cycloalkyl, spiro ring, bridged ring or fused ring is optionally further substituted with 1, 2, or 3 groups selected from $R^y$;
$R^y$ is selected from deuterium, halogen, amino, hydroxyl, cyano, =O, $SF_5$ or azido.

**16.** The compound, and the stereoisomer, pharmaceutically acceptable salt, solvate, co-crystal or deuterated product thereof according to claim 15, **characterized in that**

$R_4$, $R_5$, and $R^{7a}$ are selected from H;
$R^{6a}$ is selected from $C_{1-6}$alkyl or $C_{2-6}$alkynyl, wherein the alkyl is optionally further substituted with 1, 2, or 3 groups selected from deuterium, halogen, hydroxyl, cyano or $C_{1-2}$alkoxy;
$R^3$ is selected from 4- to 10-membered fused ring, wherein the fused ring is optionally further substituted with 1, 2, or 3 groups selected from $R^y$;
$R^y$ is selected from deuterium, halogen, amino, hydroxyl or =O.

**17.** The compound, and the stereoisomer, pharmaceutically acceptable salt, solvate, co-crystal or deuterated product thereof according to claim 14, **characterized in that** the compound has a structure of formula (IX) as follows:

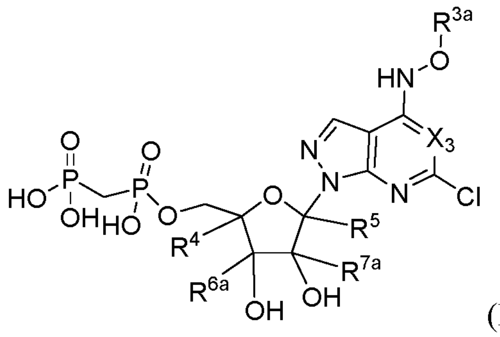

(IX)

$X_3$ is CH or N;
$R_4$, $R_5$, $R^{6a}$, and $R^{7a}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1,

2, or 3 groups selected from deuterium, halogen, hydroxyl or cyano;
$R^{3a}$ is selected from $C_{1-6}$alkyl, benzyl, 3- to 6-membered monocyclic cycloalkyl, 4- to 6-membered monocyclic heterocycloalkyl, 5- to 11-membered spiro ring, 4- to 10-membered fused ring or 5- to 9-membered bridged ring, wherein the cycloalkyl, heterocycle cycloalkyl, spiro ring, bridged ring or fused ring is optionally further substituted with 1, 2, or 3 groups selected from $R^y$;
$R^y$ is selected from deuterium, halogen, amino, hydroxyl, cyano, =O, $SF_5$ or azido.

**18.** The compound, and the stereoisomer, pharmaceutically acceptable salt, solvate, co-crystal or deuterated product thereof according to claim 17, **characterized in that**

$R_4$, $R_5$, $R^{6a}$, and $R^{7a}$ are selected from H; $R^{3a}$ is selected from $C_{1-6}$alkyl, benzyl or 3- to 6-membered monocyclic cycloalkyl, wherein the cycloalkyl is optionally further substituted with 1, 2, or 3 groups selected from $R^y$;
$R^y$ is selected from deuterium, halogen, amino, hydroxyl or =O.

**19.** The compound, and the stereoisomer, pharmaceutically acceptable salt, solvate, co-crystal or deuterated product thereof according to claim 1, **characterized in that** the compound has a structure of formula (X) as follows:

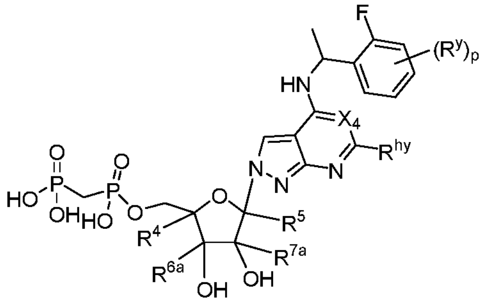

(X)

$X_4$ is CH or N;
$R_4$, $R_5$, $R^{6a}$, and $R^{7a}$ are each independently selected from H, deuterium, hydroxyl, cyano, halogen, azido, amino, $C_{1-6}$alkyl, $C_{2-6}$alkenyl or $C_{2-6}$alkynyl, wherein the amino or alkyl is optionally further substituted with 1, 2, or 3 groups selected from deuterium, halogen, hydroxyl or cyano;
$R^y$ is selected from deuterium, halogen, amino, hydroxyl, cyano, =O, $SF_5$, azido, $-P(=O)(C_{1-3}alkyl)_2$, $-P(=S)(C_{1-3}alkyl)_2$,

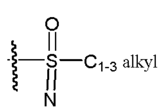 , 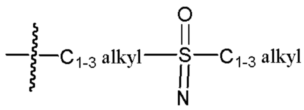 ,

$-Si(C_{1-3}alkyl)_3$, -N=S(=O)($C_{1-3}$alkyl)2, $-C_{1-3}$alkyl-S(=O)(=N), $-C_{1-3}$alkyl, $C_{1-3}$alkoxy, $C_{2-4}$alkenyl or $C_{2-4}$alkynyl, wherein the alkyl, alkoxy, alkenyl or alkynyl is optionally further substituted with 1 or more groups selected from deuterium, halogen, amino, hydroxyl, cyano, deuterated $C_{1-3}$alkyl, halo $C_{1-3}$alkyl, hydroxyl $C_{1-3}$alkyl, $C_{1-3}$alkoxy, deuterated $C_{1-3}$alkoxy, halo $C_{1-3}$alkoxy or hydroxyl $C_{1-3}$alkoxy;
p is selected from 0, 1, or 2;
$R^{hy}$ is selected from deuterium, halogen, amino, hydroxyl, cyano, =O, $SF_5$, azido, $C_{1-6}$alkyl, $C_{1-6}$alkoxy, $C_{2-6}$alkenyl or $C_{2-6}$alkynyl, wherein the alkyl, alkoxy, alkenyl or alkynyl is optionally further substituted with 1 or more groups selected from deuterium, halogen, amino, hydroxyl, cyano, deuterated $C_{1-6}$alkyl, halo $C_{1-6}$alkyl, hydroxyl $C_{1-6}$alkyl, $C_{1-6}$alkoxy, deuterated $C_{1-6}$alkoxy, halo $C_{1-6}$alkoxy, hydroxyl $C_{1-6}$alkoxy, $-O-C_{1-6}$alkyl-$C_{6-10}$aryl or $-O-C_{1-6}$alkyl-(5- to 10-membered heteroaryl).

**20.** The compound, and the stereoisomer, pharmaceutically acceptable salt, solvate, co-crystal or deuterated product thereof according to claim 19, **characterized in that**

$R_4$, $R_5$, and $R^{7a}$ are selected from H;
$R^{6a}$ is selected from $C_{1-6}$alkyl, wherein the alkyl is optionally further substituted with 1, 2, or 3 groups selected from deuterium, halogen, hydroxyl, cyano or $C_{1-2}$alkoxy;
p is selected from 0;

$R^{hy}$ is selected from F or Cl.

**21.** A compound, and a stereoisomer, a pharmaceutically acceptable salt, a solvate, a co-crystal or a deuterated product thereof, **characterized in that** the compound has a structure selected from one of the following:

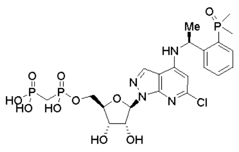
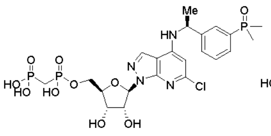
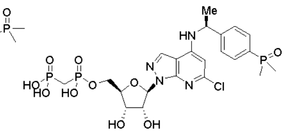
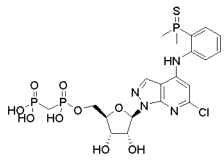
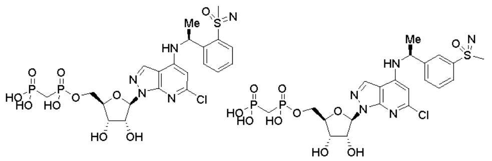
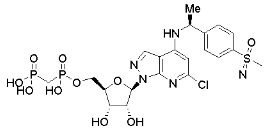
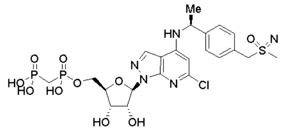
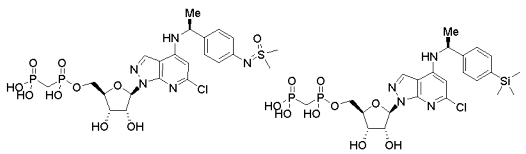
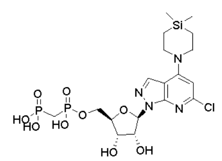
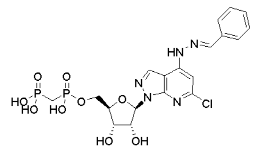
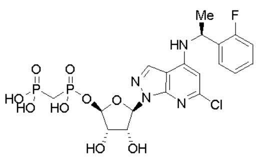
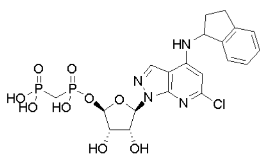
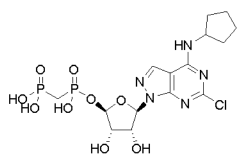
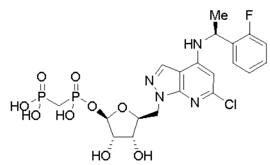
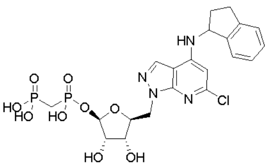
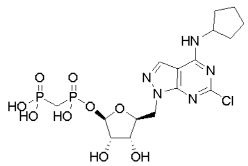
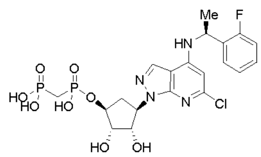
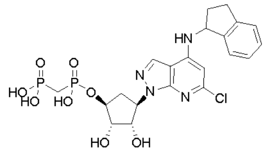
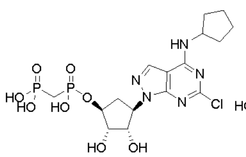
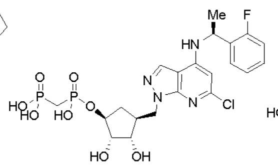
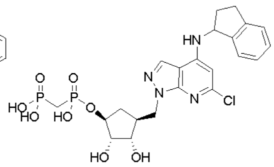
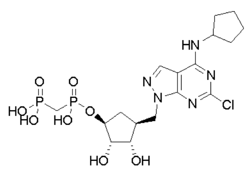
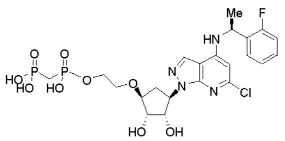
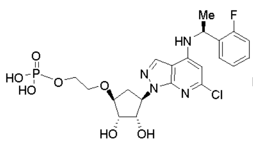
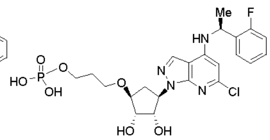
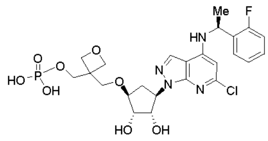
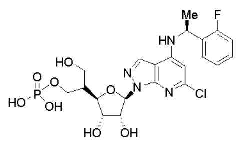
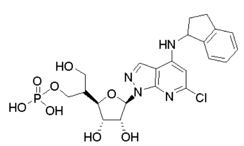
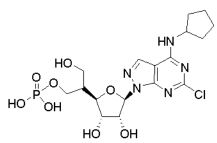
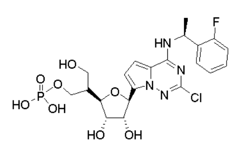

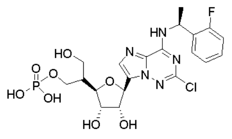
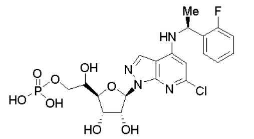
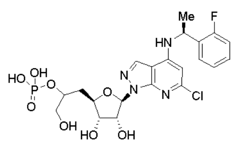
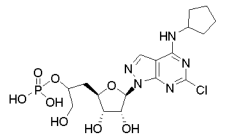
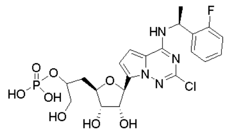
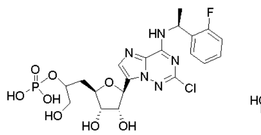
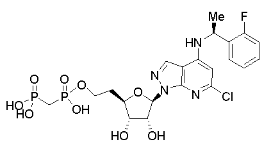
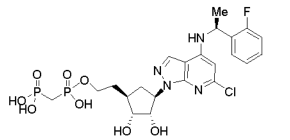
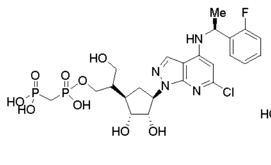
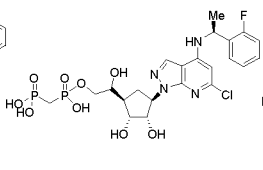
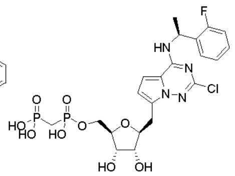
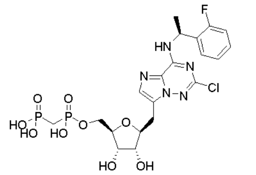
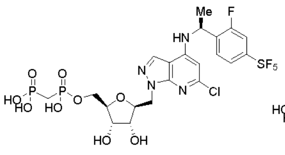
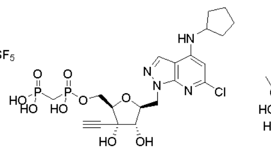
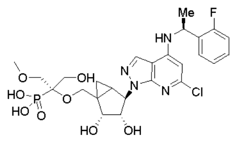
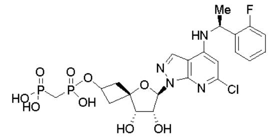
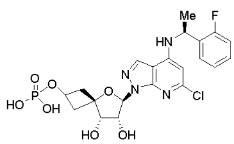
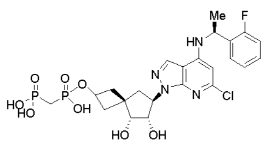
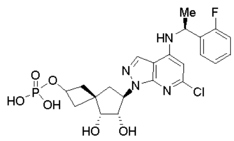
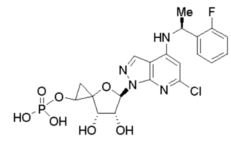
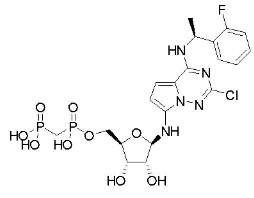
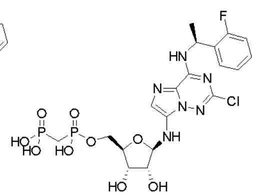
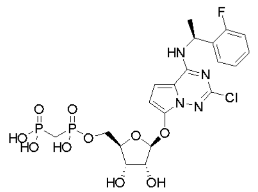
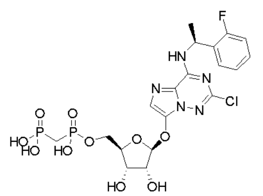
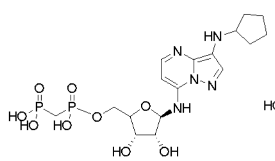
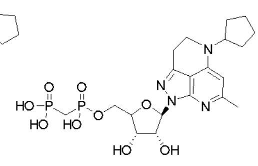
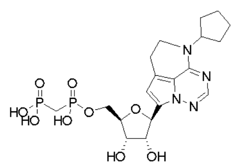
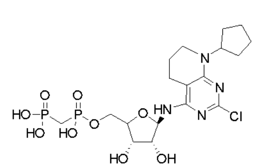
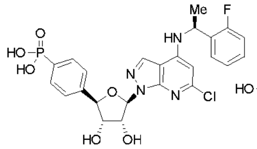
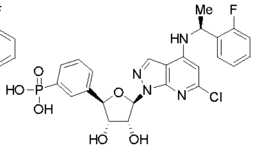
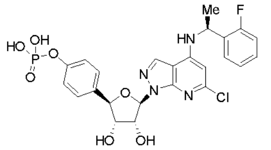
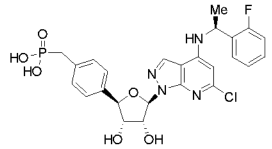

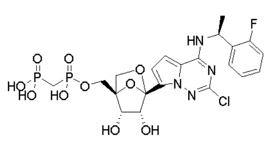 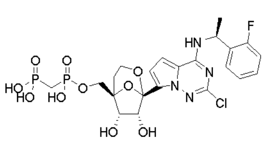 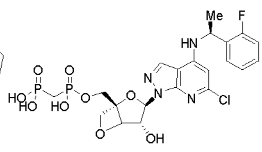 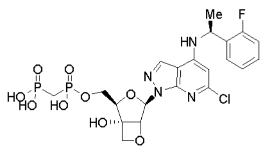

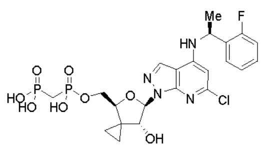 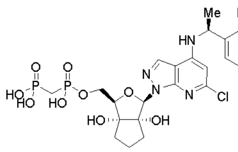 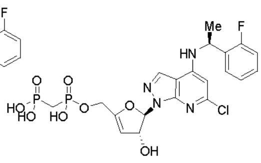 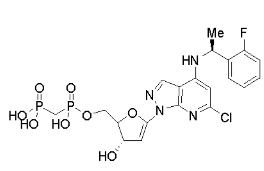

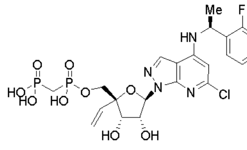 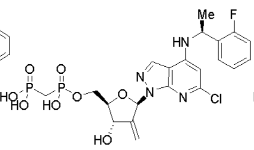 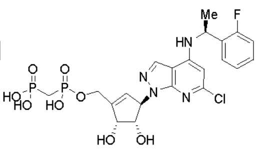 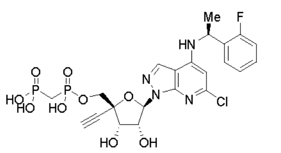

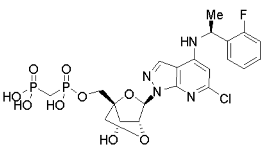 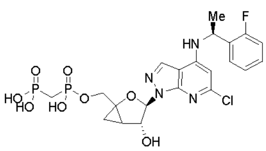 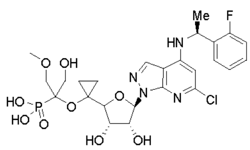 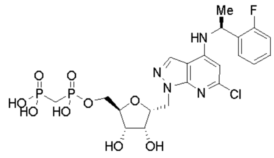

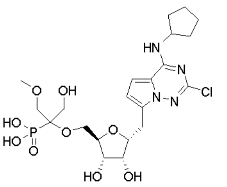 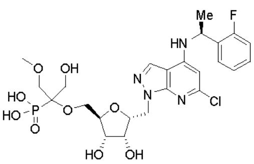 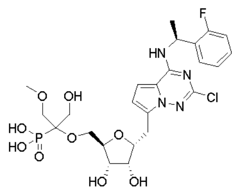 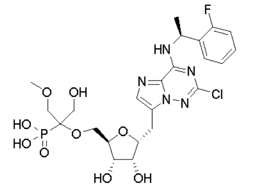

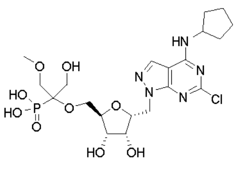 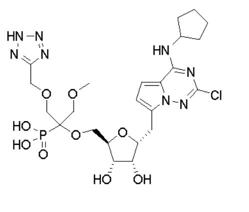 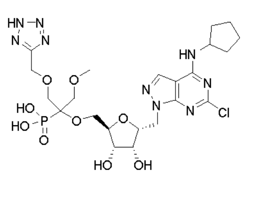 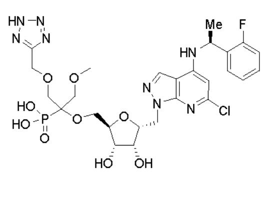

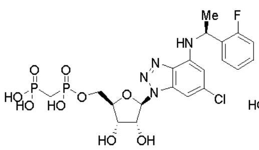 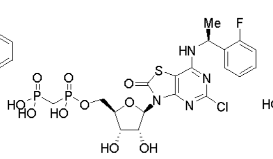 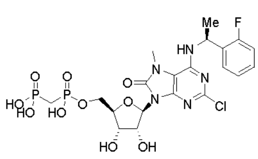 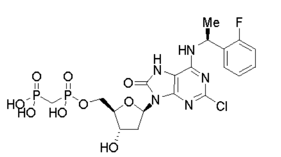

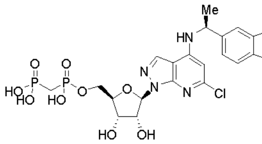 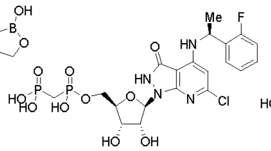 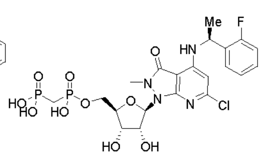 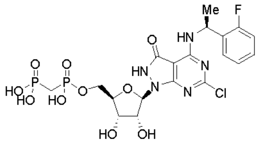

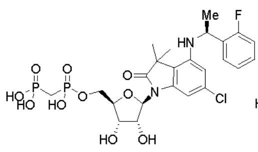 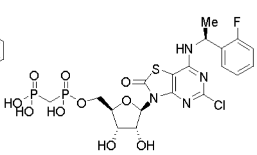 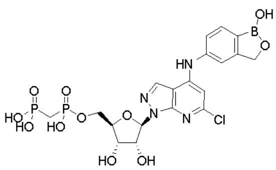 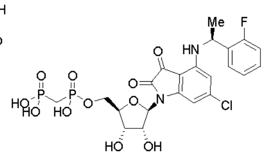

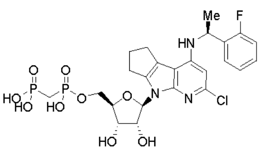 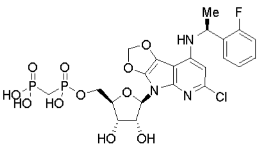 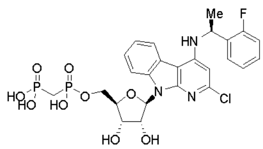 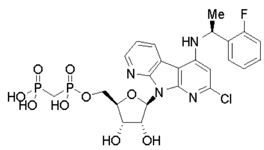

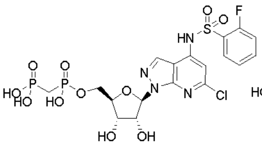 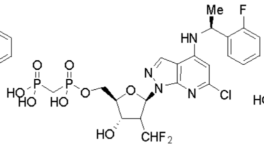 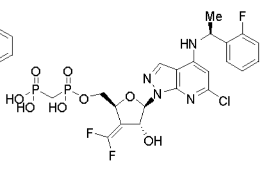 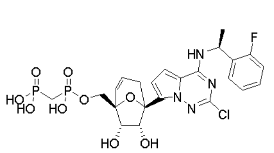

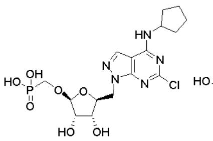 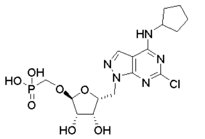 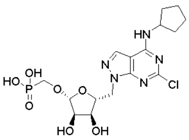 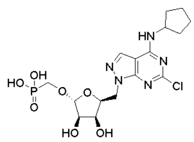 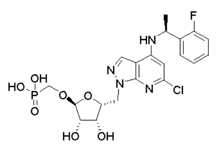

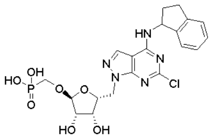 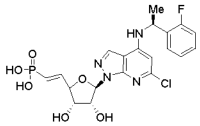 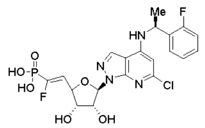 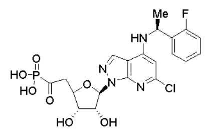 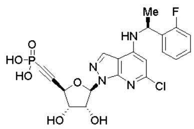

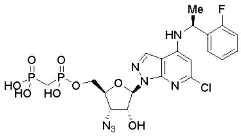 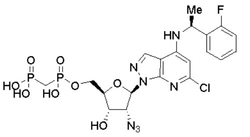 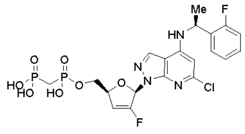 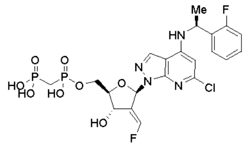

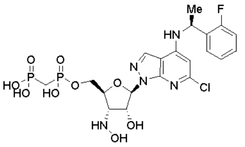 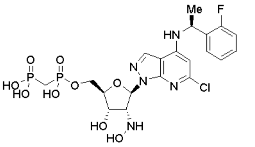 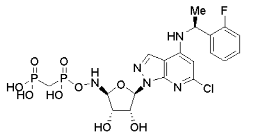 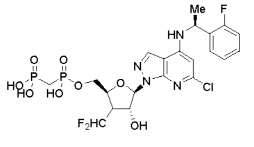

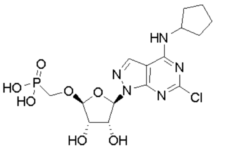 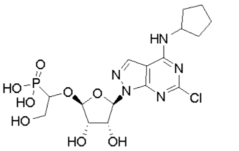 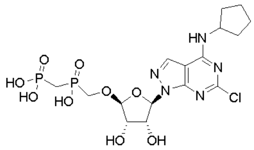 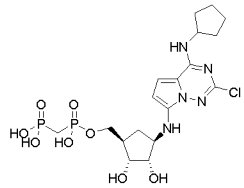

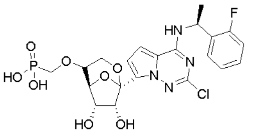 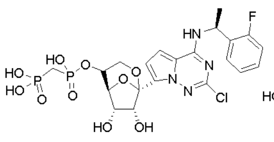 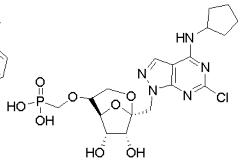 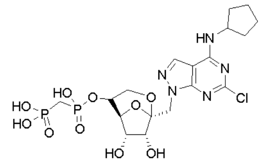

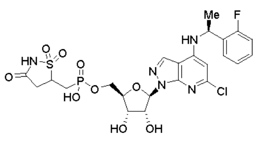
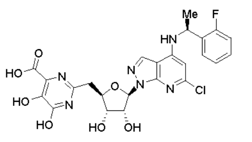
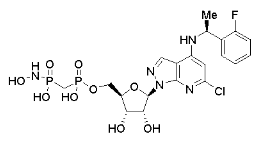
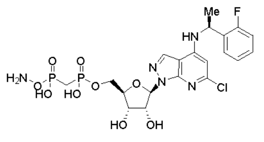

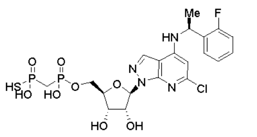
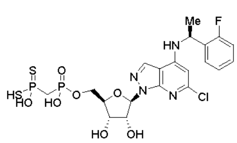

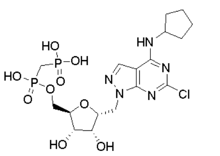
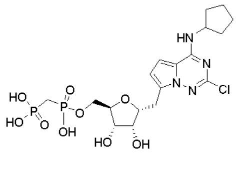
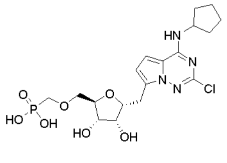
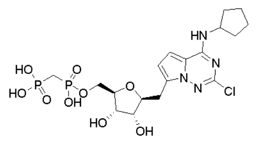

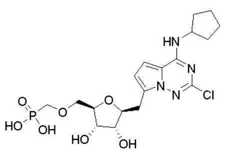
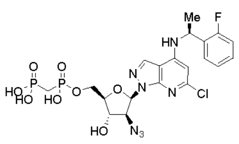
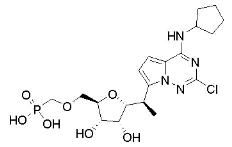
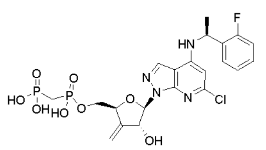

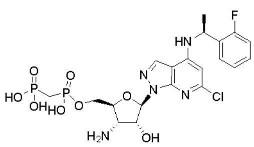
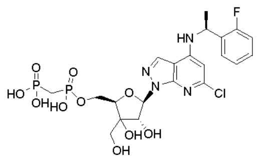
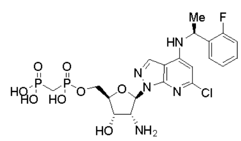
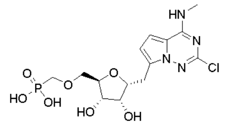

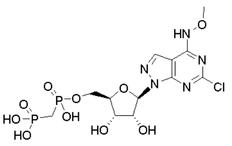
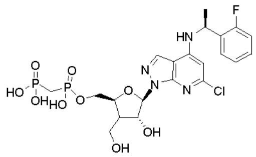
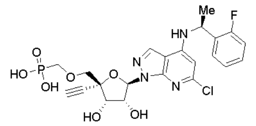

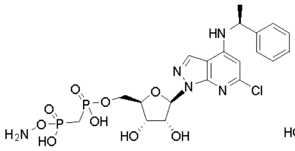
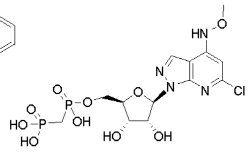
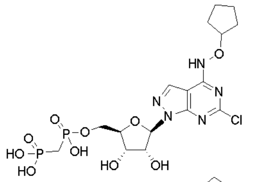
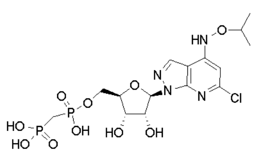

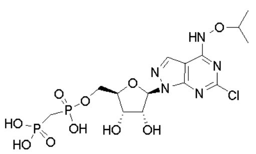
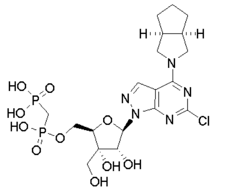
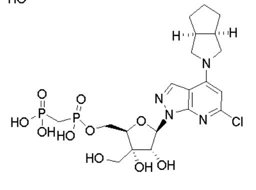
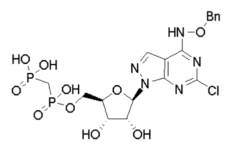

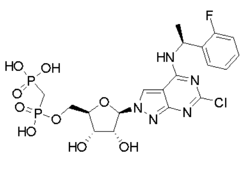
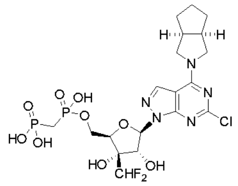
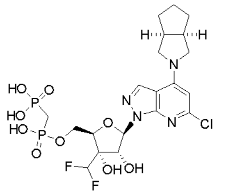
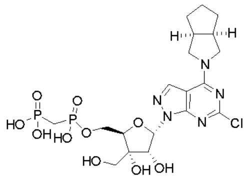
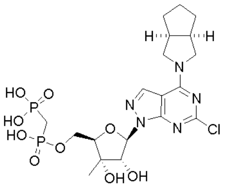
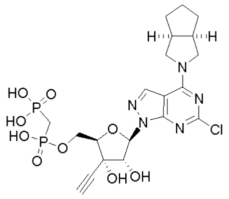
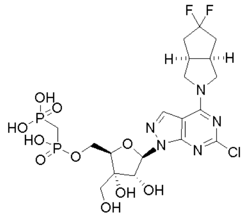
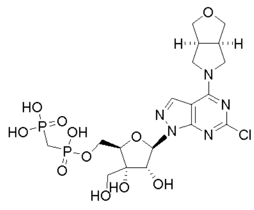
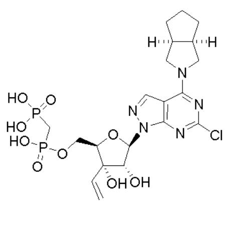
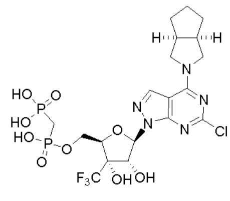
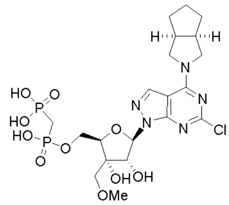
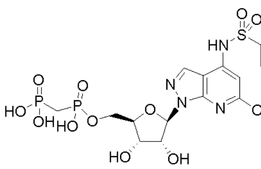
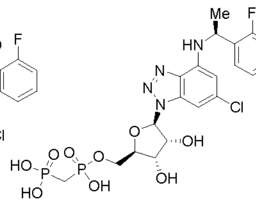
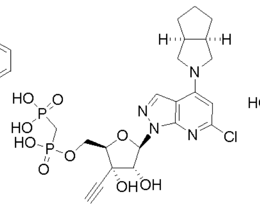
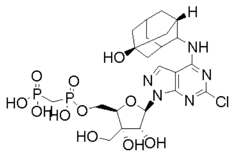
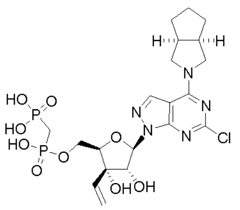
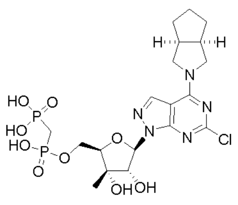
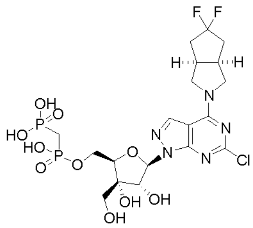
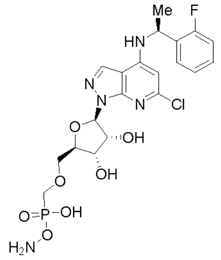
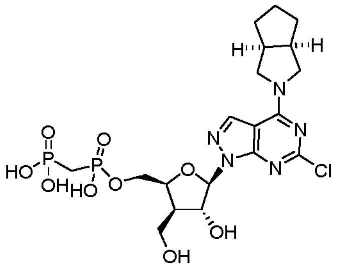
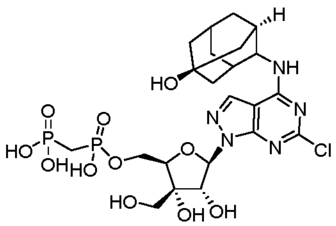

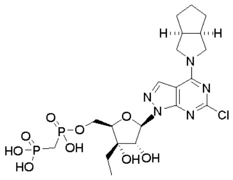 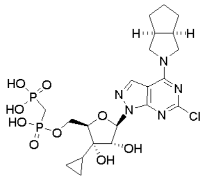 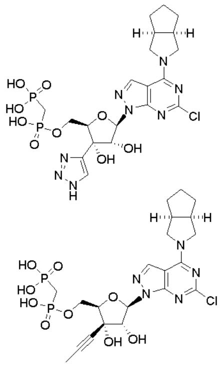 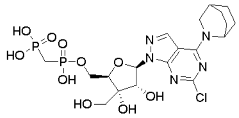

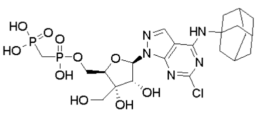 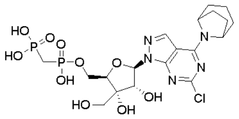 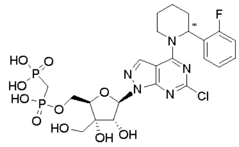

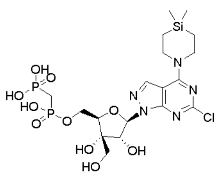 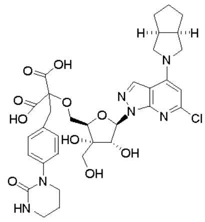 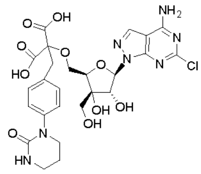 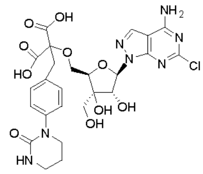 .

**22.** A pharmaceutical composition, **characterized in that** the pharmaceutical composition comprises the compound, and the stereoisomer, pharmaceutically acceptable salt, solvate, co-crystal or deuterated product thereof according to any one of claims 1-21, and a pharmaceutically acceptable adjuvant and/or carrier.

**23.** Use of the compound, and the stereoisomer, pharmaceutically acceptable salt, solvate, co-crystal or deuterated product thereof according to any one of claims 1-21, or the composition according to claim 22 in the preparation of a drug for treating a CD73-mediated disease.

**24.** The use according to claim 23, **characterized in that** the drug is used alone or in combination with an anti-PD-1 antibody.

**25.** The use according to claim 23, **characterized in that** the CD73-mediated disease is a tumor or an autoimmune disease.

**26.** A method for treating a CD73-mediated disease, **characterized in that** the method comprises administering the compound, and the stereoisomer, pharmaceutically acceptable salt, solvate or co-crystal thereof according to any one of claims 1-21, or the composition according to claim 22.

**27.** The method according to claim 26, **characterized in that** the CD73-mediated disease is a tumor or an autoimmune disease.

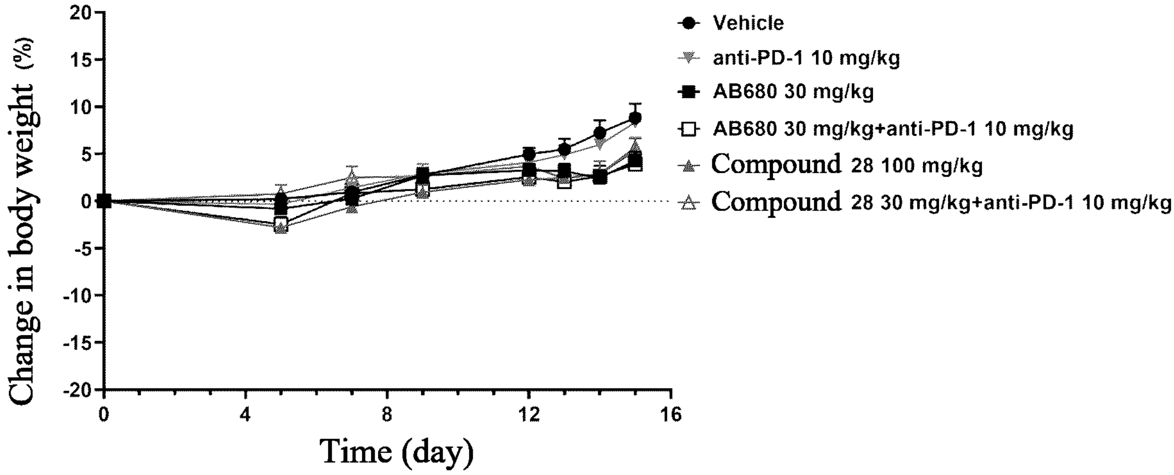
Change in body weight (%)
20
15
10
5
0
-5
-10
-15
-20
0
4
8
12
16
Time (day)
Vehicle
anti-PD-1 10 mg/kg
AB680 30 mg/kg
AB680 30 mg/kg+anti-PD-1 10 mg/kg
Compound 28 100 mg/kg
Compound 28 30 mg/kg+anti-PD-1 10 mg/kg

*FIG. 1*

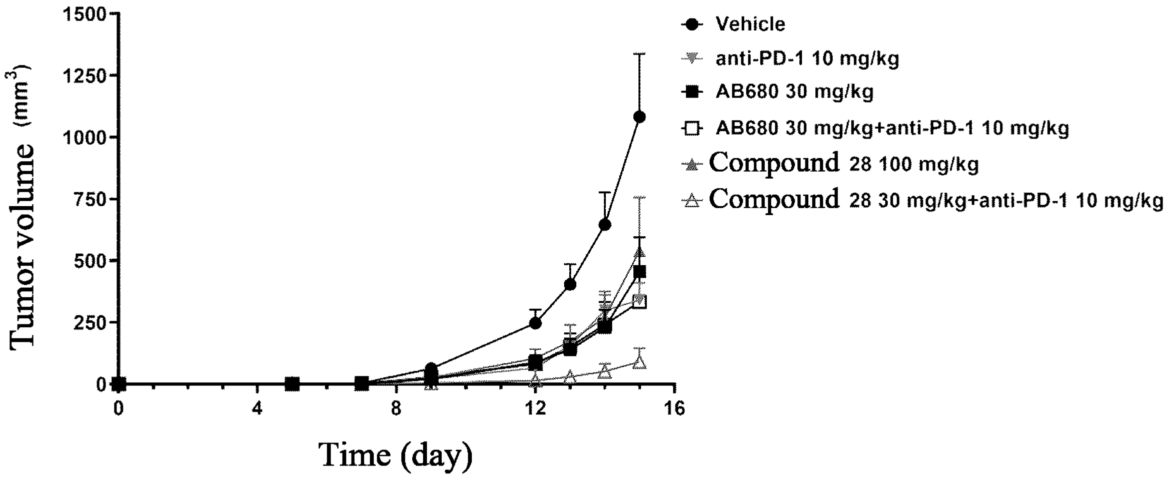
Tumor volume (mm3)
1500
1250
1000
750
500
250
0
0
4
8
12
16
Time (day)
Vehicle
anti-PD-1 10 mg/kg
AB680 30 mg/kg
AB680 30 mg/kg+anti-PD-1 10 mg/kg
Compound 28 100 mg/kg
Compound 28 30 mg/kg+anti-PD-1 10 mg/kg

*FIG. 2*

**INTERNATIONAL SEARCH REPORT**

International application No. **PCT/CN2022/091843**

**A. CLASSIFICATION OF SUBJECT MATTER**

C07H 19/16(2006.01)i; C07H 19/20(2006.01)i; C07H 19/207(2006.01)i; A61K 31/70(2006.01)i; A61K 31/7052(2006.01)i; A61K 31/7076(2006.01)i; A61P 35/00(2006.01)i

According to International Patent Classification (IPC) or to both national classification and IPC

**B. FIELDS SEARCHED**

Minimum documentation searched (classification system followed by classification symbols)

C07H A61K A61P

Documentation searched other than minimum documentation to the extent that such documents are included in the fields searched

Electronic data base consulted during the international search (name of data base and, where practicable, search terms used)

CNABS, CNKI, EPODOC, WPI, REGISTRY(STN), CAPLUS(STN): 核苷酸酶, 抑制剂, 癌, 肿瘤, CD73, nucleotinase, inhibitor, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| X | WO 2020205538 A1 (ETERNITY BIOSCIENCE INC.) 08 October 2020 (2020-10-08) abstract, and claims 1 and 10-16 | 1-27 |
| X | CN 112004541 A (ARCUS BIOSCIENCES INC.) 27 November 2020 (2020-11-27) description, paragraphs [0017], [0036]-[0049], and [0455], table 1 | 1-27 |
| X | CN 108697719 A (ARCUS BIOSCIENCES INC.) 23 October 2018 (2018-10-23) claims 8 and 30-50, and description, table 1 | 1-27 |
| X | WO 2019232319 A1 (PELOTON THERAPEUTICS, INC.) 05 December 2019 (2019-12-05) claims 1 and 69-72, and description, table 1 | 1-27 |
| X | WO 2018183635 A1 (PELOTON THERAPEUTICS, INC.) 04 October 2018 (2018-10-04) claims 1 and 64-67, and description, table 1 | 1-27 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 July 2022** | **16 August 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/091843**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **26-27**
because they relate to subject matter not required to be searched by this Authority, namely:

[1] The method according to claims 26-27 is a disease treatment method. However, the present search report was formed on the basis of a corresponding pharmaceutical use.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/091843**

| Patent document cited in search report | Publication date (day/month/year) | Patent family member(s) | Publication date (day/month/year) |
|---|---|---|---|
| WO 2020205538 A1 | 08 October 2020 | CN 113905743 A | 07 January 2022 |
| CN 112004541 A | 27 November 2020 | CA 3092585 A1 | 12 September 2019 |
| | | EP 3761993 A1 | 13 January 2021 |
| | | WO 2019173682 A1 | 12 September 2019 |
| | | KR 20200130843 A | 20 November 2020 |
| | | JP 2021515775 A | 24 June 2021 |
| | | AU 2019231781 A1 | 22 October 2020 |
| | | US 2020405629 A1 | 31 December 2020 |
| CN 108697719 A | 23 October 2018 | KR 20180100638 A | 11 September 2018 |
| | | US 2021371449 A1 | 02 December 2021 |
| | | US 2019309010 A1 | 10 October 2019 |
| | | MX 2018008350 A | 30 May 2019 |
| | | CA 3151595 A1 | 13 July 2017 |
| | | IL 260260 D0 | 31 July 2018 |
| | | CA 3009196 A1 | 13 July 2017 |
| | | PH 12018501361 A1 | 27 February 2019 |
| | | US 2021002322 A1 | 07 January 2021 |
| | | BR 112018013827 A2 | 11 December 2018 |
| | | UA 124529 C2 | 05 October 2021 |
| | | TW 201805006 A | 16 February 2018 |
| | | WO 2017120508 A1 | 13 July 2017 |
| | | EP 3399984 A1 | 14 November 2018 |
| | | AU 2017206061 A1 | 19 July 2018 |
| | | US 2017267710 A1 | 21 September 2017 |
| | | EA 201891583 A1 | 31 January 2019 |
| | | CN 114395005 A | 26 April 2022 |
| | | JP 2021167351 A | 21 October 2021 |
| | | JP 2019501223 A | 17 January 2019 |
| | | CL 2018001845 A1 | 07 December 2018 |
| | | SG 11201805506 Y A | 30 July 2018 |
| WO 2019232319 A1 | 05 December 2019 | US 2021253614 A1 | 19 August 2021 |
| | | EP 3810109 A1 | 28 April 2021 |
| | | TW 202017569 A | 16 May 2020 |
| WO 2018183635 A1 | 04 October 2018 | US 2020115404 A1 | 16 April 2020 |
| | | JP 2020515559 A | 28 May 2020 |
| | | US 2021317150 A1 | 14 October 2021 |
| | | EP 3600273 A1 | 05 February 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017120508 A1
- WO 2019213174 A1


### Non-patent literature cited in the description


- *Front Immunol,* 2020, vol. 15 (11), 508
- *Current Opinion in Pharmacology,* 2020, vol. 53, 1-11
- *Am J Physiol Cell Physiol.,* 2019, vol. 317 (6), C1079-C1092
- Synthetic Organic Chemistry. John Wiley & Sons, Inc,
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistr. John Wiley & Sons, 1992
- **J. MARCH.** Advanced Organic Chemistry: Reactions, Mechanisms and Structure. Wiley-Interscience, 1992
- Organic Synthesis: Concepts, Methods, Starting Materials. **FUHRHOP, J ; PENZLIN G.** Second, Revised and Enlarged Edition. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002